# EUROPEAN PATENT APPLICATION

(11) **EP 4 344 705 A2**
(43) Date of publication of application: **03.04.2024**
(21) Application number: 23214373.5
(22) Date of filing: 14.03.2014
(51) Int. Cl.: A61K 38/04

(54) **BIOMARKERS AND METHODS FOR PREDICTING PREECLAMPSIA**

(30) Priority: 15.03.2013 US 201361798413 P
(62) Divisional of application: 19166832.6
(71) Applicant: Sera Prognostics, Inc., Salt Lake City, UT 84109 (US)
(72) Inventor: HICKOK, Durlin Edward, Salt Lake City, UT 84109 (US); BONIFACE, John Jay, Salt Lake City, UT 84109 (US); CRITCHFIELD, Gregory Charles, Salt Lake City, UT 84109 (US); FLEISCHER, Tracey Cristine, Salt Lake City, UT 84109 (US)
(74) Representative: Graf, Roland

(57) **Abstract**

The disclosure provides biomarker panels, methods and kits for determining the probability for preeclampsia in a pregnant female. The present disclosure is based, in part, on the discovery that certain proteins and peptides in biological samples obtained from a pregnant female are differentially expressed in pregnant females that have an increased risk of developing in the future or presently suffering from preeclampsia relative to matched controls. The present disclosure is further based, in part, on the unexepected discovery that panels combining one or more of these proteins and peptides can be utilized in methods of determining the probability for preeclampsia in a pregnant female with relatively high sensitivity and specificity. These proteins and peptides dislosed herein serve as biomarkers for classifying test samples, predicting a probability of preeclampsia, monitoring of progress of preeclampsia in a pregnant female, either individually or in a panel of biomarkers.

## Description

This application claims the benefit of priority to U.S. provisional patent application number 61/798,413, filed March 15, 2013, which is herein incorporated by reference in its entirety.

The invention relates generally to the field of personalized medicine and, more specifically to compositions and methods for determining the probability for preeclampsia in a pregnant female.

### BACKGROUND

Preeclampsia (PE), a pregnancy-specific multi-system disorder characterized by hypertension and excess protein excretion in the urine, is a leading cause of maternal and fetal morbidity and mortality worldwide. Preeclampsia affects at least 5-8% of all pregnancies and accounts for nearly 18% of maternal deaths in the United States. The disorder is probably multifactorial, although most cases of preeclampsia are characterized by abnormal maternal uterine vascular remodeling by fetally derived placental trophoblast cells.

Complications of preeclampsia can include compromised placental blood flow, placental abruption, eclampsia, HELLP syndrome (hemolysis, elevated liver enzymes and low platelet count), acute renal failure, cerebral hemorrhage, hepatic failure or rupture, pulmonary edema, disseminated intravascular coagulation and future cardiovascular disease. Even a slight increase in blood pressure can be a sign of preeclampsia. While symptoms can include swelling, sudden weight gain, headaches and changes in vision, some women remain asymptomatic.

Management of preeclampsia consists of two options: delivery or observation. Management decisions depend on the gestational age at which preeclampsia is diagnosed and the relative state of health of the fetus . The only cure for preeclampsia is delivery of the fetus and placenta. However, the decision to deliver involves balancing the potential benefit to the fetus of further *in utero* development with fetal and maternal risk of progressive disease, including the development of eclampsia, which is preeclampsia complicated by maternal seizures.

There is a great need to identify women at risk for preeclampsia as most currently available tests fail to predict the majority of women who eventually develop preeclampsia. Women identified as high-risk can be scheduled for more intensive antenatal surveillance and prophylactic interventions. Reliable early detection of preeclampsia would enable planning appropriate monitoring and clinical management, potentially providing the early identification of disease complications. Such monitoring and management might include: more frequent assessment of blood pressure and urinary protein concentration, uterine artery doppler measurement, ultrasound assessment of fetal growth and prophylactic treatment with aspirin. Finally, reliable antenatal identification of preeclampsia also is crucial to cost-effective allocation of monitoring resources.

The present invention addresses this need by providing compositions and methods for determining whether a pregnant woman is at risk for developing preeclampsia. Related advantages are provided as well.

### SUMMARY

The present invention provides compositions and methods for predicting the probability of preeclampsia in a pregnant female.

In one aspect, the invention provides a panel of isolated biomarkers comprising N of the biomarkers listed in Tables 2, 3, 4, 5 and 7 through 22. In some embodiments, N is a number selected from the group consisting of 2 to 24. In additional embodiments, the biomarker panel comprises at least two of the isolated biomarkers selected from the group consisting of FSVVYAK, SPELQAEAK, VNHVTLSQPK, SSNNPHSPIVEEFQVPYNK, and VVGGLVALR. In additional embodiments, the biomarker panel comprises at least two of the isolated biomarkers selected from the group consisting of LDFHFSSDR, TVQAVLTVPK, GPGEDFR, ETLLQDFR, ATVVYQGER, GFQALGDAADIR. In additional embodiments, the biomarker panel comprises at least two of the isolated biomarkers selected from the group consisting of FSVVYAK, SPELQAEAK, VNHVTLSQPK, SSNNPHSPIVEEFQVPYNK, VVGGLVALR, LDFHFSSDR, TVQAVLTVPK, GPGEDFR, ETLLQDFR, ATVVYQGER, and GFQALGDAADIR.

In some embodiments, the invention provides a biomarker panel comprising at least two of the isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4). In additional embodiments, the invention provides a biomarker panel comprising at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4).

In some embodiments, the invention provides a biomarker panel comprising at least two of the isolated biomarkers selected from the group consisting of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG).

In other embodiments, the invention provides a biomarker panel comprising alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, subcomponent (C1S), and retinol binding protein 4 (RBP4 or RET4) cell adhesion molecule with homology to L1CAM (CHL1), complement component C5 (C5 or COS), complement component C8 beta chain (C8B or CO8B), endothelin-converting enzyme 1 (ECE1), coagulation factor XIII, B polypeptide (F13B), interleukin 5 (IL5), Peptidase D (PEPD), and plasminogen (PLMN). In another aspect, the invention provides a biomarker panel comprising at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4) cell adhesion molecule with homology to L1CAM (CHL1), complement component C5 (C5 or CO5), complement component C8 beta chain (C8B or CO8B), endothelin-converting enzyme 1 (ECE1), coagulation factor XIII, B polypeptide (F13B), interleukin 5 (IL5), Peptidase D (PEPD), and plasminogen (PLMN).

Also provided by the invention is a method of determining probability for preeclampsia in a pregnant female comprising detecting a measurable feature of each of N biomarkers selected from the biomarkers listed in Tables 2, 3, 4, 5 and 7 through 22 in a biological sample obtained from the pregnant female, and analyzing the measurable feature to determine the probability for preeclampsia in the pregnant female. In some embodiments, a measurable feature comprises fragments or derivatives of each of the N biomarkers selected from the biomarkers listed in Tables 2, 3, 4, 5 and 7 through 22. In some embodiments of the disclosed methods detecting a measurable feature comprises quantifying an amount of each of N biomarkers selected from the biomarkers listed in Tables 2, 3, 4, 5 and 7 through 22, combinations or portions and/or derivatives thereof in a biological sample obtained from the pregnant female. In additional embodiments, the disclosed methods of determining probability for preeclampsia in a pregnant female further encompass detecting a measurable feature for one or more risk indicia associated with preeclampsia.

In some embodiments, the disclosed methods of determining probability for preeclampsia in a pregnant female comprises detecting a measurable feature of each of N biomarkers, wherein N is selected from the group consisting of 2 to 24. In further embodiments, the disclosed methods of determining probability for preeclampsia in a pregnant female comprises detecting a measurable feature of each of at least two isolated biomarkers selected from the group consisting of FSVVYAK, SPELQAEAK, VNHVTLSQPK, SSNNPHSPIVEEFQVPYNK, and VVGGLVALR.

In further embodiments, the disclosed methods of determining probability for preeclampsia in a pregnant female comprises detecting a measurable feature of each of at least two isolated biomarkers selected from the group consisting of LDFHFSSDR, TVQAVLTVPK, GPGEDFR, ETLLQDFR, ATVVYQGER, GFQALGDAADIR.

In additional embodiments, the disclosed methods of determining probability for preeclampsia in a pregnant female comprises detecting a measurable feature of each of at least two isolated biomarkers selected from the group consisting of FSVVYAK, SPELQAEAK, VNHVTLSQPK, SSNNPHSPIVEEFQVPYNK, VVGGLVALR, LDFHFSSDR, TVQAVLTVPK, GPGEDFR, ETLLQDFR, ATVVYQGER, and GFQALGDAADIR.

In other embodiments, the disclosed methods of determining probability for preeclampsia in a pregnant female comprise detecting a measurable feature of each of at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4).

In some embodiments, the disclosed methods of determining probability for preeclampsia in a pregnant female comprise detecting a measurable feature of each of at least two isolated biomarkers selected from the group consisting of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG).

In further embodiments, the disclosed methods of determining probability for preeclampsia in a pregnant female comprise detecting a measurable feature of each of at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, subcomponent (C1S), and retinol binding protein 4 (RBP4 or RET4) cell adhesion molecule with homology to L1CAM (CHL1), complement component C5 (C5 or COS), complement component C8 beta chain (C8B or CO8B), endothelin-converting enzyme 1 (ECE1), coagulation factor XIII, B polypeptide (F13B), interleukin 5 (IL5), Peptidase D (PEPD), and plasminogen (PLMN).

In some embodiments of the methods of determining probability for preeclampsia in a pregnant female, the probability for preeclampsia in the pregnant female is calculated based on the quantified amount of each of N biomarkers selected from the biomarkers listed in Tables 2, 3, 4, 5 and 7 through 22. In some embodiments, the disclosed methods for determining the probability of preeclampsia encompass detecting and/or quantifying one or more biomarkers using mass sprectrometry, a capture agent or a combination thereof.

In some embodiments, the disclosed methods of determining probability for preeclampsia in a pregnant female encompass an initial step of providing a biomarker panel comprising N of the biomarkers listed in Tables 2, 3, 4, 5 and 7 through 22. In additional embodiments, the disclosed methods of determining probability for preeclampsia in a pregnant female encompass an initial step of providing a biological sample from the pregnant female.

In some embodiments, the disclosed methods of determining probability for preeclampsia in a pregnant female encompass communicating the probability to a health care provider. In additional embodiments, the communication informs a subsequent treatment decision for the pregnant female. In further embodiments, the treatment decision comprises one or more selected from the group of consisting of more frequent assessment of blood pressure and urinary protein concentration, uterine artery doppler measurement, ultrasound assessment of fetal growth and prophylactic treatment with aspirin.

In further embodiments, the disclosed methods of determining probability for preeclampsia in a pregnant female encompass analyzing the measurable feature of one or more isolated biomarkers using a predictive model. In some embodiments of the disclosed methods, a measurable feature of one or more isolated biomarkers is compared with a reference feature.

In additional embodiments, the disclosed methods of determining probability for preeclampsia in a pregnant female encompass using one or more analyses selected from a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, and a combination thereof. In one embodiment, the disclosed methods of determining probability for preeclampsia in a pregnant female encompasses logistic regression.

In some embodiments, the invention provides a method of determining probability for preeclampsia in a pregnant female encompasses quantifying in a biological sample obtained from the pregnant female an amount of each of N biomarkers selected from the biomarkers listed in Tables 2, 3, 4, 5 and 7 through 22; multiplying the amount by a predetermined coefficient, and determining the probability for preeclampsia in the pregnant female comprising adding the individual products to obtain a total risk score that corresponds to the probability.

Other features and advantages of the invention will be apparent from the detailed description, and from the claims.

### DETAILED DESCRIPTION

The present disclosure is based, in part, on the discovery that certain proteins and peptides in biological samples obtained from a pregnant female are differentially expressed in pregnant females that have an increased risk of developing in the future or presently suffering from preeclampsia relative to matched controls. The present disclosure is further based, in part, on the unexepected discovery that panels combining one or more of these proteins and peptides can be utilized in methods of determining the probability for preeclampsia in a pregnant female with relatively high sensitivity and specificity. These proteins and peptides dislosed herein serve as biomarkers for classifying test samples, predicting a probability of preeclampsia, monitoring of progress of preeclampsia in a pregnant female, either individually or in a panel of biomarkers.

The disclosure provides biomarker panels, methods and kits for determining the probability for preeclampsia in a pregnant female. One major advantage of the present disclosure is that risk of developing preeclampsia can be assessed early during pregnancy so that management of the condition can be initiated in a timely fashion. Sibai, Hypertension. In: Gabbe et al., eds. Obstetrics: Normal and Problem Pregnancies. 6th ed. Philadelphia, Pa: Saunders Elsevier; 2012:chap 35. The present invention is of particular benefit to asymptomatic females who would not otherwise be identified and treated.

By way of example, the present disclosure includes methods for generating a result useful in determining probability for preeclampsia in a pregnant female by obtaining a dataset associated with a sample, where the dataset at least includes quantitative data about biomarkers and panels of biomarkers that have been identified as predictive of preeclampsia, and inputting the dataset into an analytic process that uses the dataset to generate a result useful in determining probability for preeclampsia in a pregnant female. As described further below, this quantitative data can include amino acids, peptides, polypeptides, proteins, nucleotides, nucleic acids, nucleosides, sugars, fatty acids, steroids, metabolites, carbohydrates, lipids, hormones, antibodies, regions of interest that serve as surrogates for biological macromolecules and combinations thereof.

In addition to the specific biomarkers identified in this disclosure, for example, by accession number, sequence, or reference, the invention also contemplates contemplates use of biomarker variants that are at least 90% or at least 95% or at least 97% identical to the exemplified sequences and that are now known or later discover and that have utility for the methods of the invention. These variants may represent polymorphisms, splice variants, mutations, and the like. In this regard, the instant specification discloses multiple art-known proteins in the context of the invention and provides exemplary accession numbers associated with one or more public databases as well as exemplary references to published journal articles relating to these art-known proteins. However, those skilled in the art appreciate that additional accession numbers and journal articles can easily be identified that can provide additional characteristics of the disclosed biomarkers and that the exemplified references are in no way limiting with regard to the disclosed biomarkers. As described herein, various techniques and reagents find use in the methods of the present invention. Suitable samples in the context of the present invention include, for example, blood, plasma, serum, amniotic fluid, vaginal secretions, saliva, and urine. In some embodiments, the biological sample is selected from the group consisting of whole blood, plasma, and serum. In a particular embodiment, the biological sample is serum. As described herein, biomarkers can be detected through a variety of assays and techniques known in the art. As further described herein, such assays include, without limitation, mass spectrometry (MS)-based assays, antibody-based assays as well as assays that combine aspects of the two.

Protein biomarkers associated with the probability for preeclampsia in a pregnant female include, but are not limited to, one or more of the isolated biomarkers listed in Tables 2, 3, 4, 5, and 7 through 22. In addition to the specific biomarkers, the disclosure further includes biomarker variants that are about 90%, about 95%, or about 97% identical to the exemplified sequences. Variants, as used herein, include polymorphisms, splice variants, mutations, and the like.

Additional markers can be selected from one or more risk indicia, including but not limited to, maternal age, race, ethnicity, medical history, past pregnancy history, and obstetrical history. Such additional markers can include, for example, age, prepregnancy weight, ethnicity, race; the presence, absence or severity of diabetes, hypertension, heart disease, kidney disease; the incidence and/or frequency of prior preeclampsia, prior preeclampsia; the presence, absence, frequency or severity of present or past smoking, illicit drug use, alcohol use; the presence, absence or severity of bleeding after the 12th gestational week; cervical cerclage and transvaginal cervical length. Additional risk indicia useful for as markers can be identified using learning algorithms known in the art, such as linear discriminant analysis, support vector machine classification, recursive feature elimination, prediction analysis of microarray, logistic regression, CART, FlexTree, LART, random forest, MART, and/or survival analysis regression, which are known to those of skill in the art and are further described herein.

Provided herein are panels of isolated biomarkers comprising N of the biomarkers selected from the group listed in Tables 2, 3, 4, 5, and 7 through 22. In the disclosed panels of biomarkers N can be a number selected from the group consisting of 2 to 24. In the disclosed methods, the number of biomarkers that are detected and whose levels are determined, can be 1, or more than 1, such as 2, 3, 4, 5, 6, 7, 8, 9, 10, 11, 12, 12, 14, 15, 16, 17, 18, 19, 20, 21, 22, 23, 24, 25 or more. In certain embodiments, the number of biomarkers that are detected, and whose levels are determined, can be 1, or more than 1, such as 2, 3, 4, 5, 6, 7, 8, 9, 10, or more. The methods of this disclosure are useful for determining the probability for preeclampsia in a pregnant female.

While certain of the biomarkers listed in Tables 2, 3, 4, 5, and 7 through 22 are useful alone for determining the probability for preeclampsia in a pregnant female, methods are also described herein for the grouping of multiple subsets of the biomarkers that are each useful as a panel of three or more biomarkers. In some embodiments, the invention provides panels comprising N biomarkers, wherein N is at least three biomarkers. In other embodiments, N is selected to be any number from 3-23 biomarkers.

In yet other embodiments, N is selected to be any number from 2-5, 2-10, 2-15, 2-20, or 2-23. In other embodiments, N is selected to be any number from 3-5, 3-10, 3-15, 3-20, or 3-23. In other embodiments, N is selected to be any number from 4-5, 4-10, 4-15, 4-20, or 4-23. In other embodiments, N is selected to be any number from 5-10, 5-15, 5-20, or 5-23. In other embodiments, N is selected to be any number from 6-10, 6-15, 6-20, or 6-23. In other embodiments, N is selected to be any number from 7-10, 7-15, 7-20, or 7-23. In other embodiments, N is selected to be any number from 8-10, 8-15, 8-20, or 8-23. In other embodiments, N is selected to be any number from 9-10, 9-15, 9-20, or 9-23. In other embodiments, N is selected to be any number from 10-15, 10-20, or 10-23. It will be appreciated that N can be selected to encompass similar, but higher order, ranges.

In certain embodiments, the panel of isolated biomarkers comprises one or more, two or more, three or more, four or more, or five isolated biomarkers comprising an amino acid sequence selected from SPELQAEAK, SSNNPHSPIVEEFQVPYN, VNHVTLSQPK, VVGGLVALR, and FSVVYAK. In some embodiments, the panel of isolated biomarkers comprises one or more, two or more, three or more, four or more, five of the isolated biomarkers consisting of an amino acid sequence selected from SPELQAEAK, SSNNPHSPIVEEFQVPYN, VNHVTLSQPK, VVGGLVALR, and FSVVYAK.

In certain embodiments, the panel of isolated biomarkers comprises one or more, two or more, three or more, four or more, or five isolated biomarkers comprising an amino acid sequence selected from LDFHFSSDR, TVQAVLTVPK, GPGEDFR, ETLLQDFR, ATVVYQGER, GFQALGDAADIR. In some embodiments, the panel of isolated biomarkers comprises one or more, two or more, three or more, four or more, five of the isolated biomarkers consisting of an amino acid sequence selected from LDFHFSSDR, TVQAVLTVPK, GPGEDFR, ETLLQDFR, ATVVYQGER, GFQALGDAADIR.

In certain embodiments, the panel of isolated biomarkers comprises one or more, two or more, three or more, four or more, or five isolated biomarkers comprising an amino acid sequence selected from FSVVYAK, SPELQAEAK, VNHVTLSQPK, SSNNPHSPIVEEFQVPYNK, VVGGLVALR, LDFHFSSDR, TVQAVLTVPK, GPGEDFR, ETLLQDFR, ATVVYQGER, and GFQALGDAADIR. In some embodiments, the panel of isolated biomarkers comprises one or more, two or more, three or more, four or more, five of the isolated biomarkers consisting of an amino acid sequence selected from FSVVYAK, SPELQAEAK, VNHVTLSQPK, SSNNPHSPIVEEFQVPYNK, VVGGLVALR, LDFHFSSDR, TVQAVLTVPK, GPGEDFR, ETLLQDFR, ATVVYQGER, and GFQALGDAADIR.

In some embodiments, the panel of isolated biomarkers comprises one or more peptides comprising a fragment from alpha-1-microglobulin (AMBP) Traboni and Cortese, Nucleic Acids Res. 14 (15), 6340 (1986); ADP/ATP translocase 3 (ANT3) Cozens et al., J. Mol. Biol. 206 (2), 261-280 (1989) (NCBI Reference Sequence: NP_001627.2); apolipoprotein A-II (APOA2) Fullerton et al., Hum. Genet. 111 (1), 75-87 (2002) GenBank: AY100524.1); apolipoprotein B (APOB) Knott et al., Nature 323, 734 - 738 (1986) (GenBank: EAX00803.1); apolipoprotein C-III (APOC3), Fullerton et al., Hum. Genet. 115 (1), 36-56 (2004)(GenBank: AAS68230.1); beta-2-microglobulin (B2MG) Cunningham et al., Biochemistry 12 (24), 4811-4822 (1973) (GenBank: AI686916.1); complement component 1, s subcomponent (CIS) Mackinnon et al., Eur. J. Biochem. 169 (3), 547-553 (1987), and retinol binding protein 4 (RBP4 or RET4) Rask et al., Ann. N. Y. Acad. Sci. 359, 79-90 (1981) (UniProtKB/Swiss-Prot: P02753.3).

In some embodiments, the panel of isolated biomarkers comprises one or more peptides comprising a fragment from cell adhesion molecule with homology to L1CAM (close homolog of L1) (CHL1) (GenBank: AAI43497.1), complement component C5 (C5 or CO5) Haviland, J. Immunol. 146 (1), 362-368 (1991)( GenBank: AAA51925.1); Complement component C8 beta chain (C8B or CO8B) Howard et al., Biochemistry 26 (12), 3565-3570 (1987) (NCBI Reference Sequence: NP_000057.1), endothelin-converting enzyme 1 (ECE1) Xu et al., Cell 78 (3), 473-485 (1994) (NCBI Reference Sequence: NM_001397.2; NP_001388.1); coagulation factor XIII, B polypeptide (F13B) Grundmann et al., Nucleic Acids Res. 18 (9), 2817-2818 (1990) (NCBI Reference Sequence: NP_001985.2); Interleukin 5 (IL5), Murata et al., J. Exp. Med. 175 (2), 341-351 (1992) (NCBI Reference Sequence: NP_000870.1), Peptidase D (PEPD) Endo et al., J. Biol. Chem. 264 (8), 4476-4481 (1989) (UniProtKB/Swiss-Prot: P12955.3); Plasminogen (PLMN) Petersen et al., J. Biol. Chem. 265 (11), 6104-6111 (1990), (NCBI Reference Sequences: NP_000292.1 NP_001161810.1).

In additional embodiments, the invention provides a panel of isolated biomarkers comprising N of the biomarkers listed in Tables 2, 3, 4, 5 and 7 through 22. In some embodiments, N is a number selected from the group consisting of 2 to 24. In additional embodiments, the biomarker panel comprises at least two of the isolated biomarkers selected from the group consisting of FSVVYAK, SPELQAEAK, VNHVTLSQPK, SSNNPHSPIVEEFQVPYNK, and VVGGLVALR.

In further embodiments, the biomarker panel comprises at least two of the isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4). In another embodiment, the invention provides a biomarker panel comprising at least three isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4).

In further embodiments, the biomarker panel comprises at least two of the isolated biomarkers selected from the group consisting Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG). In another embodiment, the invention provides a biomarker panel comprising at least three isolated biomarkers selected from the group consisting of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG).

In some embodiments, the invention provides a biomarker panel comprising alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, subcomponent (C1S), and retinol binding protein 4 (RBP4 or RET4) cell adhesion molecule with homology to L1CAM (CHL1), complement component C5 (C5 or COS), complement component C8 beta chain (C8B or CO8B), endothelin-converting enzyme 1 (ECE1), coagulation factor XIII, B polypeptide (F13B), interleukin 5 (IL5), Peptidase D (PEPD), and plasminogen (PLMN). In another aspect, the invention provides a biomarker panel comprising at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4) cell adhesion molecule with homology to L1CAM (CHL1), complement component C5 (C5 or COS), complement component C8 beta chain (C8B or CO8B), endothelin-converting enzyme 1 (ECE1), coagulation factor XIII, B polypeptide (F13B), interleukin 5 (IL5), Peptidase D (PEPD), and plasminogen (PLMN).

In some embodiments, the invention provides a biomarker panel comprising Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG). In another aspect, the invention provides a biomarker panel comprising at least two isolated biomarkers selected from the group consisting of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG).

As used in this application, including the appended claims, the singular forms "a," "an," and "the" include plural references, unless the content clearly dictates otherwise, and are used interchangeably with "at least one" and "one or more."

The term "about," particularly in reference to a given quantity, is meant to encompass deviations of plus or minus five percent.

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but can include other elements not expressly listed or inherent to such process, method, product-by-process, or composition of matter.

As used herein, the term "panel" refers to a composition, such as an array or a collection, comprising one or more biomarkers. The term can also refer to a profile or index of expression patterns of one or more biomarkers described herein. The number of biomarkers useful for a biomarker panel is based on the sensitivity and specificity value for the particular combination of biomarker values.

As used herein, and unless otherwise specified, the terms "isolated" and "purified" generally describes a composition of matter that has been removed from its native environment (*e.g.*, the natural environment if it is naturally occurring), and thus is altered by the hand of man from its natural state. An isolated protein or nucleic acid is distinct from the way it exists in nature.

The term "biomarker" refers to a biological molecule, or a fragment of a biological molecule, the change and/or the detection of which can be correlated with a particular physical condition or state. The terms "marker" and "biomarker" are used interchangeably throughout the disclosure. For example, the biomarkers of the present invention are correlated with an increased likelihood of preeclampsia. Such biomarkers include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (*e.g.*, glycoproteins, ribonucleoproteins, lipoproteins). The term also encompasses portions or fragments of a biological molecule, for example, peptide fragment of a protein or polypeptide that comprises at least 5 consecutive amino acid residues, at least 6 consecutive amino acid residues, at least 7 consecutive amino acid residues, at least 8 consecutive amino acid residues, at least 9 consecutive amino acid residues, at least 10 consecutive amino acid residues, at least 11 consecutive amino acid residues, at least 12 consecutive amino acid residues, at least 13 consecutive amino acid residues, at least 14 consecutive amino acid residues, at least 15 consecutive amino acid residues, at least 5 consecutive amino acid residues, at least 16 consecutive amino acid residues, at least 17consecutive amino acid residues, at least 18 consecutive amino acid residues, at least 19 consecutive amino acid residues, at least 20 consecutive amino acid residues, at least 21 consecutive amino acid residues, at least 22 consecutive amino acid residues, at least 23 consecutive amino acid residues, at least 24 consecutive amino acid residues, at least 25 consecutive amino acid residues,or more consecutive amino acid residues.

The invention also provides a method of determining probability for preeclampsia in a pregnant female, the method comprising detecting a measurable feature of each of N biomarkers selected from the biomarkers listed in Tables 2, 3, 4, 5 and 7 through 22 in a biological sample obtained from the pregnant female, and analyzing the measurable feature to determine the probability for preeclampsia in the pregnant female. As disclosed herein, a measurable feature comprises fragments or derivatives of each of said N biomarkers selected from the biomarkers listed in Tables 2, 3, 4, 5 and 7 through 22. In some embodiments of the disclosed methods detecting a measurable feature comprises quantifying an amount of each of N biomarkers selected from the biomarkers listed in Tables 2, 3, 4, 5 and 7 through 22, combinations or portions and/or derivatives thereof in a biological sample obtained from said pregnant female.

In some embodiments, the present invention describes a method for predicting the time to onset of preeclamspsia in a pregnant female, the method comprising: (a) obtaining a biological sample from said pregnant female; (b) quantifying an amount of each of N biomarkers selected from the biomarkers listed in Tables 2, 3, 4, 5 and 7 through 22 in said biological sample; (c) multiplying or thresholding said amount by a predetermined coefficient, (d) determining predicted onset of of said preeclampsia in said pregnant female comprising adding said individual products to obtain a total risk score that corresponds to said predicted onset of said preeclampsia in said pregnant female. Although described and exemplified with reference to methods of determining probability for preeclampsia in a pregnant female, the present disclosure is similarly applicable to the method of predicting time to onset of in a pregnant female. It will be apparent to one skilled in the art that each of the aforementioned methods has specific and substantial utilities and benefits with regard maternal-fetal health considerations.

In some embodiments, the method of determining probability for preeclampsia in a pregnant female comprises detecting a measurable feature of each of N biomarkers, wherein N is selected from the group consisting of 2 to 24. In further embodiments, the disclosed methods of determining probability for preeclampsia in a pregnant female comprises detecting a measurable feature of each of at least two isolated biomarkers selected from the group consisting of FSVVYAK, SPELQAEAK, VNHVTLSQPK, SSNNPHSPIVEEFQVPYNK, and VVGGLVALR.

In further embodiments, the disclosed methods of determining probability for preeclampsia in a pregnant female comprises detecting a measurable feature of each of at least two isolated biomarkers selected from the group consisting of LDFHFSSDR, TVQAVLTVPK, GPGEDFR, ETLLQDFR, ATVVYQGER, GFQALGDAADIR.

In further embodiments, the disclosed methods of determining probability for preeclampsia in a pregnant female comprises detecting a measurable feature of each of at least two isolated biomarkers selected from the group consisting of FSVVYAK, SPELQAEAK, VNHVTLSQPK, SSNNPHSPIVEEFQVPYNK, VVGGLVALR, LDFHFSSDR, TVQAVLTVPK, GPGEDFR, ETLLQDFR, ATVVYQGER, and GFQALGDAADIR

In additional embodiments, the method of determining probability for preeclampsia in a pregnant female comprises detecting a measurable feature of each of at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4).

In additional embodiments, the method of determining probability for preeclampsia in a pregnant female comprises detecting a measurable feature of each of at least two isolated biomarkers selected from the group consisting of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG).

In further embodiments, the disclosed method of determining probability for preeclampsia in a pregnant female comprises detecting a measurable feature of each of at least two isolated biomarkers selected from the group consisting of alpha-1-microglobulin (AMBP), ADP/ATP translocase 3 (ANT3), apolipoprotein A-11 (APOA2), apolipoprotein B (APOB), apolipoprotein C-III (APOC3), beta-2-microglobulin (B2MG), complement component 1, subcomponent (C1S), and retinol binding protein 4 (RBP4 or RET4) cell adhesion molecule with homology to L1CAM (CHL1), complement component C5 (C5 or COS), complement component C8 beta chain (C8B or CO8B), endothelin-converting enzyme 1 (ECE1), coagulation factor XIII, B polypeptide (F13B), interleukin 5 (IL5), Peptidase D (PEPD), plasminogen (PLMN), of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG).

In additional embodiments, the methods of determining probability for preeclampsia in a pregnant female further encompass detecting a measurable feature for one or more risk indicia associated with preeclampsia. In additional embodiments the risk indicia are selected form the group consisting of history of preeclampsia, first pregnancy, age, obesity, diabetes, gestational diabetes, hypertension, kidney disease, multiple pregnancy, interval between pregnancies, migraine headaches, rheumatoid arthritis, and lupus.

A "measurable feature" is any property, characteristic or aspect that can be determined and correlated with the probability for preeclampsia in a subject. For a biomarker, such a measurable feature can include, for example, the presence, absence, or concentration of the biomarker, or a fragment thereof, in the biological sample, an altered structure, such as, for example, the presence or amount of a post-translational modification, such as oxidation at one or more positions on the amino acid sequence of the biomarker or, for example, the presence of an altered conformation in comparison to the conformation of the biomarker in normal control subjects, and/or the presence, amount, or altered structure of the biomarker as a part of a profile of more than one biomarker. In addition to biomarkers, measurable features can further include risk indicia including, for example, maternal age, race, ethnicity, medical history, past pregnancy history, obstetrical history. For a risk indicium, a measurable feature can include, for example, age, prepregnancy weight, ethnicity, race; the presence, absence or severity of diabetes, hypertension, heart disease, kidney disease; the incidence and/or frequency of prior preeclampsia, prior preeclampsia; the presence, absence, frequency or severity of present or past smoking, illicit drug use, alcohol use; the presence, absence or severity of bleeding after the 12th gestational week; cervical cerclage and transvaginal cervical length.

In some embodiments of the disclosed methods of determining probability for preeclampsia in a pregnant female, the probability for preeclampsia in the pregnant female is calculated based on the quantified amount of each of N biomarkers selected from the biomarkers listed in Tables 2, 3, 4, 5 and 7 through 22. In some embodiments, the disclosed methods for determining the probability of preeclampsia encompass detecting and/or quantifying one or more biomarkers using mass sprectrometry, a capture agent or a combination thereof.

In some embodiments, the disclosed methods of determining probability for preeclampsia in a pregnant female encompass an initial step of providing a biomarker panel comprising N of the biomarkers listed in Tables 2, 3, 4, 5 and 7 through 22. In additional embodiments, the disclosed methods of determining probability for preeclampsia in a pregnant female encompass an initial step of providing a biological sample from the pregnant female.

In some embodiments, the disclosed methods of determining probability for preeclampsia in a pregnant female encompass communicating the probability to a health care provider. In additional embodiments, the communication informs a subsequent treatment decision for the pregnant female.

In some embodiments, the method of determining probability for preeclampsia in a pregnant female encompasses the additional feature of expressing the probability as a risk score.

As used herein, the term "risk score" refers to a score that can be assigned based on comparing the amount of one or more biomarkers in a biological sample obtained from a pregnant female to a standard or reference score that represents an average amount of the one or more biomarkers calculated from biological samples obtained from a random pool of pregnant females. Because the level of a biomarker may not be static throughout pregnancy, a standard or reference score has to have been obtained for the gestational time point that corresponds to that of the pregnant female at the time the sample was taken. The standard or reference score can be predetermined and built into a predictor model such that the comparison is indirect rather than actually performed every time the probability is determined for a subject. A risk score can be a standard (*e.g.*, a number) or a threshold (*e.g.,* a line on a graph). The value of the risk score correlates to the deviation, upwards or downwards, from the average amount of the one or more biomarkers calculated from biological samples obtained from a random pool of pregnant females. In certain embodiments, if a risk score is greater than a standard or reference risk score, the pregnant female can have an increased likelihood of preeclampsia. In some embodiments, the magnitude of a pregnant female's risk score, or the amount by which it exceeds a reference risk score, can be indicative of or correlated to that pregnant female's level of risk.

In the context of the present invention, the term "biological sample," encompasses any sample that is taken from pregnant female and contains one or more of the biomarkers listed in Table 1. Suitable samples in the context of the present invention include, for example, blood, plasma, serum, amniotic fluid, vaginal secretions, saliva, and urine. In some embodiments, the biological sample is selected from the group consisting of whole blood, plasma, and serum. As will be appreciated by those skilled in the art, a biological sample can include any fraction or component of blood, without limitation, T cells, monocytes, neutrophils, erythrocytes, platelets and microvesicles such as exosomes and exosome-like vesicles. In a particular embodiment, the biological sample is serum.

Preeclampsia refers to a condition characterized by high blood pressure and excess protein in the urine (proteinuria) after 20 weeks of pregnancy in a woman who previously had normal blood pressure. Preeclampsia encompasses Eclampsia, a more severe form of preeclampsia that is further characterized by seizures. Preeclampsia can be further classified as mild or severe depending upon the severity of the clinical symptoms. While preeclampsia usually develops during the second half of pregnancy (after 20 weeks), it also can develop shortly after birth or before 20 weeks of pregnancy.

Preeclampsia has been characterized by some investigators as 2 different disease entities: early-onset preeclampsia and late-onset preeclampsia, both of which are intended to be encompassed by reference to preeclampsia herein. Early-onset preeclampsia is usually defined as preeclampsia that develops before 34 weeks of gestation, whereas late-onset preeclampsia develops at or after 34 weeks of gestation. Preclampsia also includes postpartum preeclampsia is a less common condition that occurs when a woman has high blood pressure and excess protein in her urine soon after childbirth. Most cases of postpartum preeclampsia develop within 48 hours of childbirth. However, postpartum preeclampsia sometimes develops up to four to six weeks after childbirth. This is known as late postpartum preeclampsia.

Clinical criteria for diagnosis of preeclampsia are well established, for example, blood pressure of at least 140/90 mm Hg and urinary excretion of at least 0.3 grams of protein in a 24-hour urinary protein excretion (or at least +1 or greater on dipstick testing), each on two occasions 4-6 hours apart. Severe preeclampsia generally refers to a blood pressure of at least 160/110 mm Hg on at least 2 occasions 6 hours apart and greater than 5 grams of protein in a 24-hour urinary protein excretion or persistent +3 proteinuria on dipstick testing. Preeclampsia can include HELLP syndrome (hemolysis, elevated liver enzymes, low platelet count). Other elements of preeclampsia can include in-utero growth restriction (IUGR) in less than the 10% percentile according to the US demographics, persistent neurologic symptoms (headache, visual disturbances), epigastric pain, oliguria (less than 500 mL/24 h), serum creatinine greater than 1.0 mg/dL, elevated liver enzymes (greater than two times normal), thrombocytopenia (<100,000 cells/µL).

In some embodiments, the pregnant female was between 17 and 28 weeks of gestation at the time the biological sample was collected. In other embodiments, the pregnant female was between 16 and 29 weeks, between 17 and 28 weeks, between 18 and 27 weeks, between 19 and 26 weeks, between 20 and 25 weeks, between 21 and 24 weeks, or between 22 and 23 weeks of gestation at the time the biological sample was collected. In further embodiments, the the pregnant female was between about 17 and 22 weeks, between about 16 and 22 weeks between about 22 and 25 weeks, between about 13 and 25 weeks, between about 26 and 28, or between about 26 and 29 weeks of gestation at the time the biological sample was collected. Accordingly, the gestational age of a pregnant female at the time the biological sample is collected can be 15, 16, 17, 18, 19, 20, 21, 22, 23, 24, 25, 26, 27, 28, 29 or 30 weeks.

In some embodiments of the claimed methods the measurable feature comprises fragments or derivatives of each of the N biomarkers selected from the biomarkers listed in Table 1. In additional embodiments of the claimed methods, detecting a measurable feature comprises quantifying an amount of each of N biomarkers selected from the biomarkers listed in Table 1, combinations or portions and/or derivatives thereof in a biological sample obtained from said pregnant female.

The term "amount" or "level" as used herein refers to a quantity of a biomarker that is detectable or measurable in a biological sample and/or control. The quantity of a biomarker can be, for example, a quantity of polypeptide, the quantity of nucleic acid, or the quantity of a fragment or surrogate. The term can alternatively include combinations thereof. The term "amount" or "level" of a biomarker is a measurable feature of that biomarker.

In some embodiments, calculating the probability for preeclampsia in a pregnant female is based on the quantified amount of each of N biomarkers selected from the biomarkers listed in Table 1. Any existing, available or conventional separation, detection and quantification methods can be used herein to measure the presence or absence (*e.g.*, readout being present vs. absent; or detectable amount vs. undetectable amount) and/or quantity (*e.g.*, readout being an absolute or relative quantity, such as, for example, absolute or relative concentration) of biomarkers, peptides, polypeptides, proteins and/or fragments thereof and optionally of the one or more other biomarkers or fragments thereof in samples. In some embodiments, detection and/or quantification of one or more biomarkers comprises an assay that utilizes a capture agent. In further ambodiments, the capture agent is an antibody, antibody fragment, nucleic acid-based protein binding reagent, small molecule or variant thereof. In additional embodiments, the assay is an enzyme immunoassay (EIA), enzyme-linked immunosorbent assay (ELISA), and radioimmunoassay (RIA). In some embodiments, detection and/or quantification of one or more biomarkers further comprises mass spectrometry (MS). In yet further embodiments, the mass spectrometry is co-immunoprecitipation-mass spectrometry (co-IP MS), where coimmunoprecipitation, a technique suitable for the isolation of whole protein complexes is followed by mass spectrometric analysis.

As used herein, the term "mass spectrometer" refers to a device able to volatilize/ionize analytes to form gas-phase ions and determine their absolute or relative molecular masses. Suitable methods of volatilization/ionization are matrix-assisted laser desorption ionization (MALDI), electrospray, laser/light, thermal, electrical, atomized/sprayed and the like, or combinations thereof. Suitable forms of mass spectrometry include, but are not limited to, ion trap instruments, quadrupole instruments, electrostatic and magnetic sector instruments, time of flight instruments, time of flight tandem mass spectrometer (TOF MS/MS), Fourier-transform mass spectrometers, Orbitraps and hybrid instruments composed of various combinations of these types of mass analyzers. These instruments can, in turn, be interfaced with a variety of other instruments that fractionate the samples (for example, liquid chromatography or solid-phase adsorption techniques based on chemical, or biological properties) and that ionize the samples for introduction into the mass spectrometer, including matrix-assisted laser desorption (MALDI), electrospray, or nanospray ionization (ESI) or combinations thereof.

Generally, any mass spectrometric (MS) technique that can provide precise information on the mass of peptides, and preferably also on fragmentation and/or (partial) amino acid sequence of selected peptides (*e.g.*, in tandem mass spectrometry, MS/MS; or in post source decay, TOF MS), can be used in the methods disclosed herein. Suitable peptide MS and MS/MS techniques and systems are well-known *per se* (see, e.g., Methods in Molecular Biology, vol. 146: "Mass Spectrometry of Proteins and Peptides", by Chapman, ed., Humana Press 2000; Biemann 1990. Methods Enzymol 193: 455-79; or Methods in Enzymology, vol. 402: "Biological Mass Spectrometry", by Burlingame, ed., Academic Press 2005) and can be used in practicing the methods disclosed herein. Accordingly, in some embodiments, the disclosed methods comprise performing quantitative MS to measure one or more biomarkers. Such quantitiative methods can be performed in an automated (Villanueva, et al., Nature Protocols (2006) 1(2):880-891) or semi-automated format. In particular embodiments, MS can be operably linked to a liquid chromatography device (LC-MS/MS or LC-MS) or gas chromatography device (GC-MS or GC-MS/MS). Other methods useful in this context include isotope-coded affinity tag (ICAT) followed by chromatography and MS/MS.

As used herein, the terms "multiple reaction monitoring (MRM)" or "selected reaction monitoring (SRM)" refer to an MS-based quantification method that is particularly useful for quantifying analytes that are in low abundance. In an SRM experiment, a predefined precursor ion and one or more of its fragments are selected by the two mass filters of a triple quadrupole instrument and monitored over time for precise quantification. Multiple SRM precursor and fragment ion pairs can be measured within the same experiment on the chromatographic time scale by rapidly toggling between the different precursor/fragment pairs to perform an MRM experiment. A series of transitions (precursor/fragment ion pairs) in combination with the retention time of the targeted analyte (*e.g.*, peptide or small molecule such as chemical entity, steroid, hormone) can constitute a definitive assay. A large number of analytes can be quantified during a single LC-MS experiment. The term "scheduled," or "dynamic" in reference to MRM or SRM, refers to a variation of the assay wherein the transitions for a particular analyte are only acquired in a time window around the expected retention time, significantly increasing the number of analytes that can be detected and quantified in a single LC-MS experiment and contributing to the selectivity of the test, as retention time is a property dependent on the physical nature of the analyte. A single analyte can also be monitored with more than one transition. Finally, included in the assay can be standards that correspond to the analytes of interest (*e.g.*, same amino acid sequence), but differ by the inclusion of stable isotopes. Stable isotopic standards (SIS) can be incorporated into the assay at precise levels and used to quantify the corresponding unknown analyte. An additional level of specificity is contributed by the co-elution of the unknown analyte and its corresponding SIS and properties of their transitions (*e.g.*, the similarity in the ratio of the level of two transitions of the unknown and the ratio of the two transitions of its corresponding SIS).

Mass spectrometry assays, instruments and systems suitable for biomarker peptide analysis can include, without limitation, matrix-assisted laser desorption/ionisation time-of-flight (MALDI-TOF) MS; MALDI-TOF post-source-decay (PSD); MALDI-TOF/TOF; surface-enhanced laser desorption/ionization time-of-flight mass spectrometry (SELDI-TOF) MS; electrospray ionization mass spectrometry (ESI-MS); ESI-MS/MS; ESI-MS/(MS)ₙ (n is an integer greater than zero); ESI 3D or linear (2D) ion trap MS; ESI triple quadrupole MS; ESI quadrupole orthogonal TOF (Q-TOF); ESI Fourier transform MS systems; desorption/ionization on silicon (DIOS); secondary ion mass spectrometry (SIMS); atmospheric pressure chemical ionization mass spectrometry (APCI-MS); APCI-MS/MS; APCI- (MS)ₙ; atmospheric pressure photoionization mass spectrometry (APPI-MS); APPI-MS/MS; and APPI- (MS)ₙ. Peptide ion fragmentation in tandem MS (MS/MS) arrangements can be achieved using manners established in the art, such as, e.g., collision induced dissociation (CID). As described herein, detection and quantification of biomarkers by mass spectrometry can involve multiple reaction monitoring (MRM), such as described among others by Kuhn et al. Proteomics 4: 1175-86 (2004). Scheduled multiple-reaction-monitoring (Scheduled MRM) mode acquisition during LC-MS/MS analysis enhances the sensitivity and accuracy of peptide quantitation. Anderson and Hunter , Molecular and Cellular Proteomics 5(4):573 (2006). As described herein, mass spectrometry-based assays can be advantageously combined with upstream peptide or protein separation or fractionation methods, such as for example with the chromatographic and other methods described herein below.

A person skilled in the art will appreciate that a number of methods can be used to determine the amount of a biomarker, including mass spectrometry approaches, such as MS/MS, LC-MS/MS, multiple reaction monitoring (MRM) or SRM and product-ion monitoring (PIM) and also including antibody based methods such as immunoassays such as Western blots, enzyme-linked immunosorbant assay (ELISA), immunopercipitation, immunohistochemistry, immunofluorescence, radioimmunoassay, dot blotting, and fluorescence-activated cell sorting (FACS). Accordingly, in some embodiments, determining the level of the at least one biomarker comprises using an immunoassay and/or mass spectrometric methods. In additional embodiments, the mass spectrometric methods are selected from MS, MS/MS, LC-MS/MS, SRM, PIM, and other such methods that are known in the art. In other embodiments, LC-MS/MS further comprises 1D LC-MS/MS, 2D LC-MS/MS or 3D LC-MS/MS. Immunoassay techniques and protocols are generally known to those skilled in the art ( Price and Newman, Principles and Practice of Immunoassay, 2nd Edition, Grove's Dictionaries, 1997; and Gosling, Immunoassays: A Practical Approach, Oxford University Press, 2000.) A variety of immunoassay techniques, including competitive and non-competitive immunoassays, can be used ( Self et al., Curr. Opin. Biotechnol., 7:60-65 (1996).

In further embodiments, the immunoassay is selected from Western blot, ELISA, immunopercipitation, immunohistochemistry, immunofluorescence, radioimmunoassay (RIA), dot blotting, and FACS. In certain embodiments, the immunoassay is an ELISA. In yet a further embodiment, the ELISA is direct ELISA (enzyme-linked immunosorbent assay), indirect ELISA, sandwich ELISA, competitive ELISA, multiplex ELISA, ELISPOT technologies, and other similar techniques known in the art. Principles of these immunoassay methods are known in the art, for example John R. Crowther, The ELISA Guidebook, 1st ed., Humana Press 2000, ISBN 0896037282. Typically ELISAs are performed with antibodies but they can be performed with any capture agents that bind specifically to one or more biomarkers of the invention and that can be detected. Multiplex ELISA allows simultaneous detection of two or more analytes within a single compartment (e.g., microplate well) usually at a plurality of array addresses (Nielsen and Geierstanger 2004. J Immunol Methods 290: 107-20 (2004) and Ling et al. 2007. Expert Rev Mol Diagn 7: 87-98 (2007)).

In some embodiments, Radioimmunoassay (RIA) can be used to detect one or more biomarkers in the methods of the invention. RIA is a competition-based assay that is well known in the art and involves mixing known quantities of radioactavely-labelled (e.g.,¹²⁵I or ¹³¹I-labelled) target analyte with antibody specific for the analyte, then adding non-labelled analyte from a sample and measuring the amount of labelled analyte that is displaced (see, e.g., An Introduction to Radioimmunoassay and Related Techniques, by Chard T, ed., Elsevier Science 1995, ISBN 0444821198 for guidance).

A detectable label can be used in the assays described herein for direct or indirect detection of the biomarkers in the methods of the invention. A wide variety of detectable labels can be used, with the choice of label depending on the sensitivity required, ease of conjugation with the antibody, stability requirements, and available instrumentation and disposal provisions. Those skilled in the art are familiar with selection of a suitable detectable label based on the assay detection of the biomarkers in the methods of the invention. Suitable detectable labels include, but are not limited to, fluorescent dyes (*e.g.,* fluorescein, fluorescein isothiocyanate (FITC), Oregon Green^{™}, rhodamine, Texas red, tetrarhodimine isothiocynate (TRITC), Cy3, Cy5, etc.), fluorescent markers (*e.g.*, green fluorescent protein (GFP), phycoerythrin, etc.), enzymes (*e.g.*, luciferase, horseradish peroxidase, alkaline phosphatase, etc.), nanoparticles, biotin, digoxigenin, metals, and the like.

For mass-sectrometry based analysis, differential tagging with isotopic reagents, *e.g.*, isotope-coded affinity tags (ICAT) or the more recent variation that uses isobaric tagging reagents, iTRAQ (Applied Biosystems, Foster City, Calif.), or tandem mass tags, TMT, (Thermo Scientific, Rockford, IL), followed by multidimensional liquid chromatography (LC) and tandem mass spectrometry (MS/MS) analysis can provide a further methodology in practicing the methods of the inventon.

A chemiluminescence assay using a chemiluminescent antibody can be used for sensitive, non-radioactive detection of protein levels. An antibody labeled with fluorochrome also can be suitable. Examples of fluorochromes include, without limitation, DAPI, fluorescein, Hoechst 33258, R-phycocyanin, B-phycoerythrin, R-phycoerythrin, rhodamine, Texas red, and lissamine. Indirect labels include various enzymes well known in the art, such as horseradish peroxidase (HRP), alkaline phosphatase (AP), beta-galactosidase, urease, and the like. Detection systems using suitable substrates for horseradish-peroxidase, alkaline phosphatase , beta-galactosidase are well known in the art.

A signal from the direct or indirect label can be analyzed, for example, using a spectrophotometer to detect color from a chromogenic substrate; a radiation counter to detect radiation such as a gamma counter for detection of ¹²⁵I; or a fluorometer to detect fluorescence in the presence of light of a certain wavelength. For detection of enzyme-linked antibodies, a quantitative analysis can be made using a spectrophotometer such as an EMAX Microplate Reader (Molecular Devices; Menlo Park, Calif.) in accordance with the manufacturer's instructions. If desired, assays used to practice the invention can be automated or performed robotically, and the signal from multiple samples can be detected simultaneously.

In some embodiments, the methods described herein encompass quantification of the biomarkers using mass spectrometry (MS). In further embodiments, the mass spectrometry can be liquid chromatography-mass spectrometry (LC-MS), multiple reaction monitoring (MRM) or selected reaction monitoring (SRM). In additional embodiments, the MRM or SRM can further encompass scheduled MRM or scheduled SRM.

As described above, chromatography can also be used in practicing the methods of the invention. Chromatography encompasses methods for separating chemical substances and generally involves a process in which a mixture of analytes is carried by a moving stream of liquid or gas ("mobile phase") and separated into components as a result of differential distribution of the analytes as they flow around or over a stationary liquid or solid phase ("stationary phase"), between the mobile phase and said stationary phase. The stationary phase can be usually a finely divided solid, a sheet of filter material, or a thin film of a liquid on the surface of a solid, or the like. Chromatography is well understood by those skilled in the art as a technique applicable for the separation of chemical compounds of biological origin, such as, *e.g.,* amino acids, proteins, fragments of proteins or peptides, *etc.*

Chromatography can be columnar (*i.e.,* wherein the stationary phase is deposited or packed in a column), preferably liquid chromatography, and yet more preferably high-performance liquid chromatography (HPLC) or ultra high performance/pressure liquid chromatography (UHPLC). Particulars of chromatography are well known in the art (Bidlingmeyer, Practical HPLC Methodology and Applications, John Wiley & Sons Inc., 1993). Exemplary types of chromatography include, without limitation, high-performance liquid chromatography (HPLC), UHPLC, normal phase HPLC (NP-HPLC), reversed phase HPLC (RP-HPLC), ion exchange chromatography (IEC), such as cation or anion exchange chromatography, hydrophilic interaction chromatography (HILIC), hydrophobic interaction chromatography (HIC), size exclusion chromatography (SEC) including gel filtration chromatography or gel permeation chromatography, chromatofocusing, affinity chromatography such as immuno-affinity, immobilised metal affinity chromatography, and the like. Chromatography, including single-, two- or more-dimensional chromatography, can be used as a peptide fractionation method in conjunction with a further peptide analysis method, such as for example, with a downstream mass spectrometry analysis as described elsewhere in this specification.

Further peptide or polypeptide separation, identification or quantification methods can be used, optionally in conjunction with any of the above described analysis methods, for measuring biomarkers in the present disclosure. Such methods include, without limitation, chemical extraction partitioning, isoelectric focusing (IEF) including capillary isoelectric focusing (CIEF), capillary isotachophoresis (CITP), capillary electrochromatography (CEC), and the like, one-dimensional polyacrylamide gel electrophoresis (PAGE), two-dimensional polyacrylamide gel electrophoresis (2D-PAGE), capillary gel electrophoresis (CGE), capillary zone electrophoresis (CZE), micellar electrokinetic chromatography (MEKC), free flow electrophoresis (FFE), etc.

In the context of the invention, the term "capture agent" refers to a compound that can specifically bind to a target, in particular a biomarker. The term includes antibodies, antibody fragments, nucleic acid-based protein binding reagents (*e.g.* aptamers, Slow Off-rate Modified Aptamers (SOMAmer^{™})), protein-capture agents, natural ligands (*i.e.* a hormone for its receptor or vice versa), small molecules or variants thereof.

Capture agents can be configured to specifically bind to a target, in particular a biomarker. Capture agents can include but are not limited to organic molecules, such as polypeptides, polynucleotides and other non polymeric molecules that are identifiable to a skilled person. In the embodiments disclosed herein, capture agents include any agent that can be used to detect, purify, isolate, or enrich a target, in particular a biomarker. Any art-known affinity capture technologies can be used to selectively isolate and enrich/concentrate biomarkers that are components of complex mixtures of biological media for use in the disclosed methods.

Antibody capture agents that specifically bind to a biomarker can be prepared using any suitable methods known in the art. *See, e.g.,* Coligan, Current Protocols in Immunology (1991); Harlow & Lane, Antibodies: A Laboratory Manual (1988); Goding, Monoclonal Antibodies: Principles and Practice (2d ed. 1986). Antibody capture agents can be any immunoglobulin or derivative therof, whether natural or wholly or partially synthetically produced. All derivatives thereof which maintain specific binding ability are also included in the term. Antibody capture agents have a binding domain that is homologous or largely homologous to an immunoglobulin binding domain and can be derived from natural sources, or partly or wholly synthetically produced. Antibody capture agents can be monoclonal or polyclonal antibodies. In some embodiments, an antibody is a single chain antibody. Those of ordinary skill in the art will appreciate that antibodies can be provided in any of a variety of forms including, for example, humanized, partially humanized, chimeric, chimeric humanized, *etc.* Antibody capture agents can be antibody fragments including, but not limited to, Fab, Fab', F(ab')2, scFv, Fv, dsFv diabody, and Fd fragments. An antibody capture agent can be produced by any means. For example, an antibody capture agent can be enzymatically or chemically produced by fragmentation of an intact antibody and/or it can be recombinantly produced from a gene encoding the partial antibody sequence. An antibody capture agent can comprise a single chain antibody fragment. Alternatively or additionally, antibody capture agent can comprise multiple chains which are linked together, for example, by disulfide linkages; and, any functional fragments obtained from such molecules, wherein such fragments retain specific-binding properties of the parent antibody molecule. Because of their smaller size as functional components of the whole molecule, antibody fragments can offer advantages over intact antibodies for use in certain immunochemical techniques and experimental applications.

Suitable capture agents useful for practicing the invention also include aptamers. Aptamers are oligonucleotide sequences that can bind to their targets specifically via unique three dimensional (3-D) structures. An aptamer can include any suitable number of nucleotides and different aptamers can have either the same or different numbers of nucleotides. Aptamers can be DNA or RNA or chemically modified nucleic acids and can be single stranded, double stranded, or contain double stranded regions, and can include higher ordered structures. An aptamer can also be a photoaptamer, where a photoreactive or chemically reactive functional group is included in the aptamer to allow it to be covalently linked to its corresponding target. Use of an aptamer capture agent can include the use of two or more aptamers that specifically bind the same biomarker. An aptamer can include a tag. An aptamer can be identified using any known method, including the SELEX (systematic evolution of ligands by exponential enrichment), process. Once identified, an aptamer can be prepared or synthesized in accordance with any known method, including chemical synthetic methods and enzymatic synthetic methods and used in a variety of applications for biomarker detection. Liu et al., Curr Med Chem. 18(27):4117-25 (2011). Capture agents useful in practicing the methods of the invention also include SOMAmers (Slow Off-Rate Modified Aptamers) known in the art to have improved off-rate characteristics. Brody et al., J Mol Biol. 422(5):595-606 (2012). SOMAmers can be generated using using any known method, including the SELEX method.

It is understood by those skilled in the art that biomarkers can be modified prior to analysis to improve their resolution or to determine their identity. For example, the biomarkers can be subject to proteolytic digestion before analysis. Any protease can be used. Proteases, such as trypsin, that are likely to cleave the biomarkers into a discrete number of fragments are particularly useful. The fragments that result from digestion function as a fingerprint for the biomarkers, thereby enabling their detection indirectly. This is particularly useful where there are biomarkers with similar molecular masses that might be confused for the biomarker in question. Also, proteolytic fragmentation is useful for high molecular weight biomarkers because smaller biomarkers are more easily resolved by mass spectrometry. In another example, biomarkers can be modified to improve detection resolution. For instance, neuraminidase can be used to remove terminal sialic acid residues from glycoproteins to improve binding to an anionic adsorbent and to improve detection resolution. In another example, the biomarkers can be modified by the attachment of a tag of particular molecular weight that specifically binds to molecular biomarkers, further distinguishing them. Optionally, after detecting such modified biomarkers, the identity of the biomarkers can be further determined by matching the physical and chemical characteristics of the modified biomarkers in a protein database (*e.g.*, SwissProt).

It is further appreciated in the art that biomarkers in a sample can be captured on a substrate for detection. Traditional substrates include antibody-coated 96-well plates or nitrocellulose membranes that are subsequently probed for the presence of the proteins. Alternatively, protein-binding molecules attached to microspheres, microparticles, microbeads, beads, or other particles can be used for capture and detection of biomarkers. The protein-binding molecules can be antibodies, peptides, peptoids, aptamers, small molecule ligands or other protein-binding capture agents attached to the surface of particles. Each protein-binding molecule can include unique detectable label that is coded such that it can be distinguished from other detectable labels attached to other protein-binding molecules to allow detection of biomarkers in multiplex assays. Examples include, but are not limited to, color-coded microspheres with known fluorescent light intensities (see e.g., microspheres with xMAP technology produced by Luminex (Austin, Tex.); microspheres containing quantum dot nanocrystals, for example, having different ratios and combinations of quantum dot colors (e.g., Qdot nanocrystals produced by Life Technologies (Carlsbad, Calif.); glass coated metal nanoparticles (see *e.g.,* SERS nanotags produced by Nanoplex Technologies, Inc. (Mountain View, Calif.); barcode materials (see e.g., sub-micron sized striped metallic rods such as Nanobarcodes produced by Nanoplex Technologies, Inc.), encoded microparticles with colored bar codes (see e.g., CellCard produced by Vitra Bioscience, vitrabio.com), glass microparticles with digital holographic code images (see *e.g.,* CyVera microbeads produced by Illumina (San Diego, Calif.); chemiluminescent dyes, combinations of dye compounds; and beads of detectably different sizes.

In another aspect, biochips can be used for capture and detection of the biomarkers of the invention. Many protein biochips are known in the art. These include, for example, protein biochips produced by Packard BioScience Company (Meriden Conn.), Zyomyx (Hayward, Calif.) and Phylos (Lexington, Mass.). In general, protein biochips comprise a substrate having a surface. A capture reagent or adsorbent is attached to the surface of the substrate. Frequently, the surface comprises a plurality of addressable locations, each of which location has the capture agent bound there. The capture agent can be a biological molecule, such as a polypeptide or a nucleic acid, which captures other biomarkers in a specific manner. Alternatively, the capture agent can be a chromatographic material, such as an anion exchange material or a hydrophilic material. Examples of protein biochips are well known in the art.

Measuring mRNA in a biological sample can be used as a surrogate for detection of the level of the corresponding protein biomarker in a biological sample. Thus, any of the biomarkers or biomarker panels described herein can also be detected by detecting the appropriate RNA. Levels of mRNA can measured by reverse transcription quantitative polymerase chain reaction (RT-PCR followed with qPCR). RT-PCR is used to create a cDNA from the mRNA. The cDNA can be used in a qPCR assay to produce fluorescence as the DNA amplification process progresses. By comparison to a standard curve, qPCR can produce an absolute measurement such as number of copies of mRNA per cell. Northern blots, microarrays, Invader assays, and RT-PCR combined with capillary electrophoresis have all been used to measure expression levels of mRNA in a sample. See Gene Expression Profiling: Methods and Protocols, Richard A. Shimkets, editor, Humana Press, 2004.

Some embodiments disclosed herein relate to diagnostic and prognostic methods of determining the probability for preeclampsia in a pregnant female. The detection of the level of expression of one or more biomarkers and/or the determination of a ratio of biomarkers can be used to determine the probability for preeclampsia in a pregnant female. Such detection methods can be used, for example, for early diagnosis of the condition, to determine whether a subject is predisposed to preeclampsia, to monitor the progress of preeclampsia or the progress of treatment protocols, to assess the severity of preeclampsia, to forecast the outcome of preeclampsia and/or prospects of recovery or birth at full term, or to aid in the determination of a suitable treatment for preeclampsia.

The quantitation of biomarkers in a biological sample can be determined, without limitation, by the methods described above as well as any other method known in the art. The quantitative data thus obtained is then subjected to an analytic classification process. In such a process, the raw data is manipulated according to an algorithm, where the algorithm has been pre-defined by a training set of data, for example as described in the examples provided herein. An algorithm can utilize the training set of data provided herein, or can utilize the guidelines provided herein to generate an algorithm with a different set of data.

In some embodiments, analyzing a measurable feature to determine the probability for preeclampsia in a pregnant female encompasses the use of a predictive model. In further embodiments, analyzing a measurable feature to determine the probability for preeclampsia in a pregnant female encompasses comparing said measurable feature with a reference feature. As those skilled in the art can appreciate, such comparison can be a direct comparison to the reference feature or an indirect comparison where the reference feature has been incorporated into the predictive model. In further embodiments, analyzing a measurable feature to determine the probability for preeclampsia in a pregnant female encompasses one or more of a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, or a combination thereof. In particular embodiments, the analysis comprises logistic regression.

An analytic classification process can use any one of a variety of statistical analytic methods to manipulate the quantitative data and provide for classification of the sample. Examples of useful methods include linear discriminant analysis, recursive feature elimination, a prediction analysis of microarray, a logistic regression, a CART algorithm, a FlexTree algorithm, a LART algorithm, a random forest algorithm, a MART algorithm, machine learning algorithms; etc.

Classification can be made according to predictive modeling methods that set a threshold for determining the probability that a sample belongs to a given class. The probability preferably is at least 50%, or at least 60%, or at least 70%, or at least 80% or higher. Classifications also can be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class.

The predictive ability of a model can be evaluated according to its ability to provide a quality metric, e.g. AUC (area under the curve) or accuracy, of a particular value, or range of values. Area under the curve measures are useful for comparing the accuracy of a classifier across the complete data range. Classifiers with a greater AUC have a greater capacity to classify unknowns correctly between two groups of interest. In some embodiments, a desired quality threshold is a predictive model that will classify a sample with an accuracy of at least about 0.7, at least about 0.75, at least about 0.8, at least about 0.85, at least about 0.9, at least about 0.95, or higher. As an alternative measure, a desired quality threshold can refer to a predictive model that will classify a sample with an AUC of at least about 0.7, at least about 0.75, at least about 0.8, at least about 0.85, at least about 0.9, or higher.

As is known in the art, the relative sensitivity and specificity of a predictive model can be adjusted to favor either the selectivity metric or the sensitivity metric, where the two metrics have an inverse relationship. The limits in a model as described above can be adjusted to provide a selected sensitivity or specificity level, depending on the particular requirements of the test being performed. One or both of sensitivity and specificity can be at least about 0.7, at least about 0.75, at least about 0.8, at least about 0.85, at least about 0.9, or higher.

The raw data can be initially analyzed by measuring the values for each biomarker, usually in triplicate or in multiple triplicates. The data can be manipulated, for example, raw data can be transformed using standard curves, and the average of triplicate measurements used to calculate the average and standard deviation for each patient. These values can be transformed before being used in the models, e.g. log-transformed, Box-Cox transformed (Box and Cox, Royal Stat. Soc., Series B, 26:211-246(1964). The data are then input into a predictive model, which will classify the sample according to the state. The resulting information can be communicated to a patient or health care provider.

To generate a predictive model for preeclampsia, a robust data set, comprising known control samples and samples corresponding to the preeclampsia classification of interest is used in a training set. A sample size can be selected using generally accepted criteria. As discussed above, different statistical methods can be used to obtain a highly accurate predictive model. Examples of such analysis are provided in Example 2.

In one embodiment, hierarchical clustering is performed in the derivation of a predictive model, where the Pearson correlation is employed as the clustering metric. One approach is to consider a preeclampsia dataset as a "learning sample" in a problem of "supervised learning." CART is a standard in applications to medicine (Singer , Recursive Partitioning in the Health Sciences, Springer(1999)) and can be modified by transforming any qualitative features to quantitative features; sorting them by attained significance levels, evaluated by sample reuse methods for Hotelling's T² statistic; and suitable application of the lasso method. Problems in prediction are turned into problems in regression without losing sight of prediction, indeed by making suitable use of the Gini criterion for classification in evaluating the quality of regressions.

This approach led to what is termed FlexTree (Huang, Proc. Nat. Acad. Sci. U.S.A 101:10529-10534(2004)). FlexTree performs very well in simulations and when applied to multiple forms of data and is useful for practicing the claimed methods. Software automating FlexTree has been developed. Alternatively, LARTree or LART can be used (Tumbull (2005) Classification Trees with Subset Analysis Selection by the Lasso, Stanford University). The name reflects binary trees, as in CART and FlexTree; the lasso, as has been noted; and the implementation of the lasso through what is termed LARS by Efron et al. (2004) Annals of Statistics 32:407-451 (2004). See, also, Huang et al.., Proc. Natl. Acad. Sci. USA. 101(29):10529-34 (2004). Other methods of analysis that can be used include logic regression. One method of logic regression Ruczinski , Journal of Computational and Graphical Statistics 12:475-512 (2003). Logic regression resembles CART in that its classifier can be displayed as a binary tree. It is different in that each node has Boolean statements about features that are more general than the simple "and" statements produced by CART.

Another approach is that of nearest shrunken centroids (Tibshirani, Proc. Natl. Acad. Sci. U.S.A 99:6567-72(2002)). The technology is k-means-like, but has the advantage that by shrinking cluster centers, one automatically selects features, as is the case in the lasso, to focus attention on small numbers of those that are informative. The approach is available as PAM software and is widely used. Two further sets of algorithms that can be used are random forests (Breiman, Machine Learning 45:5-32 (2001)) and MART (Hastie, The Elements of Statistical Learning, Springer (2001)). These two methods are known in the art as "committee methods," that involve predictors that "vote" on outcome.

To provide significance ordering, the false discovery rate (FDR) can be determined. First, a set of null distributions of dissimilarity values is generated. In one embodiment, the values of observed profiles are permuted to create a sequence of distributions of correlation coefficients obtained out of chance, thereby creating an appropriate set of null distributions of correlation coefficients (Tusher et al. , Proc. Natl. Acad. Sci. U.S.A 98, 5116-21 (2001)). The set of null distribution is obtained by: permuting the values of each profile for all available profiles; calculating the pair-wise correlation coefficients for all profile; calculating the probability density function of the correlation coefficients for this permutation; and repeating the procedure for N times, where N is a large number, usually 300. Using the N distributions, one calculates an appropriate measure (mean, median, *etc.*) of the count of correlation coefficient values that their values exceed the value (of similarity) that is obtained from the distribution of experimentally observed similarity values at given significance level.

The FDR is the ratio of the number of the expected falsely significant correlations (estimated from the correlations greater than this selected Pearson correlation in the set of randomized data) to the number of correlations greater than this selected Pearson correlation in the empirical data (significant correlations). This cut-off correlation value can be applied to the correlations between experimental profiles. Using the aforementioned distribution, a level of confidence is chosen for significance. This is used to determine the lowest value of the correlation coefficient that exceeds the result that would have obtained by chance. Using this method, one obtains thresholds for positive correlation, negative correlation or both. Using this threshold(s), the user can filter the observed values of the pair wise correlation coefficients and eliminate those that do not exceed the threshold(s). Furthermore, an estimate of the false positive rate can be obtained for a given threshold. For each of the individual "random correlation" distributions, one can find how many observations fall outside the threshold range. This procedure provides a sequence of counts. The mean and the standard deviation of the sequence provide the average number of potential false positives and its standard deviation.

In an alternative analytical approach, variables chosen in the cross-sectional analysis are separately employed as predictors in a time-to-event analysis (survival analysis), where the event is the occurrence of preeclampsia, and subjects with no event are considered censored at the time of giving birth. Given the specific pregnancy outcome (preeclampsia event or no event), the random lengths of time each patient will be observed, and selection of proteomic and other features, a parametric approach to analyzing survival can be better than the widely applied semi-parametric Cox model. A Weibull parametric fit of survival permits the hazard rate to be monotonically increasing, decreasing, or constant, and also has a proportional hazards representation (as does the Cox model) and an accelerated failure-time representation. All the standard tools available in obtaining approximate maximum likelihood estimators of regression coefficients and corresponding functions are available with this model.

In addition the Cox models can be used, especially since reductions of numbers of covariates to manageable size with the lasso will significantly simplify the analysis, allowing the possibility of a nonparametric or semi-parametric approach to prediction of time to preeclampsia. These statistical tools are known in the art and applicable to all manner of proteomic data. A set of biomarker, clinical and genetic data that can be easily determined, and that is highly informative regarding the probability for preeclampsia and predicted time to a preeclampsia event in said pregnant female is provided. Also, algorithms provide information regarding the probability for preeclampsia in the pregnant female.

In the development of a predictive model, it can be desirable to select a subset of markers, *i.e.* at least 3, at least 4, at least 5, at least 6, up to the complete set of markers. Usually a subset of markers will be chosen that provides for the needs of the quantitative sample analysis, e.g. availability of reagents, convenience of quantitation, etc., while maintaining a highly accurate predictive model. The selection of a number of informative markers for building classification models requires the definition of a performance metric and a user-defined threshold for producing a model with useful predictive ability based on this metric. For example, the performance metric can be the AUROC, the sensitivity and/or specificity of the prediction as well as the overall accuracy of the prediction model.

As will be understood by those skilled in the art, an analytic classification process can use any one of a variety of statistical analytic methods to manipulate the quantitative data and provide for classification of the sample. Examples of useful methods include, without limitation, linear discriminant analysis, recursive feature elimination, a prediction analysis of microarray, a logistic regression, a CART algorithm, a FlexTree algorithm, a LART algorithm, a random forest algorithm, a MART algorithm, and machine learning algorithms.

As described in Example 2, various methods are used in a training model. The selection of a subset of markers can be for a forward selection or a backward selection of a marker subset. The number of markers can be selected that will optimize the performance of a model without the use of all the markers. One way to define the optimum number of terms is to choose the number of terms that produce a model with desired predictive ability (e.g. an AUC>0.75, or equivalent measures of sensitivity/specificity) that lies no more than one standard error from the maximum value obtained for this metric using any combination and number of terms used for the given algorithm.

**Table 1. Transitions with p-values less than 0.05 in univariate Cox Proportional Hazards to predict Gestational Age of time to event (preeclampsia).**

| | | |
|---|---|---|
| TSDQIHFFFAK_447.56_512.3 | 0.00 | ANT3_HUMAN |
| DPNGLPPEAQK_583.3_669.4 | 0.00 | RET4_HUMAN |
| SVSLPSLDPASAK_636.35_885.5 | 0.00 | APOB_HUMAN |
| SSNNPHSPIVEEFQVPYNK_729.36_261 .2 | 0.00 | C1S_HUMAN |
| IEGNLIFDPNNYLPK_873.96_414.2 | 0.00 | APOB_HUMAN |
| YWGVASFLQK_599.82_849.5 | 0.00 | RET4_HUMAN |
| ITENDIQIALDDAK_779.9_632.3 | 0.00 | APOB_HUMAN |
| IEGNLIFDPNNYLPK_873.96_845.5 | 0.00 | APOB_HUMAN |
| GWVTDGFSSLK_598.8_953.5 | 0.00 | APOC3_HUMAN |
| TGISPLALIK_506.82_741.5 | 0.00 | APOB HUMAN |
| SVSLPSLDPASAK_636.35_473.3 | 0.00 | APOB_HUMAN |
| IIGGSDADIK_494.77_762.4 | 0.00 | C1S_HUMAN |
| TGISPLALIK_506.82_654.5 | 0.00 | APOB_HUMAN |
| TLLIANETLR_572.34_703.4 | 0.00 | IL5_HUMAN |
| YWGVASFLQK_599.82_350.2 | 0.00 | RET4_HUMAN |
| VSALLTPAEQTGTWK_801.43_371.2 | 0.00 | APOB_HUMAN |
| DPNGLPPEAQK_583.3_497.2 | 0.00 | RET4_HUMAN |
| VNHVTLSQPK_561.82_673.4 | 0.00 | B2MG_HUMAN |
| DALSSVQESQVAQQAR_572.96_502.3 | 0.00 | APOC3_HUMAN |
| IAQYYYTFK_598.8_884.4 | 0.00 | F13B_HUMAN |
| IEEIAAK_387.22_531.3 | 0.00 | CO5_HUMAN |
| GWVTDGFSSLK_598.8_854.4 | 0.00 | APOC3_HUMAN |
| VNHVTLSQPK_561.82_351.2 | 0.00 | B2MG_HUMAN |
| ITENDIQIALDDAK_779.9_873.5 | 0.00 | APOB_HUMAN |
| VSALLTPAEQTGTWK_801.43_585.4 | 0.00 | APOB_HUMAN |
| VILGAHQEVNLEPHVQEIEVSR_832.78_ 860.4 | 0.00 | PLMN_HUMAN |
| SPELQAEAK_486.75_788.4 | 0.00 | APOA2_HUMAN |
| SPELQAEAK_486.75_659.4 | 0.00 | APOA2_HUMAN |
| DYWSTVK_449.72_620.3 | 0.00 | APOC3_HUMAN |
| VPLALFALNR_557.34_620.4 | 0.00 | PLPD_HUMAN |
| TSDQIHFFFAK_447.56_659.4 | 0.00 | ANT3_HUMAN |
| DALSSVQESQVAQQAR_572.96_672.4 | 0.00 | APOC3_HUMAN |
| VIAVNEVGR_478.78_284.2 | 0.00 | CHL1_HUMAN |
| LLEVPEGR_456.76_686.3 | 0.00 | C1S_HUMAN |
| VEPLYELVTATDFAYSSTVR_754.38_5 49.3 | 0.00 | CO8B_HUMAN |
| HHGPTITAK_321.18_275.1 | 0.01 | AMBP_HUMAN |
| ALNFGGIGVVVGHELTHAFDDQGR_8 37.09_299.2 | 0.01 | ECE1_HUMAN |
| ETLLQDFR 511,27_565.3 | 0.01 | AMBP_HUMAN |
| HHGPTITAK_321.18_432.3 | 0.01 | AMBP_HUMAN |
| IIGGSDADIK_494.77_260.2 | 0.01 | C1S_HUMAN |

**Table 2. Top 40 transitions with p-values less than 0.05 in univariate Cox Proportional Hazards to predict Gestational Age of time to event (preeclampsia), sorted by protein ID.**

| **Transition** | **cox pvalues** | **protein** |
|---|---|---|
| HHGPTITAK_321.18_275.1 | 0.01 | AMBP_HUMAN |
| ETLLQDFR_511.27_565.3 | 0.01 | AMBP_HUMAN |
| HHGPTITAK_321.18_432.3 | 0.01 | AMBP_HUMAN |
| TSDQIHFFFAK_447.56_512.3 | 0.00 | ANT3_HUMAN |
| TSDQIHFFFAK_447.56_659.4 | 0.00 | ANT3_HUMAN |
| SPELQAEAK_486.75_788.4 | 0.00 | APOA2_HUMAN |
| SPELQAEAK_486.75_659.4 | 0.00 | APOA2_HUMAN |
| SVSLPSLDPASAK_636.35_885.5 | 0.00 | APOB_HUMAN |
| IEGNLIFDPNNYLPK_873.96_414.2 | 0.00 | APOB_HUMAN |
| ITENDIQIALDDAK_779.9_632.3 | 0.00 | APOB_HUMAN |
| IEGNLIFDPNNYLPK_873.96_845.5 | 0.00 | APOB_HUMAN |
| TGISPLALIK_506.82_741.5 | 0.00 | APOB_HUMAN |
| SVSLPSLDPASAK_636.35_473.3 | 0.00 | APOB_HUMAN |
| TGISPLALIK_506.82_654.5 | 0.00 | APOB_HUMAN |
| VSALLTPAEQTGTWK_801.43_371.2 | 0.00 | APOB_HUMAN |
| ITENDIQIALDDAK_779.9_873.5 | 0.00 | APOB_HUMAN |
| VSALLTPAEQTGTWK_801.43_585.4 | 0.00 | APOB_HUMAN |
| GWVTDGFSSLK_598.8_953.5 | 0.00 | APOC3_HUMAN |
| DALSSVQESQVAQQAR_572.96_502.3 | 0.00 | APOC3_HUMAN |
| GWVTDGFSSLK_598.8_854.4 | 0.00 | APOC3_HUMAN |
| DYWSTVK_449.72_620.3 | 0.00 | APOC3_HUMAN |
| DALSSVQESQVAQQAR_572.96_672.4 | 0.00 | APOC3_HUMAN |
| VNHVTLSQPK_561.82_673.4 | 0.00 | B2MG_HUMAN |
| VNHVTLSQPK_561.82_351.2 | 0.00 | B2MG_HUMAN |
| SSNNPHSPIVEEFQVPYNK_729.36_261.2 | 0.00 | C1S_HUMAN |
| IIGGSDADIK_494.77_762.4 | 0.00 | C1S_HUMAN |
| LLEVPEGR_456.76_686.3 | 0.00 | C1S_HUMAN |
| IIGGSDADIK_494.77_260.2 | 0.01 | C1S_HUMAN |
| VIAVNEVGR_478.78_284.2 | 0.00 | CHL1_HUMAN |
| IEEIAAK_387.22_531.3 | 0.00 | CO5_HUMAN |
| VEPLYELVTATDFAYSSTVR_754.38_549.3 | 0.00 | CO8B_HUMAN |
| ALNFGGIGVVVGHELTHAFDDQGR_837.09_299.2 | 0.01 | ECE1_HUMAN |
| IAQYYYTFK_598.8_884.4 | 0.00 | F13B_HUMAN |
| TLLIANETLR_572.34_703.4 | 0.00 | IL5_HUMAN |
| VPLALFALNR_557.34_620.4 | 0.00 | PEPD_HUMAN |
| VILGAHQEVNLEPHVQEIEVSR_832.78_860.4 | 0.00 | PLMN_HUMAN |
| DPNGLPPEAQK_583.3_669.4 | 0.00 | RET4_HUMAN |
| YWGVASFLQK 599.82 849.5 | 0.00 | RET4_HUMAN |
| YWGVASFLQK_599.82_350.2 | 0.00 | RET4_HUMAN |
| DPNGLPPEAQK_583.3_497.2 | 0.00 | RET4_HUMAN |

**Table 3. Transitions selected by Cox stepwise AIC analysis**

| Transition | coef | exp(coef ) | see(coef) | z | Pr(>\|z\|) |
|---|---|---|---|---|---|
| Collection.Window.GA.in.Days | 0.43 | 1.54E+0 0 | 0.19 | 2.22 | 0.03 |
| IIGGSDADIK_494.77_762.4 | 44.40 | 1.91E+1 9 | 18.20 | 2.44 | 0.01 |
| GGEGTGYFVDFSVR_745.85_869.5 | 6.91 | 1.00E+0 3 | 2.76 | 2.51 | 0.01 |
| SPEQQETVLDGNLIIR_906.48_685.4 | 17.28 | 3.21E+0 7 | 7.49 | 2.31 | 0.02 |
| EPGLCTWQSLR_673.83_790.4 | -2.08 | 1.25E-01 | 1.02 | -2.05 | 0.04 |

**Table 4. Transitions selected by Cox lasso analysis**

| Transition | coef | exp(coef) | se(coef) | z | Pr(>\|z\|) |
|---|---|---|---|---|---|
| Collection.Window.GA.in.Days | 0.05069 | 1.052 | 0.02348 | 2.159 | 0.0309 |
| SPELQAEAK_486.75_788.4 | 0.68781 | 1.98936 | 0.4278 | 1.608 | 0.1079 |
| SSNNPHSPIVEEFQVPYNK_72 9.36_261.2 | 2.63659 | 13.96553 | 1.69924 | 1.552 | 0.1208 |

**Table 5. Area under the ROC curve for individual analytes to discriminate preeclampsia subjects from non-preeclampsia subjects. The 196 transitions with the highest ROC area are shown.**

| Transition | ROC area |
|---|---|
| SPELQAEAK_486.75_788.4 | 0.92 |
| SSNNPHSPIVEEFQVPYNK_729.36_261.2 | 0.88 |
| VNHVTLSQPK_561.82_673.4 | 0.85 |
| TLLIANETLR_572.34_703.4 | 0.84 |
| SSNNPHSPIVEEFQVPYNK_729.36_521.3 | 0.83 |
| IIGGSDADIK_494.77_762.4 | 0.82 |
| VVGGLVALR_442.29_784.5 | 0.82 |
| ALNFGGIGVVVGHELTHAFDDQGR_837.09_299.2 | 0.81 |
| DYWSTVK_449.72_620.3 | 0.81 |
| FSVVYAK_407.23_579.4 | 0.81 |
| GWVTDGFSSLK_598.8_953.5 | 0.81 |
| IIGGSDADIK_494.77_260.2 | 0.81 |
| LLEVPEGR_456.76_356.2 | 0.81 |
| DALSSVQESQVAQQAR_572.96_672.4 | 0.80 |
| DPNGLPPEAQK_583.3_497.2 | 0.80 |
| FSVVYAK_407.23_381.2 | 0.80 |
| LLEVPEGR_456.76_686.3 | 0.80 |
| SPELQAEAK_486.75_659.4 | 0.80 |
| VVLSSGSGPGLDLPLVLGLPLQLK_791.48_598.4 | 0.79 |
| ETLLQDFR_511.27_565.3 | 0.79 |
| VNHVTLSQPK_561.82_351.2 | 0.79 |
| VVGGLVALR_442.29_685.4 | 0.79 |
| YTTEIIK_434.25_603.4 | 0.79 |
| DPNGLPPEAQK_583.3_669.4 | 0.78 |
| EDTPNSVWEPAK_686.82_315.2 | 0.78 |
| GWVTDGFSSLK_598.8_854.4 | 0.78 |
| HHGPTITAK_321.18_432.3 | 0.78 |
| LHEAFSPVSYQHDLALLR_699.37_251.2 | 0.78 |
| GA.of.Time.to.Event.in.Days | 0.77 |
| DALSSVQESQVAQQAR_572.96_502.3 | 0.77 |
| DYWSTVK_449.72_347.2 | 0.77 |
| IAQYYYTFK_598.8_395.2 | 0.77 |
| YWGVASFLQK_599.82_849.5 | 0.77 |
| AHYDLR_387.7_288.2 | 0.76 |
| EDTPNSVWEPAK_686.82_630.3 | 0.76 |
| GDTYPAELYITGSILR_884.96_922.5 | 0.76 |
| SVSLPSLDPASAK_636.35_885.5 | 0.76 |
| TSESGELHGLTTEEEFVEGIYK_819.06_310.2 | 0.76 |
| ALEQDLPVNIK_620.35_570.4 | 0.75 |
| HHGPTITAK_321.18_275.1 | 0.75 |
| IAQYYYTFK_598.8_884.4 | 0.75 |
| ITENDIQIALDDAK_779.9_632.3 | 0.75 |
| LPNNVLQEK_527.8_844.5 | 0.75 |
| YWGVASFLQK_599.82_350.2 | 0.75 |
| FQLPGQK_409.23_276.1 | 0.75 |
| HTLNQIDEVK_598.82_958.5 | 0.75 |
| VVLSSGSGPGLDLPLVLGLPLQLK_791.48_768.5 | 0.75 |
| DADPDTFFAK_563.76_302.1 | 0.74 |
| DADPDTFFAK_563.76_825.4 | 0.74 |
| FQLPGQK_409.23_429.2 | 0.74 |
| HFQNLGK_422.23_527.2 | 0.74 |
| VIAVNEVGR_478.78_284.2 | 0.74 |
| VPLALFALNR_557.34_620.4 | 0.74 |
| ETLLQDFR_511.27_322.2 | 0.73 |
| FNAVLTNPQGDYDTSTGK_964.46_262.1 | 0.73 |
| SVSLPSLDPASAK_636.35_473.3 | 0.73 |
| AHYDLR_387.7_566.3 | 0.72 |
| ALNHLPLEYNSALYSR_620.99_538.3 | 0.72 |
| AWVAWR_394.71_258.1 | 0.72 |
| AWVAWR_394.71_531.3 | 0.72 |
| ETAASLLQAGYK_626.33_879.5 | 0.72 |
| IALGGLLFPASNLR_481.29_657.4 | 0.72 |
| IAPQLSTEELVSLGEK_857.47_533.3 | 0.72 |
| ITENDIQIALDDAK_779.9_873.5 | 0.72 |
| VAPEEHPVLLTEAPLNPK_652.03_869.5 | 0.71 |
| EPGLCTWQSLR_673.83_375.2 | 0.71 |
| IAPQLSTEELVSLGEK_857.47_333.2 | 0.71 |
| SPEQQETVLDGNLIIR_906.48_699.3 | 0.71 |
| VSALLTPAEQTGTWK_801.43_371.2 | 0.71 |
| VSALLTPAEQTGTWK_801.43_585.4 | 0.71 |
| VSEADSSNADWVTK_754.85_347.2 | 0.71 |
| GDTYPAELYITGSILR_884.96_274.1 | 0.70 |
| IPGIFELGISSQSDR_809.93_849.4 | 0.70 |
| IQTHSTTYR_369.52_540.3 | 0.70 |
| LLDSLPSDTR_558.8_890.4 | 0.70 |
| QLGLPGPPDVPDHAAYHPF_676.67_299.2 | 0.70 |
| SYELPDGQVITIGNER_895.95_251.1 | 0.70 |
| VILGAHQEVNLEPHVQEIEVSR_832.78_860.4 | 0.70 |
| WGAAPYR_410.71_577.3 | 0.69 |
| DFHINLFQVLPWLK_885.49_543.3 | 0.69 |
| LLDSLPSDTR_558.8_276.2 | 0.69 |
| VEPLYELVTATDFAYSSTVR_754.38_549.3 | 0.69 |
| VPTADLEDVLPLAEDITNILSK_789.43_841.4 | 0.69 |
| GGEGTGYFVDFSVR_745.85_869.5 | 0.69 |
| HTLNQIDEVK_598.82_951.5 | 0.69 |
| LIENGYFHPVK_439.57_627.4 | 0.69 |
| LPNNVLQEK_527.8_730.4 | 0.69 |
| NKPGVYTDVAYYLAWIR_677.02_545.3 | 0.69 |
| NTVISVNPSTK_580.32_845.5 | 0.69 |
| QLGLPGPPDVPDHAAYHPF_676.67_263.1 | 0.69 |
| YTTEIIK_434.25_704.4 | 0.69 |
| LPDATPK_371.21_628.3 | 0.68 |
| IEGNLIFDPNNYLPK_873.96_845.5 | 0.68 |
| LEQGENVFLQATDK_796.4_822.4 | 0.68 |
| TLYSSSPR_455.74_533.3 | 0.68 |
| TLYSSSPR_455.74_696.3 | 0.68 |
| VSEADSSNADWVTK_754.85_533.3 | 0.68 |
| DGSPDVTTADIGANTPDATK_973.45_844.4 | 0.67 |
| EWVAIESDSVQPVPR_856.44_486.2 | 0.67 |
| IALGGLLFPASNLR_481.29_412.3 | 0.67 |
| IEEIAAK_387.22_531.3 | 0.67 |
| IEGNLIFDPNNYLPK_873.96_414.2 | 0.67 |
| LYYGDDEK_501.72_726.3 | 0.67 |
| TGISPLALIK_506.82_741.5 | 0.67 |
| VPTADLEDVLPLAEDITNILSK_789.43_940.5 | 0.67 |
| ADSQAQLLLSTVVGVFTAPGLHLK_822.46_983.6 | 0.66 |
| AYSDLSR_406.2_577.3 | 0.66 |
| DFHINLFQVLPWLK_885.49_400.2 | 0.66 |
| DLHLSDVFLK_396.22_260.2 | 0.66 |
| EWVAIESDSVQPVPR_856.44_468.3 | 0.66 |
| FNAVLTNPQGDYDTSTGK_964.46_333.2 | 0.66 |
| LSSPAVITDK_515.79_743.4 | 0.66 |
| LYYGDDEK_501.72_563.2 | 0.66 |
| SGFSFGFK_438.72_732.4 | 0.66 |
| IIEVEEEQEDPYLNDR_995.97_777.4 | 0.66 |
| AVYEAVLR_460.76_750.4 | 0.66 |
| WGAAPYR_410.71_634.3 | 0.66 |
| FTFTLHLETPKPSISSSNLNPR_829.44_874.4 | 0.65 |
| DAQYAPGYDK_564.25_315.1 | 0.65 |
| YGLVTYATYPK_638.33_334.2 | 0.65 |
| DGSPDVTTADIGANTPDATK_973.45_531.3 | 0.65 |
| ETAASLLQAGYK_626.33_679.4 | 0.65 |
| ALNHLPLEYNSALYSR_620.99_696.4 | 0.65 |
| DISEVVTPR_508.27_787.4 | 0.65 |
| IS.2_662.3_313.1 | 0.65 |
| IVLGQEQDSYGGK_697.35_261.2 | 0.65 |
| IVLGQEQDSYGGK_697.35_754.3 | 0.65 |
| TLEAQLTPR 514.79 685.4 | 0.65 |
| VPVAVQGEDTVQSLTQGDGVAK_733.38_775.4 | 0.65 |
| VAPEEHPVLLTEAPLNPK_652.03_568.3 | 0.64 |
| ADSQAQLLLSTVVGVFTAPGLHLK_822.46_664.4 | 0.64 |
| AEAQAQYSAAVAK_654.33_908.5 | 0.64 |
| DISEVVTPR_508.27_472.3 | 0.64 |
| ELLESYIDGR_597.8_710.3 | 0.64 |
| TGISPLALIK_506.82_654.5 | 0.64 |
| TNLESILSYPK_632.84_807.5 | 0.64 |
| DAQYAPGYDK_564.25_813.4 | 0.63 |
| LPTAVVPLR_483.31_755.5 | 0.63 |
| DSPVLIDFFEDTER_841.9_512.3 | 0.63 |
| FAFNLYR_465.73_712.4 | 0.63 |
| FVFGTTPEDILR_697.87_843.5 | 0.63 |
| GDSGGAFAVQDPNDK_739.33_473.2 | 0.63 |
| SLDFTELDVAAEK_719.36_316.2 | 0.63 |
| SLLQPNK_400.24_599.4 | 0.63 |
| TLLIANETLR_572.34_816.5 | 0.63 |
| VILGAHQEVNLEPHVQEIEVSR_832.78_603.3 | 0.63 |
| VQEAHLTEDQIFYFPK_655.66_701.4 | 0.63 |
| FTFTLHLETPKPSISSSNLNPR_829.44_787.4 | 0.63 |
| AYSDLSR_406.2_375.2 | 0.62 |
| DDLYVSDAFHK_655.31_344.1 | 0.62 |
| DDLYVSDAFHK_655.31_704.3 | 0.62 |
| DPDQTDGLGLSYLSSHIANVER_796.39_456.2 | 0.62 |
| ESDTSYVSLK_564.77_347.2 | 0.62 |
| ESDTSYVSLK_564.77_696.4 | 0.62 |
| FVFGTTPEDILR_697.87_742.4 | 0.62 |
| ILDDLSPR_464.76_587.3 | 0.62 |
| LEQGENVFLQATDK_796.4_675.4 | 0.62 |
| LHEAFSPVSYQHDLALLR_699.37_380.2 | 0.62 |
| LIENGYFHPVK_439.57_343.2 | 0.62 |
| SLPVSDSVLSGFEQR_810.92_836.4 | 0.62 |
| TWDPEGVIFYGDTNPK_919.93_403.2 | 0.62 |
| VGEYSLYIGR_578.8_708.4 | 0.62 |
| VIAVNEVGR_478.78_744.4 | 0.62 |
| VPGTSTSATLTGLTR_731.4_761.5 | 0.62 |
| YEVQGEVFTKPQLWP_910.96_293.1 | 0.62 |
| AFTECCVVASQLR_770.87_673.4 | 0.61 |
| APLTKPLK_289.86_357.3 | 0.61 |
| DSPVLIDFFEDTER_841.9_399.2 | 0.61 |
| ELLESYIDGR_597.8_839.4 | 0.61 |
| FLQEQGHR_338.84_369.2 | 0.61 |
| IQTHSTTYR_369.52_627.3 | 0.61 |
| IS.3_432.6_397.3 | 0.61 |
| IS.4_706.3_780.3 | 0.61 |
| IS.4_706.3_927.4 | 0.61 |
| IS.5_726.3_876.3 | 0.61 |
| ISLLLIESWLEPVR_834.49_500.3 | 0.61 |
| LQGTLPVEAR_542.31_842.5 | 0.61 |
| NKPGVYTDVAYYLAWIR_677.02_821.5 | 0.61 |
| SLDFTELDVAAEK_719.36_874.5 | 0.61 |
| SYTITGLQPGTDYK_772.39_352.2 | 0.61 |
| TASDFITK_441.73_710.4 | 0.61 |
| VLSALQAVQGLLVAQGR_862.02_941.6 | 0.61 |
| VTGWGNLK_437.74_617.3 | 0.61 |
| YEVQGEVFTKPQLWP_910.96_392.2 | 0.61 |
| AFIQLWAFDAVK_704.89_650.4 | 0.60 |
| APLTKPLK_289.86_260.2 | 0.60 |
| GYVIIKPLVWV_643.9_304.2 | 0.60 |
| IITGLLEFEVYLEYLQNR_738.4_822.4 | 0.60 |
| ILDDLSPR_464.76_702.3 | 0.60 |
| LSSPAVITDK_515.79_830.5 | 0.60 |
| TDAPDLPEENQAR 728.34 843.4 | 0.60 |
| TFTLLDPK_467.77_359.2 | 0.60 |
| TFTLLDPK_467.77_686.4 | 0.60 |
| VLEPTLK_400.25_587.3 | 0.60 |
| YEFLNGR_449.72_606.3 | 0.60 |
| YGLVTYATYPK_638.33_843.4 | 0.60 |

**Table 6. AUROCs for random forest, boosting, lasso, and logistic regression models for a specific number of transitions permitted in the model, as estimated by 100 rounds of bootstrap resampling.**

| Number of transitions | rf | boosting | logit | lasso |
|---|---|---|---|---|
| 1 | 0.81 | 0.75 | 0.48 | 0.92 |
| 2 | 0.95 | 0.85 | 0.61 | 0.86 |
| 3 | 0.95 | 0.83 | 0.56 | 0.93 |
| 4 | 0.94 | 0.82 | 0.52 | 0.92 |
| 5 | 0.95 | 0.81 | 0.51 | 0.94 |
| 6 | 0.95 | 0.81 | 0.49 | 0.93 |
| 7 | 0.95 | 0.83 | 0.46 | 0.93 |
| 8 | 0.96 | 0.79 | 0.49 | 0.91 |
| 9 | 0.95 | 0.82 | 0.46 | 0.88 |
| 10 | 0.94 | 0.80 | 0.50 | 0.85 |
| 11 | 0.93 | 0.78 | 0.49 | 0.84 |
| 12 | 0.94 | 0.79 | 0.47 | 0.82 |
| 13 | 0.92 | 0.80 | 0.48 | 0.84 |
| 14 | 0.95 | 0.73 | 0.47 | 0.83 |
| 15 | 0.93 | 0.73 | 0.49 | 0.83 |

**Table 7. Top 15 transitions selected by each multivariate method, ranked by importance for that method.**

| | rf | boosting | lasso | logit |
|---|---|---|---|---|
| 1 | FSVVYAK_407. 23_579.4 | DPNGLPPEAQK_58 3.3_497.2 | SPELQAEAK_48 6.75_788.4 | AFIQLWAFDAVK_70 4.89_650.4 |
| 2 | SPELQAEAK_4 86.75_788.4 | ALNFGGIGVVVGH ELTHAFDDQGR_8 37.09_299.2 | VILGAHQEVNL EPHVQEIEVSR_ 832.78_860.4 | AFIQLWAFDAVK_70 4.89_836.4 |
| 3 | VNHVTLSQPK _561.82_673.4 | ALEQDLPVNIK_62 0.35_570.4 | VVGGLVALR_44 2.29_784.5 | AEAQAQYSAAVAK_ 654.33_709.4 |
| 4 | SSNNPHSPIVE EFQVPYNK_72 9.36_261.2 | DALSSVQESQVAQ QAR_572.96_502.3 | TSESGELHGLTT EEEFVEGIYK_81 9.06_310.2 | AFTECCVVASQLR_7 70.87_574.3 |
| 5 | SSNNPHSPIVE EFQVPYNK_72 9.36_521.3 | AHYDLR 387.7_28 8.2 | SSNNPHSPIVEE FQVPYNK_729.3 6_261.2 | ADSQAQLLLSTVVG VFTAPGLHLK_822.46 _664.4 |
| 6 | VVGGLVALR_ 442.29_784.5 | FQLPGQK_409.23_ 276.1 | VVLSSGSGPGL DLPLVLGLPLQL K_791.48_598.4 | AEAQAQYSAAVAK_ 654.33_908.5 |
| 7 | FQLPGQK_409. 23_276.1 | AFTECCVVASQLR _770.87_673.4 | ALEQDLPVNIK_ 620.35_570.4 | ADSQAQLLLSTVVG VFTAPGLHLK_822.46 _983.6 |
| 8 | TLLIANETLR_ 572.34_703.4 | ALNHLPLEYNSAL YSR_620.99_538.3 | IQTHSTTYR_369 .52_540.3 | AFTECCVVASQLR 7 70.87_673.4 |
| 9 | DYWSTVK_44 9.72_620.3 | ADSQAQLLLSTVV GVFTAPGLHLK_82 2.46_664.4 | SSNNPHSPIVEE FQVPYNK_729.3 6_21.3 | Collection.Window.GA. in.Days |
| 10 | VVGGLVALR_ 442.29_685.4 | AEAQAQYSAAVA K_654.33_908.5 | FSVVYAK_407.2 3_579.4 | AHYDLR_387.7_288.2 |
| 11 | DPNGLPPEAQ K_583.3_497.2 | ADSQAQLLLSTVV GVFTAPGLHLK_82 2.46_983.6 | IAQYYYTFK 59 8.8_884.4 | AHYDLR_387.7_566.3 |
| 12 | LLEVPEGR_45 6.76_356.2 | AITPPHPASQANIIF DITEGNLR_825.77_ 459.3 | IAQYYYTFK_59 8.8_395.2 | AITPPHPASQANIIFDI TEGNLR_825.77_459.3 |
| 13 | GWVTDGFSSL K_598.8_953.5 | Collection.Window.G A.in.Days | GDTYPAELYITG SILR_884.96_922. 5 | AITPPHPASQANIIFDI TEGNLR_825.77_917.5 |
| 14 | VILGAHQEVN LEPHVQEIEVS R_832.78_860.4 | AEAQAQYSAAVA K_654.33_709.4 | SPEQQETVLDG NLIIR_906.48_69 9.3 | ALEQDLPVNIK_620.3 5_570.4 |
| 15 | FQLPGQK_409. 23_429.2 | AFIQLWAFDAVK_ 704.89_650.4 | IAPQLSTEELVS LGEK_857.47_53 3.3 | ALEQDLPVNIK_620.3 5_798.5 |

In yet another aspect, the invention provides kits for determining probability of preeclampsia, wherein the kits can be used to detect N of the isolated biomarkers listed in Tables 2, 3, 4, 5 and 7 through 22. For example, the kits can be used to detect one or more, two or more, three or more, four or more, or five of the isolated biomarkers selected from the group consisting of SPELQAEAK, SSNNPHSPIVEEFQVPYN, VNHVTLSQPK, VVGGLVALR, and FSVVYAK, LDFHFSSDR, TVQAVLTVPK, GPGEDFR, ETLLQDFR, ATVVYQGER, and GFQALGDAADIR. In another aspect, the kits can be used to detect one or more, two or more, three or more, four or more, five or more, six or more, seven or more, or eight of the isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4), Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), Sex hormone-binding globulin (SHBG).

The kit can include one or more agents for detection of biomarkers, a container for holding a biological sample isolated from a pregnant female; and printed instructions for reacting agents with the biological sample or a portion of the biological sample to detect the presence or amount of the isolated biomarkers in the biological sample. The agents can be packaged in separate containers. The kit can further comprise one or more control reference samples and reagents for performing an immunoassay.

In one embodiment, the kit comprises agents for measuring the levels of at least N of the isolated biomarkers listed in Tables 2, 3, 4, 5 and 7 through 22. The kit can include antibodies that specifically bind to these biomarkers, for example, the kit can contain at least one of an antibody that specifically binds to alpha-1-microglobulin (AMBP), an antibody that specifically binds to ADP/ATP translocase 3 (ANT3), an antibody that specifically binds to apolipoprotein A-II (APOA2), an antibody that specifically binds to apolipoprotein C-III (APOC3), an antibody that specifically binds to apolipoprotein B (APOB), an antibody that specifically binds to beta-2-microglobulin (B2MG), an antibody that specifically binds to retinol binding protein 4 (RBP4 or RET4), an antibody that specifically binds to Inhibin beta C chain (INHBC), an antibody that specifically binds to Pigment epithelium-derived factor (PEDF), an antibody that specifically binds to Prostaglandin-H2 D-isomerase (PTGDS), an antibody that specifically binds to alpha-1-microglobulin (AMBP), an antibody that specifically binds to Beta-2-glycoprotein 1 (APOH), an antibody that specifically binds to Metalloproteinase inhibitor 1 (TIMP1), an antibody that specifically binds to Coagulation factor XIII B chain (F13B), an antibody that specifically binds to Alpha-2-HS-glycoprotein (FETUA), and an antibody that specifically binds to Sex hormone-binding globulin (SHBG).

The kit can comprise one or more containers for compositions contained in the kit. Compositions can be in liquid form or can be lyophilized. Suitable containers for the compositions include, for example, bottles, vials, syringes, and test tubes. Containers can be formed from a variety of materials, including glass or plastic. The kit can also comprise a package insert containing written instructions for methods of determining probability of preeclampsia.

From the foregoing description, it will be apparent that variations and modifications can be made to the invention described herein to adopt it to various usages and conditions. Such embodiments are also within the scope of the following claims.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

All patents and publications mentioned in this specification are herein incorporated by reference to the same extent as if each independent patent and publication was specifically and individually indicated to be incorporated by reference.

The following examples are provided by way of illustration, not limitation.

### EXAMPLES

### Example 1. Development of Sample Set for Discovery and Validation of Biomarkers for Preeclampsia

A standard protocol was developed governing conduct of the Proteomic Assessment of Preterm Risk (PAPR) clinical study. This protocol also provided the option that the samples and clinical information could be used to study other pregnancy complications. Specimens were obtained from women at 11 Internal Review Board (IRB) approved sites across the United States. After providing informed consent, serum and plasma samples were obtained, as well as pertinent information regarding the patient's demographic characteristics, past medical and pregnancy history, current pregnancy history and concurrent medications. Following delivery, data were collected relating to maternal and infant conditions and complications. Serum and plasma samples were processed according to a protocol that requires standardized refrigerated centrifugation, aliquoting of the samples into 0.5 ml 2-D bar-coded cryovials and subsequent freezing at -80°C.

Following delivery, preeclampsia cases were individually reviewed. Only preterm preeclampsia cases were used for this analysis. For discovery of biomarkers of preeclampsia, 20 samples collected between 17-28 weeks of gestation were analyzed. Samples included 9 cases, 9 term controls matched within one week of sample collection and 2 random term controls. The samples were processed in batches of 24 that included 20 clinical samples and 4 identical human gold standards (HGS). HGS samples are identical aliquots from a pool of human blood and were used for quality control. HGS samples were placed in position 1, 8, 15 and 24 of a batch with patient samples processed in the remaining 20 positions. Matched cases and controls were always processed adjacently.

The samples were subsequently depleted of high abundance proteins using the Human 14 Multiple Affinity Removal System (MARS 14), which removes 14 of the most abundant proteins that are essentially uninformative with regard to the identification for disease-relevant changes in the serum proteome. To this end, equal volumes of each clinical or HGS sample were diluted with column buffer and filtered to remove precipitates. Filtered samples were depleted using a MARS-14 column (4.6 x 100 mm, Cat. #5188-6558, Agilent Technologies). Samples were chilled to 4°C in the autosampler, the depletion column was run at room temperature, and collected fractions were kept at 4°C until further analysis. The unbound fractions were collected for further analysis.

A second aliquot of each clinical serum sample and of each HGS was diluted into ammonium bicarbonate buffer and depleted of the 14 high and approximately 60 additional moderately abundant proteins using an IgY14-SuperMix (Sigma) hand-packed column, comprised of 10 mL of bulk material (50% slurry, Sigma). Shi et al., Methods, 56(2):246-53 (2012). Samples were chilled to 4°C in the autosampler, the depletion column was run at room temperature, and collected fractions were kept at 4°C until further analysis. The unbound fractions were collected for further analysis.

Depleted serum samples were denatured with trifluorethanol, reduced with dithiotreitol, alkylated using iodoacetamide, and then digested with trypsin at a 1:10 trypsin: protein ratio. Following trypsin digestion, samples were desalted on a C18 column, and the eluate lyophilized to dryness. The desalted samples were resolubilized in a reconstitution solution containing five internal standard peptides.

Depleted and trypsin digested samples were analyzed using a scheduled Multiple Reaction Monitoring method (sMRM). The peptides were separated on a 150 mm x 0.32 mm Bio-Basic C18 column (ThermoFisher) at a flow rate of 5 µl/min using a Waters Nano Acquity UPLC and eluted using an acetonitrile gradient into a AB SCIEX QTRAP 5500 with a Turbo V source (AB SCIEX, Framingham, MA). The sMRM assay measured 1708 transitions that correspond to 854 peptides and 236 proteins. Chromatographic peaks were integrated using Rosetta Elucidator software (Ceiba Solutions).

Transitions were excluded from analysis, if their intensity area counts were less than 10000 and if they were missing in more than three samples per batch. Intensity area counts were log transformed and Mass Spectrometry run order trends and depletion batch effects were minimized using a regression analysis.

### Example 2. Analysis of Transitions to Identify PE Biomarkers

The objective of these analyses was to examine the data collected in Example 1 to identify transitions and proteins that predict preeclampsia. The specific analyses employed were (i) Cox time-to-event analyses and (ii) models with preeclampsia as a binary categorical dependent variable. The dependent variable for all the Cox analyses was Gestational Age of time to event (where event is preeclampsia). For the purpose of the Cox analyses, preeclampsia subjects have the event on the day of birth. Non-preeclampsia subjects are censored on the day of birth. Gestational age on the day of specimen collection is a covariate in all Cox analyses.

The assay data obtained in Example 1 were previously adjusted for run order and log transformed. The data was not further adjusted. There were 9 matched non-preeclampsia subjects, and two unmatched non-preeclampsia subjects, where matching was done according to center, gestational age and ethnicity.

### Univariate Cox Proportional Hazards Analyses

Univariate Cox Proportional Hazards analyses was performed to predict Gestational Age of time to event (preeclampsia), including Gestational age on the day of specimen collection as a covariate. Table 1 shows the 40 transitions with p-values less than 0.05. Table 2 shows the same transitions sorted by protein ID. There are 8 proteins that have multiple transitions with p-values less than 0.05: AMBP, ANT3, APOA2, APOB, APOC3, B2MG, C1S, and RET4.

### Multivariate Cox Proportional Hazards Analyses: Stepwise AIC selection

Cox Proportional Hazards analyses was performed to predict Gestational Age of time to event (preeclampsia), including Gestational age on the day of specimen collection as a covariate, using stepwise and lasso models for variable selection. The stepwise variable selection analysis used the Akaike Information Criterion (AIC) as the stopping criterion. Table 3 shows the transitions selected by the stepwise AIC analysis. The coefficient of determination (R²) for the stepwise AIC model is 0.87 of a maximum possible 0.9.

### Multivariate Cox Proportional Hazards Analyses: lasso selection

Lasso variable selection was utilized as the second method of multivariate Cox Proportional Hazards analyses to predict Gestational Age of time to event (preeclampsia), including Gestational age on the day of specimen collection as a covariate. Lasso regression models estimate regression coefficients using penalized optimization methods, where the penalty discourages the model from considering large regression coefficients since we usually believe such large values are not very likely. As a result, some regression coefficients are forced to be zero (i.e., excluded from the model). Here, the resulting model included analytes with non-zero regression coefficients only. The number of these analytes (with non-zero regression coefficients) depends on the severity of the penalty. Cross-validation was used to choose an optimum penalty level. Table 4 shows the results. The coefficient of determination (R²) for the lasso model is 0.53 of a maximum possible 0.9.

### Univariate ROC analysis of preeclampsia as a binary categorical dependent variable

Univariate analyses was used to discriminate preeclampsia subjects from non-preeclampsia subjects (preeclampsia as a binary categorical variable) as estimated by area under the receiver operating characteristic (ROC) curve. Table 5 shows the area under the ROC curve for the 196 transitions with the highest ROC area of 0.6 or greater.

### Multivariate analysis of preeclampsia as a binary categorical dependent variable

Multivariate analyses was performed to predict preeclampsia as a binary categorical dependent variable, using random forest, boosting, lasso, and logistic regression models. Random forest and boosting models grow many classification trees. The trees vote on the assignment of each subject to one of the possible classes. The forest chooses the class with the most votes over all the trees.

For each of the four methods (random forest, boosting, lasso, and logistic regression) each method was allowed to select and rank its own best 15 transitions. We then built models with 1 to 15 transitions. Each method sequentially reduces the number of nodes from 15 to 1 independently. A recursive option was used to reduce the number nodes at each step: To determine which node to be removed, the nodes were ranked at each step based on their importance from a nested cross-validation procedure. The least important node was eliminated. The importance measures for lasso and logistic regression are z-values. For random forest and boosting, the variable importance was calculated from permuting out-of-bag data: for each tree, the classification error rate on the out-of-bag portion of the data was recorded; the error rate was then recalculated after permuting the values of each variable (i.e., transition); if the transition was in fact important, there would have been be a big difference between the two error rates; the difference between the two error rates were then averaged over all trees, and normalized by the standard deviation of the differences. The AUCs for these models are shown in Table 6 and in Figure 1, as estimated by 100 rounds of bootstrap resampling. Table 7 shows the top 15 transitions selected by each multivariate method, ranked by importance for that method. These multivariate analyses suggest that models that combine 2 or more transitions give AUC greater than 0.9, as estimated by bootstrap.

In multivariate models, random forest (rf) and lasso models gave the best area under the ROC curve as estimated by bootstrap. The following transitions were selected by these two models for having high univariate ROC's:.
FSVVYAK_407.23_579.4
SPELQAEAK_486.75_788.4
VNHVTLSQPK_561.82_673.4
SSNNPHSPIVEEFQVPYNK_729.36_261.2
SSNNPHSPIVEEFQVPYNK_729.36_521.3
VVGGLVALR_442.29_784.5

In summary, univariate and multivariate Cox analyses were performed using transitions collected in Example 1 to predict Gestational Age at Birth, including Gestational age on the day of specimen collection as a covariate. In the univariate Cox analyses, 8 proteins were identified with multiple transitions with p-value less than 0.05. In multivariate Cox analyses, stepwise AIC variable analysis selected 4 transitions, while the lasso model selected 2 transitions. Univariate (ROC) and multivariate (random forest, boosting, lasso, and logistic regression) analyses were performed to predict preeclampsia as a binary categorical variable. Univariate analyses identify 78 analytes with AUROC of 0.7 or greater and 196 analytes with AUROC of 0.6 or greater. Multivariate analyses suggest that models that combine 2 or more transitions give AUC greater than 0.9, as estimated by bootstrap.

From the foregoing description, it will be apparent that variations and modifications can be made to the invention described herein to adopt it to various usages and conditions. Such embodiments are also within the scope of the following claims.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

All patents and publications mentioned in this specification are herein incorporated by reference to the same extent as if each independent patent and publication was specifically and individually indicated to be incorporated by reference.

### Example 3. Study II Shotgun Identification of Preeclampsia Biomarkers

A further study used a hypothesis-independent shotgun approach to identify and quantify additional biomarkers not present on our multiplexed hypothesis dependent MRM assay. Samples were processed as described in the preceding Examples unless noted below.

Serum samples were depleted of the 14 most abundant serum samples by MARS 14 as described in Example 1. Depleted serum was then reduced with dithiothreitol, alkylated with iodacetamide, and then digested with trypsin at a 1:20 trypsin to protein ratio overnight at 37°C. Following trypsin digestion, the samples were desalted on an Empore C18 96-well Solid Phase Extraction Plate (3M Company) and lyophilized to dryness. The desalted samples were resolubilized in a reconstitution solution containing five internal standard peptides.

Tryptic digests of MARS depleted patient (preeclampsia cases and normal pregnancycontrols) samples were fractionated by two-dimensional liquid chromatography and analyzed by tandem mass spectrometry. Aliquots of the samples, equivalent to 3-4 µl of serum, were injected onto a 6 cm x 75µm self-packed strong cation exchange (Luna SCX, Phenomenex) column. Peptides were eluded from the SCX column with salt (15, 30, 50, 70, and 100% B, where B = 250mM ammonium acetate, 2% acetonitrile, 0.1% formic acid in water) and consecutively for each salt elution, were bound to a 0.5 µl C18 packed stem trap (Optimize Technologies, Inc.) and further fractionated on a 10 cm x 75 µm reversed phase ProteoPep II PicoFrit column (New Objective). Peptides were eluted from the reversed phase column with an acetonitrile gradient containing 0.1% formic acid and directly ionized on an LTQ-Orbitrap (ThermoFisher). For each scan, peptide parent ion masses were obtained in the Orbitrap at 60K resolution and the top seven most abundant ions were fragmented in the LTQ to obtain peptide sequence information.

Parent and fragment ion data were used to search the Human RefSeq database using the Sequest (Eng et al., J. Am. Soc. Mass Spectrom 1994; 5:976-989) and X!Tandem (Craig and Beavis, Bioinformatics 2004; 20:1466-1467) algorithms. For Sequest, data was searched with a 20 ppm tolerance for the parent ion and 1 AMU for the fragment ion. Two missed trypsin cleavages were allowed, and modifications included static cysteine carboxyamidomethylation and methionine oxidation. After searching the data was filtered by charge state vs. Xcorr scores (charge +1 ≥ 1.5 Xcorr, charge +2 ≥ 2.0, charge +3 ≥ 2.5). Similar search parameters were used for X!tandem, except the mass tolerance for the fragment ion was 0.8 AMU and there is no Xcorr filtering. Instead, the PeptideProphet algorithm (Keller et al., Anal. Chem 2002;74:5383-5392) was used to validate each X! Tandem peptide-spectrum assignment and protein assignments were validated using ProteinProphet algorithm (Nesvizhskii et al., Anal. Chem 2002; 74:5383-5392). Data was filtered to include only the peptide-spectrum matches that had PeptideProphet probability of 0.9 or more. After compiling peptide and protein identifications, spectral count data for each peptide were imported into DAnTE software (Polpitiya et al., Bioinformatics. 2008; 24:1556-1558). Log transformed data was mean centered and missing values were filtered, by requiring that a peptide had to be identified in at least 2 cases and 2 controls. To determine the significance of an analyte, Receiver Operating Characteristic (ROC) curves for each analyte were created where the true positive rate (Sensitivity) is plotted as a function of the false positive rate (1-Specificity) for different thresholds that separate the SPTB and Term groups. The area under the ROC curve (AUC) is equal to the probability that a classifier will rank a randomly chosen positive instance higher than a randomly chosen negative one. Peptides with AUC greater than or equal to 0.6 identified by both approaches are found in Table 8 and those found uniquely by Sequest or Xtandem are found in Tables 9 and 10, respectively.

The differentially expressed proteins identified by the hypothesis-independent strategy above, not already present in our MRM-MS assay, were candidates for incorporation into the MRM-MS assay. Candidates were prioritized by AUC and biological function, with preference given for new pathways. Sequences for each protein of interest, were imported into Skyline software which generated a list of tryptic peptides, m/z values for the parent ions and fragment ions, and an instrument-specific collision energy (McLean et al. Bioinformatics (2010) 26 (7): 966-968.McLean et al. Anal. Chem (2010) 82 (24): 10116-10124).

The list was refined by eliminating peptides containing cysteines and methionines, where possible, and by using the shotgun data to select the charge state(s) and a subset of potential fragment ions for each peptide that had already been observed on a mass spectrometer.

After prioritizing parent and fragment ions, a list of transitions was exported with a single predicted collision energy. Approximately 100 transitions were added to a single MRM run. For development, MRM data was collected on either a QTRAP 5500 (AB Sciex) or a 6490 QQQ (Agilent). Commercially available human female serum (from pregnant and non-pregnant donors), was depleted and processed to tryptic peptides, as described above, and used to "scan" for peptides of interest. For development, peptides from the digested serum were separated with a 15 min acetonitrile.e gradient at 100 ul/min on a 2.1 x 50 mM Poroshell 120 EC-C18 column (Agilent) at 40°C.

The MS/MS data was imported back into Skyline, where all chromatograms for each peptide were overlayed and used to identify a concensus peak corresponding to the peptide of interest and the transitions with the highest intensities and the least noise. Table 11, contains a list of the most intensely observed candidate transitions and peptides for transfer to the MRM assay.

Next, the top 2-10 transitions per peptide and up to 7 peptides per protein were selected for collision energy (CE) optimization on the Agilent 6490. Using Skyline or MassHunter Qual software, the optimized CE value for each transition was determined based on the peak area or signal to noise. The two transitions with the largest peak areas per peptide and at least two peptides per protein were chosen for the final MRM method. Substitutions of transitions with lower peak areas were made when a transition with a larger peak area had a high background level or had a low m/z value that has more potential for interference.

Lastly, the retention times of selected peptides were mapped using the same column and gradient as our established sMRM assay. The newly discovered analytes were subsequently added to the sMRM method and used in a further hypothesis-dependent discovery study described in Example 4 below.

The above method was typical for most proteins. However, in some cases, the differentially expressed peptide identified in the shotgun method did not uniquely identify a protein, for example, in protein families with high sequence identity. In these cases, a MRM method was developed for each family member. Also, let it be noted that, for any given protein, peptides in addition to those found to be significant and fragment ions not observed on the Orbitrap may have been included in MRM optimization and added to the final sMRM method if those yielded the best signal intensities. In some cases, transition selection and CEs were re-optmized using purified, synthetic peptides.

**Table 8. Preeclampsia: Peptides significant with AUC > 0.6 by X! Tandem and Sequest**

| Protein description | Uniprot ID (name) | Peptide | XT_AUC | S_AUC |
|---|---|---|---|---|
| afamin | P43652 (AFAM HUMAN) | R.IVQIYKDLLR.N | 0.67 | 0.63 |
| afamin | P43652 (AFAM HUMAN) | K.VMNHICSK.Q | 0.73 | 0.74 |
| afamin | P43652 (AFAM HUMAN) | R.RHPDLSIPELLR.I | 0.86 | 0.83 |
| afamin | P43652 (AFAM HUMAN) | K.HFQNLGK.D | 0.71 | 0.75 |
| alpha-1-antichymotrypsin | P01011 (AACT_HUMAN) | K.ITLLSALVETR.T | 0.68 | 0.70 |
| alpha-1-antichymotrypsin | P01011 (AACT_HUMAN) | R.LYGSEAFATDFQDSAAA K.K | 0.70 | 0.78 |
| alpha-1-antichymotrypsin | P01011 (AACT_HUMAN) | R.NLAVSQVVHK.A | 0.81 | 0.79 |
| alpha-1B-glycoprotein | P04217 (A1BG_HUMAN) | R.CEGPIPDVTFELLR.E | 0.78 | 0.60 |
| alpha-1B-glycoprotein | P04217 (A1BG_HUMAN) | R.LHDNQNGWSGDSAPVEL ILSDETLPAPEFSPEPESGR. A | 0.72 | 0.66 |
| alpha-1B-glycoprotein | P04217 (A1BG_HUMAN) | R.CEGPIPDVTFELLR.E | 0.64 | 0.60 |
| alpha-1B-glycoprotein | P04217 (A1BG_HUMAN) | R.TPGAAANLELIFVGPQHA GNYR.C | 0.71 | 0.67 |
| alpha-1B-glycoprotein | P04217 (A1BG_HUMAN) | K.LLELTGPK.S | 0.70 | 0.66 |
| alpha-1B-glycoprotein | P04217 (A1BG_HUMAN) | R.ATWSGAVLAGR.D | 0.84 | 0.74 |
| alpha-2-antiplasmin | P08697 (A2AP_HUMAN) | K.HQM*DLVATLSQLGLQE LFQAPDLR.G | 0.67 | 0.67 |
| alpha-2-antiplasmin | P08697 (A2AP_HUMAN) | K.LGNQEPGGQTALK.S | 0.83 | 0.83 |
| alpha-2-antiplasmin | P08697 (A2AP_HUMAN) | K.GFPIKEDFLEQSEQLFGA KPVSLTGK.Q | 0.68 | 0.65 |
| alpha-2-HS-glycoprotein preproprotein | P02765 (FETUA_HUMAN ) | R.QPNCDDPETEEAALVAID YINQNLPWGYK.H | 0.61 | 0.61 |
| alpha-2-HS-glycoprotein preproprotein | P02765 (FETUA_HUMAN ) | K.VWPQQPSGELFEIEIDTL ETTCHVLDPTPVAR.C | 0.79 | 0.67 |
| alpha-2-HS-glycoprotein preproprotein | P02765 (FETUA_HUMAN ) | K.EHAVEGDCDFQLLK.L | 0.90 | 0.77 |
| alpha-2-HS-glycoprotein preproprotein | P02765 (FETUA_HUMAN ) | R.QPNCDDPETEEAALVAID YINQNLPWGYK.H | 0.63 | 0.61 |
| alpha-2-HS-glycoprotein preproprotein | P02765 (FETUA_HUMAN ) | K.HTLNQIDEVK.V | 0.70 | 0.68 |
| alpha-2-HS-glycoprotein preproprotein | P02765 (FETUA_HUMAN ) | R.TVVQPSVGAAAGPVVPP CPGR.I | 0.83 | 0.83 |
| angiotensinogen preproprotein | P01019 (ANGT_HUMAN) | K.TGCSLMGASVDSTLAFN TYVHFQGK.M | 0.75 | 0.67 |
| angiotensinogen preproprotein | P01019 (ANGT_HUMAN) | R.AAM*VGMLANFLGFR.I | 0.65 | 0.63 |
| angiotensinogen preproprotein | P01019 (ANGT_HUMAN) | R.AAMVGMLANFLGFR.I | 0.65 | 0.64 |
| angiotensinogen preproprotein | P01019 (ANGT_HUMAN) | R.AAM*VGM*LANFLGFR.I | 0.65 | 0.65 |
| angiotensinogen preproprotein | P01019 (ANGT_HUMAN) | R.AAMVGM*LANFLGFR.I | 0.65 | 0.74 |
| angiotensinogen preproprotein | P01019 (ANGT_HUMAN) | K.QPFVQGLALYTPVVLPR. S | 0.60 | 0.74 |
| angiotensinogen preproprotein | P01019 (ANGT_HUMAN) | R.AAM*VGMLANFLGFR.I | 0.64 | 0.63 |
| angiotensinogen preproprotein | P01019 (ANGT_HUMAN) | R.AAMVGMLANFLGFR.I | 0.64 | 0.64 |
| angiotensinogen preproprotein | P01019 (ANGT_HUMAN) | R.AAM*VGM*LANFLGFR.I | 0.64 | 0.65 |
| angiotensinogen preproprotein | P01019 (ANGT_HUMAN) | R.AAMVGM*LANFLGFR.I | 0.64 | 0.74 |
| angiotensinogen preproprotein | P01019 (ANGT_HUMAN) | K.VLSALQAVQGLLVAQGR .A | 0.74 | 0.77 |
| angiotensinogen preproprotein | P01019 (ANGT_HUMAN) | K.QPFVQGLALYTPVVLPR. S | 0.75 | 0.74 |
| angiotensinogen preproprotein | P01019 (ANGT_HUMAN) | R.ADSQAQLLLSTVVGVFT APGLHLK.Q | 0.78 | 0.77 |
| antithrombin-III | P01008 (ANT3_HUMAN) | R.ITDVIPSEAINELTVLVLV NTIYFK.G | 0.78 | 0.78 |
| antithrombin-III | P01008 (ANT3_HUMAN) | K.NDNDNIFLSPLSISTAFA MTK.L | 0.87 | 0.83 |
| antithrombin-III | P01008 (ANT3_HUMAN) | R.EVPLNTIIFMGR.V | 0.69 | 0.62 |
| antithrombin-III | P01008 (ANT3_HUMAN) | R.EVPLNTIIFM*GR.V | 0.69 | 0.69 |
| antithrombin-III | P01008 (ANT3_HUMAN) | R.VAEGTQVLELPFKGDDIT M*VLILPKPEK.S | 0.83 | 0.92 |
| antithrombin-III | P01008 (ANT3_HUMAN) | R.VAEGTQVLELPFKGDDIT MVLILPKPEK.S | 0.83 | 0.96 |
| antithrombin-III | P01008 (ANT3_HUMAN) | K.EQLQDMGLVDLFSPEK.S | 0.85 | 0.86 |
| antithrombin-III | P01008 (ANT3_HUMAN) | R.VAEGTQVLELPFKGDDIT M*VLILPKPEK.S | 0.94 | 0.92 |
| antithrombin-III | P01008 (ANT3_HUMAN) | R.VAEGTQVLELPFKGDDIT MVLILPKPEK.S | 0.94 | 0.96 |
| antithrombin-III | P01008 (ANT3_HUMAN) | R.EVPLNTIIFMGR.V | 0.63 | 0.62 |
| antithrombin-III | P01008 (ANT3_HUMAN) | R.EVPLNTIIFM*GR.V | 0.63 | 0.69 |
| antithrombin-III | P01008 (ANT3_HUMAN) | R.DIPMNPMCIYR.S | 0.71 | 0.70 |
| apolipoprotein | P02652 | K.EPCVESLVSQYFQTVTD | 0.83 | 0.83 |
| A-II preproprotein | (APOA2_HUMAN ) | YGK.D | | |
| apolipoprotein A-IV | P06727 (APOA4_HUMAN ) | K.SLAELGGHLDQQVEEFR. R | 0.67 | 0.67 |
| apolipoprotein A-IV | P06727 (APOA4_HUMAN ) | R.LAPLAEDVR.G | 0.67 | 0.90 |
| apolipoprotein A-IV | P06727 (APOA4_HUMAN ) | R.VLRENADSLQASLRPHA DELK.A | 0.79 | 0.63 |
| apolipoprotein A-IV | P06727 (APOA4_HUMAN ) | R.SLAPYAQDTQEKLNHQL EGLTFQMK.K | 0.90 | 0.65 |
| apolipoprotein A-IV | P06727 (APOA4_HUMAN ) | R.SLAPYAQDTQEKLNHQL EGLTFQM*K.K | 0.90 | 0.69 |
| apolipoprotein A-IV | P06727 (APOA4_HUMAN ) | K.LGPHAGDVEGHLSFLEK. D | 0.63 | 0.73 |
| apolipoprotein A-IV | P06727 (APOA4_HUMAN ) | K.SELTQQLNALFQDKLGE VNTYAGDLQK.K | 0.68 | 0.68 |
| apolipoprotein A-IV | P06727 (APOA4_HUMAN ) | R.SLAPYAQDTQEKLNHQL EGLTFQMK.K | 0.71 | 0.65 |
| apolipoprotein A-IV | P06727 (APOA4_HUMAN ) | R.SLAPYAQDTQEKLNHQL EGLTFQM*K.K | 0.71 | 0.69 |
| apolipoprotein A-IV | P06727 (APOA4_HUMAN ) | R.LLPHANEVSQK.I | 0.62 | 0.79 |
| apolipoprotein A-IV | P06727 (APOA4_HUMAN ) | K.SLAELGGHLDQQVEEFR R.R | 0.67 | 0.69 |
| apolipoprotein A-IV | P06727 (APOA4_HUMAN ) | K.SELTQQLNALFQDK.L | 0.68 | 0.62 |
| apolipoprotein B-100 | P04114 (APOB_HUMAN) | K.GFEPTLEALFGK.Q | 0.73 | 0.76 |
| apolipoprotein B-100 | P04114 (APOB_HUMAN) | K.ALYWVNGQVPDGVSK.V | 0.78 | 0.67 |
| apolipoprotein B-100 | P04114 (APOB_HUMAN) | K.FIIPSPK.R | 0.90 | 0.90 |
| apolipoprotein B-100 | P04114 (APOB_HUMAN) | R.TPALHFK.S | 0.68 | 0.81 |
| apolipoprotein B-100 | P04114 (APOB_HUMAN) | K.TEVIPPLIENR.Q | 0.62 | 0.64 |
| apolipoprotein B-100 | P04114 (APOB_HUMAN) | R.NLQNNAEWVYQGAIR.Q | 0.65 | 0.60 |
| apolipoprotein B-100 | P04114 (APOB_HUMAN) | K.LPQQANDYLNSFNWER. Q | 0.65 | 0.62 |
| apolipoprotein B-100 | P04114 (APOB_HUMAN) | R.LAAYLMLMR.S | 0.60 | 0.73 |
| apolipoprotein B-100 | P04114 (APOB_HUMAN) | R.VIGNMGQTMEQLTPELK. S | 0.68 | 0.67 |
| apolipoprotein B-100 | P04114 (APOB_HUMAN) | K.LIVAMSSWLQK.A | 0.74 | 0.86 |
| apolipoprotein B-100 | P04114 (APOB_HUMAN) | R.TSSFALNLPTLPEVK.F | 0.79 | 0.70 |
| apolipoprotein B-100 | P04114 (APOB_HUMAN) | K.IADFELPTIIVPEQTIEIPSI K.F | 0.62 | 0.61 |
| apolipoprotein B-100 | P04114 (APOB_HUMAN) | K.IEGNLIFDPNNYLPK.E | 0.63 | 0.62 |
| apolipoprotein B-100 | P04114 (APOB_HUMAN) | R.TSSFALNLPTLPEVKFPE VDVLTK.Y | 0.66 | 0.72 |
| apolipoprotein B-100 | P04114 (APOB_HUMAN) | R.LELELRPTGEIEQYSVSA TYELQR.E | 0.78 | 0.78 |
| apolipoprotein C-II | P02655 (APOC2_HUMAN ) | K.STAAMSTYTGIFTDQVLS VLK.G | 0.73 | 0.73 |
| apolipoprotein C-III | P02656 (APOC3_HUMAN ) | R.GWVTDGFSSLKDYWST VKDK.F | 1.00 | 1.00 |
| apolipoprotein E | P02649 (APOE_HUMAN) | R.WELALGR.F | 0.60 | 0.63 |
| apolipoprotein E | P02649 (APOE_HUMAN) | R.LAVYQAGAR.E | 0.61 | 0.64 |
| apolipoprotein E | P02649 (APOE_HUMAN) | K.SWFEPLVEDMQR.Q | 0.83 | 0.73 |
| apolipoprotein E | P02649 (APOE_HUMAN) | R.AATVGSLAGQPLQER.A | 0.67 | 0.67 |
| apolipoprotein(a) | P08519 (APOA_HUMAN) | R.TPEYYPNAGLIMNYCR.N | 0.72 | 0.61 |
| beta-2-glycoprotein 1 | P02749 (APOH_HUMAN) | K.TFYEPGEEITYSCKPGYV SR.G | 0.66 | 0.76 |
| beta-2-glycoprotein 1 | P02749 (APOH_HUMAN) | K.FICPLTGLWPINTLK.C | 0.72 | 0.70 |
| bone marrow proteoglycan | P13727 (PRG2_HUMAN) | R.SLQTFSQAWFTCR.R | 0.82 | 0.72 |
| ceruloplasmin | P00450 (CERU_HUMAN) | K.HYYIGIIETTWDYASDHG EKK.L | 0.78 | 0.89 |
| ceruloplasmin | P00450 (CERU_HUMAN) | R.EYTDASFTNRK.E | 0.63 | 0.63 |
| ceruloplasmin | P00450 (CERU_HUMAN) | K.M*YYSAVDPTKDIFTGLI GPMK.I | 0.66 | 0.68 |
| ceruloplasmin | P00450 (CERU_HUMAN) | K.M*YYSAVDPTKDIFTGLI GPM*K.I | 0.66 | 0.76 |
| ceruloplasmin | P00450 (CERU_HUMAN) | R.SGAGTEDSACIPWAYYS TVDQVKDLYSGLIGPLIVC R.R | 0.95 | 0.95 |
| ceruloplasmin | P00450 (CERU_HUMAN) | R.KAEEEHLGILGPQLHAD VGDKVK.I | 0.85 | 0.77 |
| ceruloplasmin | P00450 (CERU_HUMAN) | K.EVGPTNADPVCLAK.M | 0.62 | 0.77 |
| ceruloplasmin | P00450 (CERU_HUMAN) | R.MYSVNGYTFGSLPGLSM CAEDR.V | 0.63 | 0.71 |
| ceruloplasmin | P00450 (CERU_HUMAN) | K.DIASGLIGPLIICK.K | 0.63 | 0.66 |
| ceruloplasmin | P00450 (CERU_HUMAN) | R.QKDVDKEFYLFPTVFDE NESLLLEDNIR.M | 0.64 | 0.66 |
| ceruloplasmin | P00450 (CERU_HUMAN) | R.GPEEEHLGILGPVIWAEV GDTIR.V | 0.65 | 0.61 |
| ceruloplasmin | P00450 (CERU_HUMAN) | K.M*YYSAVDPTKDIFTGLI GPMK.I | 0.67 | 0.68 |
| ceruloplasmin | P00450 (CERU_HUMAN) | K.M*YYSAVDPTKDIFTGLI GPM*K.I | 0.67 | 0.76 |
| ceruloplasmin | P00450 (CERU_HUMAN) | K.M*YYSAVDPTKDIFTGLI GPMK.I | 0.67 | 0.68 |
| ceruloplasmin | P00450 (CERU_HUMAN) | K.M*YYSAVDPTKDIFTGLI GPM*K.I | 0.67 | 0.76 |
| ceruloplasmin | P00450 (CERU_HUMAN) | K.GAYPLSIEPIGVR.F | 0.67 | 0.63 |
| ceruloplasmin | P00450 (CERU_HUMAN) | R.GVYSSDVFDIFPGTYQTL EM*FPR.T | 0.67 | 0.67 |
| ceruloplasmin | P00450 (CERU_HUMAN) | K.DIASGLIGPLIICKK.D | 0.67 | 0.73 |
| ceruloplasmin | P00450 (CERU_HUMAN) | R.SGAGTEDSACIPWAYYS TVDQVK.D | 0.70 | 0.70 |
| ceruloplasmin | P00450 (CERU_HUMAN) | R.IYHSHIDAPK.D | 0.77 | 0.76 |
| ceruloplasmin | P00450 (CERU_HUMAN) | R.ADDKVYPGEQYTYMLL ATEEQSPGEGDGNCVTR.I | 0.77 | 0.80 |
| ceruloplasmin | P00450 (CERU_HUMAN) | K.DLYSGLIGPLIVCR.R | 0.78 | 0.82 |
| ceruloplasmin | P00450 (CERU_HUMAN) | R.TTIEKPVWLGFLGPIIK.A | 0.88 | 0.85 |
| cholinesterase | P06276 (CHLE_HUMAN) | K.IFFPGVSEFGK.E | 0.87 | 0.76 |
| cholinesterase | P06276 (CHLE_HUMAN) | R.AILQSGSFNAPWAVTSLY EAR.N | 1.00 | 0.83 |
| coagulation factor XII | P00748 (FA12_HUMAN) | R.LHEAFSPVSYQHDLALL R.L | 0.72 | 0.76 |
| coagulation factor XIII B chain | P05160 (F13B_HUMAN) | R.GDTYPAELYITGSILR.M | 0.67 | 0.83 |
| coagulation factor XIII B chain | P05160 (F13B_HUMAN) | K.VLHGDLIDFVCK.Q | 0.69 | 0.60 |
| complement C1r subcomponent | P00736 (C1R_HUMAN) | K.LVFQQFDLEPSEGCFYD YVK.I | 0.69 | 0.66 |
| complement C1s subcomponent | P09871 (C1S_HUMAN) | R.VKNYVDWIMK.T | 0.69 | 0.60 |
| complement C1s subcomponent | P09871 (C1S_HUMAN) | K.SNALDIIFQTDLTGQK.K | 0.75 | 0.70 |
| complement C2 | P06681 (CO2_HUMAN) | R.DFHINLFR.M | 0.75 | 0.72 |
| complement C2 | P06681 (CO2_HUMAN) | R.GALISDQWVLTAAHCFR. D | 0.60 | 0.75 |
| complement C2 | P06681 (CO2_HUMAN) | K.KNQGILEFYGDDIALLK. L | 0.62 | 0.67 |
| complement C3 | P01024 (CO3_HUMAN) | R.IHWESASLLR.S | 0.80 | 0.77 |
| complement C4-B-like preproprotein | P0C0L5 (CO4B_HUMAN) | R.VHYTVCIWR.N | 0.67 | 0.65 |
| complement C4-B-like preproprotein | P0C0L5 (CO4B_HUMAN) | K.AEMADQAAAWLTR.Q | 0.78 | 0.89 |
| complement C4-B-like preproprotein | P0C0L5 (CO4B_HUMAN) | K.M*RPSTDTITVMVENSH GLR.V | 0.65 | 0.65 |
| complement C4-B-like preproprotein | P0C0L5 (CO4B_HUMAN) | K.MRPSTDTITVMVENSHG LR.V | 0.65 | 0.72 |
| complement C4-B-like preproprotein | P0C0L5 (CO4B_HUMAN) | R.VQQPDCREPFLSCCQFAE SLRK.K | 0.67 | 0.60 |
| complement C4-B-like preproprotein | P0C0L5 (CO4B_HUMAN) | K.LVNGQSHISLSK.A | 0.73 | 0.73 |
| complement C4-B-like preproprotein | P0C0L5 (CO4B_HUMAN) | R.GQIVFMNREPK.R | 0.80 | 0.62 |
| complement C4-B-like preproprotein | P0C0L5 (CO4B_HUMAN) | K.VGLSGM*AIADVTLLSGF HALR.A | 0.80 | 0.80 |
| complement C4- | P0C0L5 | K.VGLSGMAIADVTLLSGF | 0.80 | 0.83 |
| B-like preproprotein | (CO4B_HUMAN) | HALR.A | | |
| complement C4-B-like preproprotein | P0C0L5 (CO4B_HUMAN) | R.GHLFLQTDQPIYNPGQR. V | 0.70 | 0.68 |
| complement C4-B-like preproprotein | P0C0L5 (CO4B_HUMAN) | K.M*RPSTDTITVMVENSH GLR.V | 0.75 | 0.65 |
| complement C4-B-like preproprotein | P0C0L5 (CO4B_HUMAN) | K.MRPSTDTITVMVENSHG LR.V | 0.75 | 0.72 |
| complement C4-B-like preproprotein | P0C0L5 (CO4B_HUMAN) | K.SHALQLNNR.Q | 0.76 | 0.70 |
| complement C4-B-like preproprotein | P0C0L5 (CO4B_HUMAN) | R.YVSHFETEGPHVLLYFDS VPTSR.E | 0.88 | 0.89 |
| complement C4-B-like preproprotein | P0C0L5 (CO4B_HUMAN) | R.GSSTWLTAFVLK.V | 0.61 | 0.72 |
| complement C4-B-like preproprotein | P0C0L5 (CO4B_HUMAN) | R.YIYGKPVQGVAYVR.F | 0.63 | 0.73 |
| complement C4-B-like preproprotein | P0C0L5 (CO4B_HUMAN) | K.SCGLHQLLR.G | 0.65 | 0.65 |
| complement C4-B-like preproprotein | P0C0L5 (CO4B_HUMAN) | R.GPEVQLVAHSPWLK.D | 0.69 | 0.73 |
| complement C4-B-like preproprotein | P0C0L5 (CO4B_HUMAN) | R.KKEVYM*PSSIFQDDFVI PDISEPGTWK.I | 0.70 | 0.67 |
| complement C4-B-like preproprotein | P0C0L5 (CO4B_HUMAN) | R.KKEVYMPSSIFQDDFVIP DISEPGTWK.I | 0.70 | 0.69 |
| complement C4-B-like preproprotein | P0C0L5 (CO4B_HUMAN) | R.VQQPDCREPFLSCCQFAE SLR.K | 0.76 | 0.74 |
| complement C4-B-like preproprotein | P0C0L5 (CO4B_HUMAN) | K.VGLSGM*AIADVTLLSGF HALR.A | 0.80 | 0.80 |
| complement C4-B-like preproprotein | P0C0L5 (CO4B_HUMAN) | K.VGLSGMAIADVTLLSGF HALR.A | 0.80 | 0.83 |
| complement C4-B-like preproprotein | P0C0L5 (CO4B_HUMAN) | K.ASAGLLGAHAAAITAYA LTLTK.A | 0.85 | 0.83 |

| Protein description | Uniprot ID (name) | Peptide | XT AUC | S AUC |
|---|---|---|---|---|
| complement C5 preproprotein | P01031 (CO5 HUMAN) | K.ITHYNYLILSK.G | 0.73 | 0.73 |
| complement C5 preproprotein | P01031 (COS HUMAN) | R.KAFDICPLVK.I | 0.83 | 0.87 |
| complement C5 preproprotein | P01031 (COS HUMAN) | R.IPLDLVPK.T | 0.90 | 0.63 |
| complement C5 preproprotein | P01031 (COS HUMAN) | R.MVETTAYALLTSLNLKD INYVNPVIK.W | 0.92 | 0.75 |
| complement C5 preproprotein | P01031 (COS HUMAN) | K.ALLVGEHLNIIVTPK.S | 1.00 | 0.87 |
| complement C5 preproprotein | P01031 (COS HUMAN) | K.LKEGMLSIMSYR.N | 0.62 | 0.75 |
| complement C5 preproprotein | P01031 (COS HUMAN) | R.YIYPLDSLTWIEYWPR.D | 0.70 | 0.69 |
| complement C5 preproprotein | P01031 (COS HUMAN) | K.GGSASTWLTAFALR.V | 0.63 | 0.83 |
| complement C5 preproprotein | P01031 (COS HUMAN) | R.YGGGFYSTQDTINAIEGL TEYSLLVK.Q | 0.73 | 0.74 |
| complement component C6 | P13671 (CO6_HUMAN) | K.AKDLHLSDVFLK.A | 0.63 | 0.62 |
| complement component C6 | P13671 (CO6_HUMAN) | K.ALNHLPLEYNSALYSR.I | 0.60 | 0.62 |
| complement component C7 | P10643 (CO7_HUMAN) | R.LSGNVLSYTFQVK.I | 0.71 | 0.63 |
| complement component C8 alpha chain | P07357 (CO8A_HUMAN) | R.KDDIMLDEGMLQSLMEL PDQYNYGMYAK.F | 0.78 | 0.89 |
| complement component C8 beta chain preproprotein | P07358 (CO8B_HUMAN) | R.DFGTHYITEAVLGGIYEY TLVMNK.E | 0.80 | 0.73 |
| complement component C8 beta chain preproprotein | P07358 (CO8B_HUMAN) | R.DTMVEDLVVLVR.G | 0.88 | 0.76 |
| complement component C8 beta chain preproprotein | P07358 (CO8B_HUMAN) | R.YYAGGCSPHYILNTR.F | 0.70 | 0.71 |
| complement component C8 gamma chain | P07360 (CO8G_HUMAN) | R.SLPVSDSVLSGFEQR.V | 0.79 | 0.81 |
| complement component C8 gamma chain | P07360 (CO8G_HUMAN) | R.VQEAHLTEDQIFYFPK.Y | 0.98 | 0.84 |
| complement component C9 | P02748 (CO9_HUMAN) | R.TAGYGINILGMDPLSTPF DNEFYNGLCNR.D | 0.62 | 0.64 |
| complement component C9 | P02748 (CO9_HUMAN) | R.RPWNVASLIYETK.G | 0.60 | 0.74 |
| complement component C9 | P02748 (CO9_HUMAN) | R.AIEDYINEFSVRK.C | 0.67 | 0.67 |
| complement component C9 | P02748 (CO9_HUMAN) | R.AIEDYINEFSVR.K | 0.77 | 0.79 |
| complement factor B preproprotein | P00751 (CFAB_HUMAN) | R.LEDSVTYHCSR.G | 0.60 | 0.60 |
| complement factor B preproprotein | P00751 (CFAB_HUMAN) | R.FIQVGVISWGVVDVCK.N | 0.67 | 0.79 |
| complement factor B preproprotein | P00751 (CFAB_HUMAN) | R.DFHINLFQVLPWLK.E | 0.78 | 0.76 |
| complement factor B preproprotein | P00751 (CFAB_HUMAN) | K.YGQTIRPICLPCTEGTTR. A | 0.60 | 0.70 |
| complement factor B preproprotein | P00751 (CFAB_HUMAN) | R.LLQEGQALEYVCPSGFY PYPVQTR.T | 0.74 | 0.74 |
| complement factor H | P08603 (CFAH_HUMAN) | R.RPYFPVAVGK.Y | 0.67 | 0.70 |
| complement factor H | P08603 (CFAH_HUMAN) | K.CTSTGWIPAPR.C | 0.70 | 0.66 |
| complement factor H | P08603 (CFAH_HUMAN) | K.CLHPCVISR.E | 0.94 | 0.64 |
| complement factor H | P08603 (CFAH_HUMAN) | R.EIMENYNIALR.W | 0.67 | 0.71 |
| complement factor H | P08603 (CFAH_HUMAN) | K.CLHPCVISR.E | 0.75 | 0.64 |
| complement factor H | P08603 (CFAH_HUMAN) | K.AVYTCNEGYQLLGEINY R.E | 0.73 | 0.62 |
| complement factor H | P08603 (CFAH_HUMAN) | R.SITCIHGVWTQLPQCVAI DK.L | 0.61 | 0.61 |
| complement factor H | P08603 (CFAH_HUMAN) | R.WQSIPLCVEK.I | 0.65 | 0.65 |
| complement factor H | P08603 (CFAH_HUMAN) | K.TDCLSLPSFENAIPMGEK. K | 0.74 | 0.77 |
| complement factor H | P08603 (CFAH_HUMAN) | K.CFEGFGIDGPAIAK.C | 0.76 | 0.69 |
| complement factor H | P08603 (CFAH_HUMAN) | K.CFEGFGIDGPAIAK.C | 0.83 | 0.69 |
| complement factor H | P08603 (CFAH_HUMAN) | K.IDVHLVPDR.K | 0.61 | 0.67 |
| complement factor H | P08603 (CFAH_HUMAN) | K.SSNLIILEEHLK.N | 0.77 | 0.69 |

| Protein description | Uniprot ID (name) | Peptide | XT_AUC | S_AUC |
|---|---|---|---|---|
| complement factor I preproprotein | P05156 (CFAI_HUMAN) | R.AQLGDLPWQVAIK.D | 0.66 | 0.69 |
| complement factor I preproprotein | P05156 (CFAI_HUMAN) | R.VFSLQWGEVK.L | 0.69 | 0.77 |
| corticosteroid-binding globulin | P08185 (CBG_HUMAN) | R.WSAGLTSSQVDLYIPK.V | 0.63 | 0.61 |
| fibrinogen alpha chain | P02671 (FIBA_HUMAN) | K.TFPGFFSPMLGEFVSETE SR.G | 0.80 | 0.78 |
| gelsolin | P06396 (GELS_HUMAN) | R.IEGSNKVPVDPATYGQF YGGDSYIILYNYR.H | 0.78 | 0.78 |
| gelsolin | P06396 (GELS_HUMAN) | R.AQPVQVAEGSEPDGFWE ALGGK.A | 0.62 | 0.65 |
| gelsolin | P06396 (GELS_HUMAN) | K.TPSAAYLWVGTGASEAE KTGAQELLR.V | 0.78 | 0.78 |
| gelsolin | P06396 (GELS_HUMAN) | R.VEKFDLVPVPTNLYGDF FTGDAYVILK.T | 0.61 | 0.63 |
| gelsolin | P06396 (GELS_HUMAN) | R.EVQGFESATFLGYFK.S | 0.87 | 0.88 |
| gelsolin | P06396 (GELS_HUMAN) | K.NWRDPDQTDGLGLSYLS SHIANVER.V | 0.89 | 0.89 |
| gelsolin | P06396 (GELS_HUMAN) | K.TPSAAYLWVGTGASEAE K.T | 0.87 | 0.77 |
| glutathione peroxidase 3 | P22352 (GPX3_HUMAN) | K.FLVGPDGIPIMR.W | 0.85 | 0.77 |
| hemopexin | P02790 (HEMO_HUMAN) | R.LEKEVGTPHGIILDSVDA AFICPGSSR.L | 0.93 | 0.74 |
| hemopexin | P02790 (HEMO_HUMAN) | R.WKNFPSPVDAAFR.Q | 0.64 | 0.82 |
| hemopexin | P02790 (HEMO_HUMAN) | R.GECQAEGVLFFQGDREW FWDLATGTMK.E | 0.60 | 0.64 |
| hemopexin | P02790 (HEMO_HUMAN) | R.GECQAEGVLFFQGDREW FWDLATGTM*K.E | 0.60 | 0.83 |
| hemopexin | P02790 (HEMO_HUMAN) | R.GECQAEGVLFFQGDREW FWDLATGTMK.E | 0.93 | 0.64 |
| hemopexin | P02790 (HEMO_HUMAN) | R.GECQAEGVLFFQGDREW FWDLATGTM*K.E | 0.93 | 0.83 |
| hemopexin | P02790 (HEMO_HUMAN) | K.EVGTPHGIILDSVDAAFI CPGSSR.L | 0.62 | 0.69 |
| hemopexin | P02790 (HEMO_HUMAN) | R.LWWLDLK.S | 0.64 | 0.64 |
| hemopexin | P02790 (HEMO_HUMAN) | K.NFPSPVDAAFR.Q | 0.65 | 0.72 |
| hemopexin | P02790 (HEMO_HUMAN) | R.EWFWDLATGTMK.E | 0.68 | 0.65 |
| hemopexin | P02790 (HEMO_HUMAN) | K.GGYTLVSGYPK.R | 0.69 | 0.65 |
| hemopexin | P02790 (HEMO_HUMAN) | K.LYLVQGTQVYVFLTK. G | 0.69 | 0.76 |
| heparin cofactor 2 | P05546 (HEP2_HUMAN) | R.EYYFAEAQIADFSDPAFI SK.T | 0.80 | 0.78 |
| heparin cofactor 2 | P05546 (HEP2_HUMAN) | K.QFPILLDFK.T | 0.62 | 1.00 |
| heparin cofactor 2 | P05546 (HEP2_HUMAN) | K.QFPILLDFK.T | 0.64 | 1.00 |
| heparin cofactor 2 | P05546 (HEP2_HUMAN) | K.FAFNLYR.V | 0.70 | 0.60 |
| histidine-rich glycoprotein | P04196 (HRG_HUMAN) | R.DGYLFQLLR.I | 0.65 | 0.65 |
| insulin-like growth factor-binding protein complex acid labile subunit | P35858 (ALS_HUMAN) | R.SFEGLGQLEVLTLDHNQ LQEVK.A | 0.75 | 0.83 |
| insulin-like growth factor-binding protein complex acid labile subunit | P35858 (ALS_HUMAN) | R.TFTPQPPGLER.L | 0.75 | 0.60 |
| insulin-like growth factor-binding protein complex acid labile subunit | P35858 (ALS_HUMAN) | R.AFWLDVSHNR.L | 0.77 | 0.75 |
| insulin-like growth factor-binding protein complex acid labile subunit | P35858 (ALS_HUMAN) | R.LAELPADALGPLQR.A | 0.66 | 0.64 |
| insulin-like growth factor-binding protein complex acid labile subunit | P35858 (ALS_HUMAN) | R.LEALPNSLLAPLGR.L | 0.70 | 0.67 |
| insulin-like growth factor-binding protein complex acid labile subunit | P35858 (ALS_HUMAN) | R.NLIAAVAPGAFLGLK.A | 0.70 | 0.68 |
| inter-alpha-trypsin inhibitor heavy chain H1 | P19827 (ITIH1_HUMAN) | R.QAVDTAVDGVFIR.S | 0.60 | 0.64 |
| inter-alpha- | P19827 | K.TAFISDFAVTADGNAFIG | 0.81 | 0.86 |
| trypsin inhibitor heavy chain H1 | (ITIH1_HUMAN) | DIK.D | | |
| inter-alpha-trypsin inhibitor heavy chain H1 | P19827 (ITIH1_HUMAN) | R.GHMLENHVER.L | 0.63 | 0.61 |
| inter-alpha-trypsin inhibitor heavy chain H1 | P19827 (ITIH1_HUMAN) | R.GHM*LENHVER.L | 0.63 | 0.70 |
| inter-alpha-trypsin inhibitor heavy chain H1 | P19827 (ITIH1_HUMAN) | K.TAFISDFAVTADGNAFIG DIKDKVTAWK.Q | 0.75 | 0.60 |
| inter-alpha-trypsin inhibitor heavy chain H1 | P19827 (ITIH1_HUMAN) | R.GIEILNQVQESLPELSNH ASILIMLTDGDPTEGVTDR. S | 0.80 | 0.80 |
| inter-alpha-trypsin inhibitor heavy chain H1 | P19827 (ITIH1_HUMAN) | K.ILGDM*QPGDYFDLVLF GTR.V | 0.85 | 0.79 |
| inter-alpha-trypsin inhibitor heavy chain H1 | P19827 (ITIH1_HUMAN) | K.LDAQASFLPK.E | 0.88 | 0.75 |
| inter-alpha-trypsin inhibitor heavy chain H1 | P19827 (ITIH1_HUMAN) | R.GFSLDEATNLNGGLLR.G | 0.80 | 0.80 |
| inter-alpha-trypsin inhibitor heavy chain H1 | P19827 (ITIH1_HUMAN) | K.TAFISDFAVTADGNAFIG DIKDK.V | 0.93 | 0.96 |
| inter-alpha-trypsin inhibitor heavy chain H1 | P19827 (ITIH1_HUMAN) | K.GSLVQASEANLQAAQDF VR.G | 0.60 | 0.65 |
| inter-alpha-trypsin inhibitor heavy chain H1 | P19827 (ITIH1_HUMAN) | R.GHMLENHVER.L | 0.64 | 0.61 |
| inter-alpha-trypsin inhibitor heavy chain H1 | P19827 (ITIH1_HUMAN) | R.GHM*LENHVER.L | 0.64 | 0.70 |
| inter-alpha-trypsin inhibitor heavy chain H1 | P19827 (ITIH1_HUMAN) | R.LWAYLTIQELLAK.R | 0.72 | 0.74 |
| inter-alpha-trypsin inhibitor heavy chain H1 | P19827 (ITIH1_HUMAN) | R.EVAFDLEIPK.T | 0.78 | 0.62 |
| inter-alpha-trypsin inhibitor heavy chain H2 | P19823 (ITIH2_HUMAN) | R.SILQMSLDHHIVTPLTSL VIENEAGDER.M | 0.76 | 0.76 |
| inter-alpha-trypsin inhibitor heavy chain H2 | P19823 (ITIH2_HUMAN) | R.SILQM*SLDHHIVTPLTSL VIENEAGDER.M | 0.76 | 0.80 |
| inter-alpha-trypsin inhibitor heavy chain H2 | P19823 (ITIH2_HUMAN) | R.SILQMSLDHHIVTPLTSL VIENEAGDER.M | 0.77 | 0.76 |
| inter-alpha-trypsin inhibitor heavy chain H2 | P19823 (ITIH2_HUMAN) | R.SILQM*SLDHHIVTPLTSL VIENEAGDER.M | 0.77 | 0.80 |
| inter-alpha-trypsin inhibitor heavy chain H2 | P19823 (ITIH2_HUMAN) | K.AGELEVFNGYFVHFFAP DNLDPIPK.N | 0.79 | 0.76 |
| inter-alpha-trypsin inhibitor heavy chain H2 | P19823 (ITIH2_HUMAN) | R.ETAVDGELVVLYDVK.R | 0.94 | 0.97 |
| inter-alpha-trypsin inhibitor heavy chain H2 | P19823 (ITIH2_HUMAN) | R.NVQFNYPHTSVTDVTQN NFHNYFGGSEIVVAGK.F | 0.74 | 0.83 |
| inter-alpha-trypsin inhibitor heavy chain H2 | P19823 (ITIH2_HUMAN) | R.FLHVPDTFEGHFDGVPVI SK.G | 0.81 | 0.81 |
| inter-alpha-trypsin inhibitor heavy chain H4 | Q14624 (ITIH4_UMAN) | K.YIFHNFM*ER.L | 0.70 | 0.73 |
| inter-alpha-trypsin inhibitor heavy chain H4 | Q14624 (ITIH4_HUMAN) | R.SFAAGIQALGGTNINDA MLMAVQLLDSSNQEER.L | 0.75 | 0.75 |
| inter-alpha-trypsin inhibitor heavy chain H4 | Q14624 (ITIH4_HUMAN) | R.NMEQFQVSVSVAPNAK.I | 1.00 | 1.00 |
| inter-alpha-trypsin inhibitor heavy chain H4 | Q14624 (ITIH4_UMAN) | R.VQGNDHSATR.E | 0.85 | 0.86 |
| inter-alpha-trypsin inhibitor heavy chain H4 | Q14624 (ITIH4_UMAN) | K.WKETLFSVMPGLK.M | 0.66 | 0.69 |
| inter-alpha-trypsin inhibitor heavy chain H4 | Q14624 (ITIH4_UMAN) | K.AGFSWIEVTFK.N | 0.78 | 0.82 |
| inter-alpha-trypsin inhibitor heavy chain H4 | Q14624 (ITIH4_HUMAN) | R.DQFNLIVFSTEATQWRPS LVPASAENVNK.A | 0.61 | 0.60 |
| inter-alpha-trypsin inhibitor heavy chain H4 | Q14624 (ITIH4_UMAN) | R.LWAYLTIQQLLEQTVSA SDADQQALR.N | 0.66 | 0.66 |
| kallistatin | P29622 (KAIN HUMAN) | K.FSISGSYVLDQILPR.L | 0.79 | 0.72 |
| kininogen-1 | P01042 (KNG1 HUMAN) | K.AATGECTATVGKR.S | 0.76 | 0.60 |
| kininogen-1 | P01042 (KNG1_HUMAN) | K.ENFLFLTPDCK.S | 0.71 | 0.68 |
| kininogen-1 | P01042 (KNG1_HUMAN) | R.DIPTNSPELEETLTHTITK. L | 0.65 | 0.64 |
| kininogen-1 | P01042 (KNG1_HUMAN) | K.IYPTVNCQPLGM*ISLMK .R | 0.66 | 0.60 |
| kininogen-1 | P01042 (KNG1_HUMAN) | K.IYPTVNCQPLGMISLMK. R | 0.66 | 0.62 |
| kininogen-1 | P01042 (KNG1_HUMAN) | K.IYPTVNCQPLGMISLM*K .R | 0.66 | 0.63 |
| kininogen-1 | P01042 (KNG1_HUMAN) | R.IGEIKEETTSHLR.S | 0.67 | 0.70 |
| kininogen-1 | P01042 (KNG1_HUMAN) | K.YNSQNQSNNQFVLYR.I | 0.76 | 0.65 |
| kininogen-1 | P01042 (KNG1_HUMAN) | K.TVGSDTFYSFK.Y | 0.78 | 0.77 |
| leucine-rich alpha-2-glycoprotein | P02750 (A2GL_HUMAN) | R.DGFDISGNPWICDQNLSD LYR.W | 0.73 | 0.73 |
| leucine-rich alpha-2-glycoprotein | P02750 (A2GL_HUMAN) | R.NALTGLPPGLFQASATLD TLVLK.E | 0.79 | 0.79 |
| leucine-rich alpha-2-glycoprotein | P02750 (A2GL_HUMAN) | K.ALGHLDLSGNR.L | 0.71 | 0.71 |
| leucine-rich alpha-2-glycoprotein | P02750 (A2GL_HUMAN) | R.VAAGAFQGLR.Q | 0.71 | 0.77 |
| lipopolysacchari de-binding protein | P18428 (LBP_HUMAN) | R.SPVTLLAAVMSLPEEHN K.M | 0.65 | 0.61 |
| lumican | P51884 (LUM HUMAN) | K.SLEYLDLSFNQIAR.L | 0.93 | 0.96 |
| monocyte differentiation antigen CD 14 | P08571 (CD 14 HUMAN) | R.LTVGAAQVPAQLLVGAL R.V | 0.68 | 0.63 |
| N-acetylmuramoyl-L-alanine amidase | Q96PD5 (PGRP2_HUMAN) | R.EGKEYGVVLAPDGSTVA VEPLLAGLEAGLQGR.R | 0.64 | 0.64 |
| N-acetylmuramoyl-L-alanine amidase | Q96PD5 (PGRP2_HUMAN) | K.EFTEAFLGCPAIHPR. C | 0.63 | 0.62 |
| N-acetylmuramoyl-L-alanine amidase | Q96PD5 (PGRP2_HUMAN) | R.TDCPGDALFDLLR.T | 0.88 | 0.86 |
| phosphatidylinos itol-glycan-specific phospholipase D | P80108 (PHLD_HUMAN) | K.VAFLTVTLHQGGATR.M | 0.63 | 0.65 |
| pigment epithelium-derived factor | P36955 (PEDF_HUMAN) | R.ALYYDLISSPDIHGTYKE LLDTVTAPQK.N | 0.69 | 0.65 |
| pigment epithelium-derived factor | P36955 (PEDF_HUMAN) | K.TVQAVLTVPK.L | 0.72 | 0.62 |
| pigment epithelium-derived factor | P36955 (PEDF_HUMAN) | R.LDLQEINNWVQAQMK.G | 0.67 | 0.68 |
| plasma kallikrein preproprotein | P03952 (KLKB1_HUMAN ) | R.LVGITSWGEGCAR.R | 1.00 | 0.67 |
| plasma protease C1 inhibitor | P05155 (IC1_HUMAN) | K.TNLESILSYPKDFTCVHQ ALK.G | 0.83 | 0.83 |
| plasma protease C1 inhibitor | P05155 (IC1_HUMAN) | R.LVLLNAIYLSAK.W | 0.64 | 0.61 |
| plasma protease C1 inhibitor | P05155 (IC1_HUMAN) | K.FQPTLLTLPR.I | 0.86 | 0.77 |
| plasminogen | P00747 (PLMN HUMAN) | R.HSIFTPETNPR.A | 0.66 | 0.64 |
| plasminogen | P00747 (PLMN HUMAN) | R.FVTWIEGVMR.N | 0.65 | 0.74 |
| PREDICTED: complement C4-A | P0C0L4 (CO4A_HUMAN) | R.GQIVFMNR.E | 0.75 | 0.61 |
| PREDICTED: complement C4-A | P0C0L4 (CO4A_HUMAN) | R.DSSTWLTAFVLK.V | 0.65 | 0.67 |
| PREDICTED: complement C4-A | P0C0L4 (CO4A_HUMAN) | R.YLDKTEQWSTLPPETK.D | 0.70 | 0.60 |
| PREDICTED: complement C4-A | P0C0L4 (CO4A_HUMAN) | R.DFALLSLQVPLK.D | 0.78 | 0.62 |
| PREDICTED: complement C4-A | P0C0L4 (CO4A_HUMAN) | R.TLEIPGNSDPNMIPDGDF NSYVR.V | 0.74 | 0.78 |
| PREDICTED: complement C4-A | P0C0L4 (CO4A_HUMAN) | R.EMSGSPASGIPVK.V | 0.88 | 0.88 |
| PREDICTED: complement C4-A | P0C0L4 (CO4A_HUMAN) | K.LHLETDSLALVALGALD TALYAAGSK.S | 0.68 | 0.64 |
| PREDICTED: complement C4-A | P0C0L4 (CO4A_HUMAN) | R.GCGEQTMIYLAPTLAAS R.Y | 0.71 | 0.67 |
| pregnancy zone protein | P20742 (PZP_HUMAN) | R.NELIPLIYLENPR.R | 1.00 | 0.67 |
| pregnancy zone protein | P20742 (PZP_HUMAN) | K.LEAGINQLSFPLSSEPIQG SYR.V | 1.00 | 0.73 |
| pregnancy zone protein | P20742 (PZP_HUMAN) | R.NQGNTWLTAFVLK.T | 0.73 | 0.78 |
| pregnancy zone protein | P20742 (PZP_HUMAN) | R.AFQPFFVELTMPYSVIR.G | 0.83 | 0.88 |
| pregnancy zone protein | P20742 (PZP_HUMAN) | R.IQHPFTVEEFVLPK.F | 0.65 | 0.79 |
| pregnancy zone protein | P20742 (PZP_HUMAN) | K.ALLAYAFSLLGK.Q | 0.69 | 0.74 |
| pregnancy-specific beta-1-glycoprotein 1 /8/4 | P11464 (PSG1_HUMAN)/ Q9UQ74 (PSG8_HUMAN)/ Q00888 (PSG4_HUMAN) | R.TLFLLGVTK.Y | 0.74 | 0.83 |
| protein AMBP preproprotein | P02760 (AMBP_HUMAN) | R.TVAACNLPIVR.G | 0.78 | 0.77 |
| protein AMBP preproprotein | P02760 (AMBP_HUMAN) | K.WYNLAIGSTCPWLK.K | 0.80 | 0.80 |
| protein Z-dependent protease inhibitor | Q9UK55 (ZPI_HUMAN) | K.LILVDYILFK.G | 0.69 | 0.62 |
| prothrombin preproprotein | P00734 (THRB_HUMAN) | R.KSPQELLCGASLISDR.W | 0.63 | 0.65 |
| prothrombin preproprotein | P00734 (THRB_HUMAN) | R.TATSEYQTFFNPR.T | 0.79 | 0.61 |
| prothrombin preproprotein | P00734 (THRB_HUMAN) | R.VTGWGNLKETWTANVG K.G | 1.00 | 0.71 |
| prothrombin preproprotein | P00734 (THRB_HUMAN) | R.IVEGSDAEIGMSPWQVM LFR.K | 0.65 | 0.61 |
| prothrombin preproprotein | P00734 (THRB_HUMAN) | K.HQDFNSAVQLVENFCR. N | 0.65 | 0.64 |
| prothrombin preproprotein | P00734 (THRB_HUMAN) | R.IVEGSDAEIGM*SPWQV MLFR.K | 0.65 | 0.80 |
| prothrombin preproprotein | P00734 (THRB_HUMAN) | R.IVEGSDAEIGMSPWQVM *LFR.K | 0.65 | 1.00 |
| prothrombin preproprotein | P00734 (THRB_HUMAN) | R.RQECSIPVCGQDQVTVA MTPR.S | 0.74 | 0.73 |
| prothrombin preproprotein | P00734 (THRB HUMAN) | R.LAVTTHGLPCLAWASAQ AK.A | 0.76 | 0.80 |
| prothrombin preproprotein | P00734 (THRB_HUMAN) | K.GQPSVLQVVNLPIVERPV CK.D | 0.76 | 0.67 |
| retinol-binding protein 4 | P02753 (RET4_HUMAN) | R.LLNLDGTCADSYSFVFSR .D | 0.70 | 0.66 |
| sex hormone-binding globulin | P04278 (SHBG_HUMAN) | R.LFLGALPGEDSSTSFCLN GLWAQGQR.L | 0.72 | 0.72 |
| sex hormone-binding globulin | P04278 (SHBG_HUMAN) | R.TWDPEGVIFYGDTNPKD DWFMLGLR.D | 0.75 | 0.76 |
| sex hormone-binding globulin | P04278 (SHBG_HUMAN) | R.IALGGLLFPASNLR.L | 0.62 | 0.72 |
| sex hormone-binding globulin | P04278 (SHBG_HUMAN) | K.VVLSSGSGPGLDLPLVLG LPLQLK.L | 0.65 | 0.68 |
| thyroxine-binding globulin | P05543 (THBG_HUMAN) | K.AVLHIGEK.G | 0.64 | 0.75 |
| thyroxine-binding globulin | P05543 (THBG_HUMAN) | K.GWVDLFVPK.F | 0.60 | 0.61 |
| thyroxine-binding globulin | P05543 (THBG_HUMAN) | K.FSISATYDLGATLLK.M | 0.62 | 0.64 |
| thyroxine-binding globulin | P05543 (THBG_HUMAN) | R.SILFLGK.V | 0.66 | 0.63 |
| transforming growth factor-beta-induced protein ig-h3 | Q15582 (BGH3_HUMAN) | R.LTLLAPLNSVFK.D | 0.78 | 0.65 |
| vitamin D-binding protein | P02774 (VTDB_HUMAN) | K.EYANQFMWEYSTNYGQ APLSLLVSYTK.S | 0.67 | 0.64 |
| vitamin D-binding protein | P02774 (VTDB_HUMAN) | K.EYANQFM*WEYSTNYG QAPLSLLVSYTK.S | 0.67 | 0.67 |
| vitamin D-binding protein | P02774 (VTDB_HUMAN) | K.ELPEHTVK.L | 0.79 | 0.74 |
| vitamin D-binding protein | P02774 (VTDB_HUMAN) | R.RTHLPEVFLSK.V | 0.63 | 0.76 |
| vitamin D-binding protein | P02774 (VTDB_HUMAN) | K.TAMDVFVCTYFMPAAQ LPELPDVELPTNK.D | 0.66 | 0.63 |
| vitamin D-binding protein | P02774 (VTDB_HUMAN) | K.LPDATPTELAK.L | 0.67 | 0.73 |
| vitamin D-binding protein | P02774 (VTDB_HUMAN) | K.EYANQFMWEYSTNYGQ APLSLLVSYTK.S | 0.65 | 0.64 |
| vitamin D-binding protein | P02774 (VTDB_HUMAN) | K.EYANQFM*WEYSTNYG QAPLSLLVSYTK.S | 0.65 | 0.67 |
| vitamin D-binding protein | P02774 (VTDB_HUMAN) | K.ELSSFIDKGQELCADYSE NTFTEYKK.K | 0.71 | 0.73 |
| vitamin D-binding protein | P02774 (VTDB_HUMAN) | K.EDFTSLSLVLYSR.K | 0.71 | 0.75 |
| vitamin D-binding protein | P02774 (VTDB_HUMAN) | K.HQPQEFPTYVEPTNDEIC EAFRK.D | 0.77 | 0.75 |
| vitamin D-binding protein | P02774 (VTDB_HUMAN) | K.HQPQEFPTYVEPTNDEIC EAFR.K | 0.60 | 0.67 |
| vitamin D-binding protein | P02774 (VTDB_HUMAN) | R.KFPSGTFEQVSQLVK.E | 0.62 | 0.61 |
| vitamin D-binding protein | P02774 (VTDB_HUMAN) | K.ELSSFIDKGQELCADYSE NTFTEYK.K | 0.64 | 0.64 |
| vitamin D-binding protein | P02774 (VTDB_HUMAN) | K.EFSHLGKEDFTSLSLVLY SR.K | 0.66 | 0.64 |
| vitamin D-binding protein | P02774 (VTDB_HUMAN) | K.SYLSMVGSCCTSASPTV CFLK.E | 0.68 | 0.77 |
| vitronectin | P04004 (VTNC_HUMAN) | R.IYISGMAPRPSLAK.K | 0.63 | 0.66 |
| vitronectin | P04004 (VTNC_HUMAN) | R.IYISGMAPRPSLAK.K | 0.64 | 0.66 |
| vitronectin | P04004 (VTNC_HUMAN) | K.LIRDVWGIEGPIDAAFTR. I | 0.81 | 0.75 |
| von Willebrand factor preproprotein | P04275 (VWF_HUMAN) | R.IGWPNAPILIQDFETLPR. E | 0.67 | 0.67 |

| | | | | |
|---|---|---|---|---|
| * = Oxidation of Methionine | | | | |

**Table 9. Preeclampsia: Additional peptides significant with AUC > 0.6 by Sequest only**

| Protein description | Uniprot ID (name) | Peptide | S_AUC |
|---|---|---|---|
| afamin | P43652 (AFAM_HUMAN) | R.LCFFYNKK.S | 0.67 |
| afamin | P43652 (AFAM_HUMAN) | R.RPCFESLK.A | 0.81 |
| afamin | P43652 (AFAM_HUMAN) | R.IVQIYK.D | 0.61 |
| afamin | P43652 (AFAM_HUMAN) | R.FLVNLVK.L | 0.60 |
| afamin | P43652 (AFAM_HUMAN) | K.LPNNVLQEK.I | 0.67 |
| alpha-1-antichymotrypsin | P01011 (AACT_HUMAN) | R.LYGSEAFATDFQDSAAAK K.L | 0.61 |
| alpha-1-antichymotrypsin | P01011 (AACT_HUMAN) | K.EQLSLLDRFTEDAKR.L | 0.71 |
| alpha-1-antichymotrypsin | P01011 (AACT_HUMAN) | R.EIGELYLPK.F | 0.68 |
| alpha-1-antichymotrypsin | P01011 (AACT_HUMAN) | R.WRDSLEFR.E | 0.71 |
| alpha-1-antichymotrypsin | P01011 (AACT_HUMAN) | K.RLYGSEAFATDFQDSAAA K.K | 0.89 |
| alpha-1B-glycoprotein | P04217 (A1BG_HUMAN) | R.FALVR.E | 1.00 |
| alpha-1B-glycoprotein | P04217 (A1BG_HUMAN) | R.GVTFLLRR.E | 0.67 |
| alpha-1B-glycoprotein | P04217 (A1BG_HUMAN) | R.RGEKELLVPR.S | 0.71 |
| alpha-1B-glycoprotein | P04217 (A1BG_HUMAN) | K.ELLVPR.S | 0.61 |
| alpha-1B-glycoprotein | P04217 (A1BG_HUMAN) | K.NGVAQEPVHLDSPAIK.H | 0.64 |
| alpha-2-antiplasmin | P08697 (A2AP_HUMAN) | R.NKFDPSLTQR.D | 0.60 |
| alpha-2-antiplasmin | P08697 (A2AP_HUMAN) | R.QLTSGPNQEQVSPLTLLK. L | 0.67 |
| alpha-2-antiplasmin | P08697 (A2AP_HUMAN) | K.HQM*DLVATLSQLGLQEL FQAPDLR.G | 0.67 |
| angiotensinogen preproprotein | P01019 (ANGT_HUMAN) | R.FM*QAVTGWK.T | 0.60 |
| angiotensinogen preproprotein | P01019 (ANGT_HUMAN) | K.PKDPTFIPAPIQAK.T | 0.83 |
| angiotensinogen preproprotein | P01019 (ANGT_HUMAN) | R. SLDFTELDVAAEK.I | 0.60 |
| ankyrin repeat and protein kinase domain-containing protein 1 | Q8NFD2 (ANKK1_HUMAN) | R.KNLVPR.D | 1.00 |
| antithrombin-III | P01008 (ANT3_HUMAN) | R.RVWELSK.A | 0.68 |
| apolipoprotein A-IV | P06727 (APOA4_HUMAN) | K.VKIDQTVEELRR.S | 0.62 |
| apolipoprotein A-IV | P06727 (APOA4_HUMAN) | K.DLRDKVNSFFSTFK.E | 0.92 |
| apolipoprotein A-IV | P06727 (APOA4_HUMAN) | K.LVPFATELHER.L | 0.71 |
| apolipoprotein A-IV | P06727 (APOA4_HUMAN) | R.RVEPYGENFNK.A | 0.86 |
| apolipoprotein A-IV | P06727 (APOA4_HUMAN) | K.VNSFFSTFK.E | 0.87 |
| apolipoprotein B-100 | P04114 (APOB_HUMAN) | K.AVSM*PSFSILGSDVR.V | 0.70 |
| apolipoprotein B-100 | P04114 (APOB_HUMAN) | K.AVSMPSFSILGSDVR.V | 0.66 |
| apolipoprotein B-100 | P04114 (APOB_HUMAN) | K.AVSMPSFSILGSDVR.V | 0.66 |
| apolipoprotein B-100 | P04114 (APOB_HUMAN) | K.AVSM*PSFSILGSDVR.V | 0.70 |
| apolipoprotein B-100 | P04114 (APOB_HUMAN) | K.VNWEEEAASGLLTSLKD NVPK.A | 0.60 |
| apolipoprotein B-100 | P04114 (APOB_HUMAN) | R.DLKVEDIPLAR.I | 0.70 |
| apolipoprotein C-I | P02654 (APOC1_HUMAN) | K.MREWFSETFQK.V | 0.73 |
| apolipoprotein C-II | P02655 (APOC2_HUMAN) | K.STAAMSTYTGIFTDQVLS VLKGEE.- | 0.68 |
| apolipoprotein E | P02649 (APOE_HUMAN) | R.AKLEEQAQQIR.L | 0.67 |
| apolipoprotein E | P02649 (APOE_HUMAN) | R.FWDYLR.W | 0.67 |
| apolipoprotein E | P02649 (APOE_HUMAN) | R.LKSWFEPLVEDMQR.Q | 0.65 |
| beta-2-glycoprotein 1 | P02749 (APOH_HUMAN) | K.VSFFCK.N | 0.67 |
| beta-2-glycoprotein 1 | P02749 (APOH_HUMAN) | R.VCPFAGILENGAVR.Y | 0.63 |
| beta-2-microglobulin | P61769 (B2MG_HUMAN) | K.SNFLNCYVSGFHPSDIEVD LLK.N | 0.60 |
| biotinidase | P43251 (BTD_HUMAN) | R.LSSGLVTAALYGR.L | 1.00 |
| carboxypeptidase B2 preproprotein | Q96IY4 (CBPB2_HUMAN) | K.IAWHVIR.N | 0.90 |
| carboxypeptidase N subunit 2 | P22792 (CPN2_HUMAN) | K.LSNNALSGLPQGVFGK.L | 0.62 |
| carboxypeptidase N subunit 2 | P15169 (CBPN_HUMAN) | R.DHLGFQVTWPDESK.A | 0.93 |
| ceruloplasmin | P00450 (CERU_HUMAN) | K.VYVHLK.N | 0.67 |
| ceruloplasmin | P00450 (CERU_HUMAN) | K.LISVDTEHSNIYLQNGPDR .I | 0.62 |
| ceruloplasmin | P00450 (CERU_HUMAN) | K.M*YYSAVDPTKDIFTGLIG PM*K.I | 0.76 |
| ceruloplasmin | P00450 (CERU_HUMAN) | K.M*YYSAVDPTKDIFTGLIG PMK.I | 0.68 |
| ceruloplasmin | P00450 (CERU_HUMAN) | R.QKDVDKEFYLFPTVFDEN ESLLLEDNIR.M | 0.66 |
| ceruloplasmin | P00450 (CERU_HUMAN) | K.DVDKEFYLFPTVFDENES LLLEDNIR.M | 0.60 |
| ceruloplasmin | P00450 (CERU_HUMAN) | K.DIFTGLIGPMK.I | 0.62 |
| ceruloplasmin | P00450 (CERU_HUMAN) | R.SVPPSASHVAPTETFTYE WTVPK.E | 0.66 |
| ceruloplasmin | P00450 (CERU_HUMAN) | R.GVYSSDVFDIFPGTYQTLE M*FPR.T | 0.67 |
| ceruloplasmin | P00450 (CERU_HUMAN) | K.DIFTGLIGPMK.I | 0.62 |
| ceruloplasmin | P00450 (CERU_HUMAN) | K.VNKDDEEFIESNK.M | 0.78 |
| clusterin preproprotein | P10909 (CLUS_HUMAN) | R.KYNELLK.S | 0.75 |
| coagulation factor XII | P00748 (FA12_HUMAN) | R.TTLSGAPCQPWASEATYR .N | 0.64 |
| complement C1q subcomponent subunit A | P02745 (C1QA_HUMAN) | K.GHIYQGSEADSVFSGFLIF PSA.- | 0.64 |
| complement C1q subcomponent subunit C | P02747 (C1QC_HUMAN) | K.FQSVFTVTR.Q | 0.65 |
| complement C1r subcomponent | P00736 (C1R_HUMAN) | R.WILTAAHTLYPK.E | 0.68 |
| complement C1r subcomponent | P00736 (C1R_HUMAN) | K.VLNYVDWIKK.E | 0.81 |
| complement C1s subcomponent | P09871 (C1S_HUMAN) | R.LPVAPLRK.C | 0.63 |
| complement C2 | P06681 (CO2_HUMAN) | R.PICLPCTMEANLALR.R | 0.78 |
| complement C2 | P06681 (CO2_HUMAN) | R.QHLGDVLNFLPL.- | 0.70 |
| complement C4-B-like preproprotein | P0C0L5 (CO4B_HUMAN) | K.LGQYASPTAKR.C | 0.89 |
| complement C4-B-like preproprotein | P0C0L5 (CO4B_HUMAN) | K.M*RPSTDTITVMVENSHG LR.V | 0.65 |
| complement C4-B-like preproprotein | P0C0L5 (CO4B_HUMAN) | K.MRPSTDTITVMVENSHGL R.V | 0.72 |
| complement C5 preproprotein | P01031 (CO5_HUMAN) | K.EFPYRIPLDLVPK.T | 0.67 |
| complement C5 preproprotein | P01031 (CO5_HUMAN) | R.VFQFLEK.S | 0.60 |
| complement C5 preproprotein | P01031 (CO5_HUMAN) | R.MVETTAYALLTSLNLK.D | 0.61 |
| complement C5 preproprotein | P01031 (CO5_HUMAN) | R.ENSLYLTAFTVIGIR.K | 0.81 |
| complement component C8 alpha chain | P07357 (CO8A HUMAN) | K.YNPVVIDFEMQPIHEVLR. H | 0.62 |
| complement component C8 beta chain preproprotein | P07358 (CO8B_HUMAN) | K.IPGIFELGISSQSDR.G | 0.61 |
| complement component C8 gamma chain | P07360 (CO8G_HUMAN) | R.RPASPISTIQPK.A | 0.71 |
| complement component C8 gamma chain | P07360 (CO8G_HUMAN) | R.FLQEQGHR.A | 0.87 |
| complement factor B preproprotein | P00751 (CFAB_HUMAN) | K.VSVGGEKR.D | 0.60 |
| complement factor B preproprotein | P00751 (CFAB_HUMAN) | K.CLVNLIEK.V | 0.69 |
| complement factor B preproprotein | P00751 (CFAB_HUMAN) | K.KDNEQHVFK.V | 0.68 |
| complement factor B preproprotein | P00751 (CFAB_HUMAN) | K.ISVIRPSK.G | 0.63 |
| complement factor B preproprotein | P00751 (CFAB_HUMAN) | K.KCLVNLIEK.V | 0.63 |
| complement factor B preproprotein | P00751 (CFAB_HUMAN) | R.LPPTTTCQQQKEELLPAQ DIK.A | 0.64 |
| complement factor B preproprotein | P00751 (CFAB_HUMAN) | K.LQDEDLGFL.- | 0.66 |
| complement factor H | P08603 (CFAH_HUMAN) | K.SCDIPVFMNAR.T | 0.60 |
| complement factor H | P08603 (CFAH_HUMAN) | K.HGGLYHENMR.R | 0.75 |
| complement factor H | P08603 (CFAH_HUMAN) | K.IIYKENER.F | 0.69 |
| complement factor I preproprotein | P05156 (CFAI_HUMAN) | K.RAQLGDLPWQVAIK.D | 0.68 |
| conserved oligomeric Golgi complex subunit 6 isoform | Q9Y2V7 (COG6_HUMAN) | K.ISNLLK.F | 0.71 |
| cornulin | Q9UBG3 (CRNN_HUMAN) | R.RYARTEGNCTALTR.G | 0.81 |
| FERM domain-containing protein 8 | Q9BZ67 (FRMD8_HUMAN) | R.VQLGPYQPGRPAACDLR. E | 0.63 |
| gelsolin | P06396 (GELS_HUMAN) | R.VPEARPNSMVVEHPEFLK. A | 0.61 |
| gelsolin | P06396 (GELS_HUMAN) | K.AGKEPGLQIWR.V | 0.70 |
| glucose-induced degradation protein 8 homolog | Q9NWU2 (GID8_HUMAN) | K.VWSEVNQAVLDYENRES TPK.L | 0.83 |
| hemK methyltransferase family member 1 | Q9Y5R4 (HEMK1_HUMAN) | R.M*LWALLSGPGRRGSTR. G | 0.61 |
| hemopexin | P02790 (HEMO_HUMAN) | R.ELISER.W | 0.82 |
| hemopexin | P02790 (HEMO_HUMAN) | R.DVRDYFM*PCPGR.G | 0.70 |
| hemopexin | P02790 (HEMO_HUMAN) | K.GDKVWVYPPEKK.E | 0.71 |
| hemopexin | P02790 (HEMO_HUMAN) | R.DVRDYFMPCPGR.G | 0.60 |
| hemopexin | P02790 (HEMO_HUMAN) | R.EWFWDLATGTMK.E | 0.65 |
| hemopexin | P02790 (HEMO_HUMAN) | R.YYCFQGNQFLR.F | 0.68 |
| hemopexin | P02790 (HEMO_HUMAN) | R.RLWWLDLK.S | 0.65 |
| heparin cofactor 2 | P05546 (HEP2_HUMAN) | R. LNILNAK. F | 0.75 |
| heparin cofactor 2 | P05546 (HEP2_HUMAN) | R.NFGYTLR.S | 0.66 |
| histone deacetylase complex subunit SAP25 | Q8TEE9 (SAP25_HUMAN) | K.LLPPPPIM*SARVLPR.P | 0.63 |
| hyaluronan-binding protein 2 | Q14520 (HABP2_HUMAN) | K.RPGVYTQVTK.F | 0.68 |
| hyaluronan-binding protein 2 | Q14520 (HABP2_HUMAN) | K.FLNWIK.A | 0.62 |
| immediate early response gene 5-like protein | Q5T953 (IER5L_HUMAN) | - .MECALDAQSLISISLRKIHSS R.T | 0.93 |
| inactive caspase-12 | Q6UXS9 (CASPC_HUMAN) | K.AGADTHGRLLQGNICND AVTK.A | 0.60 |
| insulin-like growth factor-binding protein complex acid labile subunit | P35858 (ALS_HUMAN) | K.ANVFVQLPR.L | 0.62 |
| inter-alpha-trypsin inhibitor heavy chain H1 | P19827 (ITIH1_HUMAN) | K.ELAAQTIKK.S | 0.71 |
| inter-alpha-trypsin inhibitor heavy chain H1 | P19827 (ITIH1_HUMAN) | K.ILGDM*QPGDYFDLVLFG TR.V | 0.79 |
| inter-alpha-trypsin inhibitor heavy chain H1 | P19827 (ITIH1_HUMAN) | K.VTFQLTYEEVLKR.N | 0.70 |
| inter-alpha-trypsin inhibitor heavy chain H1 | P19827 (ITIH1_HUMAN) | R.TMEQFTIHLTVNPQSK.V | 0.61 |
| inter-alpha-trypsin inhibitor heavy chain H1 | P19827 (ITIH1_HUMAN) | R.FAHYVVTSQVVNTANEA R.E | 0.63 |
| inter-alpha-trypsin inhibitor heavy chain H2 | P19823 (ITIH2_HUMAN) | R.SSALDMENFRTEVNVLPG AK.V | 0.89 |
| inter-alpha-trypsin inhibitor heavy chain H2 | P19823 (ITIH2_HUMAN) | K.MKQTVEAMK.T | 0.93 |
| inter-alpha-trypsin inhibitor heavy chain H2 | P19823 (ITIH2_HUMAN) | R.IYLQPGR.L | 0.66 |
| inter-alpha-trypsin inhibitor heavy chain H2 | P19823 (ITIH2_HUMAN) | K.HLEVDVWVIEPQGLR.F | 0.61 |
| inter-alpha-trypsin inhibitor heavy chain H2 | P19823 (ITIH2_HUMAN) | K.FYNQVSTPLLR.N | 0.89 |
| inter-alpha-trypsin inhibitor heavy chain H2 | P19823 (ITIH2_HUMAN) | R.KLGSYEHR.I | 0.69 |
| inter-alpha-trypsin inhibitor heavy chain H4 | Q14624 (ITIH4_UMAN) | K.GSEMVVAGK.L | 1.00 |
| inter-alpha-trypsin inhibitor heavy chain H4 | Q14624 (ITIH4_HUMAN) | R.MNFRPGVLSSR.Q | 0.72 |
| inter-alpha-trypsin inhibitor heavy chain H4 | Q14624 (ITIH4_UMAN) | K.YIFHNFM*ER.L | 0.73 |
| inter-alpha-trypsin inhibitor heavy chain H4 | Q14624 (ITIH4_UMAN) | K.ETLFSVMPGLK.M | 0.60 |
| inter-alpha-trypsin inhibitor heavy chain H4 | Q14624 (ITIH4_HUMAN) | R.FKPTLSQQQK.S | 0.64 |
| inter-alpha-trypsin inhibitor heavy chain H4 | Q14624 (ITIH4_UMAN) | K.WKETLFSVMPGLK.M | 0.69 |
| inter-alpha-trypsin inhibitor heavy chain H4 | Q14624 (ITIH4_UMAN) | R.RLGVYELLLK.V | 0.65 |
| inter-alpha-trypsin inhibitor heavy chain H4 | Q14624 (ITIH4_HUMAN) | R.DTDRFSSHVGGTLGQFYQ EVLWGSPAASDDGRR.T | 0.69 |
| inter-alpha-trypsin inhibitor heavy chain H4 | Q14624 (ITIH4_HUMAN) | K.VRPQQLVK.H | 0.62 |
| inter-alpha-trypsin inhibitor heavy chain H4 | Q14624 (ITIH4_HUMAN) | R.NVHSAGAAGSR.M | 0.69 |
| kallistatin | P29622 (KAIN_HUMAN) | R.LGFTDLFSK.W | 0.63 |
| kallistatin | P29622 (KAIN_HUMAN) | R.VGSALFLSHNLK.F | 0.62 |
| kininogen-1 | P01042 (KNG1_HUMAN) | R.VQVVAGKK.Y | 0.68 |
| leucine-rich alpha-2-glycoprotein | P02750 (A2GL_HUMAN) | R.LHLEGNKLQVLGK.D | 0.75 |
| lumican | P51884 (LUM_HUMAN) | R.FNALQYLR.L | 0.77 |
| m7GpppX diphosphatase | Q96C86 (DCPS_HUMAN) | R.IVFENPDPSDGFVLIPDLK. W | 0.94 |
| MAGUK p55 subfamily member 5 | Q8N3R9 (MPP5_HUMAN) | K.ILEIEDLFSSLK.H | 0.69 |
| MBT domain-containing protein 1 | Q05BQ5 (MBTD1_HUMAN) | K.WFDYLR.E | 0.63 |
| obscurin | Q5VST9 (OBSCN_HUMAN) | R.CELQIRGLAVEDTGEYLC VCGQERTSATLTVR.A | 0.73 |
| olfactory receptor 1L1 | Q8NH94 (OR1L1_HUMAN) | K.DMKQGLAKLM*HR.M | 0.89 |
| phosphatidylinositol -glycan-specific phospholipase D | P80108 (PHLD_HUMAN) | K.GIVAAFYSGPSLSDKEK.L | 0.79 |
| phosphatidylinositol -glycan-specific phospholipase D | P80108 (PHLD_HUMAN) | R.TLLLVGSPTWK.N | 0.65 |
| phosphatidylinositol -glycan-specific phospholipase D | P80108 (PHLD_HUMAN) | R.WYVPVKDLLGIYEK.L | 0.92 |
| pigment epithelium-derived factor | P36955 (PEDF_HUMAN) | R.SSTSPTTNVLLSPLSVATA LSALSLGAEQR.T | 0.63 |
| plasma protease C 1 inhibitor | P05155 (IC1_HUMAN) | K.GVTSVSQIFHSPDLAIR.D | 0.60 |
| PREDICTED: complement C4-A | P0C0L4 (CO4A_HUMAN) | R.DKGQAGLQR.A | 0.67 |
| PREDICTED: complement C4-A | P0C0L4 (CO4A_HUMAN) | K.SHKPLNMGK.V | 0.87 |
| PREDICTED: complement C4-A | P0C0L4 (CO4A_HUMAN) | R.KKEVYM*PSSIFQDDFVIP DISEPGTWK.I | 0.67 |
| PREDICTED: complement C4-A | P0C0L4 (CO4A_HUMAN) | R.FGLLDEDGKK.T | 0.64 |
| PREDICTED: complement C4-A | P0C0L4 (CO4A_HUMAN) | R.KKEVYMPSSIFQDDFVIPD ISEPGTWK.I | 0.69 |
| PREDICTED: complement C4-A | P0C0L4 (CO4A_HUMAN) | K.GLCVATPVQLR.V | 0.78 |
| PREDICTED: complement C4-A | P0C0L4 (CO4A_HUMAN) | R.YRVFALDQK.M | 0.63 |
| PREDICTED: complement C4-A | P0C0L4 (CO4A_HUMAN) | K.AEFQDALEKLNMGITDLQ GLR.L | 0.60 |
| PREDICTED: complement C4-A | P0C0L4 (CO4A_HUMAN) | R.ECVGFEAVQEVPVGLVQP ASATLYDYYNPERR.C | 0.60 |
| PREDICTED: complement C4-A | P0C0L4 (CO4A_HUMAN) | K.AEFQDALEKLNMGITDLQ GLR.L | 0.60 |
| PREDICTED: complement C4-A | P0C0L4 (CO4A_HUMAN) | R.VTASDPLDTLGSEGALSP GGVASLLR.L | 0.61 |
| pregnancy zone protein | P20742 (PZP_HUMAN) | R.NELIPLIYLENPRR.N | 0.60 |
| pregnancy zone protein | P20742 (PZP_HUMAN) | K.AVGYLITGYQR.Q | 0.67 |
| protein AMBP preproprotein | P02760 (AMBP_HUMAN) | R.AFIQLWAFDAVK.G | 0.70 |
| protein CBFA2T2 | O43439 (MTG8R_HUMAN) | R.LTEREWADEWKHLDHAL NCIMEMVEK.T | 0.61 |
| protein NLRC3 | Q7RTR2 (NLRC3_HUMAN) | K.ALM*DLLAGKGSQGSQA PQALDR.T | 0.83 |
| prothrombin preproprotein | P00734 (THRB_HUMAN) | R.TFGSGEADCGLRPLFEK.K | 0.69 |
| ras-related GTP-binding protein A | Q7L523 (RRAGA_HUMAN) | K.ISNIIK.Q | 0.68 |
| retinol-binding protein 4 | P02753 (RET4_HUMAN) | R.FSGTWYAMAK.K | 0.64 |
| retinol-binding protein 4 | P02753 (RET4_HUMAN) | R.LLNNWDVCADMVGTFTD TEDPAKFK.M | 0.61 |
| retinol-binding protein 4 | P02753 (RET4_HUMAN) | K.YWGVASFLQK.G | 0.63 |
| serum amyloid P-component | P02743 (SAMP_HUMAN) | R.GYVIIKPLVWV.- | 0.60 |
| sex hormone-binding globulin | P04278 (SHBG_HUMAN) | R.LPLVPALDGCLR.R | 0.63 |
| spectrin beta chain, non-erythrocytic 1 | Q13813 (SPTN1_HUMAN) | R.NELIRQEKLEQLAR.R | 0.88 |
| TATA element modulatory factor | P82094 (TMF1_HUMAN) | K.EELATRLNSSETADLLK.E | 0.71 |
| testicular haploid expressed gene protein-like | P0DJG4 (THEGL_HUMAN) | R.QCLLNRPFSDNSAR.D | 0.67 |
| thyroxine-binding globulin | P05543 (THBG_HUMAN) | K.NALALFVLPK.E | 0.61 |
| thyroxine-binding globulin | P05543 (THBG_HUMAN) | R.SFMLLILER.S | 0.64 |
| titin | Q8WZ42 (TITIN_HUMAN) | K.TEPKAPEPISSK.P | 0.89 |
| transthyretin | P02766 (TTHY_HUMAN) | R.GSPAINVAVHVFR.K | 0.61 |
| tripartite motif-containing protein 5 | Q9C035 (TRIM5_HUMAN) | R.ELISDLEHRLQGSVM*ELL QGVDGVIK.R | 0.92 |
| vitamin D-binding protein | P02774 (VTDB_HUMAN) | K.TAMDVFVCTYFMPAAQL PELPDVELPTNKDVCDPGN TK.V | 0.88 |
| vitamin D-binding protein | P02774 (VTDB_HUMAN) | K.VM*DKYTFELSR.R | 0.70 |
| vitamin D-binding protein | P02774 (VTDB_HUMAN) | K.LAQKVPTADLEDVLPLAE DITNILSK.C | 0.61 |
| vitamin D-binding protein | P02774 (VTDB_HUMAN) | K.SCESNSPFPVHPGTAECCT K.E | 0.68 |
| vitamin D-binding protein | P02774 (VTDB_HUMAN) | R.KLCMAALK.H | 0.71 |
| vitamin D-binding protein | P02774 (VTDB_HUMAN) | K.LCDNLSTK.N | 0.60 |
| vitamin D-binding protein | P02774 (VTDB_HUMAN) | K.VM*DKYTFELSR.R | 0.70 |
| vitronectin | P04004 (VTNC_HUMAN) | R.IYISGM*APR.P | 0.75 |
| vitronectin | P04004 (VTNC_HUMAN) | R.ERVYFFK.G | 0.67 |
| vitronectin | P04004 (VTNC_HUMAN) | R.IYISGMAPR.P | 0.81 |
| vitronectin | P04004 (VTNC_HUMAN) | K.AVRPGYPK.L | 0.63 |
| zinc finger protein 142 | P52746 (ZN142_HUMAN) | K.TRFLLR.T | 0.67 |

| | | | |
|---|---|---|---|
| * = Oxidation of methionine | | | |

**Table 10. Preeclampsia: Additional peptides significant with AUC > 0.6 by X! Tandem only**

| Protein description | Uniprot ID (name) | Peptide | XT_AUC |
|---|---|---|---|
| afamin | P43652 (AFAM_HUMAN) | K.TYVPPPFSQDLFTFHADMCQSQN EELQR.K | 0.76 |
| afamin | P43652 (AFAM_HUMAN) | K.KSDVGFLPPFPTLDPEEK.C | 0.62 |
| alpha-1-antichymotrypsin | P01011 (AACT_HUMAN) | R.GTHVDLGLASANVDFAFSLYK.Q | 0.69 |
| alpha-1B-glycoprotein | P04217 (A1BG_HUMAN) | K.SLPAPWLSM*APVSWITPGLK.T | 0.67 |
| alpha-1B-glycoprotein | P04217 (A1BG_HUMAN) | K.SLPAPWLSM*APVSWITPGLK.T | 0.67 |
| alpha-1B-glycoprotein | P04217 (A1BG_HUMAN) | R.C^LAPLEGAR.F | 0.62 |
| alpha-2-antiplasmin | P08697 (A2AP_HUMAN) | R.WFLLEQPEIQVAHFPFK.N | 0.60 |
| alpha-2-antiplasmin | P08697 (A2AP_HUMAN) | R.LCQDLGPGAFR.L | 0.92 |
| alpha-2-antiplasmin | P08697 (A2AP_HUMAN) | K.HQMDLVATLSQLGLQELFQAPDL R.G | 0.67 |
| alpha-2-HS-glycoprotein preproprotein | P02765 (FETUA_HUMAN) | R.QLKEHAVEGDCDFQLLK.L | 0.63 |
| alpha-2-HS-glycoprotein preproprotein | P02765 (FETUA_HUMAN) | R.Q^LKEHAVEGDCDFQLLK.L | 0.65 |
| alpha-2-HS-glycoprotein preproprotein | P02765 (FETUA_HUMAN) | K.C^NLLAEK.Q | 0.61 |
| angiotensinogen preproprotein | P01019 (ANGT_HUMAN) | R.SLDFTELDVAAEKIDR.F | 0.62 |
| angiotensinogen preproprotein | P01019 (ANGT_HUMAN) | K.DPTFIPAPIQAK.T | 0.78 |
| apolipoprotein A-II preproprotein | P02652 (APOA2_HUMAN) | K.EPCVESLVSQYFQTVTDYGKDLM EK.V | 0.67 |
| apolipoprotein B-100 | P04114 (APOB_HUMAN) | K.FSVPAGIVIPSFQALTAR.F | 0.66 |
| apolipoprotein B-100 | P04114 (APOB_HUMAN) | K.EQHLFLPFSYK.N | 0.90 |
| apolipoprotein B-100 | P04114 (APOB_HUMAN) | R.GIISALLVPPETEEAK.Q | 0.70 |
| beta-2-glycoprotein 1 | P02749 (APOH_HUMAN) | K.C^FKEHSSLAFWK.T | 0.70 |
| beta-2-glycoprotein 1 | P02749 (APOH_HUMAN) | K.EHSSLAFWK.T | 0.62 |
| ceruloplasmin | P00450 (CERU_HUMAN) | R.FNKNNEGTYYSPNYNPQSR. S | 0.64 |
| ceruloplasmin | P00450 (CERU_HUMAN) | K.HYYIGIIETTWDYASDHGEK.K | 0.63 |
| ceruloplasmin | P00450 (CERU_HUMAN) | K.M*YYSAVDPTKDIFTGLIGPM*K.I | 0.66 |
| ceruloplasmin | P00450 (CERU_HUMAN) | K.M*YYSAVDPTKDIFTGLIGPM*K.I | 0.66 |
| ceruloplasmin | P00450 (CERU_HUMAN) | K.M*YYSAVDPTKDIFTGLIGPMK.I | 0.67 |
| ceruloplasmin | P00450 (CERU_HUMAN) | K.M*YYSAVDPTKDIFTGLIGPMK.I | 0.67 |
| ceruloplasmin | P00450 (CERU_HUMAN) | K.MYYSAVDPTKDIFTGLIGPM*K.I | 0.67 |
| ceruloplasmin | P00450 (CERU_HUMAN) | K.MYYSAVDPTKDIFTGLIGPM*K.I | 0.67 |
| ceruloplasmin | P00450 (CERU_HUMAN) | R.GVYSSDVFDIFPGTYQTLEM*FPR. T | 0.67 |
| coagulation factor XII | P00748 (FA12_HUMAN) | R.VVGGLVALR.G | 0.64 |
| complement C1q subcomponent subunit A | P02745 (C1QA_HUMAN) | K.KGHIYQGSEADSVFSGFLIFPSA.- | 0.81 |
| complement C1q subcomponent subunit C | P02747 (C1QC_HUMAN) | R.Q^THQPPAPNSLIR.F | 0.64 |
| complement C1s subcomponent | P09871 (CIS_HUMAN) | R.Q^FGPYCGHGFPGPLNIETK.S | 0.71 |
| complement C2 | P06681 (CO2_HUMAN) | R.QPYSYDFPEDVAPALGTSFSHML GATNPTQK.T | 0.63 |
| complement C2 | P06681 (CO2_HUMAN) | R.LLGMETMAWQEIR.H | 0.70 |
| complement C4-B-like preproprotein | P0C0L5 (CO4B_HUMAN) | R.AVGSGATFSHYYYM*ILSR.G | 0.67 |
| complement C4-B-like preproprotein | P0C0L5 (CO4B_HUMAN) | R.FGLLDEDGKKTFFR.G | 0.61 |
| complement C4-B-like preproprotein | P0C0L5 (CO4B_HUMAN) | K.ITQVLHFTK.D | 0.67 |
| complement C4-B-like preproprotein | P0C0L5 (CO4B_HUMAN) | K.M*RPSTDTITVM*VENSHGLR.V | 0.65 |
| complement C4-B-like preproprotein | P0C0L5 (CO4B_HUMAN) | K.M*RPSTDTITVM*VENSHGLR.V | 0.75 |
| complement C5 preproprotein | P01031 (CO5_HUMAN) | R.IVACASYKPSR.E | 0.67 |
| complement C5 preproprotein | P01031 (CO5_HUMAN) | R.SYFPESWLWEVHLVPR.R | 0.60 |
| complement C5 preproprotein | P01031 (CO5_HUMAN) | K.Q^LPGGQNPVSYVYLEVVSK.H | 0.74 |
| complement C5 preproprotein | P01031 (CO5_HUMAN) | K.TLLPVSKPEIR.S | 0.78 |
| complement component C8 beta chain preproprotein | P07358 (CO8B_HUMAN) | R.GGASEHITTLAYQELPTADLMQE WGDAVQYNPAIIK.V | 0.60 |
| complement factor B preproprotein | P00751 (CFAB_HUMAN) | K.GTDYHKQPWQAK.I | 0.89 |
| complement factor B preproprotein | P00751 (CFAB_HUMAN) | K.VKDISEVVTPR.F | 0.64 |
| complement factor B preproprotein | P00751 (CFAB_HUMAN) | K.Q^VPAHAR.D | 0.63 |
| complement factor B preproprotein | P00751 (CFAB_HUMAN) | R.GDSGGPLIVHKR.S | 0.79 |
| complement factor B preproprotein | P00751 (CFAB_HUMAN) | R.FLCTGGVSPYADPNTCR.G | 0.71 |
| complement factor B preproprotein | P00751 (CFAB_HUMAN) | K.KEAGIPEFYDYDVALIK.L | 0.74 |
| complement factor B preproprotein | P00751 (CFAB_HUMAN) | R.YGLVTYATYPK.I | 0.88 |
| complement factor H | P08603 (CFAH_HUMAN) | K.EFDHNSNIR.Y | 1.00 |
| complement factor H | P08603 (CFAH_HUMAN) | K.WSSPPQCEGLPCK.S | 0.71 |
| complement factor H | P08603 (CFAH_HUMAN) | R.KGEWVALNPLR.K | 0.67 |
| complement factor I preproprotein | P05156 (CFAI_HUMAN) | K.SLECLHPGTK.F | 0.60 |
| corticosteroid-binding globulin | P08185 (CBG_HUMAN) | R.GLASANVDFAFSLYK.H | 0.62 |
| fetuin-B | Q9UGM5 (FETUB_HUMAN) | K.LVVLPFPK.E | 0.74 |
| fetuin-B | Q9UGM5 (FETUB_HUMAN) | R.ASSQWVVGPSYFVEYLIK.E | 0.61 |
| ficolin-3 | O75636 (FCN3_HUMAN) | R.LLGEVDHYQLALGK.F | 0.61 |
| gelsolin | P06396 (GELS_HUMAN) | K.QTQVSVLPEGGETPLFK.Q | 0.69 |
| hemopexin | P02790 (HEMO_HUMAN) | K.VDGALCMEK.S | 0.60 |
| hemopexin | P02790 (HEMO_HUMAN) | K.SGAQATWTELPWPHEKVDGALC M*EK.S | 0.66 |
| hemopexin | P02790 (HEMO_HUMAN) | K.SGAQATWTELPWPHEKVDGALC M*EK.S | 0.66 |
| hemopexin | P02790 (HEMO_HUMAN) | R.EWFWDLATGTMK.E | 0.68 |
| hemopexin | P02790 (HEMO_HUMAN) | R.Q^GHNSVFLIK.G | 0.67 |
| heparin cofactor 2 | P05546 (HEP2_HUMAN) | K.TLEAQLTPR.V | 0.67 |
| histidine-rich glycoprotein | P04196 (HRG_HUMAN) | K.DSPVLIDFFEDTER.Y | 0.60 |
| insulin-like growth factor-binding protein complex acid labile subunit | P35858 (ALS_HUMAN) | K.ALRDFALQNPSAVPR.F | 0.89 |
| insulin-like growth factor-binding protein complex acid labile subunit | P35858 (ALS_HUMAN) | R.LWLEGNPWDCGCPLK.A | 0.60 |
| inter-alpha-trypsin inhibitor heavy chain H1 | P19827 (ITIH1_HUMAN) | K.ILGDM*QPGDYFDLVLFGTR.V | 0.85 |
| inter-alpha-trypsin inhibitor heavy chain H2 | P19823 (ITIH2_HUMAN) | R.SSALDMENFR.T | 0.63 |
| inter-alpha-trypsin inhibitor heavy chain H2 | P19823 (ITIH2_HUMAN) | R.SLAPTAAAK.R | 0.83 |
| inter-alpha-trypsin inhibitor heavy chain H2 | P19823 (ITIH2_HUMAN) | R.LSNENHGIAQR.I | 0.76 |
| inter-alpha-trypsin inhibitor heavy chain H2 | P19823 (ITIH2_HUMAN) | R.IYGNQDTSSQLKK.F | 0.63 |
| inter-alpha-trypsin inhibitor heavy chain H4 | Q14624 (ITIH4_HUMAN) | K.TGLLLLSDPDKVTIGLLFWDGR.G | 0.60 |
| inter-alpha-trypsin inhibitor heavy chain H4 | Q14624 (ITIH4_UMAN) | K.YIFHNFM*ER.L | 0.70 |
| inter-alpha-trypsin inhibitor heavy chain H4 | Q14624 (ITIH4_UMAN) | K.IPKPEASFSPR.R | 0.65 |
| inter-alpha-trypsin inhibitor heavy chain H4 | Q14624 (ITIH4_HUMAN) | R.QGPVNLLSDPEQGVEVTGQYER. E | 0.64 |
| inter-alpha-trypsin inhibitor heavy chain H4 | Q14624 (ITIH4_UMAN) | R.ANTVQEATFQMELPK.K | 0.61 |
| inter-alpha-trypsin inhibitor heavy chain H4 | Q14624 (ITIH4_UMAN) | K.WKETLFSVMPGLK.M | 0.66 |
| inter-alpha-trypsin inhibitor heavy chain H4 | Q14624 (ITIH4_HUMAN) | R.RLDYQEGPPGVEISCWSVEL.- | 0.69 |
| inter-alpha-trypsin inhibitor heavy chain H4 | Q14624 (ITIH4_HUMAN) | K.SPEQQETVLDGNLIIR.Y | 0.66 |
| kallistatin | P29622 (KAIN_HUMAN) | K.ALWEKPFISSR.T | 0.65 |
| kininogen-1 | P01042 (KNG1_HUMAN) | R.Q^VVAGLNFR.I | 0.67 |
| kininogen-1 | P01042 (KNG1_HUMAN) | R.QVVAGLNFR.I | 0.71 |
| kininogen-1 | P01042 (KNG1_HUMAN) | K.LGQSLDCNAEVYVVPWEK.K | 0.62 |
| kininogen-1 | P01042 (KNG1_HUMAN) | R.IASFSQNCDIYPGKDFVQPPTK.I | 0.64 |
| leucine-rich alpha-2-glycoprotein | P02750 (A2GL_HUMAN) | R.C^AGPEAVKGQTLLAVAK.S | 0.70 |
| leucine-rich alpha-2-glycoprotein | P02750 (A2GL_HUMAN) | K.GQTLLAVAK.S | 0.67 |
| leucine-rich alpha-2-glycoprotein | P02750 (A2GL_HUMAN) | K.DLLLPQPDLR.Y | 0.71 |
| lumican | P51884 (LUM_HUMAN) | K.ILGPLSYSK.I | 0.83 |
| PREDICTED: complement C4-A | P0C0L4 (CO4A_HUMAN) | R.QGSFQGGFR.S | 0.83 |
| PREDICTED: complement C4-A | P0C0L4 (CO4A HUMAN) | K.YVLPNFEVK.I | 0.69 |
| PREDICTED: complement C4-A | P0C0L4 (CO4A_HUMAN) | R.LLATLCSAEVCQCAEGK.C | 0.60 |
| PREDICTED: complement C4-A | P0C0L4 (CO4A_HUMAN) | R.VGDTLNLNLR.A | 0.66 |
| PREDICTED: complement C4-A | P0C0L4 (CO4A_HUMAN) | R.EPFLSCCQFAESLR.K | 0.62 |
| PREDICTED: complement C4-A | P0C0L4 (CO4A_HUMAN) | R.EELVYELNPLDHR.G | 0.60 |
| PREDICTED: complement C4-A | P0C0L4 (CO4A_HUMAN) | R.GSFEFPVGDAVSK.V | 0.62 |
| PREDICTED: complement C4-A | P0C0L4 (CO4A_HUMAN) | R.GCGEQTMIYLAPTLAASR.Y | 0.71 |
| pregnancy zone protein | P20742 (PZP_HUMAN) | K.GSFALSFPVESDVAPIAR.M | 0.63 |
| protein AMBP preproprotein | P02760 (AMBP_HUMAN) | R.VVAQGVGIPEDSIFTMADRGECV PGEQEPEPILIPR.V | 0.62 |
| prothrombin preproprotein | P00734 (THRB_HUMAN) | R.SGIECQLWR.S | 0.65 |
| thyroxine-binding globulin | P05543 (THBG_HUMAN) | K.MSSINADFAFNLYR.R | 0.63 |
| vitronectin | P04004 (VTNC_HUMAN) | R.MDWLVPATCEPIQSVFFFSGDKY YR.V | 1.00 |
| vitronectin | P04004 (VTNC_HUMAN) | R.IYISGM*APRPSLAK.K | 0.64 |
| vitronectin | P04004 (VTNC_HUMAN) | R.IYISGMAPRPSLAK.K | 0.63 |
| vitronectin | P04004 (VTNC_HUMAN) | R.DVWGIEGPIDAAFTR.I | 0.61 |
| zinc finger CCHC domain-containing protein 9 | Q8N567 (ZCHC9_HUMAN) | R.SCPDNPK.G | 0.68 |

| | | | |
|---|---|---|---|
| * = Oxidation of Methionine, ^ = cyclic pyrolidone derivative by the loss of NH3 (-17 Da) | | | |

**Table 11. Candidate peptides and transitions for transferring to the MRM assay**

| Protein | Peptide | m/z, charge | fragment ion, m/z, charge, rank | area |
|---|---|---|---|---|
| inter-alpha-trypsin inhibitor heavy chain H1 ITIH1_HUMAN | K.AAISGENAGLVR.A | 579.317 3++ | S [y9] - 902.4690+[1] | 518001 |
| | | | G [y8] - 815.4370+[2] | 326256 |
| | | | N [y6] - 629.3729+[3] | 296670 |
| | | | S [b4] - 343.1976+[4] | 258172 |
| inter-alpha-trypsin inhibitor heavy chain H1 ITIH1_HUMAN | K.GSLVQASEANLQAA QDFVR.G | 668.676 3+++ | A [y7] - 806.4155+[1] | 304374 |
| | | | V [b4] - 357.2132+[3] | 294094 |
| | | | A [b13] - 635.3253++[7] | 249287 |
| | | | A [y6] - 735.3784+[2] | 193844 |
| | | | F [y3] - 421.2558+[4] | 167816 |
| | | | L [b11] - 535.7775++[6] | 156882 |
| | | | A [b6] - 556.3089+[5] | 149216 |
| | | | A [y14] - 760.3786++[8] | 123723 |
| inter-alpha-trypsin inhibitor heavy chain H1 ITIH1_HUMAN | K.TAFISDFAVTADGNA FIGDIK.D | 1087.04 42++ | G [y4] - 432.2453+[1] | 22362 |
| | | | V [b9] - 952.4775+[2] | 9508 |
| | | | I [y5] - 545.3293+[3] | 8319 |
| | | | A [b8] - 853.4090+[4] | 7006 |
| | | | G [y9] - 934.4993+[5] | 6755 |
| | | | F [y6] - 692.3978+[6] | 6193 |
| inter-alpha-trypsin inhibitor heavy chain H1 ITIH1_HUMAN | K.VTYDVSR.D | 420.216 5++ | T [b2] - 201.1234+[1] | 792556 |
| | | | Y [y5] - 639.3097+[2] | 609348 |
| | | | V [y3] - 361.2194+[3] | 256946 |
| | | | D [y4] - 476.2463+[4] | 169546 |
| | | | Y [y5] - 320.1585++[5] | 110608 |
| | | | S [y2] - 262.1510+[6] | 50268 |
| | | | D [b4] - 479.2136+[7] | 13662 |
| | | | Y [b3] - 182.5970++[8] | 10947 |
| inter-alpha-trypsin inhibitor heavy chain H1 ITIH1_HUMAN | R.EVAFDLEIPK.T | 580.813 5++ | P [y2] - 244.1656+[1] | 2032509 |
| | | | D [y6] - 714.4032+[2] | 672749 |
| | | | A [y8] - 932.5088+[3] | 390837 |
| | | | F [y7] - 861.4716+[4] | 305087 |
| | | | L [y5] - 599.3763+[5] | 255527 |
| inter-alpha-trypsin inhibitor heavy chain H1 ITIH1_HUMAN | R.LWAYLTIQELLAK.R | 781.453 1++ | W [b2] - 300.1707+[1] | 602601 |
| | | | A [b3] - 371.2078+[2] | 356967 |
| | | | T [y8] - 915.5510+[3] | 150419 |
| | | | Y [b4] - 534.2711+[4] | 103449 |
| | | | L [b5] - 647.3552+[5] | 99820 |
| | | | I [y7] - 814.5033+[6] | 72044 |
| | | | Q [y6] - 701.4192+[7] | 66989 |
| | | | E [y5] - 573.3606+[8] | 44843 |
| inter-alpha-trypsin inhibitor heavy chain H2 ITIH2_HUMAN | K.FYNQVSTPLLR.N | 669.364 2++ | S [y6] - 686.4196+[1] | 367330 |
| | | | V [y7] - 785.4880+[2] | 182396 |
| | | | P [y4] - 498.3398+[3] | 103638 |
| | | | Q [b4] - 553.2405+[4] | 54270 |
| | | | Y [b2] - 311.1390+[5] | 52172 |
| | | | N [b3] - 425.1819+[6] | 34567 |
| inter-alpha-trypsin inhibitor heavy chain H2 ITIH2_HUMAN | K.HLEVDVWVIEPQGL R.F | 597.324 7+++ | P [y5] - 570.3358+[1] | 303693 |
| | | | I [y7] - 812.4625+[2] | 206996 |
| | | | E [y6] - 699.3784+[3] | 126752 |
| | | | P [y5] - 285.6715++[4] | 79841 |
| inter-alpha-trypsin inhibitor heavy chain H2 ITIH2_HUMAN | K.TAGLVR.S | 308.692 5++ | G [y4] - 444.2929+[1] | 789068 |
| | | | A [b2] - 173.0921+[2] | 460019 |
| | | | V [y2] - 274.1874+[3] | 34333 |
| | | | L [y3] - 387.2714+[4] | 29020 |
| | | | G [b3] - 230.1135+[5] | 15169 |
| inter-alpha-trypsin inhibitor heavy chain H2 ITIH2_HUMAN | R.IYLQPGR.L | 423.745 2++ | L [y5] - 570.3358+[1] | 638209 |
| | | | Y [b2] - 277.1547+[2] | 266889 |
| | | | P [y3] - 329.1932+[3] | 235194 |
| | | | Q [y4] - 457.2518+[4] | 171389 |
| inter-alpha-trypsin inhibitor heavy chain H2 ITIH2_HUMAN | R.LSNENHGIAQR.I | 413.546 1+++ | N [y9] - 519.7574++[1] | 325409 |
| | | | G [y5] - 544.3202+[2] | 139598 |
| | | | S [b2] - 201.1234+[3] | 54786 |
| | | | N [y7] - 398.2146++[4] | 39521 |
| | | | E [y8] - 462.7359++[5] | 30623 |
| inter-alpha-trypsin inhibitor heavy chain H2 ITIH2_HUMAN | R.SLAPTAAAKR.R | 415.242 5++ | A [y7] - 629.3617+[1] | 582421 |
| | | | P [y6] - 558.3246+[2] | 463815 |
| | | | L [b2] - 201.1234+[3] | 430584 |
| | | | A [b3] - 272.1605+[4] | 204183 |
| | | | T [y5] - 461.2718+[5] | 47301 |
| pregnancy-specific beta-1-glycoprotein 1 PSG1_HUMAN | K.FQLPGQK.L | 409.232 0++ | L [y5] - 542.3297+[3] | 192218 |
| | | | P [y4] - 429.2456+[2] | 252933 |
| | | | Q [y2] - 275.1714+[6] | 15366 |
| | | | Q [b2] - 276.1343+[1] | 305361 |
| | | | L [b3] - 389.2183+[4] | 27279 |
| | | | G [b5] - 543.2926+[5] | 18416 |
| pregnancy-specific beta-1-glycoprotein 1 PSG1_HUMAN | R.DLYHYITSYVVDGEIII YGPAYSGR.E | 955.476 2+++ | G [y7] - 707.3471+[1] | 66891 |
| | | | Y [y8] - 870.4104+[2] | 45076 |
| | | | P [y6] - 650.3257+[3] | 28437 |
| | | | I [y9] - 983.4945+[4] | 20423 |
| | | | V [b10] - 628.3033++[5] | 17864 |
| | | | E [b14] - 828.3830++[6] | 13690 |
| | | | V [b11] - 677.8375++[7] | 12354 |
| | | | I [b6] - 805.3879+[8] | 11186 |
| | | | V [y15] - 805.4147++[9] | 10573 |
| | | | G [b13] - 763.8617++[10] | 10407 |
| pregnancy-specific beta-1-glycoprotein 4 PSG4_HUMAN | TLFIFGVTK | 513.305 1++ | F [y7] - 811.4713+[1] | 102139 |
| | | | L [b2] - 215.1390+[2] | 86272 |
| | | | F [y5] - 551.3188+[3] | 49520 |
| | | | I [y6] - 664.4028+[4] | 26863 |
| | | | T [y2] - 248.1605+[5] | 18671 |
| | | | F [b3] - 362.2074+[6] | 17343 |
| | | | G [y4] - 404.2504+[7] | 17122 |
| pregnancy-specific beta-1-glycoprotein 4 PSG4_HUMAN | NYTYIWWLNGQSLPV SPR | 1097.55 76++ | W [b6] - 841.3879+[1] | 25756 |
| | | | G [y9] - 940.5211+[2] | 25018 |
| | | | Y [b4] - 542.2245+[3] | 19778 |
| PSG8_HUMAN | LQLSETNR | 480.759 1++ | T [y3] - 390.2096+[1] | 185568 |
| pregnancy-specific beta-1-glycoprotein 8 | | | Q [b2] - 242.1499+[2] | 120644 |
| | | | N [y2] - 289.1619+[3] | 95164 |
| | | | S [y5] - 606.2842+[4] | 84314 |
| | | | L [b3] - 355.2340+[5] | 38587 |
| | | | E [y4] - 519.2522+[6] | 34807 |
| | | | L [y6] - 719.3682+[7] | 17482 |
| | | | E [b5] - 571.3086+[8] | 8855 |
| | | | S [b4] - 442.2660+[9] | 7070 |
| Pan-PSG | ILILPSVTR | 506.331 7++ | P [y5] - 559.3198+[1] | 484395 |
| | | | L [b2] - 227.1754+[2] | 102774 |
| | | | L [b4] - 227.1754++[3] | 102774 |
| | | | I [y7] - 785.4880+[4] | 90153 |
| | | | I [b3] - 340.2595+[5] | 45515 |
| | | | L [y6] - 672.4039+[6] | 40368 |
| thyroxine-binding globulin THBG_HUMAN | K.ELELQIGNALFIGK.H | 515.627 | E [b3] - 186.5919++[1] | 48549 |
| | | 6+++ | E [b3] - 372.1765+[2] | 28849 |
| | | | G [y2] - 204.1343+[3] | 27487 |
| | | | F [b11] - 614.8322++[4] | 14892 |
| | | | L [b4] - 485.2606+[5] | 14552 |
| | | | L [b2] - 243.1339+[6] | 10169 |
| | | | L [b4] - 243.1339++[7] | 10169 |
| thyroxine-binding globulin THBG_HUMAN | K.AQWANPFDPSK.T | 630.804 0++ | A [b4] - 457.2194+[1] | 48405 |
| | | | S [y2] - 234.1448+[2] | 43781 |
| | | | D [y4] - 446.2245+[3] | 26549 |
| | | | D [y4] - 446.2245+[4] | 25148 |
| thyroxine-binding globulin THBG_HUMAN | K.TEDSSSFLIDK.T | 621.298 4++ | E [b2] - 231.0975+[1] | 37113 |
| | | | D [y2] - 262.1397+[2] | 14495 |
| thyroxine-binding globulin THBG_HUMAN | K.AVLHIGEK.G | 433.758 4++ | V [b2] - 171.1128+[1] | 151828 |
| | | | L [y6] - 696.4039+[2] | 102903 |
| | | | H [y5] - 583.3198+[3] | 73288 |
| | | | I [y4] - 446.2609+[4] | 54128 |
| | | | G [y3] - 333.1769+[5] | 32717 |
| | | | H [b4] - 421.2558+[6] | 22662 |
| thyroxine-binding globulin THBG_HUMAN | K.AVLHIGEK.G | 289.508 0+++ | L [y6] - 348.7056++[1] | 2496283 |
| | | | V [b2] - 171.1128+[2] | 551283 |
| | | | I [y4] - 446.2609+[3] | 229168 |
| | | | H [y5] - 292.1636++[4] | 212709 |
| | | | H [y5] - 583.3198+[5] | 160132 |
| | | | G [y3] - 333.1769+[6] | 117961 |
| | | | H [b4] - 421.2558+[7] | 56579 |
| | | | I [y4] - 223.6341++[8] | 36569 |
| | | | H [b4] - 211.1315++[9] | 19460 |
| | | | L [b3] - 284.1969+[10] | 15758 |
| thyroxine-binding globulin THBG_HUMAN | K.FLNDVK.T | 368.205 4++ | N [y4] - 475.2511+[1] | 298227 |
| | | | V [y2] - 246.1812+[2] | 252002 |
| | | | L [b2] - 261.1598+[3] | 98700 |
| | | | D [y3] - 361.2082+[4] | 29215 |
| | | | D [b4] - 490.2296+[5] | 27258 |
| | | | N [b3] - 375.2027+[6] | 10971 |
| thyroxine-binding globulin THBG_HUMAN | K.FSISATYDLGATLLK. M | 800.435 1++ | S [b2] - 235.1077+[1] | 50075 |
| | | | G [y6] - 602.3872+[2] | 46373 |
| | | | D [y8] - 830.4982+[3] | 43372 |
| | | | Y [y9] - 993.5615+[4] | 40970 |
| | | | T [y4] - 474.3286+[5] | 22161 |
| | | | L [y7] - 715.4713+[6] | 19710 |
| | | | S [b4] - 435.2238+[7] | 19310 |
| | | | L [y3] - 373.2809+[8] | 14157 |
| | | | I [b3] - 348.1918+[9] | 13207 |
| thyroxine-binding globulin THBG_HUMAN | K.LSNAAHK.A | 370.706 1++ | H [y2] - 284.1717+[4] | 19319 |
| | | | S [b2] - 201.1234+[1] | 60611 |
| | | | N [b3] - 315.1663+[2] | 42142 |
| | | | A [b4] - 386.2034+[3] | 31081 |
| thyroxine-binding globulin THBG_HUMAN | K.GWVDLFVPK.F | 530.794 9++ | V [y7] - 817.4818+[2] | 297536 |
| | | | D [y6] - 718.4134+[4] | 226951 |
| | | | L [y5] - 603.3865+[8] | 60712 |
| | | | F [y4] - 490.3024+[9] | 45586 |
| | | | V [y3] - 343.2340+[6] | 134588 |
| | | | P [y2] - 244.1656+[1] | 1619888 |
| | | | V [b3] - 343.1765+[7] | 126675 |
| | | | D [b4] - 458.2034+[10] | 14705 |
| | | | F [b6] - 718.3559+[5] | 208674 |
| | | | V [b7] - 817.4243+[3] | 270156 |
| thyroxine-binding globulin THBG_HUMAN | K.NALALFVLPK.E | 543.339 5++ | L [b3] - 299.1714+[1] | 365040 |
| | | | P [y2] - 244.1656+[2] | 274988 |
| | | | A [y7] - 787.5076+[3] | 237035 |
| | | | L [y6] - 716.4705+[4] | 107838 |
| | | | L [y3] - 357.2496+[5] | 103847 |
| | | | L [y8] - 900.5917+[6] | 97265 |
| | | | F [y5] - 603.3865+[7] | 88231 |
| | | | A [b4] - 370.2085+[8] | 82559 |
| | | | V [y4] - 456.3180+[9] | 32352 |
| | | | L [b5] - 483.2926+[10] | 11974 |
| thyroxine-binding globulin THBG_HUMAN | R.SILFLGK.V | 389.247 1++ | L [y5] - 577.3708+[1] | 564222 |
| | | | I [b2] - 201.1234+[2] | 384240 |
| | | | G [y2] - 204.1343+[3] | 302557 |
| | | | L [y3] - 317.2183+[4] | 282436 |
| | | | F [y4] - 464.2867+[5] | 194047 |
| | | | L [b3] - 314.2074+[6] | 27878 |
| leucine-rich alpha-2-glycoprotein A2GL_HUMAN | R.VLDLTR.N | 358.718 7++ | D [y4] - 504.2776+[1] | 629222 |
| | | | L [y5] - 617.3617+[2] | 236165 |
| | | | L [b2] - 213.1598+[3] | 171391 |
| | | | L [y3] - 389.2507+[4] | 167609 |
| | | | R [y1] - 175.1190+[5] | 41213 |
| | | | T [y2] - 276.1666+[6] | 37194 |
| | | | D [b3] - 328.1867+[7] | 27029 |
| leucine-rich alpha-2-glycoprotein A2GL_HUMAN | K.ALGHLDLSGNR.L | 576.809 6++ | G [y9] - 484.7490++[1] | 46334 |
| | | | L [y7] - 774.4104+[2] | 44285 |
| | | | D [y6] - 661.3264+[3] | 40188 |
| | | | H [y8] - 456.2383++[4] | 29392 |
| | | | H [b4] - 379.2088+[5] | 26871 |
| | | | L [y5] - 546.2994+[6] | 17178 |
| | | | L [b5] - 492.2929+[7] | 14578 |
| leucine-rich alpha-2-glycoprotein A2GL_HUMAN | K.LPPGLLANFTLLR.T | 712.934 8++ | R [y1] - 175.1190+[1] | 34435 |
| | | | A [b7] - 662.4236+[2] | 25768 |
| | | | G [y10] - 1117.6728+[3] | 11662 |
| leucine-rich alpha-2-glycoprotein A2GL_HUMAN | R.TLDLGENQLETLPPD LLR.G | 1019.04 68++ | P [y6] - 710.4196+[1] | 232459 |
| | | | L [y7] - 823.5036+[2] | 16075 |
| | | | E [y9] - 1053.5939+[3] | 15839 |
| | | | D [b3] - 330.1660+[4] | 15524 |
| leucine-rich alpha-2-glycoprotein | R.GPLQLER.L | 406.734 9++ | P [b2] - 155.0815+[1] | 144054 |
| A2GL_HUMAN | | | Q [y4] - 545.3042+[2] | 103146 |
| | | | L [y5] - 658.3883+[3] | 77125 |
| | | | L [y3] - 417.2456+[4] | 65928 |
| | | | R [y1] - 175.1190+[5] | 27585 |
| | | | E [y2] - 304.1615+[6] | 22956 |
| leucine-rich alpha-2-glycoprotein | R.LHLEGNK.L | 405.727 1++ | H [b2] - 251.1503+[1] | 79532 |
| A2GL_HUMAN | | | L [y5] - 560.3039+[2] | 54272 |
| | | | G [b5] - 550.2984+[3] | 49019 |
| | | | G [y3] - 318.1772+[4] | 18570 |
| | | | L [b3] - 364.2343+[5] | 14068 |
| | | | E [y4] - 447.2198+[6] | 13318 |
| leucine-rich alpha-2-glycoprotein | K.LQVLGK.D | 329.218 3++ | V [y4] - 416.2867+[1] | 141056 |
| A2GL_HUMAN | | | G [y2] - 204.1343+[2] | 102478 |
| | | | Q [b2] - 242.1499+[3] | 98414 |
| | | | L [y3] - 317.2183+[4] | 60587 |
| | | | Q [y5] - 544.3453+[5] | 50833 |
| leucine-rich alpha-2-glycoprotein | K.DLLLPQPDLR.Y | 590.340 2++ | P [y6] - 725.3941+[1] | 592715 |
| A2GL_HUMAN | | | L [b3] - 342.2023+[2] | 570948 |
| | | | L [b2] - 229.1183+[3] | 403755 |
| | | | P [y6] - 363.2007++[4] | 120157 |
| | | | L [y2] - 288.2030+[5] | 89508 |
| | | | L [y7] - 838.4781+[6] | 76185 |
| | | | L [b4] - 455.2864+[7] | 60422 |
| | | | L [y7] - 419.7427++[8] | 45849 |
| | | | P [y4] - 500.2827+[9] | 45223 |
| | | | L [y8] - 951.5622+[10] | 22393 |
| | | | Q [y5] - 628.3413+[11] | 15450 |
| leucine-rich alpha-2-glycoprotein | R.VAAGAFQGLR.Q | 495.280 0++ | A [y8] - 819.4472+[1] | 183637 |
| A2GL_HUMAN | | | G [y7] - 748.4100+[2] | 110920 |
| | | | F [y5] - 620.3515+[3] | 85535 |
| | | | A [y9] - 890.4843+[4] | 45894 |
| | | | G [y3] - 345.2245+[5] | 45644 |
| | | | Q [y4] - 473.2831+[6] | 40579 |
| | | | A [y8] - 410.2272++[7] | 39266 |
| | | | A [b3] - 242.1499+[8] | 35890 |
| | | | A [y6] - 691.3886+[9] | 29637 |
| | | | G [b4] - 299.1714+[10] | 19195 |
| | | | A [b5] - 370.2085+[11] | 14944 |
| | | | A [y9] - 445.7458++[12] | 11567 |
| leucine-rich alpha-2-glycoprotein A2GL_HUMAN | R.WLQAQK.D | 387.218 9++ | L [y5] - 587.3511+[1] | 80533 |
| | | | Q [y4] - 474.2671+[2] | 57336 |
| | | | A [y3] - 346.2085+[3] | 35952 |
| | | | L [b2] - 300.1707+[4] | 22509 |
| leucine-rich alpha-2-glycoprotein A2GL_HUMAN | K.GQTLLAVAK.S | 450.779 3++ | Q [b2] - 186.0873+[1] | 110213 |
| | | | T [y7] - 715.4713+[2] | 81127 |
| | | | L [y5] - 501.3395+[3] | 52292 |
| | | | L [y6] - 614.4236+[4] | 46349 |
| | | | A [y4] - 388.2554+[5] | 41283 |
| | | | A [y2] - 218.1499+[6] | 38843 |
| | | | V [y3] - 317.2183+[7] | 28961 |
| | | | T [b3] - 287.1350+[8] | 23831 |
| leucine-rich alpha-2-glycoprotein | R.YLFLNGNK.L | 484.763 6++ | F [y6] - 692.3726+[1] | 61861 |
| A2GL_HUMAN | | | L [b2] - 277.1547+[2] | 39468 |
| | | | F [b3] - 424.2231+[3] | 21454 |
| | | | L [y5] - 545.3042+[4] | 20016 |
| | | | N [y4] - 432.2201+[5] | 18077 |
| leucine-rich alpha-2-glycoprotein | R.NALTGLPPGLFQASA TLDTLVLK.E | 780.777 3+++ | T [y8] - 902.5557+[1] | 44285 |
| A2GL_HUMAN | | | P [y17] - 886.0036++[2] | 39557 |
| | | | D [y6] - 688.4240+[3] | 19464 |
| alpha-1B-glycoprotein | K.NGVAQEPVHLDSPAI K.H | 837.944 1++ | P [y10] - 1076.6099+[1] | 130137 |
| A1BG_HUMAN | | | V [b3] - 271.1401+[2] | 110650 |
| | | | A [y13] - 702.8777++[3] | 75803 |
| | | | 5 [y5] - 515.3188+[4] | 63197 |
| | | | G [b2] - 172.0717+[5] | 57307 |
| | | | E [b6] - 599.2784+[6] | 49765 |
| | | | A [b4] - 342.1772+[7] | 36058 |
| | | | E [y11] - 1205.6525+[8] | 34131 |
| | | | P [y4] - 428.2867+[9] | 31158 |
| | | | H [y8] - 880.4887+[10] | 28296 |
| | | | D [y6] - 630.3457+[11] | 20534 |
| | | | L [y7] - 743.4298+[12] | 17946 |
| alpha-1B-glycoprotein | K.HQFLLTGDTQGR.Y | 686.852 0++ | Q [b2] - 266.1248+[1] | 1144372 |
| A1BG_HUMAN | | | F [y10] - 1107.5793+[2] | 725830 |
| | | | T [y7] - 734.3428+[3] | 341528 |
| | | | L [y8] - 847.4268+[4] | 297048 |
| | | | F [b3] - 413.1932+[5] | 230163 |
| | | | G [y6] - 633.2951+[6] | 226694 |
| | | | T [y4] - 461.2467+[7] | 217446 |
| | | | L [y9] - 960.5109+[8] | 215574 |
| | | | L [b4] - 526.2772+[9] | 184306 |
| | | | L [b5] - 639.3613+[10] | 157607 |
| | | | Q [y11] - 1235.6379+[11] | 117366 |
| | | | Q [y11] - 618.3226++[12] | 109274 |
| | | | D [b8] - 912.4574+[13] | 53233 |
| | | | T [b6] - 740.4090+[14] | 49104 |
| | | | D [y5] - 576.2736+[15] | 35232 |
| alpha-1B-glycoprotein | R.SGLSTGWTQLSK.L | 632.830 2++ | G [y7] - 819.4359+[1] | 1138845 |
| A1BG_HUMAN | | | L [b3] - 258.1448+[2] | 1128060 |
| | | | S [y9] - 1007.5156+[3] | 877313 |
| | | | S [y2] - 234.1448+[4] | 653032 |
| | | | T [y8] - 920.4836+[5] | 651216 |
| | | | T [y5] - 576.3352+[6] | 538856 |
| | | | W [y6] - 762.4145+[7] | 406137 |
| | | | L [y3] - 347.2289+[8] | 313255 |
| | | | Q [y4] - 475.2875+[9] | 209919 |
| | | | L [y10] - 560.8035++[10] | 103666 |
| | | | W [b7] - 689.3253+[11] | 48587 |
| | | | Q [b9] - 918.4316+[12] | 27677 |
| | | | T [b8] - 790.3730+[13] | 26742 |
| | | | L [b10] - 1031.5156+[14] | 23936 |
| alpha-1B-glycoprotein | K.LLELTGPK.S | 435.768 4++ | E [y6] - 644.3614+[1] | 6043967 |
| A1BG_HUMAN | | | L [b2] - 227.1754+[2] | 2185138 |
| | | | L [y7] - 757.4454+[3] | 1878211 |
| | | | L [y5] - 515.3188+[4] | 923148 |
| | | | T [y4] - 402.2347+[5] | 699198 |
| | | | G [y3] - 301.1870+[6] | 666018 |
| | | | P [y2] - 244.1656+[7] | 430183 |
| | | | E [b3] - 356.2180+[8] | 244199 |
| alpha-1B-glycoprotein | R.GVTFLLR.R | 403.250 2++ | T [y5] - 649.4032+[1] | 4135468 |
| A1BG_HUMAN | | | L [y3] - 401.2871+[2] | 2868709 |
| | | | V [b2] - 157.0972+[3] | 2109754 |
| | | | F [y4] - 548.3555+[4] | 1895653 |
| | | | R [y1] - 175.1190+[5] | 918856 |
| | | | L [y2] - 288.2030+[6] | 780084 |
| | | | T [b3] - 258.1448+[7] | 478494 |
| | | | T [y5] - 325.2052++[8] | 415711 |
| | | | F [y4] - 274.6814++[9] | 140533 |
| | | | L [b6] - 631.3814+[10] | 129473 |
| alpha-1B-glycoprotein | K.ELLVPR.S | 363.729 1++ | P [y2] - 272.1717+[1] | 9969478 |
| A1BG_HUMAN | | | L [y4] - 484.3242+[2] | 3676023 |
| | | | V [y3] - 371.2401+[3] | 2971809 |
| | | | L [b2] - 243.1339+[4] | 809753 |
| | | | L [y5] - 597.4083+[5] | 159684 |
| alpha-1B-glycoprotein | R.SSTSPDR.I | 375.174 8++ | S [b2] - 175.0713+[1] | 89016 |
| A1BG_HUMAN | | | R [y1] - 175.1190+[2] | 82740 |
| | | | P [y3] - 387.1987+[3] | 76299 |
| | | | T [y5] - 575.2784+[4] | 75253 |
| | | | D [b6] - 575.2307+[5] | 71180 |
| | | | S [y4] - 474.2307+[6] | 53784 |
| alpha-1B-glycoprotein | R.LELHVDGPPPRPQLR .A | 862.483 7++ | D [b6] - 707.3723+[1] | 49322 |
| A1BG_HUMAN | | | G [y9] - 1017.5952+[2] | 32049 |
| | | | G [y9] - 509.3012++[3] | 27715 |
| alpha-1B-glycoprotein | R.LELHVDGPPPRPQLR .A | 575.324 9+++ | V [y11] - 616.3489++[1] | 841163 |
| A1BG_HUMAN | | | D [y10] - 566.8147++[2] | 621546 |
| | | | E [b2] - 243.1339+[3] | 581025 |
| | | | H [y12] - 684.8784++[4] | 485731 |
| | | | R [y5] - 669.4155+[5] | 477653 |
| | | | L [y13] - 741.4204++[6] | 369224 |
| | | | H [b4] - 493.2769+[7] | 219485 |
| | | | D [b6] - 707.3723+[8] | 195842 |
| | | | V [b5] - 592.3453+[9] | 170689 |
| | | | R [y1] - 175.1190+[10] | 160049 |
| | | | L [b3] - 356.2180+[11] | 63902 |
| | | | G [b7] - 764.3937+[12] | 62128 |
| | | | P [y4] - 513.3144+[13] | 33888 |
| alpha-1B-glycoprotein | R.ATWSGAVLAGR.D | 544.796 0++ | S [y8] - 730.4206+[1] | 1933290 |
| A1BG_HUMAN | | | G [y7] - 643.3886+[2] | 1828931 |
| | | | L [y4] - 416.2616+[3] | 869412 |
| | | | V [y5] - 515.3300+[4] | 615117 |
| | | | A [y3] - 303.1775+[5] | 584118 |
| | | | A [y6] - 586.3671+[6] | 471353 |
| | | | W [y9] - 458.7536++[7] | 466690 |
| | | | W [y9] - 916.4999+[8] | 454934 |
| | | | G [y2] - 232.1404+[9] | 338886 |
| | | | S [b4] - 446.2034+[10] | 165831 |
| | | | W [b3] - 359.1714+[11] | 139166 |
| | | | R [y1] - 175.1190+[12] | 83145 |
| | | | A [b6] - 574.2620+[13] | 65281 |
| | | | G [b5] - 503.2249+[14] | 30473 |
| | | | V [b7] - 673.3304+[15] | 30408 |
| alpha-1B-glycoprotein | R. TPGAAAN LEU FVGP QHAGNYR.C | 1148.59 53++ | G [y9] - 999.4755+[1] | 39339 |
| A1BG_HUMAN | | | F [y11] - 1245.6123+[2] | 22329 |
| | | | V [y10] - 1098.5439+[3] | 14054 |
| | | | I [b11] - 1051.5782+[4] | 12281 |
| | | | P [y8] - 942.4540+[5] | 10574 |
| alpha-1B-glycoprotein | R.TPGAAANLELIFVGP QHAGNYR.C | 766.065 9+++ | G [y9] - 999.4755+[1] | 426098 |
| A1BG_HUMAN | | | P [y8] - 942.4540+[2] | 191245 |
| | | | V [y10] - 1098.5439+[3] | 183889 |
| | | | F [y11] - 1245.6123+[4] | 172790 |
| | | | G [b3] - 256.1292+[5] | 172068 |
| | | | A [y5] - 580.2838+[6] | 170557 |
| | | | A [b4] - 327.1663+[7] | 146455 |
| | | | H [y6] - 717.3427+[8] | 127934 |
| | | | E [b9] - 825.4101+[9] | 119922 |
| | | | G [y4] - 509.2467+[10] | 107378 |
| | | | L [b10] - 938.4942+[11] | 102387 |
| | | | A [b5] - 398.2034+[12] | 86428 |
| | | | L [b10] - 469.7507++[13] | 68959 |
| | | | E [y14] - 800.9152++[14] | 67711 |
| | | | I [y12] - 679.8518++[15] | 65740 |
| | | | N [b7] - 583.2835+[16] | 58648 |
| | | | A [y17] - 949.9972++[17] | 55561 |
| | | | G [y20] - 1049.5451++[18] | 51555 |
| | | | I [b11] - 1051.5782+[19] | 51489 |
| | | | L [y13] - 736.3939++[20] | 49190 |
| | | | L [y15] - 857.4572++[21] | 48534 |
| | | | A [y18] - 985.5158++[22] | 48337 |
| | | | L [b8] - 696.3675+[23] | 47352 |
| | | | N [y16] - 914.4787++[24] | 43280 |
| | | | A [b6] - 469.2405+[25] | 38091 |
| | | | Q [y7] - 845.4013+[26] | 32443 |
| insulin-like growth factor-binding protein complex | R.SLALGTFAHTPALAS LGLSNNR.L | 737.734 2+++ | G [y6] - 660.3424+[1] | 37287 |
| acid labile subunit | | | | |
| ALS_HUMAN | | | A [b3] - 272.1605+[2] | 21210 |
| | | | S [y8] - 860.4585+[3] | 15266 |
| | | | S [y4] - 490.2368+[4] | 12497 |
| | | | L [y5] - 603.3209+[5] | 9592 |
| insulin-like growth factor-binding protein complex acid labile subunit | R.ELVLAGNR.L | 436.253 4++ | A [y4] - 417.2205+[1] | 74710 |
| ALS_HUMAN | | | L [y5] - 530.3045+[2] | 71602 |
| | | | G [y3] - 346.1833+[3] | 39449 |
| | | | V [y6] - 629.3729+[4] | 30127 |
| insulin-like growth factor-binding protein complex acid labile subunit | R.LAYLQPALFSGLAELR .E | 881.498 5++ | P [y11] - 1173.6626+[1] | 47285 |
| ALS_HUMAN | | | Y [b3] - 348.1918+[2] | 27425 |
| | | | Q [b5] - 589.3344+[3] | 18779 |
| | | | L [b4] - 461.2758+[4] | 13442 |
| insulin-like growth factor-binding protein complex acid labile subunit | | 588.001 4+++ | 5 [y7] - 745.4203+[1] | 29519 |
| ALS_HUMAN | | | A [y4] - 488.2827+[2] | 23305 |
| | | | G [y6] - 658.3883+[3] | 22089 |
| | | | F [y8] - 892.4887+[4] | 16888 |
| | | | Q [b5] - 589.3344+[5] | 15807 |
| | | | L [y2] - 288.2030+[6] | 15266 |
| | | | Y [b3] - 348.1918+[7] | 12835 |
| | | | L [y5] - 601.3668+[8] | 12024 |
| insulin-like growth factor-binding protein complex acid labile subunit | R.ELDLSR.N | 366.698 0++ | S [y2] - 262.1510+[1] | 91447 |
| ALS_HUMAN | | | D [b3] - 358.1609+[2] | 85115 |
| | | | D [y4] - 490.2620+[3] | 75618 |
| | | | L [y3] - 375.2350+[4] | 37835 |
| insulin-like growth factor-binding protein complex acid labile subunit | K.ANVFVQLPR.L | 522.303 5++ | N [b2] - 186.0873+[1] | 90097 |
| ALS_HUMAN | | | F [y6] - 759.4512+[2] | 61085 |
| | | | P [y2] - 272.1717+[3] | 46657 |
| | | | V [y5] - 612.3828+[4] | 43595 |
| | | | V [b3] - 285.1557+[5] | 31451 |
| | | | Q [y4] - 513.3144+[6] | 28908 |
| | | | V [y7] - 858.5196+[7] | 15725 |
| | | | L [y3] - 385.2558+[8] | 14324 |
| | | | Q [y4] - 257.1608++[9] | 13753 |
| insulin-like growth factor-binding protein complex acid labile subunit | R.NLIAAVAPGAFLGLK. A | 727.940 1++ | L [b2] - 228.1343+[1] | 26729 |
| ALS_HUMAN | | | I [b3] - 341.2183+[2] | 25535 |
| | | | P [y8] - 802.4822+[3] | 25120 |
| | | | A [y9] - 873.5193+[4] | 17542 |
| | | | A [y12] - 1114.6619+[5] | 14895 |
| insulin-like growth factor-binding protein complex acid labile subunit | R.VAGLLEDTFPGLLGL R.V | 835.977 4++ | P [y7] - 725.4668+[1] | 22005 |
| ALS_HUMAN | | | L [b4] - 341.2183+[2] | 13753 |
| | | | E [y11] - 1217.6525+[3] | 12611 |
| | | | D [y10] - 1088.6099+[4] | 11003 |
| insulin-like growth factor-binding protein complex acid labile subunit | R.SFEGLGQLEVLTLDH NQLQEVK.A | 833.102 6+++ | Q [y4] - 503.2824+[1] | 328959 |
| ALS_HUMAN | | | T [y11] - 662.8464++[2] | 54479 |
| | | | G [b4] - 421.1718+[3] | 24263 |
| insulin-like growth factor-binding protein complex acid labile subunit | R.NLPEQVFR.G | 501.772 0++ | P [y6] - 775.4097+[1] | 88417 |
| ALS_HUMAN | | | E [y5] - 678.3570+[2] | 13620 |
| insulin-like growth factor-binding protein complex acid labile subunit | R.IRPHTFTGLSGLR.R | 485.612 4+++ | S [y4] - 432.2565+[1] | 82619 |
| ALS_HUMAN | | | L [y5] - 545.3406+[2] | 70929 |
| | | | T [b5] - 303.1795++[3] | 56677 |
| insulin-like growth factor-binding protein complex acid labile subunit | K.LEYLLLSR.N | 503.800 2++ | Y [y6] - 764.4665+[1] | 67619 |
| ALS_HUMAN | | | E [b2] - 243.1339+[2] | 56261 |
| | | | L [y4] - 488.3191+[3] | 32890 |
| | | | L [y5] - 601.4032+[4] | 24224 |
| | | | L [y3] - 375.2350+[5] | 21139 |
| insulin-like growth factor-binding protein complex acid labile subunit | R.LAELPADALGPLQR. A | 732.414 5++ | E [b3] - 314.1710+[1] | 57859 |
| ALS_HUMAN | | | P [y10] - 1037.5738+[2] | 45907 |
| | | | P [y10] - 519.2905++[3] | 22723 |
| | | | L [b4] - 427.2551+[4] | 14054 |
| insulin-like growth factor-binding protein complex acid labile subunit | R.LEALPNSLLAPLGR.L | 732.432 7++ | A [b3] - 314.1710+[1] | 52485 |
| ALS_HUMAN | | | P [y10] - 1037.6102+[2] | 37028 |
| | | | E [b2] - 243.1339+[3] | 24846 |
| | | | P [y10] - 519.3087++[4] | 15601 |
| | | | P [y4] - 442.2772+[5] | 12327 |
| insulin-like growth factor-binding protein complex acid labile subunit | R.TFTPQPPGLER.L | 621.827 5++ | P [y6] - 668.3726+[1] | 57877 |
| ALS_HUMAN | | | P [y8] - 447.2456++[2] | 50606 |
| | | | P [b4] - 447.2238+[3] | 50606 |
| | | | F [b2] - 249.1234+[4] | 42083 |
| | | | P [y8] - 893.4839+[5] | 34716 |
| | | | T [y9] - 497.7694++[6] | 24220 |
| | | | T [b3] - 350.1710+[7] | 22053 |
| insulin-like growth factor-binding protein complex acid labile subunit | R.DFALQNPSAVPR.F | 657.843 7++ | A [b3] - 334.1397+[1] | 28905 |
| ALS_HUMAN | | | P [y6] - 626.3620+[2] | 23750 |
| | | | P [y2] - 272.1717+[3] | 20860 |
| | | | F [b2] - 263.1026+[4] | 17536 |
| | | | N [y7] - 740.4050+[5] | 15320 |
| | | | Q [y8] - 868.4635+[6] | 12525 |
| beta-2-glycoprotein 1 | K.FICPLTGLWPINTLK.C | 886.992 0++ | C [b3] - 421.1904+[1] | 546451 |
| APOH_HUMAN | | | C [y13] - 756.9158++[2] | 438858 |
| | | | P [y6] - 685.4243+[3] | 229375 |
| | | | I [b2] - 261.1598+[4] | 188092 |
| | | | W [y7] - 871.5036+[5] | 143885 |
| | | | G [y9] - 1041.6091+[6] | 143458 |
| | | | T [b13] - 757.3972++[7] | 127058 |
| | | | T [y10] - 1142.6568+[8] | 89126 |
| | | | T [b6] - 732.3749+[9] | 51907 |
| | | | L [b5] - 631.3272+[10] | 43351 |
| | | | L [b8] - 902.4804+[11] | 38788 |
| | | | N [y4] - 475.2875+[12] | 38574 |
| | | | W [b9] - 1088.5597+[13] | 37148 |
| | | | T [y3] - 361.2445+[14] | 34153 |
| | | | G [b7] - 789.3964+[15] | 22460 |
| | | | P [b4] - 518.2432+[16] | 19893 |
| | | | L [y8] - 984.5877+[17] | 19180 |
| beta-2-glycoprotein 1 | K.FICPLTGLWPINTLK. C | 591.663 8+++ | P [y6] - 685.4243+[1] | 541745 |
| APOH_HUMAN | | | P [y6] - 343.2158++[2] | 234580 |
| | | | G [b7] - 789.3964+[3] | 99108 |
| | | | W [y7] - 871.5036+[4] | 89126 |
| | | | L [b8] - 902.4804+[5] | 68306 |
| | | | C [b3] - 421.1904+[6] | 58396 |
| | | | N [y4] - 475.2875+[7] | 54474 |
| | | | I [y5] - 588.3715+[8] | 54403 |
| | | | W [y7] - 436.2554++[9] | 44706 |
| | | | I [b2] - 261.1598+[10] | 40214 |
| | | | T [y3] - 361.2445+[11] | 20535 |
| beta-2-glycoprotein 1 | R.VCPFAGILENGAVR. Y | 751.892 8++ | P [y12] - 622.3433++[1] | 431648 |
| APOH_HUMAN | | | C [b2] - 260.1063+[2] | 223667 |
| | | | P [y12] - 1243.6793+[3] | 134827 |
| | | | G [y9] - 928.5211+[4] | 89980 |
| | | | L [y7] - 758.4155+[5] | 85773 |
| | | | A [y10] - 999.5582+[6] | 69303 |
| | | | A [b5] - 575.2646+[7] | 47913 |
| | | | E [y6] - 645.3315+[8] | 44705 |
| | | | N [y5] - 516.2889+[9] | 23244 |
| | | | I [y8] - 871.4996+[10] | 20320 |
| | | | G [y4] - 402.2459+[11] | 19180 |
| | | | I [b7] - 745.3702+[12] | 18966 |
| | | | F [b4] - 504.2275+[13] | 16399 |
| beta-2-glycoprotein 1 | R.VCPFAGILENGAVR. Y | 501.597 7+++ | E [y6] - 645.3315+[1] | 131191 |
| APOH_HUMAN | | | N [y5] - 516.2889+[2] | 130264 |
| | | | I [b7] - 745.3702+[3] | 112154 |
| | | | G [b6] - 632.2861+[4] | 102743 |
| | | | G [y4] - 402.2459+[5] | 82779 |
| | | | C [b2] - 260.1063+[6] | 65453 |
| | | | L [y7] - 758.4155+[7] | 54330 |
| | | | I [b7] - 373.1887++[8] | 39143 |
| | | | L [y7] - 379.7114++[9] | 29661 |
| | | | V [y2] - 274.1874+[10] | 28377 |
| | | | P [y12] - 622.3433++[11] | 28163 |
| beta-2-glycoprotein 1 | K.CTEEGK.W | 362.152 5++ | E [y3] - 333.1769+[1] | 59464 |
| APOH_HUMAN | | | E [b3] - 391.1282+[2] | 21675 |
| beta-2-glycoprotein 1 | K.WSPELPVCAPIICPPP SIPTFATLR.V | 940.492 3+++ | P [y12] - 648.8692++[1] | 294510 |
| APOH_HUMAN | | | P [y11] - 600.3428++[2] | 206026 |
| | | | P [y7] - 805.4567+[3] | 122891 |
| | | | P [y10] - 1102.6255+[4] | 75113 |
| | | | L [b5] - 613.2980+[5] | 74578 |
| | | | P [y11] - 1199.6783+[6] | 72855 |
| | | | A [b9] - 1040.4870+[7] | 28643 |
| | | | T [y3] - 195.1290++[8] | 28524 |
| | | | S [b2] - 274.1186+[9] | 23770 |
| | | | P [y10] - 551.8164++[10] | 22284 |
| | | | C [y13] - 728.8845++[11] | 20918 |
| | | | E [b4] - 500.2140+[12] | 17114 |
| beta-2-glycoprotein 1 | K.ATFGCHDGYSLDGP EEIECTK.L | 796.003 6+++ | P [y8] - 503.2315++[1] | 67031 |
| APOH_HUMAN | | | E [y4] - 537.2337+[2] | 59841 |
| | | | C [b5] - 537.2126+[3] | 56454 |
| | | | I [y5] - 650.3178+[4] | 55384 |
| | | | C [y3] - 408.1911+[5] | 46946 |
| | | | E [y6] - 779.3604+[6] | 45282 |
| | | | T [b2] - 173.0921+[7] | 37675 |
| | | | G [y9] - 1062.4772+[8] | 36843 |
| | | | C [y17] - 1005.4144++[9] | 35774 |
| | | | P [y8] - 1005.4557+[10] | 33991 |
| | | | D [y10] - 1177.5041+[11] | 30366 |
| | | | E [y7] - 908.4030+[12] | 26503 |
| | | | T [y2] - 248.1605+[13] | 24840 |
| | | | Y [b9] - 1009.3832+[14] | 19491 |
| | | | G [y9] - 531.7422++[15] | 17946 |
| | | | S [b10] - 1096.4153+[16] | 17352 |
| beta-2-glycoprotein 1 | K.ATVVYQGER.V | 511.766 9++ | Y [y5] - 652.3049+[1] | 762897 |
| APOH_HUMAN | | | V [y6] - 751.3733+[2] | 548908 |
| | | | T [b2] - 173.0921+[3] | 252556 |
| | | | V [y7] - 850.4417+[4] | 231995 |
| | | | V [b3] - 272.1605+[5] | 223140 |
| | | | Q [y4] - 489.2416+[6] | 165023 |
| | | | G [y3] - 361.1830+[7] | 135013 |
| | | | V [b4] - 371.2289+[8] | 86760 |
| | | | V [y7] - 425.7245++[9] | 54314 |
| beta-2-glycoprotein 1 | K.VSFFCK.N | 394.194 0++ | S [y5] - 688.3123+[1] | 384559 |
| APOH_HUMAN | | | F [y4] - 601.2803+[2] | 321951 |
| | | | C [y2] - 307.1435+[3] | 265521 |
| | | | S [b2] - 187.1077+[4] | 237662 |
| | | | F [y3] - 454.2119+[5] | 168104 |
| beta-2-glycoprotein 1 | K.CSYTEDAQCIDGTIE VPK.C | 1043.45 88++ | P [y2] - 244.1656+[1] | 34574 |
| APOH_HUMAN | | | V [y3] - 343.2340+[2] | 9173 |
| | | | E [y4] - 472.2766+[3] | 7291 |
| | | | Y [b3] - 411.1333+[4] | 6233 |
| beta-2-glycoprotein 1 | K.CSYTEDAQCIDGTIE VPK.C | 695.975 0+++ | D [b11] - 672.2476++[1] | 37044 |
| APOH_HUMAN | | | D [y8] - 858.4567+[2] | 18816 |
| | | | D [b6] - 756.2505+[3] | 12289 |
| | | | V [y3] - 343.2340+[4] | 11348 |
| | | | A [b7] - 414.1474++[5] | 9761 |
| | | | G [y7] - 743.4298+[6] | 8644 |
| beta-2-glycoprotein 1 | K.EHSSLAFWK.T | 552.777 3++ | H [b2] - 267.1088+[1] | 237907 |
| APOH_HUMAN | | | S [y7] - 838.4458+[2] | 200568 |
| | | | W [y2] - 333.1921+[3] | 101078 |
| | | | S [y6] - 751.4137+[4] | 54920 |
| | | | A [y4] - 551.2976+[5] | 52920 |
| | | | F [y3] - 480.2605+[6] | 40102 |
| | | | L [y5] - 664.3817+[7] | 30341 |
| | | | F [b7] - 772.3624+[8] | 27871 |
| | | | S [b3] - 354.1408+[9] | 27754 |
| | | | A [b6] - 625.2940+[10] | 25931 |
| beta-2-glycoprotein 1 | K.TDASDVKPC.- | 496.721 3++ | D [b2] - 217.0819+[1] | 323810 |
| APOH_HUMAN | | | P [y2] - 276.1013+[2] | 119128 |
| | | | A [y7] - 776.3607+[3] | 86083 |
| | | | S [y6] - 705.3236+[4] | 79262 |
| | | | A [b3] - 288.1190+[5] | 77498 |
| | | | D [y5] - 618.2916+[6] | 70501 |
| | | | K [y3] - 404.1962+[7] | 55801 |
| | | | V [y4] - 503.2646+[8] | 46217 |
| transforming growth factor-beta-induced protein ig-h3 | K.SPYQLVLQHSR.L | 443.242 1+++ | Y [y9] - 572.3171++[1] | 560916 |
| BGH3_HUMAN | | | P [b2] - 185.0921+[2] | 413241 |
| | | | H [y3] - 399.2099+[3] | 320572 |
| | | | L [y5] - 640.3525+[4] | 313309 |
| | | | Q [y4] - 527.2685+[5] | 244398 |
| | | | L [y7] - 426.7561++[6] | 215854 |
| | | | V [y6] - 739.4209+[7] | 172897 |
| | | | L [y7] - 852.5050+[8] | 164959 |
| | | | Q [y8] - 490.7854++[9] | 149814 |
| | | | L [y5] - 320.6799++[10] | 127463 |
| | | | L [b5] - 589.2980+[11] | 118061 |
| | | | S [y2] - 262.1510+[12] | 110123 |
| | | | V [y6] - 370.2141++[13] | 97399 |
| | | | P [y10] - 620.8435++[14] | 94640 |
| | | | V [b6] - 688.3665+[15] | 87772 |
| | | | Q [b4] - 476.2140+[16] | 74203 |
| | | | Y [b3] - 348.1554+[17] | 65984 |
| | | | H [y3] - 200.1086++[18] | 55624 |
| | | | Q [y4] - 264.1379++[19] | 41606 |
| | | | L [b7] - 801.4505+[20] | 18241 |
| | | | V [b6] - 344.6869++[21] | 17678 |
| | | | L [b7] - 401.2289++[22] | 14976 |
| transforming growth factor-beta-induced protein ig-h3 | R.VLTDELK.H | 409.2 369++ | T [y5] - 605.3141+[1] | 937957 |
| BGH3_HUMAN | | | L [b2] - 213.1598+[2] | 298671 |
| | | | L [y6] - 718.3981+[3] | 244116 |
| | | | L [y2] - 260.1969+[4] | 135739 |
| | | | D [y4] - 504.2664+[5] | 52472 |
| | | | E [y3] - 389.2395+[6] | 50839 |
| transforming growth factor-beta-induced protein ig-h3 | K.VISTITNNIQQIIEIED TFETLR.A | 897.4 798+++ | E [y8] - 1010.4789+[1] | 282865 |
| BGH3_HUMAN | | | D [y7] - 881.4363+[2] | 237234 |
| | | | I [y9] - 1123.5630+[3] | 195581 |
| | | | T [y6] - 766.4094+[4] | 186875 |
| | | | I [b2] - 213.1598+[5] | 174492 |
| | | | T [y3] - 389.2507+[6] | 145598 |
| | | | F [y5] - 665.3617+[7] | 143872 |
| | | | E [y4] - 518.2933+[8] | 108148 |
| | | | Q [b11] - 606.8328++[9] | 106647 |
| | | | I [b5] - 514.3235+[10] | 82030 |
| | | | N [b8] - 843.4571+[11] | 75125 |
| | | | T [b4] - 401.2395+[12] | 71448 |
| | | | I [b12] - 663.3748++[13] | 58314 |
| | | | N [b7] - 365.2107++[14] | 54862 |
| | | | I [b9] - 956.5411+[15] | 51034 |
| | | | L [y2] - 288.2030+[16] | 50734 |
| | | | S [b3] - 300.1918+[17] | 48708 |
| | | | Q [b10] - 542.8035++[18] | 43754 |
| | | | Q [b11] - 1212.6583+[19] | 37375 |
| | | | T [b6] - 615.3712+[20] | 33322 |
| | | | I [b9] - 478.7742++[21] | 29570 |
| | | | Q [b10] - 1084.5997+[22] | 25817 |
| | | | T [y6] - 383.7083++[23] | 17187 |
| | | | N [b8] - 422.2322++[24] | 17111 |
| | | | I [b13] - 719.9168++[25] | 16661 |
| transforming growth factor-beta-induced protein ig-h3 | K.IPSETLNR.I | 465.2 562++ | S [y6] - 719.3682+[1] | 326570 |
| BGH3_HUMAN | | | P [y7] - 816.4210+[2] | 168951 |
| | | | E [y5] - 632.3362+[3] | 102452 |
| | | | P [b2] - 211.1441+[4] | 85885 |
| | | | T [y4] - 503.2936+[5] | 67650 |
| | | | L [y3] - 402.2459+[6] | 20939 |
| | | | N [y2] - 289.1619+[7] | 13979 |
| transforming growth factor-beta-induced protein ig-h3 | R.ILGDPEALR.D | 492.2 796++ | P [y5] - 585.3355+[1] | 1431619 |
| BGH3_HUMAN | | | G [y7] - 757.3839+[2] | 1066060 |
| | | | L [b2] - 227.1754+[3] | 742225 |
| | | | L [y8] - 870.4680+[4] | 254257 |
| | | | D [b4] - 399.2238+[5] | 159932 |
| | | | G [b3] - 284.1969+[6] | 66816 |
| | | | D [y6] - 700.3624+[7] | 65780 |
| | | | A [y3] - 359.2401+[8] | 62730 |
| | | | E [y4] - 488.2827+[9] | 23711 |
| | | | L [y2] - 288.2030+[10] | 16344 |
| transforming growth factor-beta-induced protein ig-h3 | R.DLLNNHILK.S | 360.5 451+++ | L [y7] - 426.2585++[1] | 1488651 |
| BGH3_HUMAN | | | L [b2] - 229.1183+[2] | 591961 |
| | | | N [y6] - 369.7165++[3] | 366710 |
| | | | N [y5] - 624.3828+[4] | 103993 |
| | | | L [y2] - 260.1969+[5] | 75103 |
| | | | N [b4] - 228.6263++[6] | 66125 |
| | | | N [y6] - 738.4257+[7] | 49493 |
| | | | H [y4] - 510.3398+[8] | 43681 |
| | | | N [y5] - 312.6950++[9] | 41551 |
| | | | I [y3] - 373.2809+[10] | 40285 |
| | | | L [b3] - 342.2023+[11] | 33494 |
| | | | L [y8] - 482.8006++[12] | 33034 |
| transforming growth factor-beta-induced protein ig-h3 | K.AIISNK.D | 323.2 001++ | I [y4] - 461.2718+[1] | 99850 |
| BGH3_HUMAN | | | I [b2] - 185.1285+[2] | 43105 |
| | | | S [y3] - 348.1878+[3] | 39192 |
| | | | N [y2] - 261.1557+[4] | 24516 |
| transforming growth factor-beta-induced protein ig-h3 | K.DILATNGVIHYIDELLI PDSAK.T | 804.1 003+++ | P [y5] - 517.2617+[1] | 400251 |
| BGH3_HUMAN | | | I [b2] - 229.1183+[2] | 306709 |
| | | | L [b3] - 342.2023+[3] | 147923 |
| | | | I [y6] - 630.3457+[4] | 91265 |
| | | | S [y3] - 305.1819+[5] | 61472 |
| | | | L [y7] - 743.4298+[6] | 57894 |
| | | | A [b4] - 413.2395+[7] | 52430 |
| | | | H [y13] - 757.3985++[8] | 30183 |
| | | | G [y16] - 891.9855++[9] | 27711 |
| | | | D [y10] - 1100.5834+[10] | 24979 |
| | | | A [y19] - 1035.0493++[11] | 23223 |
| | | | L [y8] - 856.5138+[12] | 22507 |
| | | | L [y20] - 1091.5913++[13] | 16783 |
| transforming growth factor-beta-induced protein ig-h3 | K.TLFELAAESDVSTAID LFR.Q | 1049. 5388++ | D [y4] - 550.2984+[1] | 64464 |
| BGH3_HUMAN | | | S [y8] - 922.4993+[2] | 47291 |
| | | | S [y11] - 1223.6266+[3] | 44234 |
| | | | A [b6] - 675.3712+[4] | 35972 |
| | | | L [b5] - 604.3341+[5] | 34997 |
| | | | A [b7] - 746.4083+[6] | 33045 |
| | | | E [b4] - 491.2500+[7] | 31744 |
| | | | D [y10] - 1136.5946+[8] | 30183 |
| | | | E [b8] - 875.4509+[9] | 26475 |
| | | | F [y2] - 322.1874+[10] | 25044 |
| | | | T [y7] - 835.4672+[11] | 21596 |
| | | | I [y5] - 663.3824+[12] | 21011 |
| | | | L [y3] - 435.2714+[13] | 20295 |
| | | | L [b2] - 215.1390+[14] | 20295 |
| | | | V [y9] - 1021.5677+[15] | 18929 |
| | | | A [y6] - 734.4196+[16] | 17694 |
| | | | F [b3] - 362.2074+[17] | 14441 |
| transforming growth factor-beta-induced protein ig-h3 | R.QAGLGNHLSGSER.L | 442.5 567+++ | G [y9] - 478.7309++[1] | 180677 |
| BGH3_HUMAN | | | L [y10] - 535.2729++[2] | 147807 |
| | | | S [y5] - 535.2471+[3] | 129825 |
| | | | G [y11] - 563.7836++[4] | 84584 |
| | | | L [y6] - 648.3311+[5] | 51642 |
| | | | A [b2] - 200.1030+[6] | 26469 |
| | | | G [y4] - 448.2150+[7] | 26397 |
| | | | H [y7] - 393.1987++[8] | 25390 |
| | | | A [y12] - 599.3022++[9] | 21434 |
| | | | N [y8] - 450.2201++[10] | 19276 |
| transforming growth factor-beta-induced protein ig-h3 | R.LTLLAPLNSVFK.D | 658.4 028++ | P [y7] - 804.4614+[1] | 1635673 |
| BGH3_HUMAN | | | A [y8] - 875.4985+[2] | 869779 |
| | | | L [b3] - 328.2231+[3] | 516429 |
| | | | T [b2] - 215.1390+[4] | 415472 |
| | | | L [y9] - 988.5826+[5] | 334225 |
| | | | L [b4] - 441.3071+[6] | 209200 |
| | | | L [y10] - 1101.6667+[7] | 174268 |
| | | | A [b5] - 512.3443+[8] | 160217 |
| | | | A [y8] - 438.2529++[9] | 83264 |
| | | | N [y5] - 594.3246+[10] | 54512 |
| | | | F [y2] - 294.1812+[11] | 51649 |
| | | | L [y9] - 494.7949++[12] | 34541 |
| | | | L [y6] - 707.4087+[13] | 34086 |
| | | | S [y4] - 480.2817+[14] | 30053 |
| | | | T [y11] - 1202.7143+[15] | 16653 |
| transforming growth factor-beta-induced protein ig-h3 | K.DGTPPIDAHTR.N | 393.8 633+++ | P [y8] - 453.7432++[1] | 355240 |
| BGH3_HUMAN | | | P [y7] - 405.2169++[2] | 88181 |
| | | | T [b3] - 274.1034+[3] | 81204 |
| | | | G [b2] - 173.0557+[4] | 40062 |
| | | | D [y5] - 599.2896+[5] | 37689 |
| | | | A [y4] - 242.6350++[6] | 29633 |
| | | | P [y7] - 809.4264+[7] | 22153 |
| | | | I [y6] - 712.3737+[8] | 16327 |
| transforming growth factor-beta-induced protein ig-h3 | K.YLYHGQTLETLGGK. K | 527.2 753+++ | E [y6] - 604.3301+[1] | 483222 |
| BGH3_HUMAN | | | Y [y12] - 652.3357++[2] | 264640 |
| | | | T [y5] - 475.2875+[3] | 239600 |
| | | | G [y3] - 261.1557+[4] | 206272 |
| | | | L [b2] - 277.1547+[5] | 134992 |
| | | | L [y13] - 708.8777++[6] | 119379 |
| | | | T [b7] - 863.4046+[7] | 104307 |
| | | | L [y4] - 374.2398+[8] | 100344 |
| | | | H [y11] - 570.8040++[9] | 93318 |
| | | | L [y7] - 717.4141+[10] | 91276 |
| | | | G [b13] - 717.3566++[11] | 80707 |
| | | | T [y8] - 818.4618+[12] | 57888 |
| | | | Q [b6] - 762.3570+[13] | 54766 |
| | | | G [y10] - 1003.5419+[14] | 51523 |
| | | | T [b7] - 432.2060++[15] | 49121 |
| | | | G [y2] - 204.1343+[16] | 45518 |
| | | | T [y8] - 409.7345++[17] | 44437 |
| | | | L [y7] - 359.2107++[18] | 33028 |
| | | | T [b10] - 603.7931++[19] | 26902 |
| | | | G [b5] - 634.2984+[20] | 21858 |
| | | | Q [b6] - 381.6821++[21] | 17595 |
| | | | H [b4] - 577.2769+[22] | 16093 |
| | | | L [b8] - 488.7480++[23] | 15133 |
| | | | T [y5] - 238.1474++[24] | 15013 |
| | | | E [b9] - 553.2693++[25] | 12370 |
| transforming growth factor-beta-induced protein ig-h3 | R.EGVYTVFAPTNEAFR .A | 850.9 176++ | P [y7] - 834.4104+[1] | 364143 |
| BGH3_HUMAN | | | F [y9] - 1052.5160+[2] | 269144 |
| | | | A [y8] - 905.4476+[3] | 176007 |
| | | | V [b3] - 286.1397+[4] | 107490 |
| | | | V [y10] - 1151.5844+[5] | 74822 |
| | | | T [b5] - 550.2508+[6] | 47560 |
| | | | V [b6] - 649.3192+[7] | 45398 |
| | | | G [b2] - 187.0713+[8] | 43056 |
| | | | Y [b4] - 449.2031+[9] | 33148 |
| | | | F [b7] - 796.3876+[10] | 24440 |
| | | | A [b8] - 867.4247+[11] | 24020 |
| | | | E [y4] - 522.2671+[12] | 17174 |
| | | | A [y3] - 393.2245+[13] | 14712 |
| | | | F [y2] - 322.1874+[14] | 12611 |
| transforming growth factor-beta-induced protein ig-h3 | R.LLGDAK.E | 308.6 869++ | A [y2] - 218.1499+[1] | 206606 |
| BGH3_HUMAN | | | G [y4] - 390.1983+[2] | 204445 |
| | | | L [y5] - 503.2824+[3] | 117829 |
| | | | L [b2] - 227.1754+[4] | 43998 |
| transforming growth factor-beta-induced protein ig-h3 | K.ELANILK.Y | 400.7 475++ | A [y5] - 558.3610+[1] | 963502 |
| BGH3_HUMAN | | | L [y2] - 260.1969+[2] | 583986 |
| | | | N [y4] - 487.3239+[3] | 326252 |
| | | | I [y3] - 373.2809+[4] | 302352 |
| | | | I [b5] - 541.2980+[5] | 179670 |
| | | | L [b2] - 243.1339+[6] | 74642 |
| | | | L [y6] - 671.4450+[7] | 38792 |
| | | | N [b4] - 428.2140+[8] | 14952 |
| transforming growth factor-beta-induced protein ig-h3 | K.YHIGDEILVSGGIGAL VR.L | 935.0 151++ | H [b2] - 301.1295+[1] | 24601 |
| BGH3_HUMAN | | | S [y9] - 829.4890+[2] | 15456 |
| transforming growth factor-beta-induced protein ig-h3 | K.YHIGDEILVSGGIGAL VR.L | 623.6 791+++ | S [y9] - 829.4890+[1] | 917445 |
| BGH3_HUMAN | | | G [y5] - 515.3300+[2] | 654048 |
| | | | I [b7] - 828.3886+[3] | 553713 |
| | | | G [y8] - 742.4570+[4] | 467481 |
| | | | L [b8] - 941.4727+[5] | 322194 |
| | | | G [y7] - 685.4355+[6] | 228428 |
| | | | E [b6] - 715.3046+[7] | 199383 |
| | | | V [y10] - 928.5574+[8] | 141616 |
| | | | G [b4] - 471.2350+[9] | 126224 |
| | | | L [b8] - 471.2400++[10] | 117080 |
| | | | H [b2] - 301.1295+[11] | 107162 |
| | | | I [y6] - 628.4141+[12] | 105488 |
| | | | A [y4] - 458.3085+[13] | 103491 |
| | | | L [y3] - 387.2714+[14] | 73094 |
| | | | I [b3] - 414.2136+[15] | 72515 |
| | | | S [y9] - 415.2482++[16] | 65044 |
| | | | V [b9] - 1040.5411+[17] | 61760 |
| | | | V [y2] - 274.1874+[19] | 56093 |
| | | | I [b7] - 414.6980++[18] | 56093 |
| | | | V [b9] - 520.7742++[20] | 39413 |
| | | | L [y11] - 1041.6415+[21] | 38962 |
| | | | D [b5] - 586.2620+[22] | 36257 |
| | | | S [b10] - 564.2902++[23] | 32329 |
| | | | I [y6] - 314.7107++[24] | 30526 |
| | | | A [b15] - 741.8830++[25] | 27692 |
| | | | V [y10] - 464.7824++[26] | 26340 |
| | | | L [y11] - 521.3244++[27] | 20415 |
| | | | G [b12] - 621.3117++[28] | 18612 |
| | | | G [b12] - 1241.6161+[29] | 13073 |
| transforming growth factor-beta-induced protein ig-h3 | K.LEVSLK.N | 344.7 156++ | V [y4] - 446.2973+[1] | 120860 |
| BGH3_HUMAN | | | E [y5] - 575.3399+[2] | 82786 |
| | | | E [b2] - 243.1339+[3] | 76794 |
| | | | S [y3] - 347.2289+[4] | 36335 |
| | | | L [y2] - 260.1969+[5] | 24932 |
| transforming growth factor-beta-induced protein ig-h3 | K.NNVVSVNK.E | 437.2 431++ | V [y5] - 546.3246+[1] | 17073 |
| BGH3_HUMAN | | | N [b2] - 229.0931+[2] | 14045 |
| transforming growth factor-beta-induced protein ig-h3 | R.GDELADSALEIFK.Q | 704.3 537++ | E [b3] - 302.0983+[1] | 687754 |
| BGH3_HUMAN | | | A [y9] - 993.5251+[2] | 431716 |
| | | | D [y8] - 922.4880+[3] | 368670 |
| | | | D [b2] - 173.0557+[4] | 358545 |
| | | | F [y2] - 294.1812+[5] | 200930 |
| | | | L [b4] - 415.1823+[6] | 197364 |
| | | | S [y7] - 807.4611+[7] | 187412 |
| | | | I [y3] - 407.2653+[8] | 129601 |
| | | | A [b5] - 486.2195+[9] | 121605 |
| | | | E [y4] - 536.3079+[10] | 108432 |
| | | | A [y6] - 720.4291+[11] | 107627 |
| | | | L [y5] - 649.3919+[12] | 95662 |
| | | | L [y10] - 1106.6092+[13] | 79325 |
| | | | D [b6] - 601.2464+[14] | 42625 |
| | | | A [b8] - 759.3155+[15] | 28647 |
| | | | S [b7] - 688.2784+[16] | 20709 |
| transforming growth factor-beta-induced protein ig-h3 | K.QASAFSR.A | 383.6 958++ | F [y3] - 409.2194+[1] | 64604 |
| BGH3_HUMAN | | | S [y5] - 567.2885+[2] | 60496 |
| | | | S [y2] - 262.1510+[3] | 42825 |
| | | | A [y4] - 480.2565+[4] | 25211 |
| transforming growth factor-beta-induced protein ig-h3 | R.LAPVYQK.L | 409.7 422++ | P [y5] - 634.3559+[1] | 416225 |
| BGH3_HUMAN | | | Y [y3] - 438.2347+[2] | 171715 |
| | | | V [y4] - 537.3031+[3] | 98187 |
| | | | Q [y2] - 275.1714+[4] | 42056 |
| | | | A [y6] - 705.3930+[5] | 32429 |
| ceruloplasmin | K.LISVDTEHSNIYLQNG PDR.I | 724.362 4+++ | I [b2] - 227.1754+[1] | 168111 |
| CERU_HUMAN | | | N [y5] - 558.2630+[2] | 87133 |
| | | | G [y4] - 444.2201+[3] | 86682 |
| | | | L [y7] - 799.4057+[4] | 84956 |
| | | | Q [y6] - 686.3216+[5] | 79928 |
| | | | Y [y8] - 962.4690+[6] | 64167 |
| | | | S [b3] - 314.2074+[7] | 39476 |
| | | | N [y10] - 1189.5960+[8] | 24691 |
| | | | P [y3] - 387.1987+[9] | 22065 |
| | | | I [y18] - 1029.4980++[10] | 20714 |
| | | | N [b10] - 1096.5269+[11] | 18087 |
| | | | I [y9] - 1075.5531+[12] | 15460 |
| ceruloplasmin | K.ALYLQYTDETFR.T | 760.375 0++ | Y [b3] - 348.1918+[1] | 681082 |
| CERU_HUMAN | | | Y [y7] - 931.4156+[2] | 405797 |
| | | | Q [y8] - 1059.4742+[3] | 343430 |
| | | | T [y6] - 768.3523+[4] | 279638 |
| | | | L [b2] - 185.1285+[5] | 229654 |
| | | | L [y9] - 1172.5582+[6] | 164660 |
| | | | L [b4] - 461.2758+[7] | 142145 |
| | | | D [y5] - 667.3046+[8] | 107547 |
| | | | Y [y10] - 668.3144++[9] | 91862 |
| | | | E [y4] - 552.2776+[10] | 76852 |
| | | | Q [b5] - 589.3344+[11] | 75200 |
| | | | T [y3] - 423.2350+[12] | 64168 |
| | | | F [y2] - 322.1874+[13] | 47807 |
| | | | Y [b6] - 752.3978+[14] | 40377 |
| | | | L [y9] - 586.7828++[15] | 40227 |
| ceruloplasmin | R.TTIEKPVWLGFLGPII K.A | 956.569 0++ | E [b4] - 445.2293+[1] | 92012 |
| CERU_HUMAN | | | K [b5] - 573.3243+[2] | 45856 |
| | | | L [y9] - 957.6132+[3] | 32272 |
| | | | G [y8] - 844.5291+[4] | 29044 |
| | | | K [y13] - 734.4579++[5] | 26118 |
| | | | G [y5] - 527.3552+[6] | 24917 |
| | | | L [y6] - 640.4392+[7] | 19738 |
| | | | I [b3] - 316.1867+[8] | 18838 |
| | | | P [y4] - 470.3337+[9] | 18012 |
| | | | W [y10] - 1143.6925+[10] | 17412 |
| | | | I [y15] - 855.5213++[11] | 14785 |
| | | | V [b7] - 769.4454+[12] | 14710 |
| ceruloplasmin | R.TTIEKPVWLGFLGPII K.A | 638.048 4+++ | G [y8] - 844.5291+[1] | 1645779 |
| CERU_HUMAN | | | G [y5] - 527.3552+[2] | 1180842 |
| | | | L [y6] - 640.4392+[3] | 920117 |
| | | | T [b2] - 203.1026+[4] | 775570 |
| | | | F [y7] - 787.5076+[5] | 416229 |
| | | | P [y4] - 470.3337+[6] | 285341 |
| | | | W [b8] - 955.5247+[7] | 275960 |
| | | | I [y2] - 260.1969+[8] | 256597 |
| | | | V [b7] - 769.4454+[9] | 230104 |
| | | | E [b4] - 445.2293+[10] | 117754 |
| | | | W [b8] - 478.2660++[11] | 105521 |
| | | | P [y12] - 670.4105++[13] | 104020 |
| | | | P [b6] - 670.3770+[12] | 104020 |
| | | | G [b10] - 1125.6303+[14] | 93363 |
| | | | F [y7] - 394.2575++[15] | 76176 |
| | | | K [b5] - 573.3243+[16] | 63718 |
| | | | I [b3] - 316.1867+[17] | 52986 |
| | | | L [b9] - 1068.6088+[18] | 33548 |
| | | | I [y3] - 373.2809+[19] | 20864 |
| ceruloplasmin | K.VYVHLK.N | 379.731 6++ | V [y4] - 496.3242+[1] | 228979 |
| CERU_HUMAN | | | Y [y5] - 659.3875+[2] | 196857 |
| | | | H [y3] - 397.2558+[3] | 89610 |
| | | | Y [b2] - 263.1390+[4] | 88034 |
| | | | L [y2] - 260.1969+[5] | 85482 |
| | | | Y [y5] - 330.1974++[6] | 31821 |
| ceruloplasmin | R.!YHSH!DAPK.D | 590.809 1++ | H [y8] - 452.7354++[1] | 167209 |
| CERU_HUMAN | | | P [y2] - 244.1656+[2] | 84831 |
| | | | A [y3] - 315.2027+[3] | 78036 |
| | | | S [y7] - 767.4046+[4] | 75864 |
| | | | H [b3] - 414.2136+[5] | 67808 |
| | | | Y [y9] - 534.2671++[6] | 50296 |
| | | | H [y8] - 904.4635+[7] | 42801 |
| | | | D [b7] - 866.4155+[8] | 28721 |
| | | | H [y6] - 680.3726+[9] | 23817 |
| | | | A [b8] - 937.4526+[10] | 19964 |
| | | | D [y4] - 430.2296+[11] | 17653 |
| | | | Y [b2] - 277.1547+[12] | 16742 |
| ceruloplasmin | R.!YHSH!DAPK.D | 394.208 5+++ | H [y8] - 452.7354++[1] | 402227 |
| CERU_HUMAN | | | Y [y9] - 534.2671++[2] | 305348 |
| | | | P [y2] - 244.1656+[5] | 101993 |
| | | | A [y3] - 315.2027+[3] | 97580 |
| | | | Y [b2] - 277.1547+[4] | 93377 |
| | | | D [y4] - 430.2296+[6] | 89734 |
| | | | S [y7] - 767.4046+[7] | 88263 |
| | | | S [y7] - 384.2060++[8] | 60663 |
| | | | I [y5] - 543.3137+[9] | 44692 |
| | | | H [y6] - 680.3726+[11] | 38528 |
| | | | A [b8] - 469.2300++[10] | 37547 |
| | | | H [b5] - 638.3045+[12] | 36146 |
| | | | H [b3] - 414.2136+[13] | 23467 |
| ceruloplasmin | R.HYYIAAEEIIWNYAPS GIDIFTK.E | 905.454 9+++ | P [y9] - 977.5302+[1] | 253794 |
| CERU_HUMAN | | | E [b8] - 977.4363+[2] | 233479 |
| | | | Y [b2] - 301.1295+[3] | 128823 |
| | | | I [b9] - 1090.5204+[4] | 103955 |
| | | | A [y10] - 1048.5673+[5] | 78247 |
| | | | P [y9] - 489.2687++[6] | 76005 |
| | | | E [b8] - 489.2218++[7] | 76005 |
| | | | I [b10] - 1203.6045+[8] | 56671 |
| | | | F [y3] - 395.2289+[9] | 49456 |
| | | | Y [b3] - 464.1928+[10] | 46864 |
| | | | E [b7] - 848.3937+[11] | 44622 |
| | | | A [b5] - 648.3140+[12] | 42451 |
| | | | A [b6] - 719.3511+[13] | 40629 |
| | | | I [b4] - 577.2769+[14] | 39999 |
| | | | D [y5] - 623.3399+[15] | 29631 |
| | | | I [y4] - 508.3130+[16] | 28581 |
| | | | T [y2] - 248.1605+[17] | 27040 |
| | | | I [b10] - 602.3059++[18] | 24448 |
| | | | Y [y11] - 1211.6307+[19] | 24238 |
| | | | G [y7] - 793.4454+[20] | 21926 |
| | | | W [b11] - 695.3455++[21] | 18704 |
| | | | S [y8] - 880.4775+[22] | 18633 |
| ceruloplasmin | R.IGGSYK.K | 312.671 2++ | G [y5] - 511.2511+[1] | 592392 |
| CERU_HUMAN | | | G [y4] - 454.2296+[2] | 89266 |
| | | | G [b2] - 171.1128+[3] | 71261 |
| | | | Y [y2] - 310.1761+[4] | 52498 |
| | | | S [y3] - 397.2082+[5] | 22364 |
| ceruloplasmin | R.EYTDASFTNR.K | 602.267 5++ | S [y5] - 624.3100+[1] | 163623 |
| CERU_HUMAN | | | F [y4] - 537.2780+[2] | 83580 |
| | | | T [y8] - 911.4217+[3] | 83391 |
| | | | A [y6] - 695.3471+[4] | 82886 |
| | | | D [y7] - 810.3741+[5] | 76315 |
| | | | T [y3] - 390.2096+[6] | 66018 |
| | | | Y [b2] - 293.1132+[7] | 50224 |
| | | | N [y2] - 289.1619+[8] | 29376 |
| ceruloplasmin | R.GPEEEHLGILGPVIW AEVGDTIR.V | 829.767 5+++ | A [y8] - 860.4472+[1] | 259776 |
| CERU_HUMAN | | | W [y9] - 1046.5265+[2] | 210032 |
| | | | E [y7] - 789.4101+[3] | 201448 |
| | | | G [y5] - 561.2991+[4] | 189809 |
| | | | V [y6] - 660.3675+[5] | 121142 |
| | | | T [y3] - 389.2507+[6] | 80306 |
| | | | P [b2] - 155.0815+[7] | 65806 |
| | | | V [b13] - 664.8459++[8] | 65676 |
| | | | G [b11] - 1132.5633+[9] | 64765 |
| | | | I [y10] - 1159.6106+[10] | 58783 |
| | | | L [b10] - 1075.5419+[11] | 56702 |
| | | | I [b9] - 962.4578+[12] | 54101 |
| | | | L [b7] - 792.3523+[13] | 48509 |
| | | | P [b12] - 615.3117++[14] | 37715 |
| | | | D [y4] - 504.2776+[15] | 34528 |
| | | | G [b8] - 849.3737+[16] | 34008 |
| | | | I [b14] - 721.3879++[17] | 23669 |
| | | | H [b6] - 679.2682+[18] | 22174 |
| | | | W [b15] - 814.4276++[19] | 21979 |
| | | | E [b3] - 284.1241+[20] | 18272 |
| | | | G [b11] - 566.7853++[21] | 17882 |
| | | | A [b16] - 849.9461++[22] | 15476 |
| ceruloplasmin | R.1ΓTFH N K. G | 373.203 2++ | T [y5] - 646.3307+[1] | 178952 |
| CERU_HUMAN | | | F [y4] - 545.2831+[2] | 175829 |
| | | | T [b2] - 201.1234+[3] | 127758 |
| | | | N [y2] - 261.1557+[4] | 107852 |
| | | | H [y3] - 398.2146+[5] | 103754 |
| ceruloplasmin | K.GAYPLSIEPIGVR.F | 686.385 2++ | S [y8] - 870.5043+[1] | 970541 |
| CERU_HUMAN | | | P [y5] - 541.3457+[2] | 966508 |
| | | | P [y10] - 1080.6412+[3] | 590391 |
| | | | E [y6] - 670.3883+[4] | 493076 |
| | | | I [y7] - 783.4723+[5] | 391013 |
| | | | Y [b3] - 292.1292+[6] | 265598 |
| | | | L [y9] - 983.5884+[7] | 217591 |
| | | | P [b4] - 389.1819+[8] | 188839 |
| | | | S [b6] - 589.2980+[9] | 95623 |
| | | | G [y3] - 331.2088+[10] | 85605 |
| | | | L [b5] - 502.2660+[11] | 76628 |
| | | | V [y2] - 274.1874+[12] | 52365 |
| | | | I [b7] - 702.3821+[13] | 39225 |
| | | | E [b8] - 831.4247+[14] | 26866 |
| ceruloplasmin | K.NNEGTYYSPNYNPQ SR.S | 952.413 9++ | P [y4] - 487.2623+[1] | 37339 |
| CERU_HUMAN | | | S [y9] - 1062.4963+[2] | 33696 |
| | | | P [y8] - 975.4643+[3] | 29467 |
| | | | N [y5] - 601.3052+[4] | 24068 |
| | | | N [b2] - 229.0931+[5] | 19060 |
| | | | Y [y10] - 1225.5596+[6] | 16718 |
| | | | E [b3] - 358.1357+[7] | 16523 |
| ceruloplasmin | R.SVPPSASHVAPTETF TYEWTVPK.E | 844.419 9+++ | P [y2] - 244.1656+[1] | 579331 |
| CERU_HUMAN | | | T [y8] - 1023.5146+[2] | 126817 |
| | | | W [y5] - 630.3610+[3] | 101524 |
| | | | V [y3] - 343.2340+[4] | 99970 |
| | | | Y [y7] - 922.4669+[5] | 95448 |
| | | | E [y6] - 759.4036+[6] | 88030 |
| | | | T [y4] - 444.2817+[7] | 55884 |
| | | | F [y9] - 1170.5830+[8] | 55743 |
| | | | V [b2] - 187.1077+[9] | 46982 |
| | | | P [y20] - 1124.5497++[10] | 37303 |
| | | | P [b3] - 284.1605+[11] | 21690 |
| | | | E [b18] - 951.4494++[12] | 18652 |
| | | | P [b4] - 381.2132+[13] | 16956 |
| | | | T [b14] - 681.3384++[14] | 15543 |
| ceruloplasmin | K.GSLHANGR.Q | 271.143 8+++ | L [y6] - 334.1854++[1] | 154779 |
| CERU_HUMAN | | | A [y4] - 417.2205+[2] | 41628 |
| | | | S [y7] - 377.7014++[3] | 35762 |
| | | | H [y5] - 277.6433++[4] | 29542 |
| ceruloplasmin | R.QSEDSTFYLGER.T | 716.323 0++ | G [y3] - 361.1830+[1] | 157040 |
| CERU_HUMAN | | | Y [y5] - 637.3304+[2] | 126155 |
| | | | F [y6] - 784.3988+[3] | 97814 |
| | | | L [y4] - 474.2671+[4] | 80146 |
| | | | T [y7] - 443.2269++[5] | 70746 |
| | | | T [y7] - 885.4465+[6] | 54844 |
| | | | S [y8] - 972.4785+[7] | 44101 |
| | | | S [b2] - 216.0979+[8] | 42193 |
| | | | D [y9] - 1087.5055+[9] | 36186 |
| | | | E [y10] - 1216.5481+[10] | 35055 |
| | | | E [b3] - 345.1405+[11] | 20778 |
| | | | E [y2] - 304.1615+[12] | 19153 |
| ceruloplasmin | R.TYYIAAVEVEWDYSP QR.E | 1045.49 69++ | P [y3] - 400.2303+[1] | 64887 |
| CERU_HUMAN | | | Y [b3] - 428.1816+[2] | 49716 |
| | | | S [y4] - 487.2623+[3] | 37369 |
| | | | Y [b2] - 265.1183+[4] | 35596 |
| | | | E [y8] - 1080.4745+[5] | 28569 |
| | | | W [y7] - 951.4319+[6] | 26204 |
| | | | V [b7] - 782.4083+[7] | 23577 |
| | | | A [b6] - 683.3399+[8] | 23512 |
| | | | V [y9] - 1179.5429+[10] | 22526 |
| | | | D [y6] - 765.3526+[9] | 22526 |
| | | | Y [y5] - 650.3257+[11] | 19965 |
| | | | A [b5] - 612.3028+[12] | 18520 |
| ceruloplasmin | K.ELHHLQEQNVSNAF LDK.G | 674.672 8+++ | N [y6] - 707.3723+[1] | 22715 |
| CERU_HUMAN | | | L [y3] - 188.1155++[2] | 21336 |
| | | | S [y7] - 794.4043+[3] | 10176 |
| ceruloplasmin | K.GEFYIGSK.Y | 450.726 7++ | E [b2] - 187.0713+[1] | 53262 |
| CERU_HUMAN | | | F [y6] - 714.3821+[2] | 50438 |
| | | | I [y4] - 404.2504+[3] | 39602 |
| | | | Y [y5] - 567.3137+[4] | 34020 |
| | | | G [y3] - 291.1663+[5] | 33100 |
| ceruloplasmin | R.QYTDSTFR.V | 509.235 4++ | T [y6] - 726.3417+[1] | 164056 |
| CERU_HUMAN | | | S [y4] - 510.2671+[2] | 155584 |
| | | | D [y5] - 625.2940+[3] | 136472 |
| | | | T [y3] - 423.2350+[4] | 54313 |
| | | | F [y2] - 322.1874+[5] | 47220 |
| | | | Y [b2] - 292.1292+[6] | 27846 |
| | | | Y [y7] - 889.4050+[7] | 16550 |
| ceruloplasmin | K.AEEEHLGILGPQLHA DVGDK.V | 710.027 2+++ | E [b2] - 201.0870+[1] | 60743 |
| CERU_HUMAN | | | V [y4] - 418.2296+[2] | 23296 |
| | | | E [y17] - 899.9759++[3] | 14619 |
| ceruloplasmin | K.LEFALLFLVFDENES WYLDDNIK.T | 945.137 2+++ | L [y6] - 359.1925++[1] | 19544 |
| CERU_HUMAN | | | L [b5] - 574.3235+[2] | 17902 |
| ceruloplasmin | K.TYSDHPEK.V | 488.722 2++ | S [y6] - 712.3260+[1] | 93810 |
| CERU_HUMAN | | | P [y3] - 373.2082+[2] | 43778 |
| | | | Y [b2] - 265.1183+[3] | 35960 |
| | | | H [y4] - 510.2671+[4] | 16651 |
| ceruloplasmin | K.TYSDHPEK.V | 326.150 5+++ | S [y6] - 356.6667++[1] | 539251 |
| CERU_HUMAN | | | Y [y7] - 438.1983++[2] | 180506 |
| | | | Y [b2] - 265.1183+[3] | 109445 |
| | | | P [y3] - 373.2082+[4] | 84742 |
| | | | H [y4] - 255.6372++[5] | 27596 |
| | | | P [y3] - 187.1077++[6] | 25016 |
| | | | D [y5] - 625.2940+[7] | 24000 |
| | | | H [y4] - 510.2671+[8] | 20795 |
| hepatocyte growth factor activator | R.YEYLEGGDR.W | 551.246 0++ | E [b2] - 293.1132+[1] | 229354 |
| HGFA_HUMAN | | | Y [y7] - 809.3788+[2] | 204587 |
| | | | L [y6] - 646.3155+[3] | 96740 |
| | | | Y [b3] - 456.1765+[4] | 54186 |
| | | | E [y8] - 938.4214+[5] | 22065 |
| hepatocyte growth factor activator | R.VQLSPDLLATLPEPA SPGR.Q | 981.038 7++ | P [y8] - 810.4104+[1] | 51109 |
| HGFA_HUMAN | | | Q [b2] - 228.1343+[2] | 19063 |
| hepatocyte growth factor activator | R.TTDVTQTFGIEK.Y | 670.340 6++ | D [b3] - 318.1296+[1] | 104844 |
| HGFA_HUMAN | | | T [y8] - 923.4833+[2] | 93287 |
| | | | T [b2] - 203.1026+[3] | 72498 |
| | | | D [y10] - 1137.5786+[4] | 53886 |
| | | | I [y3] - 389.2395+[5] | 53811 |
| | | | Q [y7] - 822.4356+[6] | 42253 |
| | | | V [b4] - 417.1980+[7] | 38726 |
| | | | T [y6] - 694.3770+[8] | 36474 |
| | | | F [y5] - 593.3293+[9] | 26793 |
| | | | E [y2] - 276.1554+[10] | 24616 |
| | | | G [y4] - 446.2609+[11] | 22215 |
| | | | V [y9] - 1022.5517+[12] | 20564 |
| hepatocyte growth factor activator | R.EALVPLVADHK.C | 596.340 2++ | P [y7] - 779.4410+[1] | 57992 |
| HGFA_HUMAN | | | L [b3] - 314.1710+[2] | 42740 |
| hepatocyte growth factor activator | R.EALVPLVADHK.C | 397.895 9+++ | P [y7] - 390.2241++[1] | 502380 |
| HGFA_HUMAN | | | V [y5] - 569.3042+[2] | 108586 |
| | | | V [y8] - 439.7584++[3] | 100001 |
| | | | H [y2] - 284.1717+[4] | 71234 |
| | | | L [y9] - 496.3004++[5] | 65572 |
| | | | A [y4] - 470.2358+[6] | 62284 |
| hepatocyte growth factor activator | R.LHKPGVYTR.V | 357.541 7+++ | P [y6] - 692.3726+[1] | 104812 |
| HGFA_HUMAN | | | H [y8] - 479.2669++[2] | 49302 |
| | | | K [y7] - 410.7374++[3] | 30859 |
| | | | Y [y3] - 439.2300+[4] | 23829 |
| hepatocyte growth factor activator | R.VANYVDWINDR.I | 682.833 3++ | D [y6] - 818.3791+[1] | 132314 |
| HGFA_HUMAN | | | V [y7] - 917.4476+[2] | 81805 |
| | | | N [b3] - 285.1557+[3] | 70622 |
| | | | W [y5] - 703.3522+[4] | 53586 |
| | | | N [y3] - 404.1888+[5] | 37675 |
| | | | A [b2] - 171.1128+[6] | 36474 |
| alpha-1-antichymotrypsin | R.GTHVDLGLASANVD FAFSLYK.Q | 1113. 0655++ | L [b6] - 623.3148+[1] | 244118 |
| AACT_HUMAN | | | L [b8] - 793.4203+[2] | 211429 |
| | | | H [b3] - 296.1353+[3] | 204581 |
| | | | D [b5] - 510.2307+[4] | 200032 |
| | | | S [y4] - 510.2922+[5] | 195904 |
| | | | V [b4] - 395.2037+[6] | 187415 |
| | | | A [b9] - 864.4574+[7] | 167905 |
| | | | G [b7] - 680.3362+[8] | 87564 |
| | | | Y [y2] - 310.1761+[9] | 74385 |
| | | | F [y7] - 875.4662+[10] | 50794 |
| | | | F [y5] - 657.3606+[11] | 44462 |
| | | | S [b10] - 951.4894+[12] | 43899 |
| | | | D [y8] - 990.4931+[13] | 39866 |
| | | | A [y6] - 728.3978+[14] | 33300 |
| | | | A [b11] - 1022.5265+[15] | 32502 |
| | | | L [y3] - 423.2602+[16] | 29829 |
| | | | V [y9] - 1089.5615+[17] | 22043 |
| | | | N [b12] - 1136.5695+[18] | 17353 |
| alpha-1-antichymotrypsin | R.GTHVDLGLASANVD FAFSLYK.Q | 742.3 794+++ | D [y8] - 990.4931+[1] | 830612 |
| AACT_HUMAN | | | L [b8] - 793.4203+[2] | 635646 |
| | | | G [b7] - 680.3362+[3] | 582273 |
| | | | S [y4] - 510.2922+[4] | 548645 |
| | | | D [b5] - 510.2307+[5] | 471071 |
| | | | F [y7] - 875.4662+[6] | 420278 |
| | | | A [b9] - 864.4574+[7] | 411366 |
| | | | A [y6] - 728.3978+[8] | 391668 |
| | | | Y [y2] - 310.1761+[9] | 390214 |
| | | | F [y5] - 657.3606+[10] | 358134 |
| | | | T [b2] - 159.0764+[11] | 288721 |
| | | | H [b3] - 296.1353+[12] | 251998 |
| | | | L [b6] - 623.3148+[13] | 240742 |
| | | | V [y9] - 1089.5615+[14] | 197218 |
| | | | V [b4] - 395.2037+[15] | 186055 |
| | | | L [y3] - 423.2602+[16] | 173673 |
| | | | S [b10] - 951.4894+[17] | 103651 |
| | | | N [b12] - 1136.5695+[18] | 97976 |
| | | | A [b11] - 1022.5265+[19] | 76448 |
| alpha-1-antichymotrypsin | K.FNLTETSEAEIHQSFQ HLLR.T | 800.7 363+++ | A [b9] - 993.4524+[1] | 75792 |
| AACT_HUMAN | | | L [b3] - 375.2027+[2] | 59001 |
| | | | H [y9] - 1165.6225+[3] | 57829 |
| | | | L [y2] - 288.2030+[4] | 55343 |
| | | | T [b4] - 476.2504+[5] | 19323 |
| alpha-1-antichymotrypsin | K.EQLSLLDR.F | 487.2 693++ | S [y5] - 603.3461+[1] | 4247034 |
| AACT_HUMAN | | | L [y3] - 403.2300+[2] | 2094711 |
| | | | L [y6] - 716.4301+[3] | 1465135 |
| | | | L [y4] - 516.3140+[4] | 1365427 |
| | | | Q [b2] - 258.1084+[5] | 1222196 |
| | | | D [y2] - 290.1459+[6] | 957403 |
| | | | L [b3] - 371.1925+[7] | 114810 |
| alpha-1-antichymotrypsin | K.EQLSLLDR.F | 325.1 819+++ | L [y3] - 403.2300+[1] | 57123 |
| AACT_HUMAN | | | D [y2] - 290.1459+[2] | 52105 |
| alpha-1-antichymotrypsin | K.YTGNASALFILPDQD K.M | 876.9 438++ | L [y9] - 1088.5986+[1] | 39933 |
| AACT_HUMAN | | | A [b5] - 507.2198+[2] | 20117 |
| | | | D [y4] - 505.2253+[3] | 19937 |
| alpha-1-antichymotrypsin | R.EIGELYLPK.F | 531.2 975++ | P [y2] - 244.1656+[1] | 8170395 |
| AACT_HUMAN | | | G [y7] - 819.4611+[2] | 3338199 |
| | | | L [y5] - 633.3970+[3] | 2616703 |
| | | | L [y3] - 357.2496+[4] | 1922561 |
| | | | Y [y4] - 520.3130+[5] | 1527792 |
| | | | G [b3] - 300.1554+[6] | 1417240 |
| | | | I [b2] - 243.1339+[7] | 1097654 |
| | | | E [y6] - 762.4396+[8] | 302412 |
| | | | E [b4] - 429.1980+[9] | 81633 |
| | | | Y [b6] - 705.3454+[10] | 36795 |
| | | | L [b5] - 542.2821+[11] | 31993 |
| alpha-1-antichymotrypsin | R.EIGELYLPK.F | 354.5 341+++ | P [y2] - 244.1656+[1] | 189758 |
| AACT_HUMAN | | | L [y3] - 357.2496+[2] | 86952 |
| | | | G [b3] - 300.1554+[3] | 49661 |
| | | | Y [y4] - 520.3130+[4] | 45518 |
| | | | E [b4] - 429.1980+[5] | 19576 |
| | | | I [b2] - 243.1339+[6] | 18375 |
| | | | L [b5] - 542.2821+[7] | 13091 |
| alpha-1-antichymotrypsin | R.DYNLNDILLQLGIEEA FTSK.A | 1148. 5890++ | G [y9] - 981.4888+[1] | 378153 |
| AACT_HUMAN | | | F [b17] - 981.4964++[2] | 378153 |
| | | | N [b3] - 393.1405+[3] | 338897 |
| | | | L [y10] - 1094.5728+[4] | 283255 |
| | | | E [y7] - 811.3832+[5] | 180253 |
| | | | I [b7] - 848.3785+[6] | 172510 |
| | | | T [y3] - 335.1925+[7] | 162966 |
| | | | D [b6] - 735.2944+[8] | 135235 |
| | | | L [b4] - 506.2245+[9] | 131573 |
| | | | A [y5] - 553.2980+[10] | 129232 |
| | | | F [y4] - 482.2609+[11] | 124490 |
| | | | Y [b2] - 279.0975+[12] | 115367 |
| | | | L [b9] - 1074.5466+[13] | 106363 |
| | | | L [b8] - 961.4625+[14] | 101621 |
| | | | E [y6] - 682.3406+[15] | 98740 |
| | | | S [y2] - 234.1448+[16] | 75991 |
| | | | N [b5] - 620.2675+[17] | 66387 |
| | | | I [y8] - 924.4673+[18] | 61465 |
| alpha-1-antichymotrypsin | R.DYNLNDILLQLGIEEA FTSK.A | 766.0 618+++ | G [y9] - 981.4888+[1] | 309485 |
| AACT_HUMAN | | | F [b17] - 981.4964++[2] | 309485 |
| | | | E [y7] - 811.3832+[3] | 262306 |
| | | | N [b3] - 393.1405+[4] | 212306 |
| | | | T [y3] - 335.1925+[5] | 199100 |
| | | | F [y4] - 482.2609+[6] | 164346 |
| | | | A [y5] - 553.2980+[7] | 161405 |
| | | | Y [b2] - 279.0975+[8] | 149220 |
| | | | E [y6] - 682.3406+[9] | 138836 |
| | | | L [y10] - 1094.5728+[10] | 137336 |
| | | | S [y2] - 234.1448+[11] | 134094 |
| | | | I [b7] - 848.3785+[12] | 80072 |
| | | | I [y8] - 924.4673+[13] | 77791 |
| | | | L [b4] - 506.2245+[14] | 70889 |
| | | | D [b6] - 735.2944+[15] | 64706 |
| | | | L [b8] - 961.4625+[16] | 51201 |
| | | | N [b5] - 620.2675+[17] | 42677 |
| | | | L [b9] - 1074.5466+[18] | 21609 |
| alpha-1-antichymotrypsin | K.ADLSGITGAR.N | 480.7 591++ | S [y7] - 661.3628+[1] | 4360743 |
| AACT_HUMAN | | | G [y6] - 574.3307+[2] | 3966462 |
| | | | T [y4] - 404.2252+[3] | 1937824 |
| | | | D [b2] - 187.0713+[4] | 799907 |
| | | | G [y3] - 303.1775+[5] | 647883 |
| | | | I [y5] - 517.3093+[6] | 612145 |
| | | | L [b3] - 300.1554+[7] | 606995 |
| | | | S [b4] - 387.1874+[8] | 544408 |
| | | | L [y8] - 774.4468+[9] | 348247 |
| | | | G [b5] - 444.2089+[10] | 232083 |
| | | | I [b6] - 557.2930+[11] | 132531 |
| | | | A [y2] - 246.1561+[12] | 113896 |
| alpha-1-antichymotrypsin | K.ADLSGITGAR.N | 320.8 418+++ | T [y4] - 404.2252+[1] | 218597 |
| AACT_HUMAN | | | G [y3] - 303.1775+[2] | 159381 |
| | | | G [b5] - 444.2089+[3] | 46527 |
| | | | A [y2] - 246.1561+[4] | 26911 |
| | | | D [b2] - 187.0713+[5] | 22497 |
| | | | S [b4] - 387.1874+[6] | 14589 |
| alpha-1-antichymotrypsin | R.NLAVSQVVHK.A | 547.8 195++ | L [b2] - 228.1343+[1] | 1872233 |
| AACT_HUMAN | | | A [y8] - 867.5047+[2] | 1133381 |
| | | | A [b3] - 299.1714+[3] | 1126331 |
| | | | V [y7] - 796.4676+[4] | 672341 |
| | | | S [y6] - 697.3991+[5] | 650028 |
| | | | H [y2] - 284.1717+[6] | 582720 |
| | | | V [y3] - 383.2401+[7] | 211547 |
| | | | V [b4] - 398.2398+[8] | 163917 |
| | | | Q [y5] - 610.3671+[9] | 100778 |
| | | | V [y4] - 482.3085+[10] | 88456 |
| | | | S [b5] - 485.2718+[11] | 64488 |
| | | | V [b7] - 712.3988+[12] | 36045 |
| alpha-1-antichymotrypsin | R.NLAVSQVVHK.A | 365.5 487+++ | L [b2] - 228.1343+[1] | 1175923 |
| AACT_HUMAN | | | V [y3] - 383.2401+[2] | 593693 |
| | | | S [y6] - 697.3991+[3] | 587502 |
| | | | H [y2] - 284.1717+[4] | 440259 |
| | | | V [y4] - 482.3085+[5] | 375955 |
| | | | Q [y5] - 610.3671+[6] | 349044 |
| | | | A [b3] - 299.1714+[7] | 339236 |
| | | | V [b4] - 398.2398+[8] | 172805 |
| | | | 5 [b5] - 485.2718+[9] | 84594 |
| alpha-1-antichymotrypsin | K.AVLDVFEEGTEASAA TAVK.I | 954.4 835++ | D [b4] - 399.2238+[1] | 1225699 |
| AACT_HUMAN | | | G [y11] - 1005.5211+[2] | 812780 |
| | | | V [b5] - 498.2922+[3] | 741243 |
| | | | E [y12] - 1134.5637+[4] | 651070 |
| | | | V [b2] - 171.1128+[5] | 634335 |
| | | | A [y8] - 718.4094+[6] | 416106 |
| | | | S [y7] - 647.3723+[7] | 360507 |
| | | | F [b6] - 645.3606+[8] | 293935 |
| | | | T [y4] - 418.2660+[9] | 281736 |
| | | | E [y9] - 847.4520+[10] | 247592 |
| | | | A [y3] - 317.2183+[11] | 246550 |
| | | | E [b7] - 774.4032+[12] | 234044 |
| | | | T [y10] - 948.4997+[13] | 221478 |
| | | | A [y6] - 560.3402+[14] | 212344 |
| | | | A [y5] - 489.3031+[15] | 195364 |
| | | | E [b8] - 903.4458+[16] | 183901 |
| | | | L [b3] - 284.1969+[17] | 176116 |
| | | | V [y2] - 246.1812+[18] | 157419 |
| | | | T [b10] - 1061.5150+[19] | 52841 |
| | | | E [b11] - 1190.5576+[20] | 34757 |
| | | | G [b9] - 960.4673+[21] | 25807 |
| alpha-1-antichymotrypsin | K.AVLDVFEEGTEASAA TAVK.I | 636.6 581+++ | V [b2] - 171.1128+[1] | 659591 |
| AACT_HUMAN | | | S [y7] - 647.3723+[2] | 630596 |
| | | | A [y8] - 718.4094+[3] | 509467 |
| | | | D [b4] - 399.2238+[4] | 353335 |
| | | | A [y6] - 560.3402+[5] | 306747 |
| | | | A [y5] - 489.3031+[6] | 280878 |
| | | | E [y9] - 847.4520+[7] | 247347 |
| | | | T [y4] - 418.2660+[8] | 197203 |
| | | | A [y3] - 317.2183+[9] | 128853 |
| | | | V [b5] - 498.2922+[10] | 120271 |
| | | | V [y2] - 246.1812+[11] | 115428 |
| | | | L [b3] - 284.1969+[12] | 102984 |
| | | | G [y11] - 1005.5211+[13] | 91215 |
| | | | F [b6] - 645.3606+[14] | 79016 |
| | | | E [y12] - 1134.5637+[15] | 72947 |
| | | | E [b7] - 774.4032+[16] | 58358 |
| | | | T [y10] - 948.4997+[17] | 41071 |
| | | | E [b8] - 903.4458+[18] | 32918 |
| | | | G [b9] - 960.4673+[19] | 24275 |
| alpha-1-antichymotrypsin | K.ITLLSALVETR.T | 608.3 690++ | S [y7] - 775.4308+[1] | 7387615 |
| AACT_HUMAN | | | T [b2] - 215.1390+[2] | 3498457 |
| | | | L [y8] - 888.5149+[3] | 2684639 |
| | | | L [b3] - 328.2231+[4] | 2164246 |
| | | | A [y6] - 688.3988+[5] | 2045853 |
| | | | L [y5] - 617.3617+[6] | 2027311 |
| | | | L [y9] - 1001.5990+[7] | 1949318 |
| | | | V [y4] - 504.2776+[8] | 1598519 |
| | | | T [y2] - 276.1666+[9] | 1416847 |
| | | | E [y3] - 405.2092+[10] | 967259 |
| | | | A [b6] - 599.3763+[11] | 579420 |
| | | | L [b4] - 441.3071+[12] | 431556 |
| | | | S [b5] - 528.3392+[13] | 107634 |
| | | | L [b7] - 712.4604+[14] | 71104 |
| | | | V [b8] - 811.5288+[15] | 24197 |
| alpha-1-antichymotrypsin | K.ITLLSALVETR.T | 405.9 151+++ | E [y3] - 405.2092+[1] | 738128 |
| AACT_HUMAN | | | T [y2] - 276.1666+[2] | 368830 |
| | | | V [y4] - 504.2776+[3] | 328133 |
| | | | A [b6] - 599.3763+[4] | 132469 |
| | | | T [b2] - 215.1390+[5] | 126898 |
| | | | L [y5] - 617.3617+[6] | 124559 |
| | | | S [y7] - 775.4308+[7] | 54263 |
| | | | L [b3] - 328.2231+[8] | 37891 |
| | | | A [y6] - 688.3988+[9] | 29853 |
| | | | L [b4] - 441.3071+[10] | 25558 |
| | | | L [b7] - 712.4604+[11] | 13353 |
| | | | S [b5] - 528.3392+[12] | 12290 |
| Pigment epithelium-derived factor | K.LAAAVSNFGYDLYR. V | 780.3 963++ | D [b11] - 1109.5262+[1] | 136227 |
| PEDF_HUMAN* | | | F [b8] - 774.4145+[2] | 61248 |
| | | | N [b7] - 314.1767++[3] | 55532 |
| | | | A [y12] - 1375.6641+[4] | 53268 |
| | | | V [b5] - 213.6392++[5] | 35818 |
| | | | L [b12] - 1222.6103+[6] | 34918 |
| | | | G [b9] - 831.4359+[7] | 33934 |
| | | | Y [b10] - 994.4993+[8] | 32923 |
| | | | G [b9] - 416.2216++[9] | 32650 |
| | | | V [b5] - 426.2711+[10] | 15646 |
| | | | A [b2] - 185.1285+[11] | 14964 |
| | | | D [b11] - 555.2667++[12] | 13922 |
| | | | L [y3] - 226.1368++[13] | 13027 |
| | | | A [b4] - 327.2027+[14] | 12782 |
| | | | A [y12] - 688.3357++[15] | 12446 |
| | | | V [y10] - 1233.5899+[16] | 12400 |
| | | | A [y11] - 652.8171++[17] | 10793 |
| Pigment epithelium-derived factor | K.LAAAVSNFGYDLYR. V | 520.5 999+++ | G [y6] - 786.3781+[1] | 42885 |
| PEDF_HUMAN* | | | D [y4] - 566.2933+[2] | 32080 |
| | | | Y [y5] - 729.3566+[3] | 17494 |
| | | | L [y3] - 451.2663+[5] | 12304 |
| | | | Y [y2] - 338.1823+[6] | 7780 |
| Pigment epithelium-derived factor | R.ALYYDLISSPDIHGTY K.E | 652.6 632+++ | Y [y15] - 886.4305++[1] | 12278 |
| PEDF_HUMAN* | | | L [b2] - 185.1285+[2] | 7601 |
| | | | S [y10] - 1104.5320+[3] | 7345 |
| | | | Y [y14] - 804.8988++[4] | 5976 |
| Pigment epithelium-derived factor | K.ELLDTVTAPQK.N | 607.8 350++ | T [y5] - 272.6581++[1] | 59670 |
| PEDF_HUMAN* | | | Q [y2] - 275.1714+[2] | 11954 |
| Pigment epithelium-derived factor | K.ELLDTVTAPQK.N | 405.5 591+++ | L [b2] - 243.1339+[1] | 16428 |
| PEDF_HUMAN* | | | T [b7] - 386.7080++[2] | 7918 |
| | | | Q [y2] - 275.1714+[3] | 7043 |
| | | | T [y5] - 272.6581++[4] | 5237 |
| Pigment epithelium-derived factor | K.SSFVAPLEK.S | 489.2 687++ | A [y5] - 557.3293+[1] | 20068 |
| PEDF_HUMAN* | | | A [y5] - 279.1683++[2] | 5059 |
| | | | S [b2] - 175.0713+[3] | 4883 |
| Pigment epithelium-derived factor | K.SSFVAPLEK.S | 326.5 149+++ | A [y5] - 279.1683++[1] | 70240 |
| PEDF_HUMAN* | | | A [y5] - 557.3293+[2] | 63329 |
| | | | S [b2] - 175.0713+[3] | 39662 |
| | | | L [b7] - 351.6947++[4] | 5393 |
| Pigment epithelium-derived factor | K.EIPDEISILLLGVAHFK .G | 632.0 277+++ | P [y15] - 826.4745++[1] | 37871 |
| PEDF_HUMAN* | | | G [y6] - 658.3671+[2] | 20077 |
| | | | L [y7] - 771.4512+[3] | 8952 |
| Pigment epithelium-derived factor | K.TSLEDFYLDEER.T | 758.8 437++ | R [y1] - 175.1190+[1] | 8206 |
| PEDF_HUMAN* | | | D [b9] - 1084.4833+[2] | 4591 |
| | | | F [b6] - 693.3090+[3] | 4498 |
| Pigment epithelium-derived factor | K.TSLEDFYLDEER.T | 506.231 6+++ | F [b6] - 693.3090+[1] | 3526 |
| PEDF_HUMAN* | | | D [y4] - 548.2311+[2] | 3208 |
| Pigment epithelium-derived factor | K.VTQNLTLIEESLTSEFI HDIDR.E | 858.4 413+++ | T [b13] - 721.8905++[1] | 11072 |
| PEDF_HUMAN* | | | T [y17] - 1009.5075++[2] | 8442 |
| | | | D [y4] - 518.2569+[3] | 6522 |
| Pigment epithelium-derived factor | K.TVQAVLTVPK.L | 528.3 266++ | Q [y8] - 855.5298+[1] | 83536 |
| PEDF_HUMAN* | | | V [b2] - 201.1234+[2] | 64729 |
| | | | A [b4] - 200.6132++[3] | 58198 |
| | | | P [y2] - 244.1656+[4] | 43347 |
| | | | Q [y8] - 428.2686++[5] | 38398 |
| | | | A [y7] - 727.4713+[6] | 33770 |
| | | | Q [b3] - 329.1819+[7] | 17809 |
| | | | L [y5] - 557.3657+[8] | 17518 |
| | | | V [y6] - 656.4341+[9] | 17029 |
| | | | V [y6] - 328.7207++[10] | 15839 |
| | | | T [y4] - 444.2817+[11] | 13859 |
| | | | V [y3] - 343.2340+[12] | 10717 |
| | | | A [b4] - 400.2191+[13] | 9695 |
| Pigment epithelium-derived factor | K.TVQAVLTVPK.L | 352.5 535+++ | P [y2] - 244.1656+[1] | 8295 |
| PEDF_HUMAN* | | | T [y4] - 444.2817+[2] | 2986 |
| | | | A [b4] - 400.2191+[3] | 2848 |
| Pigment epithelium-derived factor | K.LSYEGEVTK.S | 513.2 611++ | V [b7] - 389.6845++[1] | 60831 |
| PEDF_HUMAN* | | | E [b6] - 679.2933+[2] | 34857 |
| | | | Y [y7] - 413.2031++[3] | 10075 |
| | | | V [b7] - 778.3618+[4] | 8920 |
| | | | Y [b3] - 364.1867+[5] | 8008 |
| Pigment epithelium-derived factor | K.LQSLFDSPDFSK.I | 692.3 432++ | S [y2] - 234.1448+[1] | 49594 |
| PEDF_HUMAN* | | | L [y9] - 1055.5044+[2] | 48160 |
| | | | P [b8] - 888.4462+[3] | 23566 |
| | | | S [b7] - 791.3934+[4] | 13766 |
| | | | P [y5] - 297.1501++[5] | 12305 |
| | | | P [y5] - 593.2930+[6] | 10702 |
| | | | F [b5] - 589.3344+[7] | 8929 |
| | | | D [b9] - 1003.4731+[8] | 8742 |
| Pigment epithelium-derived factor | K.LQSLFDSPDFSK.I | 461.8 979+++ | P [y5] - 593.2930+[1] | 9154 |
| PEDF_HUMAN* | | | P [y5] - 297.1501++[2] | 5479 |
| Pigment epithelium-derived factor | R.DTDTGALLFIGK.I | 625.8 350++ | G [y2] - 204.1343+[1] | 32092 |
| PEDF_HUMAN* | | | G [y8] - 818.5135+[2] | 29707 |
| | | | T [b2] - 217.0819+[4] | 28172 |
| | | | T [b4] - 217.0819++[3] | 28172 |
| | | | F [y4] - 464.2867+[5] | 22160 |
| | | | D [y10] - 1034.5881+[6] | 20267 |
| | | | T [y9] - 919.5611+[7] | 17083 |
| | | | L [y6] - 690.4549+[8] | 14854 |
| | | | L [y5] - 577.3708+[9] | 12349 |
| | | | T [b4] - 433.1565+[10] | 11773 |
| | | | I [y3] - 317.2183+[11] | 11575 |
| | | | D [b3] - 332.1088+[12] | 8968 |
| | | | A [y7] - 761.4920+[13] | 8598 |

| | | | | |
|---|---|---|---|---|
| * Transition scan on Agilent 6490 | | | | |

### Example 4. Study III to Identify and Confirm Preeclampsia Biomarkers

A further hypothesis-dependent study was performed using essentially the same methods described in the preceding Examples unless noted below. The scheduled MRM assay used in Examples 1 and 2 but now augmented with newly discovered analytes from the Example 3 and related studies was used. Less robust transitions (from the original 1708 described in Example 1) were removed to improve analytical performance and make room for the newly discovered analytes.

Thirty subjects with preeclampsia who delivered preterm (<37 weeks 0 days) were selected for analyses. Twenty-three subjects were available with isolated preeclampsia; thus, eight subjects were selected with additional findings as follows: 5 subjects with gestational diabetes, one subject with pre-existing type 2 diabetes, and one subject with chronic hypertension. Subjects were classified as having severe preeclampsia if it was indicated in the Case Report Form as severe or if the pregnancy was complicated by HELLP syndrome. All other cases were classified as mild preeclampsia. Cases were matched to term controls (>/= 37 weeks 0 days) without preeclampsia at a 2:1 control-to-case ratio.

The samples were processed in 4 batches with each containing 3 HGS controls. All serum samples were depleted of the 14 most abundant serum proteins using MARS 14 (Agilent), digested with trypsin, desalted, and resolubilized with reconstitution solution containing 5 internal standard peptides as described in previous examples.

The LC-MS/MS analysis was performed with an Agilent Poroshell 120 EC-C18 column (2.1x50mm, 2.7 µm) at a flow rate of 400 µl/min and eluted with an acetonitrile gradient into an AB Sciex QTRAP5500 mass spectrometer. The sMRM assay measured 750 transitions that correspond to 349 peptides and 164 proteins. Chromatographic peaks were integrated using MultiQuantTM software (AB Sciex).

Transitions were excluded from analysis if they were missing in more than 20% of the samples. Log transformed peak areas for each transition were corrected for run order and batch effects by regression. The ability of each analyte to separate cases and controls was determined by calculating univariate AUC values from ROC curves. Ranked univariate AUC values (0.6 or greater) are reported for individual gestational age window sample sets or various combinations (Tables 12-15). Multivariate classifiers were built by Lasso and Random Forest methods. 1000 rounds of bootstrap resampling were performed and the nonzero Lasso coefficients or Random Forest Gini importance values were summed for each analyte amongst panels with AUCs of 0.85 or greater. For summed Random Forest Gini Importance values an Empirical Cumulative Distribution Function was fitted and probabilities (P) were calculated. The nonzero Lasso summed coefficients calculated from the different window combinations are shown in Tables 16-19. Summed Random Forest Gini values, with P >0.9 are found in Tables 20-22.

**Table 12. Univariate AUC values all windows**

| Transition | Protein | AUC |
|---|---|---|
| LDFHFSSDR_375.2_611.3 | INHBC_HUMAN | 0.785 |
| TVQAVLTVPK_528.3_428.3 | PEDF_HUMAN | 0.763 |
| TVQAVLTVPK_528.3_855.5 | PEDF_HUMAN | 0.762 |
| ETLLQDFR_511.3_565.3 | AMBP_HUMAN | 0.756 |
| DTDTGALLFIGK_625.8_818.5 | PEDF_HUMAN | 0.756 |
| DTDTGALLFIGK_625.8_217.1 | PEDF_HUMAN | 0.756 |
| IQTHSTTYR_369.5_627.3 | F13B_HUMAN | 0.755 |
| IQTHSTTYR_369.5_540.3 | F13B_HUMAN | 0.753 |
| ETLLQDFR_511.3_322.2 | AMBP_HUMAN | 0.751 |
| LDFHFSSDR_375.2_464.2 | INHBC_HUMAN | 0.745 |
| HHGPTITAK_321.2_275.1 | AMBP_HUMAN | 0.743 |
| VNHVTLSQPK_374.9_244.2 | B2MG_HUMAN | 0.733 |
| VEHSDLSFSK_383.5_468.2 | B2MG_HUMAN | 0.732 |
| ALALPPLGLAPLLNLWAKPQGR_770.5_256.2 | SHBG_HUMAN | 0.728 |
| HHGPTITAK_321.2_432.3 | AMBP_HUMAN | 0.728 |
| FLYHK_354.2_447.2 | AMBP_HUMAN | 0.722 |
| FLYHK_354.2_284.2 | AMBP_HUMAN | 0.721 |
| IALGGLLFPASNLR_481.3_657.4 | SHBG_HUMAN | 0.719 |
| GDTYPAELYITGSILR_885.0_274.1 | F13B_HUMAN | 0.716 |
| VEHSDLSFSK_383.5_234.1 | B2MG_HUMAN | 0.714 |
| GPGEDFR_389.2_623.3 | PTGDS_HUMAN | 0.714 |
| IALGGLLFPASNLR_481.3_412.3 | SHBG_HUMAN | 0.712 |
| EVFSKPISWEELLQ_852.9_260.2 | FA40A_HUMAN | 0.708 |
| FICPLTGLWPINTLK_887.0_685.4 | APOH_HUMAN | 0.707 |
| GFQALGDAADIR_617.3_717.4 | TIMP1_HUMAN | 0.707 |
| DVLLLVHNLPQNLTGHIWYK_791.8_310.2 | PSG7_HUMAN | 0.704 |
| VVLSSGSGPGLDLPLVLGLPLQLK_791.5_598.4 | SHBG_HUMAN | 0.704 |
| ATVVYQGER_511.8_652.3 | APOH_HUMAN | 0.702 |
| ALALPPLGLAPLLNLWAKPQGR_770.5_457.3 | SHBG_HUMAN | 0.702 |
| VVLSSGSGPGLDLPLVLGLPLQLK_791.5_768.5 | SHBG_HUMAN | 0.702 |
| DVLLLVHNLPQNLTGHIWYK_791.8_883.0 | PSG7_HUMAN | 0.702 |
| AHYDLR_387.7_566.3 | FETUA_HUMAN | 0.701 |
| GPGEDFR_389.2_322.2 | PTGDS_HUMAN | 0.701 |
| FSVVYAK_407.2_579.4 | FETUA_HUMAN | 0.701 |
| TLAFVR_353.7_274.2 | FA7_HUMAN | 0.699 |
| IAPQLSTEELVSLGEK_857.5_533.3 | AFAM_HUMAN | 0.698 |
| HFQNLGK_422.2_527.2 | AFAM_HUMAN | 0.696 |
| GDTYPAELYITGSILR_885.0_922.5 | F13B_HUMAN | 0.694 |
| FICPLTGLWPINTLK_887.0_756.9 | APOH_HUMAN | 0.694 |
| EVFSKPISWEELLQ_852.9_376.2 | FA40A_HUMAN | 0.692 |
| ATVVYQGER_511.8_751.4 | APOH_HUMAN | 0.690 |
| ELIEELVNITQNQK_557.6_618.3 | IL13_HUMAN | 0.690 |
| VNHVTLSQPK_374.9_459.3 | B2MG_HUMAN | 0.687 |
| IAQYYYTFK_598.8_395.2 | F13B_HUMAN | 0.685 |
| IAPQLSTEELVSLGEK_857.5_333.2 | AFAM_HUMAN | 0.685 |
| LIENGYFHPVK_439.6_627.4 | F13B_HUMAN | 0.684 |
| FSVVYAK_407.2_381.2 | FETUA_HUMAN | 0.684 |
| HFQNLGK_422.2_285.1 | AFAM_HUMAN | 0.684 |
| AHYDLR_387.7_288.2 | FETUA_HUMAN | 0.684 |
| ELPQSIVYK_538.8_417.7 | FBLN3_HUMAN | 0.683 |
| DADPDTFFAK_563.8_825.4 | AFAM_HUMAN | 0.679 |
| DADPDTFFAK_563.8_302.1 | AFAM_HUMAN | 0.676 |
| IAQYYYTFK_598.8_884.4 | F13B_HUMAN | 0.673 |
| VVESLAK_373.2_646.4 | IBP1_HUMAN | 0.673 |
| YGIEEHGK_311.5_599.3 | CXA1_HUMAN | 0.673 |
| GFQALGDAADIR_617.3_288.2 | TIMP1_HUMAN | 0.673 |
| YTTEIIK_434.2_704.4 | C1R_HUMAN | 0.671 |
| LPDTPQGLLGEAR_683.87_427.2 | EGLN_HUMAN | 0.666 |
| TLAFVR_353.7_492.3 | FA7_HUMAN | 0.666 |
| LIENGYFHPVK_439.6_343.2 | F13B_HUMAN | 0.665 |
| ELIIEELVNITQNQK_557.6_517.3 | IL13_HUMAN | 0.665 |
| DPNGLPPEAQK_583.3_669.4 | RET4_HUMAN | 0.664 |
| TNTNEFLIDVDK_704.85_849.5 | TF_HUMAN | 0.663 |
| NTVISVNPSTK_580.3_845.5 | VCAM1_HUMAN | 0.662 |
| YEFLNGR_449.7_293.1 | PLMN_HUMAN | 0.662 |
| AIGLPEELIQK_605.86_856.5 | FABPL_HUMAN | 0.662 |
| YTTEIIK_434.2_603.4 | C1R_HUMAN | 0.661 |
| AEHPTWGDEQLFQTTR_639.3_765.4 | PGH1_HUMAN | 0.658 |
| HTLNQIDEVK_598.8_951.5 | FETUA_HUMAN | 0.658 |
| HTLNQIDEVK_598.8_958.5 | FETUA_HUMAN | 0.656 |
| LPNNVLQEK_527.8_730.4 | AFAM_HUMAN | 0.655 |
| DPNGLPPEAQK_583.3_497.2 | RET4_HUMAN | 0.655 |
| TFLTVYWTPER_706.9_401.2 | ICAM1_HUMAN | 0.653 |
| TFLTVYWTPER_706.9_502.3 | ICAM1_HUMAN | 0.653 |
| SEPRPGVLLR_375.2_454.3 | FA7_HUMAN | 0.652 |
| FTFTLHLETPKPSISSSNLNPR_829.4_787.4 | PSG1_HUMAN | 0.652 |
| DAQYAPGYDK_564.3_813.4 | CFAB_HUMAN | 0.651 |
| ALDLSLK_380.2_185.1 | ITIH3_HUMAN | 0.651 |
| NCSFSIIYPVVIK_770.4_555.4 | CRHBP_HUMAN | 0.650 |
| NTVISVNPSTK_580.3_732.4 | VCAM1_HUMAN | 0.649 |
| IPSNPSHR_303.2_610.3 | FBLN3_HUMAN | 0.649 |
| DAQYAPGYDK_564.3_315.1 | CFAB_HUMAN | 0.647 |
| TLPFSR_360.7_506.3 | LYAM1_HUMAN | 0.647 |
| LPNNVLQEK_527.8_844.5 | AFAM_HUMAN | 0.644 |
| AALAAFNAQNNGSNFQLEEISR_789.1_746.4 | FETUA_HUMAN | 0.644 |
| AEHPTWGDEQLFQTTR_639.3_569.3 | PGH1_HUMAN | 0.644 |
| NNQLVAGYLQGPNVNLEEK_700.7_999.5 | IL1RA_HUMAN | 0.642 |
| EHSSLAFWK_552.8_267.1 | APOH_HUMAN | 0.642 |
| ALNHLPLEYNSALYSR_621.0_696.4 | CO6_HUMAN | 0.641 |
| VSEADSSNADWVTK_754.9_347.2 | CFAB_HUMAN | 0.641 |
| NFPSPVDAAFR_610.8_959.5 | HEMO_HUMAN | 0.641 |
| WNFAYWAAHQPWSR_607.3_545.3 | PRG2_HUMAN | 0.638 |
| WNFAYWAAHQPWSR_607.3_673.3 | PRG2_HUMAN | 0.638 |
| TAVTANLDIR_537.3_802.4 | CHL1_HUMAN | 0.638 |
| IPSNPSHR_303.2_496.3 | FBLN3_HUMAN | 0.637 |
| YWGVASFLQK_599.8_849.5 | RET4_HUMAN | 0.637 |
| ALDLSLK_380.2_575.3 | ITIH3_HUMAN | 0.636 |
| YNSQLLSFVR_613.8_508.3 | TFR1_HUMAN | 0.636 |
| EHSSLAFWK_552.8_838.4 | APOH_HUMAN | 0.635 |
| YWGVASFLQK_599.8_350.2 | RET4_HUMAN | 0.635 |
| ALNHLPLEYNSALYSR_621.0_538.3 | CO6_HUMAN | 0.633 |
| DLYHYITSYVVDGEIIIYGPAYSGR_955.5_707.3 | PSG1_HUMAN | 0.633 |
| FTFTLHLETPKPSISSSNLNPR_829.4_874.4 | PSG1_HUMAN | 0.633 |
| YQISVNK_426.2_560.3 | FIBB_HUMAN | 0.632 |
| YEFLNGR_449.7_606.3 | PLMN_HUMAN | 0.632 |
| LNIGYIEDLK_589.3_950.5 | PAI2_HUMAN | 0.631 |
| LLEVPEGR_456.8_356.2 | C1S_HUMAN | 0.630 |
| ENPAVIDFELAPIVDLVR_670.7_811.5 | CO6_HUMAN | 0.630 |
| YYLQGAK_421.7_516.3 | ITIH4_HUMAN | 0.630 |
| ITGFLKPGK_320.9_301.2 | LBP_HUMAN | 0.629 |
| DLHLSDVFLK_396.2_260.2 | CO6_HUMAN | 0.629 |
| HELTDEELQSLFTNFANVVDK_817.1_854.4 | AFAM_HUMAN | 0.629 |
| YYLQGAK_421.7_327.1 | ITIH4_HUMAN | 0.628 |
| NCSFSIIYPVVIK_770.4_831.5 | CRHBP_HUMAN | 0.627 |
| FLNWIK_410.7_560.3 | HABP2_HUMAN | 0.627 |
| ITGFLKPGK_320.9_429.3 | LBP HUMAN | 0.627 |
| VVESLAK_373.2_547.3 | IBP1_HUMAN | 0.627 |
| NFPSPVDAAFR_610.8_775.4 | HEMO_HUMAN | 0.627 |
| AEIEYLEK_497.8_552.3 | LYAM1_HUMAN | 0.627 |
| ENPAVIDFELAPIVDLVR_670.7_601.4 | CO6_HUMAN | 0.627 |
| VQEVLLK_414.8_373.3 | HYOU1_HUMAN | 0.626 |
| TQIDSPLSGK_523.3_703.4 | VCAM1_HUMAN | 0.626 |
| VSEADSSNADWVTK_754.9_533.3 | CFAB_HUMAN | 0.625 |
| DFNQFSSGEK_386.8_189.1 | FETA_HUMAN | 0.624 |
| LPDTPQGLLGEAR_683.87_940.5 | EGLN_HUMAN | 0.623 |
| DLYHYITSYVVDGEIIIYGPAYSGR_955.5_650.3 | PSG1_HUMAN | 0.623 |
| FAFNLYR_465.8_712.4 | HEP2_HUMAN | 0.623 |
| LLELTGPK_435.8_644.4 | A1BG_HUMAN | 0.623 |
| NEIVFPAGILQAPFYTR_968.5_357.2 | ECE1_HUMAN | 0.623 |
| EFDDDTYDNDIALLQLK_1014.48_501.3 | TPA_HUMAN | 0.621 |
| FSLVSGWGQLLDR_493.3_403.2 | FA7_HUMAN | 0.621 |
| LLELTGPK_435.8_227.2 | A1BG_HUMAN | 0.621 |
| LIQDAVTGLTVNGQITGDK_972.0_640.4 | ITIH3_HUMAN | 0.621 |
| QGHNSVFLIK_381.6_520.4 | HEMO_HUMAN | 0.620 |
| ILPSVPK_377.2_244.2 | PGH1_HUMAN | 0.620 |
| STLFVPR_410.2_272.2 | PEPD_HUMAN | 0.620 |
| TLEAQLTPR_514.8_685.4 | HEP2_HUMAN | 0.619 |
| QGHNSVFLIK_381.6_260.2 | HEMO_HUMAN | 0.619 |
| LSSPAVITDK_515.8_743.4 | PLMN_HUMAN | 0.618 |
| LLEVPEGR_456.8_686.4 | C1S_HUMAN | 0.617 |
| GVTGYFTFNLYLK_508.3_260.2 | PSG5_HUMAN | 0.617 |
| EALVPLVADHK_397.9_390.2 | HGFA_HUMAN | 0.616 |
| SFRPFVPR_335.9_272.2 | LBP_HUMAN | 0.616 |
| DFNQFSSGEK_386.8_333.2 | FETA_HUMAN | 0.616 |
| GSLVQASEANLQAAQDFVR_668.7_735.4 | ITIH1_HUMAN | 0.616 |
| ITLPDFTGDLR_624.3_920.5 | LBP_HUMAN | 0.615 |
| LIQDAVTGLTVNGQITGDK_972.0_798.4 | ITIH3_HUMAN | 0.615 |
| ILPSVPK_377.2_227.2 | PGH1_HUMAN | 0.614 |
| DIIKPDPPK_511.8_342.2 | IL12B_HUMAN | 0.613 |
| QGFGNVATNTDGK_654.81_319.2 | FIBB_HUMAN | 0.613 |
| AVLHIGEK_289.5_348.7 | THBG_HUMAN | 0.613 |
| YENYTSSFFIR_713.8_756.4 | IL12B_HUMAN | 0.613 |
| LSSPAVITDK_515.8_830.5 | PLMN_HUMAN | 0.613 |
| SFRPFVPR_335.9_635.3 | LBP_HUMAN | 0.613 |
| GLQYAAQEGLLALQSELLR_1037.1_858.5 | LBP_HUMAN | 0.612 |
| VELAPLPSWQPVGK_760.9_400.3 | ICAM1_HUMAN | 0.612 |
| CRPINATLAVEK_457.9_559.3 | CGB1_HUMAN | 0.610 |
| GIVEECCFR_585.3_771.3 | IGF2_HUMAN | 0.610 |
| AVLHIGEK_289.5_292.2 | THBG_HUMAN | 0.610 |
| TLEAQLTPR_514.8_814.4 | HEP2_HUMAN | 0.610 |
| SILFLGK_389.2_577.4 | THBG_HUMAN | 0.609 |
| HVVQLR_376.2_614.4 | IL6RA_HUMAN | 0.609 |
| TQILEWAAER_608.8_761.4 | EGLN_HUMAN | 0.609 |
| NSDQEIDFK_548.3_409.2 | S10A5_HUMAN | 0.609 |
| SGAQATWTELPWPHEK_613.3_510.3 | HEMO_HUMAN | 0.607 |
| EDTPNSVWEPAK_686.8 630.3 | C1S_HUMAN | 0.607 |
| ITLPDFTGDLR_624.3_288.2 | LBP_HUMAN | 0.607 |
| TLPFSR_360.7_409.2 | LYAM1_HUMAN | 0.607 |
| GIVEECCFR_585.3_900.3 | IGF2_HUMAN | 0.606 |
| SGAQATWTELPWPHEK_613.3_793.4 | HEMO_HUMAN | 0.606 |
| VRPQQLVK_484.3_609.4 | ITIH4_HUMAN | 0.605 |
| SEYGAALAWEK_612.8_788.4 | CO6_HUMAN | 0.605 |
| LEEHYELR_363.5_288.2 | PAI2_HUMAN | 0.605 |
| FQLPGQK_409.2_275.1 | PSG1_HUMAN | 0.605 |
| IHWESASLLR_606.3_437.2 | CO3_HUMAN | 0.604 |
| NAVVQGLEQPHGLVVHPLR_688.4_890.6 | LRP1_HUMAN | 0.604 |
| VTGLDFIPGLHPILTLSK_641.04_771.5 | LEP_HUMAN | 0.603 |
| YNSQLLSFVR_613.8_734.5 | TFR1_HUMAN | 0.603 |
| ALVLELAK_428.8_672.4 | INHBE_HUMAN | 0.603 |
| FAFNLYR_465.8_565.3 | HEP2_HUMAN | 0.603 |
| VRPQQLVK_484.3_722.4 | ITIH4_HUMAN | 0.602 |
| SLQAFVAVAAR_566.8_487.3 | IL23A_HUMAN | 0.602 |
| AGFAGDDAPR_488.7_701.3 | ACTB_HUMAN | 0.601 |
| EDTPNSVWEPAK_686.8_315.2 | C1S_HUMAN | 0.601 |
| VQEVLLK_414.8_601.4 | HYOU1_HUMAN | 0.601 |
| SEYGAALAWEK_612.8_845.5 | CO6_HUMAN | 0.601 |
| TLFIFGVTK_513.3_215.1 | PSG4_HUMAN | 0.601 |
| YNQLLR_403.7_288.2 | ENOA_HUMAN | 0.600 |
| TQIDSPLSGK_523.3_816.5 | VCAM1_HUMAN | 0.600 |

**Table 13. Univariate AUC values early window**

| Transition | Protein | AUC |
|---|---|---|
| LDFHFSSDR_375.2_611.3 | INHBC_HUMAN | 0.858 |
| LDFHFSSDR_375.2_464.2 | INHBC_HUMAN | 0.838 |
| ELPQSIVYK_538.8_417.7 | FBLN3_HUMAN | 0.815 |
| VNHVTLSQPK_374.9_244.2 | B2MG_HUMAN | 0.789 |
| GFQALGDAADIR_617.3_717.4 | TIMP1_HUMAN | 0.778 |
| VEHSDLSFSK_383.5_234.1 | B2MG_HUMAN | 0.778 |
| TVQAVLTVPK_528.3_428.3 | PEDF_HUMAN | 0.775 |
| TVQAVLTVPK_528.3_855.5 | PEDF_HUMAN | 0.775 |
| DTDTGALLFIGK_625.8_217.1 | PEDF_HUMAN | 0.772 |
| ETLLQDFR_511.3_565.3 | AMBP_HUMAN | 0.772 |
| DTDTGALLFIGK_625.8_818.5 | PEDF_HUMAN | 0.769 |
| VVESLAK_373.2_646.4 | IBP1_HUMAN | 0.766 |
| FSVVYAK_407.2_381.2 | FETUA_HUMAN | 0.764 |
| HHGPTITAK_321.2_275.1 | AMBP_HUMAN | 0.764 |
| ETLLQDFR_511.3_322.2 | AMBP_HUMAN | 0.761 |
| FLYHK_354.2_447.2 | AMBP_HUMAN | 0.758 |
| GPGEDFR_389.2_623.3 | PTGDS_HUMAN | 0.755 |
| HHGPTITAK_321.2_432.3 | AMBP_HUMAN | 0.755 |
| VEHSDLSFSK_383.5_468.2 | B2MG_HUMAN | 0.752 |
| FLYHK_354.2_284.2 | AMBP_HUMAN | 0.749 |
| FSVVYAK_407.2_579.4 | FETUA_HUMAN | 0.749 |
| VNHVTLSQPK_374.9_459.3 | B2MG_HUMAN | 0.749 |
| IPSNPSHR_303.2_610.3 | FBLN3_HUMAN | 0.746 |
| VVESLAK_373.2_547.3 | IBP1_HUMAN | 0.746 |
| IPSNPSHR_303.2_496.3 | FBLN3_HUMAN | 0.746 |
| NCSFSIIYPVVIK_770.4_555.4 | CRHBP_HUMAN | 0.746 |
| GFQALGDAADIR_617.3_288.2 | TIMP1_HUMAN | 0.744 |
| IQTHSTTYR_369.5_627.3 | F13B_HUMAN | 0.744 |
| AALAAFNAQNNGSNFQLEEISR_789.1_746.4 | FETUA_HUMAN | 0.738 |
| AHYDLR_387.7_566.3 | FETUA_HUMAN | 0.738 |
| IQTHSTTYR_369.5_540.3 | F13B_HUMAN | 0.738 |
| AIGLPEELIQK_605.86_856.5 | FABPL_HUMAN | 0.735 |
| ATVVYQGER_511.8_751.4 | APOH_HUMAN | 0.735 |
| FICPLTGLWPINTLK_887.0_685.4 | APOH_HUMAN | 0.735 |
| FICPLTGLWPINTLK_887.0_756.9 | APOH_HUMAN | 0.735 |
| HTLNQIDEVK_598.8_958.5 | FETUA_HUMAN | 0.735 |
| AQETSGEEISK_589.8_979.5 | IBP1_HUMAN | 0.732 |
| DSPSVWAAVPGK_607.31_301.2 | PROF1_HUMAN | 0.732 |
| GPGEDFR_389.2_322.2 | PTGDS_HUMAN | 0.732 |
| ATVVYQGER_511.8_652.3 | APOH_HUMAN | 0.729 |
| NFPSPVDAAFR_610.8_959.5 | HEMO_HUMAN | 0.729 |
| LIENGYFHPVK_439.6_627.4 | F13B_HUMAN | 0.726 |
| AHYDLR_387.7_288.2 | FETUA_HUMAN | 0.726 |
| ELIEETVNITQNQK_557.6_618.3 | IL13_HUMAN | 0.724 |
| ETPEGAEAKPWYEPIYLGGVFQLEK_951.14_877.5 | TNFA_HUMAN | 0.724 |
| ALDLSLK_380.2_185.1 | ITIH3_HUMAN | 0.721 |
| IHWESASLLR_606.3_437.2 | CO3_HUMAN | 0.721 |
| DAQYAPGYDK_564.3_813.4 | CFAB_HUMAN | 0.718 |
| NFPSPVDAAFR_610.8_775.4 | HEMO_HUMAN | 0.718 |
| AVGYLITGYQR_620.8_523.3 | PZP_HUMAN | 0.715 |
| AVGYLITGYQR_620.8_737.4 | PZP_HUMAN | 0.712 |
| DIPHWLNPTR_416.9_600.3 | PAPP1_HUMAN | 0.712 |
| ALDLSLK_380.2_575.3 | ITIH3_HUMAN | 0.709 |
| IEGNLIFDPNNYLPK_874.0_845.5 | APOB_HUMAN | 0.709 |
| LIENGYFHPVK_439.6_343.2 | F13B_HUMAN | 0.709 |
| QTLSWTVTPK_580.8_818.4 | PZP_HUMAN | 0.709 |
| DAQYAPGYKD_564.3_315.1 | CFAB_HUMAN | 0.707 |
| GLQYAAQEGLLALQSELLR_1037.1_858.5 | LBP_HUMAN | 0.707 |
| IEGNLIFDPNNYLPK_874.0_414.2 | APOB_HUMAN | 0.707 |
| IQHPFTVEEFVLPK_562.0_861.5 | PZP_HUMAN | 0.707 |
| QTLSWTVTPK_580.8_545.3 | PZP_HUMAN | 0.707 |
| VSEADSSNADWVTK_754.9_347.2 | CFAB_HUMAN | 0.707 |
| ILPSVPK_377.2_244.2 | PGH1_HUMAN | 0.704 |
| IQHPFTVEEFVLPK_562.0_603.4 | PZP_HUMAN | 0.704 |
| NCSFSIIYPVVIK_770.4_831.5 | CRHBP_HUMAN | 0.704 |
| YNSQLLSFVR_613.8_508.3 | TFR1_HUMAN | 0.704 |
| HTLNQIDEVK_598.8_951.5 | FETUA_HUMAN | 0.701 |
| NEIWYR_440.7_637.4 | FA12_HUMAN | 0.701 |
| QGHNSVFLIK_381.6_260.2 | HEMO_HUMAN | 0.701 |
| YTTEIIK_434.2_603.4 | C1R_HUMAN | 0.701 |
| STLFVPR_410.2_272.2 | PEPD_HUMAN | 0.699 |
| EVFSKPISWEELLQ_852.9_260.2 | FA40A_HUMAN | 0.698 |
| TGISPLALIK_506.8_741.5 | APOB_HUMAN | 0.698 |
| TSESGELHGLTTEEEFVEGIYK_819.06_310.2 | TTHY_HUMAN | 0.698 |
| AEHPTWGDEQLFQTTR_639.3_569.3 | PGH1_HUMAN | 0.695 |
| AEHPTWGDEQLFQTTR_639.3_765.4 | PGH1_HUMAN | 0.695 |
| HFQNLGK_422.2_527.2 | AFAM_HUMAN | 0.695 |
| SVSLPSLDPASAK_636.4_473.3 | APOB_HUMAN | 0.695 |
| ILPSVPK_377.2_227.2 | PGH1_HUMAN | 0.692 |
| LIQDAVTGLTVNGQITGDK_972.0_640.4 | ITIH3_HUMAN | 0.692 |
| QGHNSVFLIK_381.6_520.4 | HEMO_HUMAN | 0.692 |
| TGISPLALIK_506.8_654.5 | APOB_HUMAN | 0.692 |
| YGIEEHGK_311.5_599.3 | CXA1_HUMAN | 0.692 |
| ELITEELVNITQNQK_557.6_517.3 | IL13_HUMAN | 0.689 |
| IHWESASLLR_606.3_251.2 | CO3_HUMAN | 0.689 |
| LIQDAVTGLTVNGQITGDK_972.0_798.4 | ITIH3_HUMAN | 0.689 |
| ALALPPLGLAPLLNLWAKPQGR_770.5_256.2 | SHBG_HUMAN | 0.687 |
| ALNFGGIGVVVGHELTHAFDDQGR_837.1_299.2 | ECE1_HUMAN | 0.687 |
| AQETSGEEISK_589.8_850.4 | IBP1_HUMAN | 0.687 |
| GVTGYFTFNLYLK_508.3_683.9 | PSG5_HUMAN | 0.687 |
| ITLPDFTGDLR_624.3_288.2 | LBP_HUMAN | 0.687 |
| LPDTPQGLLGEAR_683.87_427.2 | EGLN_HUMAN | 0.687 |
| SVSLPSLDPASAK_636.4_885.5 | APOB_HUMAN | 0.687 |
| TLAFVR_353.7_274.2 | FA7_HUMAN | 0.687 |
| YTTEIIK_434.2_704.4 | C1R_HUMAN | 0.687 |
| EFDDDTYDNDIALLQLK_1014.48_388.3 | TPA_HUMAN | 0.684 |
| IALGGLLFPASNLR_481.3_657.4 | SHBG_HUMAN | 0.684 |
| DFNQFSSGEK_386.8_189.1 | FETA_HUMAN | 0.681 |
| EHSSLAFWK 552.8 838.4 | APOH HUMAN | 0.681 |
| ELPQSIVYK_538.8_409.2 | FBLN3_HUMAN | 0.681 |
| ITGFLKPGK_320.9_301.2 | LBP_HUMAN | 0.681 |
| ITGFLKPGK_320.9_429.3 | LBP_HUMAN | 0.681 |
| AFQVWSDVTPLR_709.88_385.3 | MMP2_HUMAN | 0.678 |
| GLQYAAQEGLLALQSELLR_1037.1_929.5 | LBP_HUMAN | 0.678 |
| HYINLITR_515.3_301.1 | NPY_HUMAN | 0.678 |
| NAVVQGLEQPHGLVVHPLR_688.4_890.6 | LRP1_HUMAN | 0.675 |
| WWGGQPLWITATK_772.4_929.5 | ENPP2_HUMAN | 0.675 |
| YNQLLR_403.7_288.2 | ENOA_HUMAN | 0.675 |
| LDGSTHLNIFFAK_488.3_852.5 | PAPP1_HUMAN | 0.672 |
| VVGGLVALR_442.3_784.5 | FA12_HUMAN | 0.672 |
| WNFAYWAAHQPWSR_607.3_673.3 | PRG2_HUMAN | 0.672 |
| NHYTESISVAK_624.8_252.1 | NEUR1_HUMAN | 0.670 |
| NSDQEIDFK_548.3_409.2 | S10A5_HUMAN | 0.670 |
| SGAQATWTELPWPHEK_613.3_510.3 | HEMO_HUMAN | 0.670 |
| WNFAYWAAHQPWSR_607.3_545.3 | PRG2_HUMAN | 0.670 |
| SFRPFVPR_335.9_272.2 | LBP_HUMAN | 0.670 |
| AFQVWSDVTPLR_709.88_347.2 | MMP2_HUMAN | 0.667 |
| DADPDTFFAK_563.8_825.4 | AFAM_HUMAN | 0.667 |
| EHSSLAFWK_552.8_267.1 | APOH_HUMAN | 0.667 |
| ITENDIQIALDDAK_779.9_632.3 | APOB_HUMAN | 0.667 |
| ITLPDFTGDLR_624.3_920.5 | LBP_HUMAN | 0.667 |
| VQEVLLK_414.8_373.3 | HYOU1_HUMAN | 0.667 |
| VSFSSPLVAISGVALR_802.0_715.4 | PAPP1_HUMAN | 0.667 |
| HFQNLGK_422.2_285.1 | AFAM_HUMAN | 0.664 |
| ITENDIQIALDDAK_779.9_873.5 | APOB_HUMAN | 0.664 |
| ALQDQLVLVAAK_634.9_289.2 | ANGT_HUMAN | 0.661 |
| DLHLSDVFLK_396.2_260.2 | CO6_HUMAN | 0.661 |
| DLHLSDVFLK_396.2_366.2 | CO6_HUMAN | 0.661 |
| TAVTANLDIR_537.3_802.4 | CHL1_HUMAN | 0.661 |
| DADPDTFFAK_563.8_302.1 | AFAM_HUMAN | 0.658 |
| DPTFIPAPIQAK_433.2_461.2 | ANGT_HUMAN | 0.658 |
| FAFNLYR_465.8_712.4 | HEP2_HUMAN | 0.658 |
| IALGGLLFPASNLR_481.3-412.3 | SHBG_HUMAN | 0.658 |
| IAQYYYTFK_598.8_395.2 | F13B_HUMAN | 0.658 |
| LPNNVLQEK_527.8_730.4 | AFAM_HUMAN | 0.658 |
| SLDFTELDVAAEK_719.4_874.5 | ANGT_HUMAN | 0.658 |
| VELAPLPSWQPVGK_760.9_400.3 | ICAM1_HUMAN | 0.658 |
| DIIKPDPPK_511.8_342.2 | IL12B_HUMAN | 0.655 |
| EVFSKPISWEELLQ_852.9_376.2 | FA40A_HUMAN | 0.655 |
| LSETNR_360.2_330.2 | PSG1_HUMAN | 0.655 |
| NEIWYR_440.7_357.2 | FA12_HUMAN | 0.655 |
| SFRPFVPR_335.9_635.3 | LBP_HUMAN | 0.655 |
| SGAQATWTELPWPHEK_613.3_793.4 | HEMO_HUMAN | 0.655 |
| TGAQELLR_444.3_530.3 | GELS_HUMAN | 0.655 |
| VSEADSSNADWVTK_754.9_533.3 | CFAB_HUMAN | 0.655 |
| VVGGLVALR_442.3_685.4 | FA12_HUMAN | 0.655 |
| DISEVVTPR_508.3_787.4 | CFAB_HUMAN | 0.652 |
| IHPSYTNYR_575.8_598.3 | PSG2_HUMAN | 0.652 |
| VSFSSPLVAISGVALR_802.0_602.4 | PAPP1_HUMAN | 0.652 |
| YNQLLR_403.7_529.3 | ENOA_HUMAN | 0.652 |
| ALQDQLVLVAAK_634.9_956.6 | ANGT_HUMAN | 0.650 |
| IHPSYTNYR_575.8_813.4 | PSG2_HUMAN | 0.650 |
| TFLTVYWTPER_706.9_401.2 | ICAM1_HUMAN | 0.650 |
| VQEVLLK_414.8_601.4 | HYOU1_HUMAN | 0.650 |
| GDTYPAELYITGSILR_885.0_274.1 | F13B_HUMAN | 0.647 |
| GVTGYFTFNLYLK_508.3_260.2 | PSG5_HUMAN | 0.647 |
| SLDFTELDVAAEK_719.4_316.2 | ANGT_HUMAN | 0.647 |
| VVLSSGSGPGLDLPLVLGLPLQLK_791.5_598.4 | SHBG_HUMAN | 0.647 |
| YEFLNGR_449.7_293.1 | PLMN_HUMAN | 0.647 |
| AQPVQVAEGSEPDGFWEALGGK_758.0_623.4 | GELS_HUMAN | 0.644 |
| FLNWIK_410.7_561.3 | HABP2_HUMAN | 0.644 |
| IAPQLSTEELVSLGEK_857.5_533.3 | AFAM_HUMAN | 0.644 |
| NTVISVNPSTK 580.3 732.4 | VCAM1_HUMAN | 0.644 |
| SFEGLGQLEVLTLDHNQLQEVK_833.1_503.3 | ALS_HUMAN | 0.644 |
| TFLTVYWTPER_706.9_502.3 | ICAM1_HUMAN | 0.644 |
| AGFAGDDAPR_488.7_701.3 | ACTB_HUMAN | 0.641 |
| AIGLPEELIQK_605.86_355.2 | FABPL_HUMAN | 0.641 |
| DISEVVTPR_508.3_472.3 | CFAB_HUMAN | 0.641 |
| DPTFIPAPIQAK_433.2_556.3 | ANGT_HUMAN | 0.641 |
| ENPAVIDFELAPIVDLVR_670.7_811.5 | CO6_HUMAN | 0.641 |
| FAFNLYR_465.8_565.3 | HEP2_HUMAN | 0.641 |
| IAPQLSTEELVSLGEK_857.5_333.2 | AFAM_HUMAN | 0.641 |
| TNTNEFLIDVDK_704.85_849.5 | TF_HUMAN | 0.639 |
| DVLLLVHNLPQNLTGHIWYK_791.8_883.0 | PSG7_HUMAN | 0.638 |
| LDGSTHLNIFFAK_488.3_739.4 | PAPP1_HUMAN | 0.638 |
| LPDTPQGLLGEAR_683.87_940.5 | EGLN_HUMAN | 0.638 |
| VVLSSGSGPGLDLPLVLGLPLQLK_791.5_768.5 | SHBG_HUMAN | 0.638 |
| ALALPPLGLAPLLNLWAKPQGR_770.5_457.3 | SHBG_HUMAN | 0.635 |
| LPNNVLQEK_527.8_844.5 | AFAM_HUMAN | 0.635 |
| QINSYVK_426.2_496.3 | CBG_HUMAN | 0.635 |
| QINSYVK_426.2_610.3 | CBG_HUMAN | 0.635 |
| TGAQELLR_444.3_658.4 | GELS_HUMAN | 0.635 |
| TLEAQLTPR_514.8_685.4 | HEP2_HUMAN | 0.635 |
| WILTAAHTLYPK_471.9_621.4 | C1R_HUMAN | 0.635 |
| SEPRPGVLLR_375.2_454.3 | FA7_HUMAN | 0.632 |
| AGFAGDDAPR_488.7_630.3 | ACTB_HUMAN | 0.632 |
| DFNQFSSGEK_386.8_333.2 | FETA_HUMAN | 0.632 |
| DVLLLVHNLPQNLTGHIWYK_791.8_310.2 | PSG7_HUMAN | 0.632 |
| NKPGVYTDVAYYLAWIR_677.0_545.3 | FA12_HUMAN | 0.632 |
| SEYGAALAWEK_612.8_788.4 | CO6_HUMAN | 0.632 |
| YNSQLLSFVR_613.8_734.5 | TFR1_HUMAN | 0.632 |
| ALVLELAK_428.8_672.4 | INHBE_HUMAN | 0.630 |
| ENPAVIDFELAPIVDLVR_670.7_601.4 | CO6_HUMAN | 0.630 |
| NNQLVAGYLQGPNVNLEEK_700.7_999.5 | IL1RA_HUMAN | 0.630 |
| WGAAPYR_410.7_577.3 | PGRP2_HUMAN | 0.630 |
| HELTDEELQSLFTNFANVVDK_817.1_854.4 | AFAM_HUMAN | 0.627 |
| AKPALEDLR_506.8_288.2 | APOA1_HUMAN | 0.624 |
| AVLHIGEK_289.5_348.7 | THBG_HUMAN | 0.624 |
| EDTPNSVWEPAK_686.8_630.3 | CIS_HUMAN | 0.624 |
| SPELQAEAK_486.8_788.4 | APOA2_HUMAN | 0.624 |
| YENYTSSFFIR_713.8_756.4 | IL12B_HUMAN | 0.624 |
| NEIVFPAGILQAPFYTR_968.5_456.2 | ECE1_HUMAN | 0.621 |
| TAVTANLDIR_537.3_288.2 | CHL1_HUMAN | 0.621 |
| WWGGQPLWITATK_772.4_373.2 | ENPP2_HUMAN | 0.621 |
| AVDIPGLEAATPYR_736.9_399.2 | TENA_HUMAN | 0.618 |
| ALNFGGIGVVVGHELTHAFDDQGR_837.1_360.2 | ECE1_HUMAN | 0.618 |
| ALNHLPLEYNSALYSR_621.0_696.4 | CO6_HUMAN | 0.618 |
| FNAVLTNPQGDYDTSTGK_964.5_262.1 | C1QC_HUMAN | 0.618 |
| GDTYPAELYITGSILR_885.0_922.5 | F13B_HUMAN | 0.618 |
| IAQYYYTFK_598.8_884.4 | F13B_HUMAN | 0.618 |
| LEQGENVFLQATDK_796.4_822.4 | C1QB_HUMAN | 0.618 |
| LSITGTYDLK_555.8_696.4 | A1AT_HUMAN | 0.618 |
| NTVISVNPSTK_580.3_845.5 | VCAM1_HUMAN | 0.618 |
| TLAFVR_353.7_492.3 | FA7_HUMAN | 0.618 |
| TLEAQLTPR_514.8_814.4 | HEP2_HUMAN | 0.618 |
| TQIDSPLSGK_523.3_703.4 | VCAM1_HUMAN | 0.618 |
| AVLHIGEK_289.5_292.2 | THBG_HUMAN | 0.615 |
| FLIPNASQAESK_652.8_931.4 | 1433Z_HUMAN | 0.615 |
| FNAVLTNPQGDYDTSTGK_964.5_333.2 | C1QC_HUMAN | 0.615 |
| FQSVFTVTR_542.8_722.4 | C1QC_HUMAN | 0.615 |
| INPASLDK_429.2_630.4 | C163A_HUMAN | 0.615 |
| IPKPEASFSPR_410.2_506.3 | ITIH4_HUMAN | 0.615 |
| ITQDAQLK_458.8_803.4 | CBG_HUMAN | 0.615 |
| TSYQVYSK_488.2_397.2 | C163A_HUMAN | 0.615 |
| WGAAPYR_410.7_634.3 | PGRP2_HUMAN | 0.615 |
| AVDIPGLEAATPYR_736.9_286.1 | TENA_HUMAN | 0.613 |
| DVLLLVHNLPQNLPGYFWYK_810.4_328.2 | PSG9_HUMAN | 0.613 |
| SFEGLGQLEVLTLDHNQLQEVK_833.1_662.8 | ALS_HUMAN | 0.613 |
| TASDFITK_441.7_710.4 | GELS_HUMAN | 0.613 |
| AGPLQAR_356.7_584.4 | DEF4_HUMAN | 0.610 |
| DYWSTVK_449.7_347.2 | APOC3_HUMAN | 0.610 |
| FQSVFTVTR_542.79_623.4 | C1QC_HUMAN | 0.610 |
| FQSVFTVTR_542.79_722.4 | C1QC_HUMAN | 0.610 |
| SYTITGLQPGTDYK_772.4_352.2 | FINC_HUMAN | 0.610 |
| FQLSETNR_497.8_476.3 | PSG2_HUMAN | 0.607 |
| IPKPEASFSPR_410.2_359.2 | ITIH4_HUMAN | 0.607 |
| LIEIANHVDK_384.6_498.3 | ADA12_HUMAN | 0.607 |
| SILFLGK_389.2_201.1 | THBG_HUMAN | 0.607 |
| SLLQPNK_400.2_358.2 | CO8A_HUMAN | 0.607 |
| VFQFLEK_455.8_811.4 | CO5_HUMAN | 0.607 |
| VPGLYYFTYHASSR_554.3_720.3 | C1QB_HUMAN | 0.607 |
| VSAPSGTGHLPGLNPL_506.3_860.5 | PSG3_HUMAN | 0.607 |
| AGITIPR_364.2_486.3 | IL17_HUMAN | 0.604 |
| FLIPNASQAESK_652.8_261.2 | 1433Z_HUMAN | 0.604 |
| FQSVFTVTR_542.8_623.4 | C1QC_HUMAN | 0.604 |
| IRPFFPQQ_516.79_661.4 | FIBB_HUMAN | 0.604 |
| LLELTGPK_435.8_644.4 | A1BG_HUMAN | 0.604 |
| SETEIHQGFQHLHQLFAK_717.4_318.1 | CBG_HUMAN | 0.604 |
| SILFLGK_389.2_577.4 | THBG_HUMAN | 0.604 |
| STLFVPR_410.2_518.3 | PEPD_HUMAN | 0.604 |
| TEQAAVAR_423.2_487.3 | FA12_HUMAN | 0.604 |
| EDTPNSVWEPAK_686.8_315.2 | C1S_HUMAN | 0.601 |
| FLNWIK_410.7_560.3 | HABP2_HUMAN | 0.601 |
| ITQDAQLK_458.8_702.4 | CBG_HUMAN | 0.601 |
| SPELQAEAK486.8_659.4 | APOA2_HUMAN | 0.601 |
| TLLPVSKPEIR_418.3_288.2 | CO5_HUMAN | 0.601 |
| VFQFLEK_455.8_276.2 | CO5_HUMAN | 0.601 |
| YGLVTYATYPK_638.3_843.4 | CFAB_HUMAN | 0.601 |

**Table 14. Univariate AUC values early-middle combined windows**

| Transition | Protein | AUC |
|---|---|---|
| LDFHFSSDR_375.2_611.3 | INHBC_HUMAN | 0.809 |
| ETLLQDFR_511.3_565.3 | AMBP_HUMAN | 0.802 |
| HHGPTITAK_321.2_275.1 | AMBP_HUMAN | 0.801 |
| ATVVYQGER_511.8_652.3 | APOH_HUMAN | 0.799 |
| ETLLQDFR_511.3_322.2 | AMBP_HUMAN | 0.796 |
| ATVVYQGER_511.8_751.4 | APOH_HUMAN | 0.795 |
| HHGPTITAK_321.2_432.3 | AMBP_HUMAN | 0.794 |
| TVQAVLTVPK_528.3_855.5 | PEDF_HUMAN | 0.791 |
| AHYDLR_387.7_566.3 | FETUA_HUMAN | 0.789 |
| TVQAVLTVPK_528.3_428.3 | PEDF_HUMAN | 0.787 |
| FICPLTGLWPINTLK_887.0_685.4 | APOH_HUMAN | 0.785 |
| VNHVTLSQPK_374.9_244.2 | B2MG_HUMAN | 0.783 |
| AHYDLR_387.7_288.2 | FETUA HUMAN | 0.781 |
| ELIEELVNITQNQK_557.6_618.3 | IL13_HUMAN | 0.780 |
| FSVVYAK_407.2_381.2 | FETUA_HUMAN | 0.777 |
| IQTHSTTYR_369.5_627.3 | F13B_HUMAN | 0.777 |
| DTDTGALLFIGK_625.8_818.5 | PEDF_HUMAN | 0.774 |
| FICPLTGLWPINTLK_887.0_756.9 | APOH_HUMAN | 0.773 |
| DTDTGALLFIGK_625.8_217.1 | PEDF_HUMAN | 0.771 |
| FSVVYAK_407.2_579.4 | FETUA_HUMAN | 0.770 |
| IQTHSTTYR_369.5_540.3 | F13B_HUMAN | 0.769 |
| LDFHFSSDR_375.2_464.2 | INHBC_HUMAN | 0.769 |
| TLAFVR_353.7_274.2 | FA7_HUMAN | 0.769 |
| FLYHK_354.2_447.2 | AMBP_HUMAN | 0.766 |
| VNHVTLSQPK_374.9_459.3 | B2MG_HUMAN | 0.762 |
| AIGLPEELIQK_605.86_856.5 | FABPL_HUMAN | 0.752 |
| FLYHK_354.2_284.2 | AMBP_HUMAN | 0.752 |
| ELIEELVNITQNQK_557.6_517.3 | IL13_HUMAN | 0.751 |
| ETPEGAEAKPWYEPIYLGGVFQLEK_951.14_877.5 | TNFA_HUMAN | 0.751 |
| HFQNLGK_422.2_527.2 | AFAM_HUMAN | 0.749 |
| LIQDAVTGLTVNGQITGDK_972.0_640.4 | ITIH3_HUMAN | 0.749 |
| LIQDAVTGLTVNGQITGDK_972.0_798.4 | ITIH3_HUMAN | 0.747 |
| IAPQLSTEELVSLGEK_857.5_533.3 | AFAM_HUMAN | 0.745 |
| HFQNLGK_422.2_285.1 | AFAM_HUMAN | 0.740 |
| NNQLVAGYLQGPNVNLEEK_700.7_999.5 | IL1RA_HUMAN | 0.738 |
| VVESLAK_373.2_646.4 | IBP1_HUMAN | 0.738 |
| IAPQLSTEELVSLGEK_857.5_333.2 | AFAM_HUMAN | 0.737 |
| IALGGLLFPASNLR_481.3_657.4 | SHBG_HUMAN | 0.734 |
| ALALPPLGLAPLLNLWAKPQGR_770.5_256.2 | SHBG_HUMAN | 0.731 |
| ELPQSIVYK_538.8_417.7 | FBLN3_HUMAN | 0.724 |
| TFLTVYWTPER_706.9_401.2 | ICAM1_HUMAN | 0.723 |
| GVTGYFTFNLYLK_508.3_260.2 | PSG5_HUMAN | 0.717 |
| DVLLLVHNLPQNLTGHIWYK_791.8_310.2 | PSG7_HUMAN | 0.716 |
| WNFAYWAAHQPWSR_607.3_545.3 | PRG2_HUMAN | 0.716 |
| YTTEIIK_434.2_603.4 | C1R_HUMAN | 0.716 |
| YTTEIIK_434.2_704.4 | C1R_HUMAN | 0.716 |
| DIPHWLNPTR_416.9_600.3 | PAPP1_HUMAN | 0.715 |
| WNFAYWAAHQPWSR_607.3_673.3 | PRG2_HUMAN | 0.715 |
| IALGGLLFPASNLR_481.3_412.3 | SHBG_HUMAN | 0.713 |
| VVLSSGSGPGLDLPLVLGLPLQLK_791.5_598.4 | SHBG_HUMAN | 0.713 |
| GFQALGDAADIR_617.3_717.4 | TIMP1_HUMAN | 0.711 |
| VVLSSGSGPGLDLPLVLGLPLQLK_791.5_768.5 | SHBG_HUMAN | 0.711 |
| DVLLLVHNLPQNLTGHIWYK_791.8_883.0 | PSG7_HUMAN | 0.708 |
| YGIEEHGK_311.5_599.3 | CXA1_HUMAN | 0.706 |
| AEHPTWGDEQLFQTTR_639.3_765.4 | PGH1_HUMAN | 0.705 |
| VVESLAK_373.2_547.3 | IBP1_HUMAN | 0.705 |
| DADPDTFFAK_563.8_825.4 | AFAM_HUMAN | 0.704 |
| DAQYAPGYDK_564.3_813.4 | CFAB_HUMAN | 0.704 |
| GFQALGDAADIR_617.3_288.2 | TIMP1_HUMAN | 0.704 |
| AEHPTWGDEQLFQTTR_639.3_569.3 | PGH1_HUMAN | 0.702 |
| NFPSPVDAAFR_610.8_959.5 | HEMO_HUMAN | 0.702 |
| ALALPPLGLAPLLNLWAKPQGR_770.5_457.3 | SHBG_HUMAN | 0.701 |
| GVTGYFTFNLYLK_508.3_683.9 | PSG5_HUMAN | 0.701 |
| DFNQFSSGEK_386.8_189.1 | FETA_HUMAN | 0.699 |
| GDTYPAELYITGSILR_885.0_274.1 | F13B_HUMAN | 0.699 |
| TLEAQLTPR_514.8_685.4 | HEP2_HUMAN | 0.699 |
| VEHSDLSFSK_383.5_468.2 | B2MG_HUMAN | 0.699 |
| DAQYAPGYDK_564.3_315.1 | CFAB_HUMAN | 0.698 |
| VSEADSSNADWVTK_754.9_347.2 | CFAB_HUMAN | 0.698 |
| ILPSVPK_377.2_244.2 | PGH1_HUMAN | 0.695 |
| DADPDTFFAK_563.8_302.1 | AFAM_HUMAN | 0.694 |
| EVFSKPISWEELLQ_852.9_260.2 | FA40A_HUMAN | 0.694 |
| HTLNQIDEVK_598.8_958.5 | FETUA_HUMAN | 0.694 |
| NFPSPVDAAFR_610.8_775.4 | HEMO_HUMAN | 0.694 |
| VSFSSPLVAISGVALR_802.0_715.4 | PAPP1_HUMAN | 0.694 |
| TLAFVR_353.7_492.3 | FA7_HUMAN | 0.693 |
| ILPSVPK_377.2_227.2 | PGH1_HUMAN | 0.691 |
| LLEVPEGR_456.8_356.2 | C1S_HUMAN | 0.691 |
| TLEAQLTPR_514.8_814.4 | HEP2_HUMAN | 0.691 |
| IPSNPSHR_303.2_610.3 | FBLN3_HUMAN | 0.690 |
| LPNNVLQEK_527.8_730.4 | AFAM_HUMAN | 0.690 |
| NCSFSIIYPVVIK_770.4_555.4 | CRHBP_HUMAN | 0.690 |
| NCSFSIIYPVVIK_770.4_831.5 | CRHBP_HUMAN | 0.690 |
| VEHSDLSFSK_383.5_234.1 | B2MG_HUMAN | 0.690 |
| ALDLSLK_380.2_185.1 | ITIH3_HUMAN | 0.688 |
| IHWESASLLR_606.3_437.2 | CO3_HUMAN | 0.688 |
| IPSNPSHR_303.2_496.3 | FBLN3_HUMAN | 0.688 |
| LDGSTHLNIFFAK_488.3_852.5 | PAPP1_HUMAN | 0.687 |
| QGHNSVFLIK_381.6_260.2 | HEMO_HUMAN | 0.687 |
| AVLHIGEK_289.5_348.7 | THBG_HUMAN | 0.686 |
| VSEADSSNADWVTK_754.9_533.3 | CFAB_HUMAN | 0.686 |
| TNTNEFLIDVDK_704.85_849.5 | TF_HUMAN | 0.685 |
| AVLHIGEK_289.5_292.2 | THBG_HUMAN | 0.683 |
| HTLNQIDEVK_598.8_951.5 | FETUA_HUMAN | 0.683 |
| VSFSSPLVAISGVALR_802.0_602.4 | PAPP1_HUMAN | 0.683 |
| IAQYYYTFK_598.8_395.2 | F13B_HUMAN | 0.681 |
| ALDLSLK_380.2_575.3 | ITIH3_HUMAN | 0.680 |
| LLEVPEGR_456.8_686.4 | C1S_HUMAN | 0.680 |
| QGHNSVFLIK_381.6_520.4 | HEMO_HUMAN | 0.680 |
| SEPRPGVLLR_375.2_454.3 | FA7_HUMAN | 0.680 |
| SFRPFVPR_335.9_272.2 | LBP_HUMAN | 0.680 |
| AFQVWSDVTPLR_709.88_385.3 | MMP2_HUMAN | 0.679 |
| FAFNLYR_465.8_712.4 | HEP2_HUMAN | 0.679 |
| IAQYYYTFK_598.8_884.4 | F13B_HUMAN | 0.679 |
| ITGFLKPGK_320.9_429.3 | LBP_HUMAN | 0.679 |
| EHSSLAFWK_552.8_838.4 | APOH_HUMAN | 0.677 |
| GLQYAAQEGLLALQSELLR-1037.1_858.5 | LBP_HUMAN | 0.676 |
| YYLQGAK_421.7_327.1 | ITIH4_HUMAN | 0.676 |
| LIENGYFHPVK_439.6_627.4 | F13B_HUMAN | 0.675 |
| SFRPFVPR_335.9_635.3 | LBP_HUMAN | 0.675 |
| AALAAFNAQNNGSNFQLEEISR_789.1_746.4 | FETUA_HUMAN | 0.674 |
| ITGFLKPGK-320.9_301.2 | LBP_HUMAN | 0.673 |
| VQEVLLK_414.8_373.3 | HYOU1_HUMAN | 0.673 |
| YNSQLLSFVR_613.8_508.3 | TFR1_HUMAN | 0.673 |
| EHSSLAFWK_552.8_267.1 | APOH_HUMAN | 0.672 |
| FAFNLYR_465.8_565.3 | HEP2_HUMAN | 0.672 |
| GDTYPAELYITGSILR_885.0_922.5 | F13B_HUMAN | 0.672 |
| ITLPDFTGDLR_624.3_920.5 | LBP_HUMAN | 0.672 |
| NSDQEIDFK_548.3_409.2 | S10A5_HUMAN | 0.672 |
| TAVTANLDIR_537.3_802.4 | CHL1_HUMAN | 0.672 |
| YYLQGAK_421.7_516.3 | ITIH4_HUMAN | 0.672 |
| ITLPDFTGDLR_624.3_288.2 | LBP_HUMAN | 0.670 |
| AIGLPEELIQK_605.86_355.2 | FABPL_HUMAN | 0.669 |
| ALNFGGIGVVVGHELTHAFDDQGR_837.1_299.2 | ECE1_HUMAN | 0.668 |
| AQETSGEEISK_589.8_979.5 | IBP1_HUMAN | 0.668 |
| LPNNVLQEK_527.8_844.5 | AFAM_HUMAN | 0.668 |
| TGISPLALIK_506.8_654.5 | APOB_HUMAN | 0.666 |
| DFHINLFQVLPWLK_885.5_543.3 | CFAB_HUMAN | 0.665 |
| VQEVLLK_414.8_601.4 | HYOU1_HUMAN | 0.665 |
| YENYTSSFFIR_713.8_756.4 | IL12B_HUMAN | 0.665 |
| CRPINATLAVEK_457.9_559.3 | CGB1_HUMAN | 0.663 |
| LDGSTHLNIFFAK_488.3_739.4 | PAPP1_HUMAN | 0.663 |
| TGISPLALIK_506.8_741.5 | APOB_HUMAN | 0.663 |
| EVFSKPISWEELLQ_852.9_376.2 | FA40A_HUMAN | 0.662 |
| SLDFTELDVAAEK_719.4_874.5 | ANGT_HUMAN | 0.662 |
| TFLTVYWTPER_706.9_502.3 | ICAM1_HUMAN | 0.662 |
| VRPQQLVK_484.3_609.4 | ITIH4_HUMAN | 0.662 |
| GLQYAAQEGLLALQSELLR_1037.1_929.5 | LBP_HUMAN | 0.661 |
| NAVVQGLEQPHGLVVHPLR_688.4_890.6 | LRP1_HUMAN | 0.661 |
| SILFLGK_389.2_201.1 | THBG_HUMAN | 0.661 |
| DFNQFSSGEK_386.8_333.2 | FETA_HUMAN | 0.659 |
| IHWESASLLR_606.3_251.2 | CO3_HUMAN | 0.659 |
| SILFLGK_389.2_577.4 | THBG_HUMAN | 0.658 |
| SVSLPSLDPASAK_636.4_473.3 | APOB_HUMAN | 0.658 |
| WWGGQPLWITATK_772.4_929.5 | ENPP2_HUMAN | 0.658 |
| LNIGYIEDLK_589.3_950.5 | PAI2_HUMAN | 0.657 |
| DFHINLFQVLPWLK_885.5_400.2 | CFAB_HUMAN | 0.657 |
| YSHYNER_323.48_418.2 | HABP2_HUMAN | 0.657 |
| STLFVPR_410.2_272.2 | PEPD_HUMAN | 0.656 |
| AFQVWSDVTPLR_709.88_347.2 | MMP2_HUMAN | 0.655 |
| FQSVFTVTR_542.8_722.4 | C1QC_HUMAN | 0.655 |
| GPGEDFR_389.2_623.3 | PTGDS_HUMAN | 0.655 |
| LEEHYELR_363.5_288.2 | PAI2_HUMAN | 0.655 |
| LPDTPQGLLGEAR_683.87_427.2 | EGLN_HUMAN | 0.655 |
| FQSVFTVTR_542.79_722.4 | C1QC_HUMAN | 0.654 |
| FTFTLHLETPKPSISSSNLNPR_829.4_787.4 | PSG1_HUMAN | 0.654 |
| NHYTESISVAK_624.8_252.1 | NEUR1_HUMAN | 0.654 |
| YSHYNER_323.48_581.3 | HABP2_HUMAN | 0.654 |
| FQSVFTVTR_542.79_623.4 | C1QC_HUMAN | 0.652 |
| IEGNLIFDPNNYLPK_874.0_845.5 | APOB_HUMAN | 0.652 |
| VRPQQLVK_484.3_722.4 | ITIH4_HUMAN | 0.652 |
| WILTAAHTLYPK_471.9_621.4 | C1R_HUMAN | 0.652 |
| ITQDAQLK_458.8_803.4 | CBG_HUMAN | 0.651 |
| SVSLPSLDPASAK_636.4_885.5 | APOB_HUMAN | 0.651 |
| ESDTSYVSLK_564.8_347.2 | CRP_HUMAN | 0.650 |
| ESDTSYVSLK_564.8_696.4 | CRP_HUMAN | 0.650 |
| FQSVFTVTR_542.8_623.4 | C1QC_HUMAN | 0.650 |
| HELTDEELQSLFTNFANVVDK_817.1_854.4 | AFAM_HUMAN | 0.650 |
| IEGNLIFDPNNYLPK_874.0_414.2 | APOB_HUMAN | 0.650 |
| DIIKPDPPK_511.8_342.2 | IL12B_HUMAN | 0.648 |
| SPELQAEAK_486.8_788.4 | APOA2_HUMAN | 0.648 |
| VELAPLPSWQPVGK_760.9_400.3 | ICAM1_HUMAN | 0.648 |
| AQETSGEEISK_589.8_850.4 | IBP1_HUMAN | 0.647 |
| QTLSWTVTPK_580.8_545.3 | PZP_HUMAN | 0.647 |
| DISEVVTPR_508.3_787.4 | CFAB_HUMAN | 0.645 |
| DVLLLVHNLPQNLPGYFWYK_810.4_328.2 | PSG9_HUMAN | 0.645 |
| QTLSWTVTPK_580.8_818.4 | PZP_HUMAN | 0.645 |
| SGAQATWTELPWPHEK_613.3_510.3 | HEMO_HUMAN | 0.645 |
| SLDFTELDVAAEK_719.4_316.2 | ANGT_HUMAN | 0.645 |
| AVGYLITGYQR_620.8_523.3 | PZP_HUMAN | 0.644 |
| DISEVVTPR_508.3_472.3 | CFAB_HUMAN | 0.644 |
| FLNWIK_410.7_560.3 | HABP2_HUMAN | 0.644 |
| IQHPFTVEEFVLPK_562.0_861.5 | PZP_HUMAN | 0.644 |
| ALQDQLVLVAAK_634.9_289.2 | ANGT_HUMAN | 0.643 |
| AVGYLITGYQR_620.8_737.4 | PZP_HUMAN | 0.643 |
| FLNWIK_410.7_561.3 | HABP2_HUMAN | 0.643 |
| LEQGENVFLQATDK_796.4_822.4 | C1QB_HUMAN | 0.643 |
| LSITGTYDLK_555.8_797.4 | A1AT_HUMAN | 0.641 |
| SEPRPGVLLR_375.2_654.4 | FA7_HUMAN | 0.641 |
| VPGLYYFTYHASSR_554.3_720.3 | C1QB_HUMAN | 0.641 |
| APLTKPLK_289.9_357.2 | CRP_HUMAN | 0.639 |
| FNAVLTNPQGDYDTSTGK_964.5_333.2 | C1QC_HUMAN | 0.639 |
| IQHPFTVEEFVLPK_562.0_603.4 | PZP_HUMAN | 0.639 |
| LSSPAVITDK_515.8_743.4 | PLMN_HUMAN | 0.639 |
| ALNFGGIGVVVGHELTHAFDDQGR_837.1_360.2 | ECE1_HUMAN | 0.637 |
| FNAVLTNPQGDYDTSTGK_964.5_262.1 | C1QC_HUMAN | 0.637 |
| LLELTGPK_435.8_227.2 | A1BG_HUMAN | 0.637 |
| YNSQLLSFVR_613.8_734.5 | TFR1_HUMAN | 0.636 |
| DLYHYITSYVVDGEIIIYGPAYSGR_955.5_707.3 | PSG1_HUMAN | 0.634 |
| GPGEDFR_389.2_322.2 | PTGDS_HUMAN | 0.634 |
| IHPSYTNYR_575.8_813.4 | PSG2_HUMAN | 0.634 |
| SGAQATWTELPWPHEK_613.3_793.4 | HEMO_HUMAN | 0.634 |
| SPELQAEAK_486.8_659.4 | APOA2_HUMAN | 0.634 |
| ALQDQLVLVAAK_634.9_956.6 | ANGT_HUMAN | 0.633 |
| ITENDIQIALDDAK_779.9_632.3 | APOB_HUMAN | 0.632 |
| ITQDAQLK_458.8_702.4 | CBG_HUMAN | 0.632 |
| LSSPAVITDK_515.8_830.5 | PLMN_HUMAN | 0.632 |
| SLLQPNK_400.2_358.2 | CO8A_HUMAN | 0.632 |
| VPGLYYFTYHASSR_554.3_420.2 | C1QB_HUMAN | 0.632 |
| YGLVTYATYPK_638.3_843.4 | CFAB_HUMAN | 0.632 |
| AGITIPR_364.2_486.3 | IL17_HUMAN | 0.630 |
| IHPSYTNYR_575.8_598.3 | PSG2 HUMAN | 0.630 |
| QINSYVK_426.2_610.3 | CBG_HUMAN | 0.630 |
| SSNNPHSPIVEEFQVPYNK_729.4_261.2 | C1S_HUMAN | 0.630 |
| ANDQYLTAAALHNLDEAVK_686.3_317.2 | IL1A_HUMAN | 0.629 |
| ATWSGAVLAGR_544.8_730.4 | A1BG_HUMAN | 0.629 |
| TLPFSR_360.7_506.3 | LYAM1_HUMAN | 0.629 |
| TYLHTYESEI_628.3_515.3 | ENPP2_HUMAN | 0.629 |
| EFDDDTYDNDIALLQLK_1014.48_388.3 | TPA_HUMAN | 0.627 |
| EFDDDTYDNDIALLQLK_1014.48_501.3 | TPA_HUMAN | 0.627 |
| VTGLDFIPGLHPILTLSK_641.04_771.5 | LEP_HUMAN | 0.627 |
| HVVQLR_376.2_614.4 | IL6RA_HUMAN | 0.626 |
| LIENGYFHPVK_439.6_343.2 | F13B_HUMAN | 0.626 |
| LLELTGPK_435.8_644.4 | A1BG_HUMAN | 0.626 |
| YEVQGEVFTKPQLWP_911.0_392.2 | CRP_HUMAN | 0.626 |
| DPNGLPPEAQK_583.3_497.2 | RET4_HUMAN | 0.625 |
| FTFTLHLETPKPSISSSNLNPR_829.4_874.4 | PSG1_HUMAN | 0.625 |
| YGLVTYATYPK_638.3_334.2 | CFAB_HUMAN | 0.625 |
| APLTKPLK_289.9_398.8 | CRP_HUMAN | 0.623 |
| DSPSVWAAVPGK_607.31_301.2 | PROF1_HUMAN | 0.623 |
| ENPAVIDFELAPIVDLVR_670.7_811.5 | CO6_HUMAN | 0.623 |
| ILILPSVTR_506.3_559.3 | PSGx_HUMAN | 0.623 |
| SFEGLGQLEVLTLDHNQLQEVK_833.1_503.3 | ALS_HUMAN | 0.623 |
| TSESGELHGLTTEEEFVEGIYK_819.06_310.2 | TTHY_HUMAN | 0.623 |
| AGITIPR_364.2_272.2 | IL17_HUMAN | 0.622 |
| DPDQTDGLGLSYLSSHIANVER_796.4_328.1 | GELS_HUMAN | 0.622 |
| ATWSGAVLAGR_544.8_643.4 | A1BG_HUMAN | 0.620 |
| HVVQLR_376.2_515.3 | IL6RA_HUMAN | 0.620 |
| QINSYVK_426.2_496.3 | CBG_HUMAN | 0.620 |
| TLFIFGVTK_513.3_215.1 | PSG4_HUMAN | 0.620 |
| YEVQGEVFTKPQLWP_911.0_293.1 | CRP_HUMAN | 0.620 |
| YYGYTGAFR_549.3_771.4 | TRFL_HUMAN | 0.620 |
| AALAAFNAQNNGSNFQLEEISR_789.1_633.3 | FETUA_HUMAN | 0.619 |
| ALNHLPLEYNSALYSR_621.0_696.4 | CO6_HUMAN | 0.619 |
| EDTPNSVWEPAK_686.8_630.3 | C1S_HUMAN | 0.619 |
| NNQLVAGYLQGPNVNLEEK_700.7_357.2 | IL1RA_HUMAN | 0.619 |
| ELANTIK_394.7_475.3 | S10AC_HUMAN | 0.618 |
| ENPAVIDFELAPIVDLVR_670.7_601.4 | CO6_HUMAN | 0.618 |
| GEVTYTTSQVSK_650.3_913.5 | EGLN_HUMAN | 0.616 |
| NEIWYR_440.7_637.4 | FA12_HUMAN | 0.616 |
| TLFIFGVTK_513.3_811.5 | PSG4_HUMAN | 0.616 |
| DLYHYITSYVVDGEIIIYGPAYSGR_955.5_650.3 | PSG1_HUMAN | 0.615 |
| DPTFIPAPIQAK_433.2_556.3 | ANGT_HUMAN | 0.615 |
| VELAPLPSWQPVGK_760.9_342.2 | ICAM1_HUMAN | 0.615 |
| DPNGLPPEAQK_583.3_669.4 | RET4_HUMAN | 0.614 |
| GIVEECCFR_585.3_900.3 | IGF2_HUMAN | 0.614 |
| ITENDIQIALDDAK_779.9_873.5 | APOB_HUMAN | 0.614 |
| LSETNR_360.2_330.2 | PSG1_HUMAN | 0.614 |
| LSNENHGIAQR_413.5_519.8 | ITIH2_HUMAN | 0.614 |
| YEFLNGR_449.7_293.1 | PLMN_HUMAN | 0.614 |
| AEIEYLEK_497.8_552.3 | LYAM1_HUMAN | 0.612 |
| GIVEECCFR_585.3_771.3 | IGF2_HUMAN | 0.612 |
| ILDDLSPR_464.8_587.3 | ITIH4_HUMAN | 0.611 |
| IRPHTFTGLSGLR_485.6_545.3 | ALS_HUMAN | 0.611 |
| VVGGLVALR_442.3_784.5 | FA12_HUMAN | 0.609 |
| LEEHYELR_363.5_417.2 | PAI2_HUMAN | 0.609 |
| LSNENHGIAQR_413.5_544.3 | ITIH2_HUMAN | 0.609 |
| TYLHTYESEI_628.3_908.4 | ENPP2_HUMAN | 0.609 |
| VLEPTLK_400.3_587.3 | VTDB_HUMAN | 0.609 |
| ILILPSVTR_506.3_785.5 | PSGx_HUMAN | 0.608 |
| TAVTANLDIR_537.3_288.2 | CHL1_HUMAN | 0.608 |
| WWGGQPLWITATK_772.4_373.2 | ENPP2_HUMAN | 0.607 |
| ALVLELAK_428.8_672.4 | INHBE_HUMAN | 0.605 |
| EAQLPVIENK_570.8_329.2 | PLMN_HUMAN | 0.605 |
| QRPPDLDTSSNAVDLLFFTDESGDSR_961.5_866.3 | C1R_HUMAN | 0.605 |
| TDAPDLPEENQAR_728.3_613.3 | CO5_HUMAN | 0.605 |
| TLPFSR_360.7_409.2 | LYAM1_HUMAN | 0.605 |
| VQTAHFK_277.5_502.3 | CO8A_HUMAN | 0.605 |
| ANLINNIFELAGLGK_793.9_299.2 | LCAP_HUMAN | 0.604 |
| FQLPGQK_409.2_275.1 | PSG1_HUMAN | 0.604 |
| NTVISVNPSTK_580.3_845.5 | VCAM1_HUMAN | 0.604 |
| VLEPTLK_400.3_458.3 | VTDB_HUMAN | 0.604 |
| YWGVASFLQK_599.8_849.5 | RET4_HUMAN | 0.604 |
| AGPLQAR_356.7_584.4 | DEF4_HUMAN | 0.602 |
| AHQLAIDTYQEFEETYIPK_766.0_521.3 | CSH_HUMAN | 0.602 |
| DLHLSDVFLK_396.2_366.2 | CO6_HUMAN | 0.602 |
| SSNNPHSPIVEEFQVPYNK_729.4_521.3 | C1S_HUMAN | 0.602 |
| YWGVASFLQK_599.8_350.2 | RET4_HUMAN | 0.602 |
| AGPLQAR_356.7_487.3 | DEF4_HUMAN | 0.601 |
| ALNHLPLEYNSALYSR_621.0_538.3 | CO6_HUMAN | 0.601 |
| EAQLPVIENK_570.8_699.4 | PLMN_HUMAN | 0.601 |
| EDTPNSVWEPAK_686.8_315.2 | C1S_HUMAN | 0.601 |
| NTVISVNPSTK_580.3_732.4 | VCAM1_HUMAN | 0.601 |

**Table 15. Univariate AUC values middle-late combined windows**

| Transition | Protein | AUC |
|---|---|---|
| GDTYPAELYITGSILR_885.0_274.1 | F13B_HUMAN | 0.7750 |
| TVQAVLTVPK_528.3_28.3 | PEDF_HUMAN | 0.7667 |
| IQTHSTTYR_369.5_627.3 | F13B_HUMAN | 0.7667 |
| DVLLLVHNLPQNLTGHIWYK_791.8_310.2 | PSG7_HUMAN | 0.7667 |
| IQTHSTTYR_369.5_540.3 | F13B_HUMAN | 0.7646 |
| ALALPPLGLAPLLNLWAKPQGR_770.5_256.2 | SHBG_HUMAN | 0.7646 |
| VVLSSGSGPGLDLPLVLGLPLQLK_791.5_768.5 | SHBG_HUMAN | 0.7625 |
| VVLSSGSGPGLDLPLVLGLPLQLK_791.5_598.4 | SHBG_HUMAN | 0.7625 |
| TVQAVLTVPK_528.3_855.5 | PEDF_HUMAN | 0.7604 |
| GDTYPAELYITGSILR_885.0_922.5 | F13B_HUMAN | 0.7604 |
| DVLLLVHNLPQNLTGHIWYK_791.8_883.0 | PSG7_HUMAN | 0.7604 |
| TLPFSR_360.7_506.3 | LYAM1_HUMAN | 0.7563 |
| ALALPPLGLAPLLNLWAKPQGR_770.5_457.3 | SHBG_HUMAN | 0.7563 |
| IALGGLLFPASNLR_481.3_657.4 | SHBG_HUMAN | 0.7542 |
| IALGGLLFPASNLR_481.3_412.3 | SHBG_HUMAN | 0.7542 |
| DTDTGALLFIGK_625.8_217.1 | PEDF_HUMAN | 0.7500 |
| QGFGNVATNTDGK_654.81_706.3 | FIBB_HUMAN | 0.7438 |
| ETLLQDFR_511.3_565.3 | AMBP_HUMAN | 0.7438 |
| ETLLQDFR_511.3_322.2 | AMBP_HUMAN | 0.7417 |
| IAQYYYTFK_598.8_884.4 | F13B_HUMAN | 0.7396 |
| DTDTGALLFIGK_625.8_818.5 | PEDF_HUMAN | 0.7396 |
| AEIEYLEK_497.8_552.3 | LYAM1_HUMAN | 0.7396 |
| LDFHFSSDR_375.2_611.3 | INHBC_HUMAN | 0.7354 |
| YQISVNK_426.2_560.3 | FIBB_HUMAN | 0.7333 |
| IAPQLSTEELVSLGEK_857.5_533.3 | AFAM_HUMAN | 0.7313 |
| EVFSKPISWEELLQ_852.9_376.2 | FA40A_HUMAN | 0.7292 |
| TLAFVR_353.7_274.2 | FA7_HUMAN | 0.7229 |
| HHGPTITAK_321.2_275.1 | AMBP_HUMAN | 0.7229 |
| SLQAFVAVAAR_566.8_487.3 | IL23A_HUMAN | 0.7208 |
| IAQYYYTFK_598.8_395.2 | F13B_HUMAN | 0.7208 |
| EVFSKPISWEELLQ_852.9_260.2 | FA40A_HUMAN | 0.7208 |
| DPNGLPPEAQK_583.3_669.4 | RET4_HUMAN | 0.7208 |
| DPNGLPPEAQK_583.3_497.2 | RET4_HUMAN | 0.7167 |
| VEHSDLSFSK_383.5_468.2 | B2MG_HUMAN | 0.7146 |
| YQISVNK_426.2_292.1 | FIBB_HUMAN | 0.7125 |
| TLAFVR_353.7_492.3 | FA7_HUMAN | 0.7125 |
| IAPQLSTEELVSLGEK_857.5_333.2 | AFAM_HUMAN | 0.7125 |
| AEIEYLEK_497.8_389.2 | LYAM1_HUMAN | 0.7125 |
| YWGVASFLQK_599.8_849.5 | RET4_HUMAN | 0.7104 |
| TLPFSR_360.7_409.2 | LYAM1_HUMAN | 0.7104 |
| HFQNLGK_422.2_527.2 | AFAM_HUMAN | 0.7104 |
| TQILEWAAER_608.8_761.4 | EGLN_HUMAN | 0.7083 |
| HFQNLGK_422.2_285.1 | AFAM_HUMAN | 0.7063 |
| FTFTLHLETPKPSISSSNLNPR_829.4_787.4 | PSG1_HUMAN | 0.7063 |
| DPDQTDGLGLSYLSSHIANVER_796.4_456.2 | GELS_HUMAN | 0.7063 |
| DADPDTFFAK_563.8_825.4 | AFAM_HUMAN | 0.7042 |
| YWGVASFLQK_599.8_350.2 | RET4_HUMAN | 0.7021 |
| DADPDTFFAK_563.8_302.1 | AFAM_HUMAN | 0.7021 |
| HHGPTITAK_321.2_432.3 | AMBP_HUMAN | 0.6979 |
| NTVISVNPSTK_580.3_845.5 | VCAM1_HUMAN | 0.6958 |
| FLYHK_354.2_447.2 | AMBP_HUMAN | 0.6958 |
| FICPLTGLWPINTLK_887.0_685.4 | APOH_HUMAN | 0.6958 |
| FTFTLHLETPKPSISSSNLNPR_829.4_874.4 | PSG1_HUMAN | 0.6938 |
| FLYHK_354.2_284.2 | AMBP_HUMAN | 0.6938 |
| EALVPLVADHK_397.9_390.2 | HGFA_HUMAN | 0.6938 |
| LNIGYIEDLK_589.3_837.4 | PAI2_HUMAN | 0.6917 |
| QGFGNVATNTDGK_654.81_319.2 | FIBB_HUMAN | 0.6896 |
| EALVPLVADHK_397.9_439.8 | HGFA_HUMAN | 0.6896 |
| TNTNEFLIDVDK_704.85_849.5 | TF_HUMAN | 0.6875 |
| DTYVSSFPR_357.8_272.2 | TCEA1_HUMAN | 0.6813 |
| VNHVTLSQPK_374.9_244.2 | B2MG_HUMAN | 0.6771 |
| GPGEDFR_389.2_623.3 | PTGDS_HUMAN | 0.6771 |
| GEVTYTTSQVSK_650.3_913.5 | EGLN_HUMAN | 0.6771 |
| GEVTYTTSQVSK_650.3_750.4 | EGLN_HUMAN | 0.6771 |
| FICPLTGLWPINTLK_887.0_756.9 | APOH_HUMAN | 0.6771 |
| YEFLNGR_449.7_606.3 | PLMN_HUMAN | 0.6750 |
| YEFLNGR_449.7_293.1 | PLMN_HUMAN | 0.6750 |
| TLFIFGVTK_513.3_215.1 | PSG4_HUMAN | 0.6750 |
| LNIGYIEDLK_589.3_950.5 | PAI2_HUMAN | 0.6750 |
| LLELTGPK_435.8_227.2 | AlBG_HUMAN | 0.6750 |
| TPSAAYLWVGTGASEAEK_919.5_849.4 | GELS_HUMAN | 0.6729 |
| FQLPGQK_409.2_275.1 | PSG1_HUMAN | 0.6729 |
| ELIEELVNITQNQK_557.6_618.3 | IL13_HUMAN | 0.6729 |
| DLYHYITSYVVDGEIIIYGPAYSGR_955.5_707.3 | PSG1_HUMAN | 0.6729 |
| AHYDLR_387.7_566.3 | FETUA_HUMAN | 0.6729 |
| LLEVPEGR_456.8_356.2 | CIS_HUMAN | 0.6708 |
| TLFIFGVTK_513.3_811.5 | PSG4_HUMAN | 0.6688 |
| FQLPGQK_409.2_429.2 | PSG1_HUMAN | 0.6667 |
| DLYHYITSYVVDGEIIIYGPAYSGR_955.5_650.3 | PSG1_HUMAN | 0.6667 |
| YYLQGAK_421.7_516.3 | ITIH4_HUMAN | 0.6646 |
| FSVVYAK_407.2_579.4 | FETUA_HUMAN | 0.6646 |
| EQLGEFYEALDCLR_871.9_747.4 | A1AG1_HUMAN | 0.6646 |
| LDFHFSSDR_375.2_464.2 | INHBC_HUMAN | 0.6625 |
| ALNHLPLEYNSALYSR_621.0_696.4 | CO6_HUMAN | 0.6625 |
| YYLQGAK_421.7_327.1 | ITIH4_HUMAN | 0.6604 |
| YTTEIIK_434.2_704.4 | C1R_HUMAN | 0.6604 |
| VEHSDLSFSK_383.5_234.1 | B2MG_HUMAN | 0.6604 |
| SNPVTLNVLYGPDLPR_585.7_654.4 | PSG6_HUMAN | 0.6604 |
| LWAYLTIQELLAK_781.5_300.2 | ITIH1_HUMAN | 0.6604 |
| FSLVSGWGQLLDR_493.3_403.2 | FA7_HUMAN | 0.6604 |
| ATVVYQGER_511.8_652.3 | APOH_HUMAN | 0.6604 |
| TPSAAYLWVGTGASEAEK_919.5_428.2 | GELS_HUMAN | 0.6583 |
| SEPRPGVLLR_375.2_454.3 | FA7_HUMAN | 0.6583 |
| LSSPAVITDK_515.8_830.5 | PLMN_HUMAN | 0.6583 |
| GPGEDFR_389.2_322.2 | PTGDS_HUMAN | 0.6583 |
| EFDDDTYDNDIALLQLK_1014.48_501.3 | TPA_HUMAN | 0.6583 |
| TFLTVYWTPER_706.9_502.3 | ICAM1_HUMAN | 0.6563 |
| NTVISVNPSTK_580.3_732.4 | VCAM1_HUMAN | 0.6563 |
| LPNNVLQEK_527.8_730.4 | AFAM_HUMAN | 0.6563 |
| LPDTPQGLLGEAR_683.87_427.2 | EGLN_HUMAN | 0.6563 |
| VANYVDWINDR_682.8_818.4 | HGFA_HUMAN | 0.6542 |
| LSSPAVITDK_515.8_743.4 | PLMN_HUMAN | 0.6542 |
| LPNNVLQEK_527.8_844.5 | AFAM_HUMAN | 0.6542 |
| IPGIFELGISSQSDR_809.9_849.4 | CO8B_HUMAN | 0.6542 |
| GAVHVVVAETDYQSFAVLYLER_822.8_580.3 | CO8G_HUMAN | 0.6542 |
| FLNWIK_410.7_560.3 | HABP2_HUMAN | 0.6542 |
| TFLTVYWTPER_706.9_401.2 | ICAM1_HUMAN | 0.6521 |
| NKPGVYTDVAYYLAWIR_677.0_821.5 | FA12_HUMAN | 0.6521 |
| AHYDLR_387.7_288.2 | FETUA_HUMAN | 0.6521 |
| LLEVPEGR 456.8 686.4 | CIS_HUMAN | 0.6500 |
| LIENGYFHPVK_439.6_627.4 | F13B_HUMAN | 0.6500 |
| GFQALGDAADIR_617.3_717.4 | TIMP1_HUMAN | 0.6500 |
| ELIEELVNITQNQK_557.6_517.3 | IL13_HUMAN | 0.6500 |
| EAQLPVIENK_570.8_329.2 | PLMN_HUMAN | 0.6479 |
| CRPINATLAVEK_457.9_559.3 | CGB1_HUMAN | 0.6479 |
| ATVVYQGER_511.8_751.4 | APOH_HUMAN | 0.6479 |
| ALNHLPLEYNSALYSR_621.0_538.3 | CO6_HUMAN | 0.6479 |
| AHQLAIDTYQEFEETYIPK_766.0_634.4 | CSH_HUMAN | 0.6479 |
| VTGLDFIPGLHPILTLSK_641.04_771.5 | LEP_HUMAN | 0.6458 |
| VANYVDWINDR_682.8_917.4 | HGFA_HUMAN | 0.6458 |
| SSNNPHSPIVEEFQVPYNK_729.4_261.2 | C1S_HUMAN | 0.6458 |
| NKPGVYTDVAYYLAWIR_677.0_545.3 | FA12_HUMAN | 0.6458 |
| GSLVQASEANLQAAQDFVR_668.7_735.4 | ITIH1_HUMAN | 0.6458 |
| YTTEIIK_434.2_603.4 | C1R_HUMAN | 0.6438 |
| NEIVFPAGILQAPFYTR_968.5_357.2 | ECE1_HUMAN | 0.6438 |
| IPGIFELGISSQSDR_809.9_679.3 | CO8B_HUMAN | 0.6438 |
| SNPVTLNVLYGPDLPR_585.7_817.4 | PSG6_HUMAN | 0.6417 |
| LLELTGPK_435.8_644.4 | A1BG_HUMAN | 0.6417 |
| EAQLPVIENK_570.8_699.4 | PLMN_HUMAN | 0.6417 |
| AEHPTWGDEQLFQTTR_639.3_765.4 | PGH1_HUMAN | 0.6417 |
| YGIEEHGK_311.5_599.3 | CXA1_HUMAN | 0.6396 |
| TQIDSPLSGK_523.3_703.4 | VCAM1_HUMAN | 0.6396 |
| YHFEALADTGISSEFYDNANDLLSK_940.8_301.1 | CO8A_HUMAN | 0.6375 |
| SCDLALLETYCATPAK_906.9_315.2 | IGF2_HUMAN | 0.6375 |
| NAVVQGLEQPHGVVHPLR_688.4_285.2 | LRP1_HUMAN | 0.6375 |
| HVVQLR_376.2_614.4 | IL6RA_HUMAN | 0.6375 |
| NNQLVAGYLQGPNVNLEEK_700.7_999.5 | IL1RA_HUMAN | 0.6354 |
| GIVEECCFR_585.3_771.3 | IGF2_HUMAN | 0.6354 |
| DGSPDVTTADIGANTPDATK_973.5_531.3 | PGRP2_HUMAN | 0.6354 |
| AEHPTWGDEQLFQTTR_639.3_569.3 | PGH1_HUMAN | 0.6354 |
| YVVISQGLDKPR_458.9_400.3 | LRP1_HUMAN | 0.6333 |
| WGAAPYR_410.7_577.3 | PGRP2_HUMAN | 0.6333 |
| VRPQQLVK_484.3_609.4 | ITIH4_HUMAN | 0.6333 |
| AVYEAVLR_460.8_750.4 | PEPD_HUMAN | 0.6333 |
| TQIDSPLSGK_523.3_816.5 | VCAM1_HUMAN | 0.6313 |
| IPKPEASFSPR_410.2_359.2 | ITIH4_HUMAN | 0.6313 |
| HELTDEELQSLFTNFANVVDK_817.1_854.4 | AFAM_HUMAN | 0.6313 |
| GSLVQASEANLQAAQDFVR_668.7_806.4 | ITIH1_HUMAN | 0.6313 |
| GAVHVVVAETDYQSFAVLYLER_822.8_863.5 | CO8G_HUMAN | 0.6313 |
| ENPAVIDFELAPIVDLVR_670.7_811.5 | CO6_HUMAN | 0.6313 |
| VRPQQLVK_484.3_722.4 | ITIH4_HUMAN | 0.6292 |
| IRPFFPQQ_516.79_372.2 | FIBB_HUMAN | 0.6292 |
| LWAYLTIQELLAK_781.5_371.2 | ITIH1_HUMAN | 0.6271 |
| EQLGEFYEALDCLR_871.9_563.3 | A1AG1_HUMAN | 0.6271 |
| LLDFEFSSGR_585.8_553.3 | G6PE_HUMAN | 0.6250 |
| LIENGYFHPVK_439.6_343.2 | F13B_HUMAN | 0.6250 |
| ENPAVIDFELAPIVDLVR_670.7_601.4 | CO6_HUMAN | 0.6250 |
| WNFAYWAAHQPWSR_607.3_545.3 | PRG2_HUMAN | 0.6229 |
| TAVTANLDIR_537.3_802.4 | CHL1_HUMAN | 0.6229 |
| WNFAYWAAHQPWSR_607.3_673.3 | PRG2_HUMAN | 0.6208 |
| HTLNQIDEVK_598.8_951.5 | FETUA_HUMAN | 0.6208 |
| DPDQTDGLGLSYLSSHIANVER_796.4_328.1 | GELS_HUMAN | 0.6208 |
| WGAAPYR_410.7_634.3 | PGRP2_HUMAN | 0.6188 |
| TEQAAVAR_423.2_487.3 | FA12_HUMAN | 0.6188 |
| LEEHYELR_363.5_288.2 | PAI2_HUMAN | 0.6188 |
| GIVEECCFR_585.3_900.3 | IGF2_HUMAN | 0.6188 |
| YHFEALADTGISSEFYDNANDLLSK_940.8_874.5 | CO8A_HUMAN | 0.6167 |
| TQILEWAAER_608.8_632.3 | EGLN_HUMAN | 0.6167 |
| DSPSVWAAVPGK_607.31_301.2 | PROF1_HUMAN | 0.6167 |
| DLHLSDVFLK_396.2_260.2 | CO6_HUMAN | 0.6167 |
| AQPVQVAEGSEPDGFWEALGGK_758.0_574.3 | GELS_HUMAN | 0.6167 |
| YSHYNER_323.48_581.3 | HABP2_HUMAN | 0.6146 |
| YSHYNER_323.48_418.2 | HABP2_HUMAN | 0.6146 |
| VNHVTLSQPK_374.9_459.3 | B2MG_HUMAN | 0.6146 |
| EHSSLAFWK_552.8_267.1 | APOH_HUMAN | 0.6146 |
| TATSEYQTFFNPR_781.4_386.2 | THRB_HUMAN | 0.6104 |
| SGFSFGFK_438.7_732.4 | CO8B_HUMAN | 0.6104 |
| GFQALGDAADIR_617.3_288.2 | TIMP1_HUMAN | 0.6104 |
| FSVVYAK_407.2_381.2 | FETUA_HUMAN | 0.6104 |
| QTLSWTVTPK_580.8_545.3 | PZP_HUMAN | 0.6083 |
| QLGLPGPPDVPDHAAYHPF_676.7_263.1 | ITIH4_HUMAN | 0.6083 |
| LSITGTYDLK_555.8_797.4 | A1AT_HUMAN | 0.6083 |
| LPDTPQGLLGEAR_683.87_940.5 | EGLN_HUMAN | 0.6083 |
| VVESLAK_373.2_646.4 | IBP1_HUMAN | 0.6063 |
| VSEADSSNADWVTK_754.9_347.2 | CFAB_HUMAN | 0.6063 |
| TEQAAVAR_423.2_615.4 | FA12_HUMAN | 0.6063 |
| SEPRPGVLLR_375.2_654.4 | FA7_HUMAN | 0.6063 |
| QTLSWTVTPK_580.8_818.4 | PZP_HUMAN | 0.6063 |
| HYINLITR_515.3_301.1 | NPY_HUMAN | 0.6063 |
| DPTFIPAPIQAK_433.2_461.2 | ANGT_HUMAN | 0.6063 |
| VSEADSSNADWVTK_754.9_533.3 | CFAB_HUMAN | 0.6042 |
| VQEVLLK_414.8_373.3 | HYOU1_HUMAN | 0.6042 |
| SILFLGK_389.2_577.4 | THBG_HUMAN | 0.6042 |
| IQHPFTVEEFVLPK_562.0_603.4 | PZP_HUMAN | 0.6042 |
| ELPQSIVYK_538.8_417.7 | FBLN3_HUMAN | 0.6042 |
| AVGYLITGYQR_620.8_737.4 | PZP_HUMAN | 0.6042 |
| ATWSGAVLAGR_544.8_643.4 | A1BG_HUMAN | 0.6042 |
| AKPALEDLR_506.8_288.2 | APOA1_HUMAN | 0.6042 |
| SEYGAALAWEK_612.8_845.5 | CO6_HUMAN | 0.6021 |
| NVNQSLLELHK_432.2_656.3 | FRIH_HUMAN | 0.6021 |
| IQHPFTVEEFVLPK_562.0_861.5 | PZP_HUMAN | 0.6021 |
| IPKPEASFSPR_410.2_506.3 | ITIH4_HUMAN | 0.6021 |
| GVTGYFTFNLYLK_508.3_260.2 | PSG5_HUMAN | 0.6021 |
| DGSPDVTTADIGANTPDATK_973.5_844.4 | PGRP2_HUMAN | 0.6021 |
| AVGYLITGYQR_620.8_523.3 | PZP_HUMAN | 0.6021 |
| ANDQYLTAAALHNLDEAVK_686.3_317.2 | IL1A_HUMAN | 0.6021 |
| TLYSSSPR_455.7_696.3 | IC1_HUMAN | 0.6000 |
| LHKPGVYTR_357.5_479.3 | HGFA_HUMAN | 0.6000 |
| IIGGSDADIK_494.8_260.2 | C1S_HUMAN | 0.6000 |
| HELTDEELQSLFTNFANVVDK_817.1_906.5 | AFAM_HUMAN | 0.6000 |
| GGEGTGYFVDFSVR_745.9_869.5 | HRG_HUMAN | 0.6000 |
| AVLHIGEK_289.5_348.7 | THBG_HUMAN | 0.6000 |
| ALVLELAK_428.8_672.4 | INHBE_HUMAN | 0.6000 |

**Table 16. Lasso Summed Coefficients All Windows**

| Transition | Protein | SumBestCoefs_A1 1 |
|---|---|---|
| TQILEWAAER_608.8_761.4 | EGLN_HUMAN | 26.4563 |
| GFQALGDAADIR_617.3_717.4 | TIMP1_HUMAN | 17.6447 |
| AVDIPGLEAATPYR_736.9_399.2 | TENA_HUMAN | 16.2270 |
| TVQAVLTVPK_528.3_428.3 | PEDF_HUMAN | 15.1166 |
| LDFHFSSDR_375.2_611.3 | INHBC_HUMA N | 15.0029 |
| ATVVYQGER_511.8_652.3 | APOH_HUMAN | 13.2314 |
| ETLLQDFR_511.3 _65.3 | AMBP_HUMAN | 13.1219 |
| GFQALGDAADIR_617.3_288.2 | TIMP1_HUMAN | 12.1693 |
| IQTHSTTYR_369.5_627.3 | F13B_HUMAN | 9.4737 |
| GDTYPAELYITGSILR_885.0_274.1 | F13B_HUMAN | 6.1820 |
| ELPQSIVYK_538.8_417.7 | FBLN3_HUMAN | 6.1607 |
| NEIVFPAGILQAPFYTR_968.5_357.2 | ECE1_HUMAN | 5.5493 |
| AHYDLR_387.7_566.3 | FETUA_HUMA N | 5.4415 |
| HHGPTITAK_321.2_275.1 | AMBP_HUMAN | 5.0751 |
| SERPPIFEIR_415.2_564.3 | LRP1_HUMAN | 4.5620 |
| ALDLSLK_380.2_185.1 | ITIH3_HUMAN | 4.4275 |
| DTDTGALLFIGK_625.8_217.1 | PEDF_HUMAN | 4.3562 |
| ALNHLPLEYNSALYSR_621.0_696.4 | CO6_HUMAN | 3.9022 |
| ETLLQDFR_511.3_322.2 | AMBP_HUMAN | 3.3017 |
| YGIEEHGK_311.5_599.3 | CXA1_HUMAN | 2.8410 |
| IHWESASLLR_606.3_437.2 | CO3_HUMAN | 2.6618 |
| GEVTYTTSQVSK_650.3_750.4 | EGLN_HUMAN | 2.5328 |
| ELIEELVNITQNQK_557.6_618.3 | IL13_HUMAN | 2.5088 |
| DLHLSDVFLK_396.2_260.2 | CO6_HUMAN | 2.4010 |
| SYTITGLQPGTDYK_772.4_352.2 | FINC_HUMAN | 2.3304 |
| SPELQAEAK_486.8_788.4 | APOA2_HUMA N | 2.2657 |
| VNHVTLSQPK_374.9_459.3 | B2MG_HUMAN | 2.1480 |
| DTDTGALLFIGK_625.8_818.5 | PEDF_HUMAN | 2.0051 |
| LLDFEFSSGR_585.8_944.4 | G6PE_HUMAN | 1.7763 |
| GPGEDFR_389.2_623.3 | PTGDS_HUMA N | 1.6782 |
| DPNGLPPEAQK_583.3_669.4 | RET4_HUMAN | 1.6581 |
| IQTHSTTYR_369.5_540.3 | F13B_HUMAN | 1.6107 |
| VNHVTLSQPK_374.9_244.2 | B2MG_HUMAN | 1.4779 |
| STLFVPR_410.2_518.3 | PEPD_HUMAN | 1.3961 |
| GEVTYTTSQVSK_650.3_913.5 | EGLN_HUMAN | 1.3306 |
| ALVLELAK_428.8_672.4 | INHBE_HUMAN | 1.2973 |
| ANDQYLTAAALHNLDEAVK_686.3_317.2 | IL1A_HUMAN | 1.1850 |
| STLFVPR_410.2_272.2 | PEPD_HUMAN | 1.1842 |
| GPGEDFR_389.2_322.2 | PTGDS_HUMA N | 1.1742 |
| IPSNPSHR_303.2_610.3 | FBLN3_HUMAN | 1.0868 |
| HHGPTITAK_321.2_432.3 | AMBP_HUMAN | 1.0813 |
| TLAFVR_353.7_274.2 | FA7_HUMAN | 1.0674 |
| DLHLSDVFLK_396.2_366.2 | CO6_HUMAN | 0.9887 |
| EFDDDTYDNDIALLQLK_1014.48_501.3 | TPA_HUMAN | 0.9468 |
| AIGLPEELIQK_605.86_856.5 | FABPL_HUMA N | 0.7740 |
| LIENGYFHPVK_439.6_343.2 | F13B_HUMAN | 0.7740 |
| LPDTPQGLLGEAR_683.87_427.2 | EGLN_HUMAN | 0.6748 |
| EHSSLAFWK_552.8_267.1 | APOH_HUMAN | 0.6035 |
| NCSFSIIYPVVIK_770.4_831.5 | CRHBP_HUMA N | 0.6014 |
| ALNSIIDVYHK_424.9_661.3 | S10A8_HUMAN | 0.5987 |
| WGAAPYR_410.7_577.3 | PGRP2_HUMAN | 0.5699 |
| TQILEWAAER_608.8_632.3 | EGLN_HUMAN | 0.5395 |
| IPSNPSHR_303.2_496.3 | FBLN3_HUMAN | 0.4845 |
| VEHSDLSFSK_383.5_234.1 | B2MG_HUMAN | 0.4398 |
| VEHSDLSFSK_383.5_468.2 | B2MG_HUMAN | 0.3883 |
| FLYHK_354.2_284.2 | AMBP_HUMAN | 0.3410 |
| LPDTPQGLLGEAR_683.87_940.5 | EGLN_HUMAN | 0.3282 |
| EALVPLVADHK_397.9_390.2 | HGFA_HUMAN | 0.3091 |
| IEGNLIFDPNNYLPK_874.0_845.5 | APOB_HUMAN | 0.2933 |
| LIENGYFHPVK_439.6_627.4 | F13B_HUMAN | 0.2896 |
| VPLALFALNR_557.3_620.4 | PEPD_HUMAN | 0.2875 |
| FICPLTGLWPINTLK_887.0_685.4 | APOH_HUMAN | 0.2823 |
| NAVVQGLEQPHGLVVHPLR_688.4_890.6 | LRP1_HUMAN | 0.2763 |
| ALNFGGIGVVVGHELTHAFDDQGR_837.1_299 .2 | ECE1_HUMAN | 0.2385 |
| SPELQAEAK_486.8_659.4 | APOA2_HUMA N | 0.2232 |
| EVFSKPISWEELLQ_852.9_260.2 | FA40A_HUMAN | 0.1608 |
| VANYVDWINDR_682.8_917.4 | HGFA_HUMAN | 0.1507 |
| EVFSKPISWEELLQ _52.9_376.2 | FA40A_HUMAN | 0.1487 |
| HVVQLR_376.2_614.4 | IL6RA_HUMAN | 0.1256 |
| TVQAVLTVPK_528.3_855.5 | PEDF_HUMAN | 0.1170 |
| ELIEELVNITQNQK_557.6_517.3 | IL13_HUMAN | 0.1159 |
| EALVPLVADHK_397.9_439.8 | HGFA_HUMAN | 0.0979 |
| AITPPHPASQANIIFDITEGNLR_825.8_917.5 | FBLN1_HUMAN | 0.0797 |
| FLYHK_354.2_447.2 | AMBP_HUMAN | 0.0778 |
| SLLQPNK_400.2_358.2 | CO8A_HUMAN | 0.0698 |
| TGISPLALIK_506.8_654.5 | APOB_HUMAN | 0.0687 |
| ALNFGGIGVVVGHELTHAFDDQGR_837.1_360 .2 | ECE1_HUMAN | 0.0571 |
| DYWSTVK_449.7_347.2 | APOC3_HUMA N | 0.0357 |
| AITPPHPASQANIIFDITEGNLR_825.8_459.3 | FBLN1_HUMAN | 0.0313 |
| AALAAFNAQNNGSNFQLEEISR_789.1_633.3 | FETUA_HUMA N | 0.0279 |
| DPNGLPPEAQK_583.3_497.2 | RET4_HUMAN | 0.0189 |
| TLAFVR_353.7_492.3 | FA7_HUMAN | 0.0087 |

**Table 17. Lasso Summed Coefficients Early Window**

| Transition | Protein | SumBestCoefs_Early |
|---|---|---|
| LDFHFSSDR_375.2_611.3 | INHBC_HUMAN | 40.2030 |
| ELPQSIVYK_538.8_417.7 | FBLN3_HUMAN | 22.6926 |
| GFQALGDAADIR_617.3_288.2 | TIMP1_HUMAN | 17.4169 |
| GFQALGDAADIR_617.3_717.4 | TIMP1_HUMAN | 3.4083 |
| VNHVTLSQPK_374.9_459.3 | B2MG_HUMAN | 3.2559 |
| EFDDDTYDNDIALLQLK_1014.48_388.3 | TPA_HUMAN | 2.4073 |
| STLFVPR_410.2_272.2 | PEPD_HUMAN | 2.3984 |
| WGAAPYR_410.7_634.3 | PGRP2_HUMAN | 2.3564 |
| LDFHFSSDR_375.2_464.2 | INHBC_HUMAN | 1.9038 |
| VNHVTLSQPK_374.9_244.2 | B2MG_HUMAN | 1.7999 |
| DTDTGALLFIGK_625.8_217.1 | PEDF_HUMAN | 1.5802 |
| GPGEDFR_389.2_623.3 | PTGDS_HUMAN | 1.4223 |
| IHWESASLLR_606.3_437.2 | CO3_HUMAN | 1.2735 |
| ELIEELVNITQNQK_557.6_618.3 | IL13_HUMAN | 1.2652 |
| AQPVQVAEGSEPDGFWEALGGK_758.0_623. 4 | GELS_HUMAN | 1.2361 |
| FAFNLYR_465.8_565.3 | HEP2_HUMAN | 1.0876 |
| SGFSFGFK_438.7_732.4 | CO8B_HUMAN | 1.0459 |
| VVGGLVALR_442.3_784.5 | FA12_HUMAN | 0.9572 |
| IEGNLIFDPNNYLPK_874.0_845.5 | APOB_HUMAN | 0.9571 |
| ETLLQDFR_511.3_565.3 | AMBP_HUMAN | 0.7851 |
| LSIPQITTK_500.8_687.4 | PSG5_HUMAN | 0.7508 |
| TASDFITK_441.7_710.4 | GELS_HUMAN | 0.6549 |
| YGIEEHGK_311.5_599.3 | CXA1_HUMAN | 0.6179 |
| AFQVWSDVTPLR_709.88_347.2 | MMP2 _HUMAN | 0.6077 |
| TVQAVLTVPK_528.3_855.5 | PEDF_HUMAN | 0.5889 |
| LSITGTYDLK_555.8_696.4 | A1AT_HUMAN | 0.5857 |
| ELIEELVNITQNQK_557.6_517.3 | IL13_HUMAN | 0.5334 |
| LIENGYFHPVK_439.6_627.4 | F13B_HUMAN | 0.5257 |
| NEIVFPAGILQAPFYTR_968.5_357.2 | ECE1_HUMAN | 0.4601 |
| SLLQPNK_400.2_358.2 | CO8A_HUMAN | 0.4347 |
| LSIPQITTK_500.8_800.5 | PSG5_HUMAN | 0.4329 |
| GVTGYFTFNLYLK_508.3_683.9 | PSG5_HUMAN | 0.4302 |
| IQTHSTTYR_369.5_627.3 | F13B_HUMAN | 0.4001 |
| ATVVYQGER_511.8_652.3 | APOH_HUMAN | 0.3909 |
| LPDTPQGLLGEAR_683.87_427.2 | EGLN_HUMAN | 0.3275 |
| NNQLVAGYLQGPNVNLEEK_700.7_999.5 | IL1RA_HUMAN | 0.3178 |
| SERPPIFEIR_415.2_564.3 | LRP1_HUMAN | 0.3112 |
| AHYDLR_387.7_566.3 | FETUA_HUMAN | 0.2900 |
| NEIWYR_440.7_637.4 | FA12_HUMAN | 0.2881 |
| ALDLSLK_380.2_575.3 | ITIH3_HUMAN | 0.2631 |
| NKPGVYTDVAYYLAWIR_677.0_545.3 | FA12_HUMAN | 0.2568 |
| SYTITGLQPGTDYK_772.4_352.2 | FINC_HUMAN | 0.2277 |
| LFIPQITPK_528.8_683.4 | PSG11_HUMAN | 0.2202 |
| IIGGSDADIK_494.8_260.2 | C1S_HUMAN | 0.2182 |
| AVDIPGLEAATPYR_736.9_399.2 | TENA_HUMAN | 0.2113 |
| DTDTGALLFIGK_625.8_818.5 | PEDF_HUMAN | 0.2071 |
| AEIEYLEK_497.8_389.2 | LYAM1_HUMAN | 0.1925 |
| EHSSLAFWK_552.8_838.4 | APOH_HUMAN | 0.1899 |
| LPDTPQGLLGEAR_683.87_940.5 | EGLN_HUMAN | 0.1826 |
| WGAAPYR_410.7_577.3 | PGRP2_HUMAN | 0.1669 |
| LFIPQITPK_528.8_261.2 | PSG11_HUMAN | 0.1509 |
| WWGGQPLWITATK_772.4_929.5 | ENPP2_HUMAN | 0.1446 |
| DSPSVWAAVPGK_607.31_301.2 | PROF1_HUMAN | 0.1425 |
| LIQDAVTGLTVNGQITGDK_972.0_798.4 | ITIH3_HUMAN | 0.1356 |
| ALDLSLK_380.2_185.1 | ITIH3_HUMAN | 0.1305 |
| TVQAVLTVPK_528.3_428.3 | PEDF_HUMAN | 0.1249 |
| NA_VVQGLEQPHGL_VVHPLR_688.4_890.6 | LRP1_HUMAN | 0.1092 |
| NSDQEIDFK_548.3_409.2 | S10A5_HUMAN | 0.0937 |
| YNSQLLSFVR_613.8_508.3 | TFR1_HUMAN | 0.0905 |
| LLDFEFSSGR_585.8_553.3 | G6PE_HUMAN | 0.0904 |
| ALNFGGIGVVVGHELTHAFDDQGR_837.1_2 99.2 | ECE1_HUMAN | 0.0766 |
| STLFVPR_410.2_518.3 | PEPD_HUMAN | 0.0659 |
| DLHLSDVFLK_396.2_260.2 | CO6_HUMAN | 0.0506 |
| EHSSLAFWK_552.8_267.1 | APOH_HUMAN | 0.0452 |
| TQIDSPLSGK_523.3_703.4 | VCAM1_HUMA N | 0.0447 |
| HHGPTITAK_321.2_432.3 | AMBP_HUMAN | 0.0421 |
| AFQVWSDVTPLR_709.88_385.3 | MMP2_HUMAN | 0.0417 |
| TGISPLALIK_506.8_741.5 | APOB_HUMAN | 0.0361 |
| DLHLSDVFLK_396.2_366.2 | CO6_HUMAN | 0.0336 |
| NTVISVNPSTK_580.3_845.5 | VCAM1_HUMA N | 0.0293 |
| DIIKPDPPK_511.8_342.2 | IL12B_HUMAN | 0.0219 |
| TGISPLALIK_506.8_654.5 | APOB_HUMAN | 0.0170 |
| GAVHVVVAETDYQSFAVLYLER_822.8_580. 3 | CO8G_HUMAN | 0.0151 |
| LNIGYIEDLK_589.3_837.4 | PAI2_HUMAN | 0.0048 |
| GPGEDFR_389.2_322.2 | PTGDS_HUMAN | 0.0008 |

**Table 18. Lasso Summed Coefficients Early Middle Combined Windows**

| Transition | Protein | SumBestCoefs_EM |
|---|---|---|
| ELPQSIVYK_538.8_417.7 | FBLN3_HUMAN | 24.8794 |
| AHYDLR_387.7_566.3 | FETUA_HUMAN | 20.8397 |
| LDFHFSSDR_375.2_611.3 | INHBC_HUMAN | 18.6630 |
| GFQALGDAADIR_517.3_288.2 | TIMP1_HUMAN | 14.7270 |
| HHGPTITAK_321.2_432.3 | AMBP_HUMAN | 11.1473 |
| VNHVTLSQPK_374.9_459.3 | B2MG_HUMAN | 10.9421 |
| NNQLVAGYLQGPNVNLEEK_700.7_999.5 | IL1RA_HUMAN | 10.4646 |
| HHGPTITAK_321.2_275.1 | AMBP_HUMAN | 7.7034 |
| ETLLQDFR_511.3_565.3 | AMBP_HUMAN | 6.7435 |
| TVQAVLTVPK_528.3_428.3 | PEDF_HUMAN | 5.7356 |
| SLQAFVAVAAR_566.8_487.3 | IL23A_HUMAN | 4.8684 |
| YGIEEHGK_311.5_599.3 | CXA1_HUMAN | 4.4936 |
| ATVVYQGER_511.8_652.3 | APOH_HUMAN | 3.9524 |
| VNHVTLSQPK_374.9_244.2 | B2MG_HUMAN | 3.8937 |
| ELIEELVNITQNQK_557.6_618.3 | IL13_HUMAN | 3.8022 |
| ALNFGGIGVVVGHELTHAFDDQGR_837.1_299 .2 | ECE1_HUMAN | 3.7603 |
| ETLLQDFR-511.3_322.2 | AMBP_HUMAN | 3.1792 |
| TVQAVLTVPK_528.3_855.5 | PEDF_HUMAN | 3.1046 |
| AALAAFNAQNNGSNFQLEEISR_789.1_633.3 | FETUA_HUMAN | 3.0021 |
| AVDIPGLEAATPYR_736.9_399.2 | TENA_HUMAN | 2.6899 |
| DLHLSDVFLK_396.2_366.2 | CO6_HUMAN | 2.5525 |
| DTDTGALLFIGK_625.8_818.5 | PEDF_HUMAN | 2.4794 |
| SYTITGLQPGTDYK_772.4_352.2 | FINC_HUMAN | 2.4535 |
| IQTHSTTYR_369.5_627.3 | F13B_HUMAN | 2.3395 |
| AHYDLR_387.7_288.2 | FETUA_HUMAN | 2.1058 |
| NCSFSIIYPVVIK_770.4_831.5 | CRHBP_HUMAN | 2.0427 |
| AIGLPEELIQK_605.86_856.5 | FABPL_HUMAN | 1.5354 |
| GFQALGDAADIR_617.3_717.4 | TIMP1_HUMAN | 1.4175 |
| TGISPLALIK_506.8_654.5 | APOB_HUMAN | 1.3562 |
| YTTEIIK_434.2_603.4 | C1R_HUMAN | 1.2855 |
| ETPEGAEAKPWYEPIYLGGVFQLEK_951.14_8 77.5 | TNFA_HUMAN | 1.1198 |
| ANDQYLTAAALHNLDEAVK_686.3_317.2 | IL1A_HUMAN | 1.0574 |
| ILPSVPK_377.2_244.2 | PGH1 HUMAN | 1.0282 |
| ALDLSLK_380.2_185.1 | ITIH3_HUMAN | 1.0057 |
| NAVVQGLEQPHGLVVHPLR_688.4_890.6 | LRP1_HUMAN | 0.9884 |
| IEGNLIFDPNNYLPK_874.0_845.5 | APOB_HUMAN | 0.9846 |
| ALDLSLK_380.2_575.3 | ITIH3_HUMAN | 0.9327 |
| LDFHFSSDR_375.2_464.2 | INHBC_HUMAN | 0.8852 |
| LSIPQITTK_500.8_800.5 | PSG5_HUMAN | 0.7740 |
| SERPPIFEIR_415.2_564.3 | LRP1_HUMAN | 0.7013 |
| AEAQAQYSAAVAK_654.3_709.4 | ITIH4_HUMAN | 0.6752 |
| IHWESASLLR_606.3_437.2 | CO3_HUMAN | 0.6176 |
| LFIPQITPK_528.8_261.2 | PSG11_HUMAN | 0.5345 |
| FICPLTGLWPINTLK_887.0_685.4 | APOH_HUMAN | 0.5022 |
| DFNQFSSGEK_386.8_189.1 | FETA_HUMAN | 0.4932 |
| TATSEYQTFFNPR_781.4_272.2 | THRB_HUMAN | 0.4725 |
| SPELQAEAK_486.8_788.4 | APOA2_HUMAN | 0.4153 |
| FIVGFTR_420.2_261.2 | CCL20_HUMAN | 0.4111 |
| TLLPVSKPEIR_418.3_288.2 | CO5_HUMAN | 0.3409 |
| DIIKPDPPK_511.8_342.2 | IL12B_HUMAN | 0.3403 |
| DTDTGALLFIGK_625.8_217.1 | PEDF_HUMAN | 0.3073 |
| YTTEIIK_434.2_704.4 | C1R_HUMAN | 0.3050 |
| SPELQAEAK_486.8_659.4 | APOA2_HUMAN | 0.3047 |
| TGISPLALIK_506.8_741.5 | APOB_HUMAN | 0.3031 |
| VVGGLVALR_442.3_784.5 | FA12_HUMAN | 0.2960 |
| WWGGQPLWITATK_772.4_373.2 | ENPP2_HUMAN | 0.2498 |
| TQILEWAAER_608.8_632.3 | EGLN_HUMAN | 0.2342 |
| STLFVPR_410.2_272.2 | PEPD_HUMAN | 0.2035 |
| DYWSTVK_449.7_347.2 | APOC3_HUMAN | 0.2018 |
| WWGGQPLWITATK_772.4_929.5 | ENPP2_HUMAN | 0.1614 |
| SILFLGK_389.2_201.1 | THBG_HUMAN | 0.1593 |
| AFQVWSDVTPLR_709.88_385.3 | MMP2_HUMAN | 0.1551 |
| IQTHSTTYR_369.5_540.3 | F13B_HUMAN | 0.1434 |
| AFQVWSDVTPLR_709.88_347.2 | MMP2_HUMAN | 0.1420 |
| LSITGTYDLK_555.8_797.4 | A1AT_HUMAN | 0.1395 |
| LSITGTYDLK_555.8_696.4 | A1AT_HUMAN | 0.1294 |
| WGAAPYR_410.7_634.3 | PGRP2_HUMAN | 0.1259 |
| IAPQLSTEELVSLGEK_857.5_533.3 | AFAM_HUMAN | 0.1222 |
| FICPLTGLWPINTLK_887.0_756.9 | APOH_HUMAN | 0.1153 |
| QINSYVK_426.2_496.3 | CBG_HUMAN | 0.1055 |
| TATSEYQTFFNPR_781.4_386.2 | THRB_HUMAN | 0.0921 |
| AFLEVNEEGSEAAASTAVVIAGR_764.4_685.4 | ANT3_HUMAN | 0.0800 |
| AKPALEDLR_506.8_288.2 | APOA1_HUMAN | 0.0734 |
| GPGEDFR_389.2_623.3 | PTGDS_HUMAN | 0.0616 |
| SLLQPNK_400.2_358.2 | CO8A_HUMAN | 0.0565 |
| ESDTSYVSLK_564.8_347.2 | CRP_HUMAN | 0.0497 |
| FFQYDTWK_567.8_712.3 | IGF2_HUMAN | 0.0475 |
| FSVVYAK_407.2_579.4 | FETUA_HUMAN | 0.0437 |
| TQIDSPLSGK_523.3_703.4 | VCAM1_HUMAN | 0.0401 |
| LNIGYIEDLK_589.3_837.4 | PAI2_HUMAN | 0.0307 |
| IPSNPSHR_303.2_496.3 | FBLN3_HUMAN | 0.0281 |
| NEIVFPAGILQAPFYTR_968.5_456.2 | ECE1_HUMAN | 0.0276 |
| TLAFVR_353.7_274.2 | FA7_HUMAN | 0.0220 |
| AEAQAQYSAAVAK_654.3_908.5 | ITIH4_HUMAN | 0.0105 |
| AQPVQVAEGSEPDGFWEALGGK_758.0_623.4 | GELS_HUMAN | 0.0103 |
| QINSYVK_426.2_610.3 | CBG_HUMAN | 0.0080 |
| NSDQEIDFK_548.3_409.2 | S10A5_HUMAN | 0.0017 |

**Table 19. Lasso Summed Coefficients Middle-Late Combined Windows**

| Transition | Protein | SumBestCoefs_ML |
|---|---|---|
| TQILEWAAER_608.8_761.4 | EGLN_HUMAN | 45.0403 |
| GDTYPAELYITGSILR_885.0_274.1 | F13B_HUMAN | 31.4888 |
| GEVTYTTSQVSK_650.3_750.4 | EGLN_HUMAN | 22.3322 |
| GEVTYTTSQVSK_650.3_913.5 | EGLN_HUMAN | 17.0298 |
| AVDIPGLEAATPYR_736.9_286.1 | TENA_HUMAN | 8.6029 |
| AVDIPGLEAATPYR_736.9_399.2 | TENA_HUMAN | 7.9874 |
| NEIVFPAGILQAPFYTR_968.5_357.2 | ECE1_HUMAN | 7.8773 |
| ALNHLPLEYNSALYSR_621.0_696.4 | CO6_HUMAN | 6.8534 |
| DPNGLPPEAQK_583.3_669.4 | RET4_HUMAN | 5.0045 |
| GFQALGDAADIR_617.3_717.4 | TIMP1_HUMAN | 4.6191 |
| ATVVYQGER_511.8_652.3 | APOH_HUMAN | 4.2522 |
| IAQYYYTFK_598.8_395.2 | F13B_HUMAN | 3.5721 |
| NAVVQGLEQPHGLVVHPLR_688.4_285.2 | LRP1_HUMAN | 3.2886 |
| IAQYYYTFK_598.8_884.4 | F13B_HUMAN | 2.9205 |
| SERPPIFEIR_415.2_564.3 | LRP1_HUMAN | 2.4237 |
| TLAFVR_353.7_274.2 | FA7_HUMAN | 2.1925 |
| EVFSKPISWEELLQ_852.9_260.2 | FA40A_HUMAN | 2.1591 |
| EVFSKPISWEELLQ_852.9_376.2 | FA40A_HUMAN | 2.1586 |
| EFDDDTYDNDIALLQLK_1014.48_501.3 | TPA_HUMAN | 2.0892 |
| TLAFVR_353.7_492.3 | FA7_HUMAN | 2.0399 |
| EALVPLVADHK_397.9_439.8 | HGFA_HUMAN | 1.8856 |
| ETLLQDFR_511.3_565.3 | AMBP_HUMAN | 1.7809 |
| ALNSIIDVYHK_424.9_661.3 | S10A8_HUMAN | 1.6114 |
| AITPPHPASQANIIFDITEGNLR_825.8_917.5 | FBLN1_HUMAN | 1.3423 |
| EQLGEFYEALDCLR_871.9_747.4 | A1AG1_HUMAN | 1.2473 |
| TFLTVYWTPER_706.9_502.3 | ICAM1_HUMAN | 0.9851 |
| NTVISVNPSTK_580.3_845.5 | VCAM1_HUMA N | 0.9845 |
| FLNWIK_410.7_560.3 | HABP2_HUMAN | 0.9798 |
| ETPEGAEAKPWYEPIYLGGVFQLEK_951.14_99 0.6 | TNFA_HUMAN | 0.9679 |
| NVNQSTIFTHK_432.2_656.3 | FRIH_HUMAN | 0.8280 |
| VPLALFALNR_557.3_620.4 | PEPD_HUMAN | 0.7851 |
| IAPQLSTEELVSLGEK_857.5_533.3 | AFAM_HUMAN | 0.7731 |
| AVYEAVLR_460.8_750.4 | PEPD_HUMAN | 0.7452 |
| LPDTPQGLLGEAR_683.87_427.2 | EGLN_HUMAN | 0.7145 |
| TVQAVLTVPK_528.3_428.3 | PEDF_HUMAN | 0.6584 |
| YSHYNER_323.48_418.2 | HABP2_HUMAN | 0.5244 |
| LLELTGPK_435.8_644.4 | A1BG_HUMAN | 0.5072 |
| DTDTGALLFIGK_625.8_818.5 | PEDF_HUMAN | 0.5010 |
| DPNGLPPEAQK_583.3_497.2 | RET4_HUMAN | 0.4803 |
| AHYDLR_387.7_566.3 | FETUA_HUMAN | 0.4693 |
| LPNNVLQEK_527.8_844.5 | AFAM_HUMAN | 0.4640 |
| VTGLDFIPGLHPILTLSK_641.04_771.5 | LEP_HUMAN | 0.4584 |
| LLELTGPK_435.8_227.2 | A1BG_HUMAN | 0.4515 |
| YTTEIIK_434.2_704.4 | C1R_HUMAN | 0.4194 |
| SSNNPHSPIVEEFQVPYNK_729.4_261.2 | C1S_HUMAN | 0.3886 |
| ALNHLPLEYNSALYSR_621.0_538.3 | CO6_HUMAN | 0.3405 |
| HFQNLGK_422.2_527.2 | AFAM_HUMAN | 0.3368 |
| EQLGEFYEALDCLR_871.9_563.3 | A1AG1_HUMAN | 0.3348 |
| TQILEWAAER_608.8_632.3 | EGLN_HUMAN | 0.2943 |
| ALVLELAK_428.8_672.4 | INHBE_HUMAN | 0.2895 |
| LSNENHGIAQR_413.5_519.8 | ITIH2_HUMAN | 0.2835 |
| LPNNVLQEK_527.8_730.4 | AFAM_HUMAN | 0.2764 |
| DTDTGALLFIGK_625.8_217.1 | PEDF_HUMAN | 0.2694 |
| GDTYPAELYITGSILR_885.0_922.5 | F13B_HUMAN | 0.2594 |
| GPITSAAELNDPQSILLR_632.3_601.4 | EGLN_HUMAN | 0.2388 |
| ANLINNIFELAGLGK_793.9_834.5 | LCAP_HUMAN | 0.2158 |
| SEPRPGVLLR_375.2_454.3 | FA7_HUMAN | 0.1921 |
| EQSLNVSQDLDTIR_539.9_557.8 | SYNE2_HUMAN | 0.1836 |
| FICPLTGLWPINTLK_887.0_685.4 | APOH_HUMAN | 0.1806 |
| ALNFGGIGVVVGHELTHAFDDQGR_837.1_360. 2 | ECE1_HUMAN | 0.1608 |
| ANDQYLTAAALHNLDEAVK_686.3_317.2 | IL1A_HUMAN | 0.1607 |
| AQETSGEEISK_589.8_979.5 | IBP1_HUMAN | 0.1598 |
| QINSYVK_426.2_610.3 | CBG_HUMAN | 0.1592 |
| SILFLGK_389.2_577.4 | THBG_HUMAN | 0.1412 |
| DAVVYPILVEFTR_761.4_286.1 | HYOU1_HUMAN | 0.1298 |
| LIEIANHVDK_384.6_683.3 | ADA12_HUMAN | 0.1297 |
| LSSPAVITDK_515.8_830.5 | PLMN_HUMAN | 0.1272 |
| LIENGYFHPVK_439.6_343.2 | F13B_HUMAN | 0.1176 |
| AALAAFNAQNNGSNFQLEEISR_789.1_633.3 | FETUA_HUMAN | 0.1160 |
| IQTHSTTYR_369.5_540.3 | F13B_HUMAN | 0.1146 |
| IPKPEASFSPR_410.2_506.3 | ITIH4_HUMAN | 0.1001 |
| LLDFEFSSGR_585.8_944.4 | G6PE_HUMAN | 0.0800 |
| YYLQGAK_421.7_516.3 | ITIH4_HUMAN | 0.0793 |
| VRPQQLVK_484.3_722.4 | ITIH4_HUMAN | 0.0744 |
| GPGEDFR_389.2_322.2 | PTGDS_HUMAN | 0.0610 |
| ITQDAQLK_458.8_803.4 | CBG_HUMAN | 0.0541 |
| TATSEYQTFFNPR_781.4_272.2 | THRB_HUMAN | 0.0511 |
| ETLLQDFR_511.3_322.2 | AMBP_HUMAN | 0.0472 |
| YEFLNGR_449.7_293.1 | PLMN_HUMAN | 0.0345 |
| TLYSSSPR_455.7_696.3 | IC1_HUMAN | 0.0316 |
| SLLQPNK_400.2_599.4 | CO8A_HUMAN | 0.0242 |
| LLEVPEGR_456.8_686.4 | C1S_HUMAN | 0.0168 |
| GGEGTGYFVDFSVR_745.9_722.4 | HRG_HUMAN | 0.0110 |
| IQTHSTTYR_369.5_627.3 | F13B_HUMAN | 0.0046 |

**Table 20. Random Forest SummedGini All Windows**

| Transition | Protein | SumBestGini | Probability |
|---|---|---|---|
| TVQAVLTVPK_528.3_428.3 | PEDF_HUMAN | 12.6521 | 1.0000 |
| DTDTGALLFIGK_625.8_818.5 | PEDF_HUMAN | 11.9585 | 0.9985 |
| ALALPPLGLAPLLNLWAKPQGR_770.5_2 56.2 | SHBG_HUMAN | 10.5229 | 0.9971 |
| DVLLLVHNLPQNLTGHIWYK_791.8_883. 0 | PSG7_HUMAN | 10.2666 | 0.9956 |
| ETLLQDFR_511.3_565.3 | AMBP_HUMAN | 8.9862 | 0.9941 |
| ALALPPLGLAPLLNLWAKPQGR_770.5_4 57.3 | SHBG_HUMAN | 8.6349 | 0.9927 |
| IALGGLLFPASNLR_481.3_657.4 | SHBG_HUMAN | 8.5838 | 0.9912 |
| DTDTGALLFIGK_625.8_217.1 | PEDF_HUMAN | 8.2463 | 0.9897 |
| IQTHSTTYR_369.5_627.3 | F13B_HUMAN | 8.1199 | 0.9883 |
| DVLLLVHNLPQNLTGHIWYK_791.8_310. 2 | PSG7_HUMAN | 7.7393 | 0.9868 |
| IALGGLLFPASNLR_481.3_412.3 | SHBG_HUMAN | 7.5601 | 0.9853 |
| HHGPTITAK_321.2_432.3 | AMBP_HUMAN | 7.5181 | 0.9838 |
| ETLLQDFR_511.3_322.2 | AMBP_HUMAN | 7.4043 | 0.9824 |
| FICPLTGLWPINTLK_887.0_685.4 | APOH_HUMAN | 7.2072 | 0.9809 |
| GPGEDFR_389.2_623.3 | PTGDS_HUMA N | 7.1422 | 0.9794 |
| IQTHSTTYR_369.5_540.3 | F13B_HUMAN | 6.9809 | 0.9780 |
| TVQAVLTVPK_528.3_855.5 | PEDF_HUMAN | 6.6191 | 0.9765 |
| ATVVYQGER_511.8_652.3 | APOH_HUMAN | 6.5813 | 0.9750 |
| VVLSSGSGPGLDLPLVLGLPLQLK_791.5 598.4 | SHBG_HUMAN | 6.3244 | 0.9736 |
| HHGPTITAK_321.2_275.1 | AMBP_HUMAN | 6.3081 | 0.9721 |
| VVLSSGSGPGLDLPLVLGLPLQLK_791.5 | SHBG_HUMAN | 6.0654 | 0.9706 |
| 768.5 | | | |
| GDTYPAELYITGSILR_885.0_274.1 | F13B_HUMAN | 5.9580 | 0.9692 |
| ATVVYQGER_511.8_751.4 | APOH_HUMAN | 5.9313 | 0.9677 |
| LDFHFSSDR_375.2_611.3 | INHBC_HUMA N | 5.8533 | 0.9662 |
| LDFHFSSDR_375.2_464.2 | INHBC_HUMA N | 5.8010 | 0.9648 |
| EVFSKPISWEELLQ_852.9_260.2 | FA40A_HUMAN | 5.6648 | 0.9633 |
| DTYVSSFPR_357.8_272.2 | TCEA1_HUMA N | 5.6549 | 0.9618 |
| LPDTPQGLLGEAR_683.87_427.2 | EGLN_HUMAN | 5.3806 | 0.9604 |
| FLYHK_354.2_447.2 | AMBP_HUMAN | 5.3764 | 0.9589 |
| SPELQAEAK_486.8_659.4 | APOA2_HUMA N | 5.1896 | 0.9574 |
| GPGEDFR_389.2_322.2 | PTGDS_HUMA N | 5.1876 | 0.9559 |
| SGVDLADSNQK_567.3_662.3 | VGFR3_HUMA N | 5.1159 | 0.9545 |
| TNT`NEFLIDVDK_704.85_849.5 | TF_HUMAN | 4.7216 | 0.9530 |
| FICPLTGLWPINTLK_887.0_756.9 | APOH_HUMAN | 4.6421 | 0.9515 |
| LNIGYIEDLK_589.3_950.5 | PAI2_HUMAN | 4.6250 | 0.9501 |
| EVFSKPISWEELLQ_852.9_376.2 | FA40A_HUMAN | 4.4215 | 0.9486 |
| SYTITGLQPGTDYK_772.4_680.3 | FINC_HUMAN | 4.4103 | 0.9471 |
| TLPFSR_360.7_409.2 | LYAM1_HUMA N | 4.2148 | 0.9457 |
| SPELQAEAK_486.8_788.4 | APOA2_HUMA N | 4.2081 | 0.9442 |
| GDTYPAELYITGSILR_885.0_922.5 | F13B_HUMAN | 4.0672 | 0.9427 |
| AEIEYLEK_497.8_552.3 | LYAM1_HUMA N | 3.9248 | 0.9413 |
| FSLVSGWGQLLDR_493.3_403.2 | FA7_HUMAN | 3.9034 | 0.9398 |
| FLYHK_354.2_284.2 | AMBP_HUMAN | 3.8982 | 0.9383 |
| SGVDLADSNQK_567.3_591.3 | VGFR3_HUMA N | 3.8820 | 0.9369 |
| LDGSTHLNIFFAK_488.3_739.4 | PAPP1_HUMAN | 3.8770 | 0.9354 |
| HFQNLGK_422.2_527.2 | AFAM_HUMAN | 3.7628 | 0.9339 |
| IAQYYYTFK_598.8_884.4 | F13B_HUMAN | 3.7040 | 0.9325 |
| GFQALGDAADIR_617.3_717.4 | TIMP1_HUMAN | 3.6538 | 0.9310 |
| ELPQSIVYK_538.8_417.7 | FBLN3_HUMAN | 3.6148 | 0.9295 |
| IAQYYYTFK_598.8_395.2 | F13B_HUMAN | 3.5820 | 0.9280 |
| GSLVQASEANLQAAQDFVR_668.7_735.4 | ITIH1_HUMAN | 3.5283 | 0.9266 |
| TLPFSR_360.7_506.3 | LYAM1_HUMA N | 3.5064 | 0.9251 |
| VNHVTLSQPK_374.9_244.2 | B2MG_HUMAN | 3.5045 | 0.9236 |
| IAPQLSTEELVSLGEK_857.5_533.3 | AFAM_HUMAN | 3.4990 | 0.9222 |
| VEHSDLSFSK_383.5_468.2 | B2MG_HUMAN | 3.4514 | 0.9207 |
| TQILEWAAER_608.8_761.4 | EGLN_HUMAN | 3.4250 | 0.9192 |
| AHQLAIDTYQEFEETYIPK_766.0_521.3 | CSH_HUMAN | 3.3634 | 0.9178 |
| TEFLSNYLTNVDDITLVPGTLGR_846.8_6 00.3 | ENPP2_HUMAN | 3.3512 | 0.9163 |
| HFQNLGK_422.2_285.1 | AFAM_HUMAN | 3.3375 | 0.9148 |
| VEHSDLSFSK_383.5_234.1 | B2MG_HUMAN | 3.3371 | 0.9134 |
| TELRPGETLNVNFLLR_624.68_875.5 | CO3_HUMAN | 3.1889 | 0.9119 |
| YQISVNK_426.2_292.1 | FIBB_HUMAN | 3.1668 | 0.9104 |
| YGFYTHVFR_397.2_659.4 | THRB_HUMAN | 3.1188 | 0.9075 |
| SEPRPGVLLR_375.2_454.3 | FA7_HUMAN | 3.1068 | 0.9060 |
| IAPQLSTEELVSLGEK_857.5_333.2 | AFAM_HUMAN | 3.0917 | 0.9046 |
| ILILPSVTR_506.3_785.5 | PSGx_HUMAN | 3.0346 | 0.9031 |
| TLAFVR_353.7_492.3 | FA7_HUMAN | 3.0237 | 0.9016 |
| AKPALEDLR_506.8_288.2 | APOA1_HUMA N | 3.0189 | 0.9001 |

**Table 21. Random Forest SummedGini Early Window**

| Transition | Protein | SumBestGini | Probability |
|---|---|---|---|
| LSETNR 360.2 330.2 | PSG1_HUMAN | 26.3610 | 1.0000 |
| ALNFGGIGVVVGHELTHAFDDQGR_837. 1_299.2 | ECE1_HUMAN | 24.8946 | 0.9985 |
| ELPQSIVYK_538.8_417.7 | FBLN3_HUMAN | 24.8817 | 0.9971 |
| LDFHFSSDR_375.2_464.2 | INHBC_HUMA N | 24.3229 | 0.9956 |
| LDFHFSSDR_375.2_611.3 | INHBC_HUMA N | 22.2162 | 0.9941 |
| FSLVSGWGQLLDR_493.3_403.2 | FA7_HUMAN | 19.6528 | 0.9927 |
| TSESGELHGLTTEEEFVEGIYK_819.06_31 0.2 | TTHY_HUMAN | 19.2430 | 0.9912 |
| ATVVYQGER_511.8_751.4 | APOH_HUMAN | 19.1321 | 0.9897 |
| IQTHSTTYR_369.5_627.3 | F13B_HUMAN | 17.1528 | 0.9883 |
| ATVVYQGER_511.8_652.3 | APOH_HUMAN | 17.0214 | 0.9868 |
| HYINLITR_515.3_301.1 | NPY_HUMAN | 16.6713 | 0.9853 |
| FICPLTGLWPINTLK_887.0_685.4 | APOH_HUMAN | 15.0826 | 0.9838 |
| AFLEVNEEGSEAAASTAVVIAGR_764.4_ 614.4 | ANT3_HUMAN | 14.6110 | 0.9824 |
| IQTHSTTYR_369.5_540.3 | F13B_HUMAN | 14.5473 | 0.9809 |
| AHQLAIDTYQEFEETYIPK_766.0_521.3 | CSH_HUMAN | 14.0287 | 0.9794 |
| TGAQELLR_444.3_530.3 | GELS_HUMAN | 13.1389 | 0.9780 |
| DSPSVWAAVPGK_607.31_301.2 | PROF1_HUMAN | 12.9571 | 0.9765 |
| NCSFSIIYPVVIK_770.4_555.4 | CRHBP_HUMA N | 12.5867 | 0.9750 |
| ALALPPLGLAPLLNLWAKPQGR_770.5_2 56.2 | SHBG_HUMAN | 12.1138 | 0.9721 |
| DTDTGALLFIGK_625.8_818.5 | PEDF_HUMAN | 11.7054 | 0.9706 |
| TSDQIHFFFAK_447.6_512.3 | ANT3_HUMAN | 11.4261 | 0.9692 |
| IALGGLLFPASNLR_481.3_657.4 | SHBG_HUMAN | 11.0968 | 0.9677 |
| DTDTGALLFIGK_625.8_217.1 | PEDF_HUMAN | 10.9040 | 0.9662 |
| EQSLNVSQDLDTIR_539.9_758.4 | SYNE2_HUMA N | 10.6572 | 0.9648 |
| IALGGLLFPASNLR_481.3_412.3 | SHBG_HUMAN | 10.0629 | 0.9633 |
| FGFGGSTDSGPIR_649.3_745.4 | ADA12_HUMA N | 10.0449 | 0.9618 |
| ETPEGAEAKPWYEPIYLGGVFQLEK_951. 14_877.5 | TNFA_HUMAN | 10.0286 | 0.9604 |
| LPDTPQGLLGEAR_683.87_427.2 | EGLN_HUMAN | 9.8980 | 0.9589 |
| FSVVYAK_407.2_381.2 | FETUA_HUMA N | 9.7971 | 0.9574 |
| YGIEEHGK_311.5_599.3 | CXA1_HUMAN | 9.7850 | 0.9559 |
| GFQALGDAADIR_617.3_717.4 | TIMP1_HUMAN | 9.7587 | 0.9545 |
| VVLSSGSGPGLDLPLVLGLPLQLK_791.5 _598.4 | SHBG_HUMAN | 9.3421 | 0.9530 |
| HHGPTITAK_321.2_275.1 | AMBP_HUMAN | 9.2728 | 0.9515 |
| ALALPPLGLAPLLNLWAKPQGR_770.5_4 57.3 | SHBG_HUMAN | 9.2431 | 0.9501 |
| LIEIANHVDK_384.6_498.3 | ADA12_HUMA N | 9.1368 | 0.9486 |
| AFQVWSDVTPLR_709.88_347.2 | MMP2_HUMAN | 8.6789 | 0.9471 |
| AFQVWSDVTPLR_709.88_385.3 | MMP2_HUMAN | 8.6339 | 0.9457 |
| ETLLQDFR_511.3_322.2 | AMBP_HUMAN | 8.6252 | 0.9442 |
| ETLLQDFR_511.3_565.3 | AMBP_HUMAN | 8.3957 | 0.9427 |
| VNHVTLSQPK_374.9_459.3 | B2MG_HUMAN | 8.3179 | 0.9413 |
| HHGPTITAK_321.2_432.3 | AMBP_HUMAN | 8.2567 | 0.9398 |
| DTYVSSFPR_357.8_272.2 | TCEA1_HUMA N | 8.2028 | 0.9383 |
| GGEGTGYFVDFSVR_745.9_722.4 | HRG_HUMAN | 8.0751 | 0.9369 |
| DFNQFSSGEK_386.8_189.1 | FETA_HUMAN | 8.0401 | 0.9354 |
| DVLLLVHNLPQNLTGHIWYK_791.8_883. 0 | PSG7_HUMAN | 7.9924 | 0.9339 |
| VSEADSSNADWVTK_754.9_347.2 | CFAB_HUMAN | 7.8630 | 0.9325 |
| QGHNSVFLIK_381.6_260.2 | HEMO_HUMAN | 7.8588 | 0.9310 |
| AQETSGEEISK_589.8_979.5 | IBP1_HUMAN | 7.7787 | 0.9295 |
| DIPHWLNPTR_416.9_600.3 | PAPP1_HUMAN | 7.6393 | 0.9280 |
| SPELQAEAK_486.8_788.4 | APOA2_HUMA N | 7.6248 | 0.9266 |
| QGHNSVFLIK_381.6_520.4 | HEMO_HUMAN | 7.6042 | 0.9251 |
| LIENGYFHPVK_439.6_343.2 | F13B_HUMAN | 7.5771 | 0.9236 |
| DIIKPDPPK_511.8_342.2 | IL12B_HUMAN | 7.5523 | 0.9222 |
| VNHVTLSQPK_374.9_244.2 | B2MG_HUMAN | 7.5296 | 0.9207 |
| TELRPGETLNVNFLLR_624.68_875.5 | CO3_HUMAN | 7.4484 | 0.9178 |
| QINSYVK_426.2_496.3 | CBG_HUMAN | 7.3266 | 0.9163 |
| YNSQLLSFVR_613.8_734.5 | TFR1_HUMAN | 7.3262 | 0.9148 |
| TVQAVLTVPK_528.3_855.5 | PEDF_HUMAN | 7.1408 | 0.9134 |
| QTLSWTVTPK_580.8_818.4 | PZP_HUMAN | 6.9764 | 0.9119 |
| DVLLLVHNLPQNLPGYFWYK_810.4_328. | PSG9_HUMAN | 6.9663 | 0.9104 |
| 2 | | | |
| FICPLTGLWPINTLK_887.0_756.9 | APOH_HUMAN | 6.8924 | 0.9090 |
| TSYQVYSK_488.2_397.2 | C163A_HUMAN | 6.5617 | 0.9075 |
| VVLSSGSGPGLDLPLVLGLPLQLK_791.5 _768.5 | SHBG_HUMAN | 6.4615 | 0.9060 |
| QINSYVK_426.2_610.3 | CBG_HUMAN | 6.4595 | 0.9046 |
| LHKPGVYTR_357.5_479.3 | HGFA_HUMAN | 6.4062 | 0.9031 |
| ALVLELAK_428.8_672.4 | INHBE_HUMAN | 6.3684 | 0.9016 |
| YNSQLLSFVR_613.8_508.3 | TFR1_HUMAN | 6.3628 | 0.9001 |

**Table 22. Random Forest SummedGini Early-Middle Combined Windows**

| Transition | Protein | SumBestGini | Probability |
|---|---|---|---|
| ATVVYQGER_511.8_652.3 | APOH_HUMAN | 120.6132 | 1.0000 |
| ATVVYQGER_511.8_751.4 | APOH_HUMAN | 99.7548 | 0.9985 |
| IQTHSTTYR_369.5_627.3 | F13B_HUMAN | 57.5339 | 0.9971 |
| IQTHSTTYR_369.5_540.3 | F13B_HUMAN | 55.0267 | 0.9956 |
| FICPLTGLWPINTLK_887.0_685.4 | APOH_HUMAN | 49.9116 | 0.9941 |
| AHQLAIDTYQEFEETYIPK_766.0_521.3 | CSH_HUMAN | 48.9796 | 0.9927 |
| HHGPTITAK_321.2_432.3 | AMBP_HUMAN | 45.7432 | 0.9912 |
| SPELQAEAK_486.8_659.4 | APOA2_HUMA N | 42.1848 | 0.9897 |
| AHYDLR 387.7_566.3 | FETUA_HUMA N | 41.4591 | 0.9883 |
| ETLLQDFR_511.3_565.3 | AMBP_HUMAN | 39.7301 | 0.9868 |
| HHGPTITAK_321.2_275.1 | AMBP_HUMAN | 39.2096 | 0.9853 |
| ETLLQDFR_511.3_322.2 | AMBP_HUMAN | 36.8033 | 0.9838 |
| FICPLTGLWPINTLK_887.0_756.9 | APOH_HUMAN | 31.8246 | 0.9824 |
| TVQAVLTVPK_528.3_855.5 | PEDF_HUMAN | 31.1356 | 0.9809 |
| IALGGLLFPASNLR_481.3_657.4 | SHBG_HUMAN | 30.5805 | 0.9794 |
| DVLLLVHNLPQNLTGHIWYK_791.8_883. 0 | PSG7_HUMAN | 29.5729 | 0.9780 |
| AHYDLR_387.7_288.2 | FETUA_HUMA N | 29.0239 | 0.9765 |
| SPELQAEAK_486.8_788.4 | APOA2_HUMA N | 28.6741 | 0.9750 |
| ETPEGAEAKPWYEPIYLGGVFQLEK_951. 14_877.5 | TNFA_HUMAN | 26.8117 | 0.9736 |
| LDFHFSSDR_375.2_611.3 | INHBC_HUMA N | 26.0001 | 0.9721 |
| DFNQFSSGEK_386.8_189.1 | FETA_HUMAN | 25.9113 | 0.9706 |
| HFQNLGK_422.2_527.2 | AFAM_HUMAN | 25.7497 | 0.9692 |
| DPDQTDGLGLSYLSSHIANVER_796.4_32 8.1 | GELS_HUMAN | 25.7418 | 0.9677 |
| VVLSSGSGPGLDLPLVLGLPLQLK_791.5 _598.4 | SHBG_HUMAN | 25.6425 | 0.9662 |
| IALGGLLFPASNLR_481.3_412.3 | SHBG_HUMAN | 25.1737 | 0.9648 |
| LDFHFSSDR_375.2_464.2 | INHBC_HUMA N | 25.0674 | 0.9633 |
| LIQDAVTGLTVNGQITGDK_972.0_640.4 | ITIH3_HUMAN | 24.5613 | 0.9618 |
| VVLSSGSGPGLDLPLVLGLPLQLK_791.5 _768.5 | SHBG_HUMAN | 23.2995 | 0.9604 |
| DIPHWLNPTR_416.9_600.3 | PAPP1_HUMAN | 22.9504 | 0.9589 |
| VNHVTLSQPK_374.9_459.3 | B2MG_HUMAN | 22.2821 | 0.9574 |
| QINSYVK_426.2_496.3 | CBG_HUMAN | 22.2233 | 0.9559 |
| ALALPPLGLAPLLNLWAKPQGR_770.5_2 56.2 | SHBG_HUMAN | 22.1160 | 0.9545 |
| TELRPGETLNVNFLLR_624.68_875.5 | CO3_HUMAN | 21.9043 | 0.9530 |
| ITQDAQLK_458.8_803.4 | CBG_HUMAN | 21.8933 | 0.9515 |
| IAPQLSTEELVSLGEK_857.5_533.3 | AFAM_HUMAN | 21.4577 | 0.9501 |
| QINSYVK_426.2_610.3 | CBG_HUMAN | 21.3414 | 0.9486 |
| LIQDAVTGLTVNGQITGDK_972.0_798.4 | ITIH3_HUMAN | 21.2843 | 0.9471 |
| DTDTGALLFIGK_625.8_818.5 | PEDF_HUMAN | 21.2631 | 0.9457 |
| DVLLLVHNLPQNLPGYFWYK_810.4_328. 2 | PSG9_HUMAN | 21.2547 | 0.9442 |
| HFQNLGK_422.2_285.1 | AFAM_HUMAN | 20.8051 | 0.9427 |
| DTDTGALLFIGK_625.8_217.1 | PEDF_HUMAN | 20.2572 | 0.9413 |
| FLYHK_354.2_447.2 | AMBP_HUMAN | 19.6822 | 0.9398 |
| NNQLVAGYLQGPNVNLEEK_700.7_999.5 | IL1RA_HUMAN | 19.2156 | 0.9383 |
| VSFSSPLVAISGVALR_802.0_715.4 | PAPP1_HUMAN | 18.9721 | 0.9369 |
| TVQAVLTVPK_528.3_428.3 | PEDF_HUMAN | 18.9392 | 0.9354 |
| TFVNITPAEVGVLVGK_822.47_968.6 | PROF1_HUMAN | 18.9351 | 0.9339 |
| LQVLGK_329.2_416.3 | A2GL_HUMAN | 18.6613 | 0.9325 |
| TLAFVR_353.7_274.2 | FA7_HUMAN | 18.5095 | 0.9310 |
| ITQDAQLK_458.8_702.4 | CBG_HUMAN | 18.5046 | 0.9295 |
| DVLLLVHNLPQNLTGHIWYK_791.8_310. 2 | PSG7_HUMAN | 18.4015 | 0.9280 |
| VSFSSPLVAISGVALR_802.0_602.4 | PAPP1_HUMAN | 17.5397 | 0.9266 |
| IAPQLSTEELVSLGEK_857.5_333.2 | AFAM_HUMAN | 17.5338 | 0.9251 |
| TLFIFGVTK_513.3_215.1 | PSG4_HUMAN | 17.5245 | 0.9236 |
| ALNFGGIGVVVGHELTHAFDDQGR_837. 1_299.2 | ECE1_HUMAN | 17.1108 | 0.9222 |
| FLYHK_354.2_284.2 | AMBP_HUMAN | 16.9237 | 0.9207 |
| LDGSTHLNIFFAK_488.3_739.4 | PAPP1_HUMAN | 16.8260 | 0.9192 |
| ELIEELVNITQNQK_557.6_618.3 | IL13_HUMAN | 16.5607 | 0.9178 |
| YNSQLLSFVR_613.8_734.5 | TFR1_HUMAN | 16.5425 | 0.9163 |
| AFQVWSDVTPLR_709.88_385.3 | MMP2_HUMAN | 16.3293 | 0.9148 |
| LDGSTHLNIFFAK_488.3_852.5 | PAPP1_HUMAN | 15.9820 | 0.9134 |
| TPSAAYLWVGTGASEAEK_919.5_428.2 | GELS_HUMAN | 15.9084 | 0.9119 |
| YTTEIIK_434.2_603.4 | C1R_HUMAN | 15.7998 | 0.9104 |
| FSVVYAK_407.2_381.2 | FETUA_HUMA N | 15.4991 | 0.9090 |
| VNHVTLSQPK_374.9_244.2 | B2MG_HUMAN | 15.2938 | 0.9075 |
| SYTITGLQPGTDYK_772.4_680.3 | FINC_HUMAN | 14.9898 | 0.9060 |
| DIPHWLNPTR_416.9_373.2 | PAPP1_HUMAN | 14.6923 | 0.9046 |
| AFQVWSDVTPLR_709.88_347.2 | MMP2_HUMAN | 14.4361 | 0.9031 |
| IAQYYYTFK_598.8_884.4 | F13B_HUMAN | 14.4245 | 0.9016 |
| FSLVSGWGQLLDR_493.3_403.2 | FA7_HUMAN | 14.3848 | 0.9001 |

From the foregoing description, it will be apparent that variations and modifications can be made to the invention described herein to adopt it to various usages and conditions. Such embodiments are also within the scope of the following claims.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

All patents and publications mentioned in this specification are herein incorporated by reference to the same extent as if each independent patent and publication was specifically and individually indicated to be incorporated by reference.

The application discloses *inter alia* the following items:
1. A panel of isolated biomarkers comprising N of the biomarkers listed in Tables 2, 3, 4, 5 and 7 through 22.
2. The panel of item 1, wherein N is a number selected from the group consisting of 2 to 24.
3. The panel of item 2, wherein said panel comprises at least two of the isolated biomarkers selected from the group consisting of FSVVYAK, SPELQAEAK, VNHVTLSQPK, SSNNPHSPIVEEFQVPYNK, and VVGGLVALR.
4. The panel of item 2, wherein said panel comprises alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4).
5. The panel of item 2, wherein said panel comprises at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4).
6. The panel of item 2, wherein said panel comprises at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4) cell adhesion molecule with homology to L1CAM (CHL1), complement component C5 (C5 or CO5), complement component C8 beta chain (C8B or CO8B), endothelin-converting enzyme 1 (ECE1), coagulation factor XIII, B polypeptide (F13B), interleukin 5 (IL5), Peptidase D (PEPD), and plasminogen (PLMN).
7. A method of determining probability for preeclampsia in a pregnant female, the method comprising detecting a measurable feature of each of N biomarkers selected from the biomarkers listed in Tables 2, 3, 4, 5 and 7 through 22 in a biological sample obtained from said pregnant female, and analyzing said measurable features to determine the probability for preeclampsia in said pregnant female.
8. The method of item 7, wherein said measurable feature comprises fragments or derivatives of each of said N biomarkers selected from the biomarkers listed in Tables 2, 3, 4, 5 and 7 through 22.
9. The method of item 7, wherein said detecting a measurable feature comprises quantifying an amount of each of N biomarkers selected from the biomarkers listed in Tables 2, 3, 4, 5 and 7 through 22, combinations or portions and/or derivatives thereof in a biological sample obtained from said pregnant female.
10. The method of item 9, further comprising calculating the probability for preeclampsia in said pregnant female based on said quantified amount of each of N biomarkers selected from the biomarkers listed in Tables 2, 3, 4, 5 and 7 through 22.
11. The method of item 7, further comprising an initial step of providing a biomarker panel comprising N of the biomarkers listed in Tables 2, 3, 4, 5 and 7 through 22.
12. The method of item 7, further comprising an initial step of providing a biological sample from the pregnant female.
13. The method of item 7, further comprising communicating said probability to a health care provider.
14. The method of item 13, wherein said communication informs a subsequent treatment decision for said pregnant female.
15. The method of item 7, wherein N is a number selected from the group consisting of 2 to 24.
16. The method of item 15, wherein said N biomarkers comprise at least two of the isolated biomarkers selected from the group consisting of FSVVYAK, SPELQAEAK, VNHVTLSQPK, SSNNPHSPIVEEFQVPYNK, and VVGGLVALR.
17. The method of item 7, wherein said analysis comprises a use of a predictive model.
18. The method of item 17, wherein said analysis comprises comparing said measurable feature with a reference feature.
19. The method of item 18, wherein said analysis comprises using one or more selected from the group consisting of a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, and a combination thereof.
20. The method of item 19, wherein said analysis comprises logistic regression.
21. The method of item 7, wherein said probability is expressed as a risk score.
22. The method of item 7, wherein the biological sample is selected from the group consisting of whole blood, plasma, and serum.
23. The method of item 22, wherein the biological sample is serum.
24. The method of item 7, wherein said quantifying comprises mass spectrometry (MS).
25. The method of item 24, wherein said MS comprises liquid chromatography-mass spectrometry (LC-MS).
26. The method of item 24, wherein said MS comprises multiple reaction monitoring (MRM) or selected reaction monitoring (SRM).
27. The method of item 26, wherein said MRM (or SRM) comprises scheduled MRM (SRM).
28. The method of item 7, wherein said quantifying comprises an assay that utilizes a capture agent.
29. The method of item 28, wherein said capture agent is selected from the group consisting of and antibody, antibody fragment, nucleic acid-based protein binding reagent, small molecule or variant thereof.
30. The method of item 28, wherein said assay is selected from the group consisting of enzyme immunoassay (EIA), enzyme-linked immunosorbent assay (ELISA), and radioimmunoassay (RIA).
31. The method of item 30, wherein said quantifying further comprises mass spectrometry (MS).
32. The method of item 31, wherein said quantifying comprises co-immunoprecitipation-mass spectrometry (co-IP MS).
33. The method of item 7, further comprising detecting a measurable feature for one or more risk indicia.
34. The method of item 33, wherein the one or more risk indicia are selected from the group consisting of history of preeclampsia, first pregnancy, age, obesity, diabetes, gestational diabetes, hypertension, kidney disease, multiple pregnancy, interval between pregnancies, new paternity, migraine headaches, rheumatoid arthritis, and lupus.
35. A method of determining probability for preeclampsia in a pregnant female, the method comprising: (a) quantifying in a biological sample obtained from said pregnant female an amount of each of N biomarkers selected from the biomarkers listed in Tables 2, 3, 4, 5 and 7 through 22; (b) multiplying said amount by a predetermined coefficient, (c) determining the probability for preeclampsia in said pregnant female comprising adding said individual products to obtain a total risk score that corresponds to said probability.
36. The panel of item 2, wherein said panel comprises at least two of the isolated biomarkers selected from the group consisting of LDFHFSSDR, TVQAVLTVPK, GPGEDFR, ETLLQDFR, ATVVYQGER, and GFQALGDAADIR.
37. The panel of item 2, wherein said panel comprises at least two of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), and Sex hormone-binding globulin (SHBG).
38. The panel of item 2, wherein said panel comprises at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4) cell adhesion molecule with homology to L1CAM (CHL1), complement component C5 (C5 or CO5), complement component C8 beta chain (C8B or CO8B), endothelin-converting enzyme 1 (ECE1), coagulation factor XIII, B polypeptide (F13B), interleukin 5 (IL5), Peptidase D (PEPD), plasminogen (PLMN), of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), and Sex hormone-binding globulin (SHBG).
39. The method of item 7, wherein said N biomarkers comprise at least two of the isolated biomarkers selected from the group consisting of LDFHFSSDR, TVQAVLTVPK, GPGEDFR, ETLLQDFR, ATVVYQGER, and GFQALGDAADIR.
40. The method of item 7, wherein said N biomarkers comprise at least two of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), and Sex hormone-binding globulin (SHBG).
41. The method of item 7, wherein said N biomarkers comprise at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4) cell adhesion molecule with homology to L1CAM (CHL1), complement component C5 (C5 or CO5), complement component C8 beta chain (C8B or CO8B), endothelin-converting enzyme 1 (ECE1), coagulation factor XIII, B polypeptide (F13B), interleukin 5 (IL5), Peptidase D (PEPD), plasminogen (PLMN), of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), and Sex hormone-binding globulin (SHBG).
42. The method of item 35 wherein said N biomarkers comprise at least two of the isolated biomarkers selected from the group consisting of LDFHFSSDR, TVQAVLTVPK, GPGEDFR, ETLLQDFR, ATVVYQGER, and GFQALGDAADIR.
43. The method of item 35 wherein said N biomarkers comprise at least two of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), and Sex hormone-binding globulin (SHBG).
44. The method of item 35, herein said N biomarkers comprise at least two isolated biomarkers selected from the group consisting of alpha-1-microglobulin (AMBP), ADP/ATP translocase 3 (ANT3), apolipoprotein A-II (APOA2), apolipoprotein B (APOB), apolipoprotein C-III (APOC3), beta-2-microglobulin (B2MG), complement component 1, s subcomponent (C1S), and retinol binding protein 4 (RBP4 or RET4) cell adhesion molecule with homology to L1CAM (CHL1), complement component C5 (C5 or CO5), complement component C8 beta chain (C8B or CO8B), endothelin-converting enzyme 1 (ECE1), coagulation factor XIII, B polypeptide (F13B), interleukin 5 (IL5), Peptidase D (PEPD), plasminogen (PLMN), of Inhibin beta C chain (INHBC), Pigment epithelium-derived factor (PEDF), Prostaglandin-H2 D-isomerase (PTGDS), alpha-1-microglobulin (AMBP), Beta-2-glycoprotein 1 (APOH), Metalloproteinase inhibitor 1 (TIMP1), Coagulation factor XIII B chain (F13B), Alpha-2-HS-glycoprotein (FETUA), and Sex hormone-binding globulin (SHBG).

## Claims

1. A panel of isolated biomarkers comprising two or more biomarkers, wherein said two or more biomarkers comprise sex hormone-binding globulin (SHBG) and one or more biomarkers selected from the group consisting of Inhibin beta C chain (INHBC), monocyte differentiation antigen CD14 (CD14), afamin (AFAM), alpha-1-antichymotrypsin (AACT), alpha-1Bglycoprotein (A1BG), alpha-2-antiplasmin (A2AP), alpha-2-HSglycoprotein preproprotein (FETUA), angiotensinogen preproprotein (ANGT), antithrombin-III (ANT3), apolipoprotein A-II preproprotein (APOA2), apolipoprotein A-IV (APOA4), apolipoprotein B-100 (APOB), apolipoprotein C-II (APOC2), apolipoprotein C-III (APOC3), apolipoprotein E (APOE), beta-2-glycoprotein 1 (APOH), bone marrow proteoglycan (PRG2), ceruloplasmin (CERU), cholinesterase (CHLE), coagulation factor XII (FA12), coagulation factor XIII B chain (F13B), complement C1r subcomponent (C1R), complement C1s subcomponent (C1S), complement C2 (CO2), complement C3 (CO3), complement C4-B-like preproprotein (CO4B), complement C5 preproprotein (CO5), complement component C6 (CO6), complement component C7 (CO7), complement component C8 alpha chain (CO8A), complement component C8 beta chain preproprotein (CO8B), complement component C8 gamma chain (CO8G), complement component C9 (CO9), complement factor B preproprotein (CFAB), complement factor H (CFAH), complement factor I preproprotein (CFAI), corticosteroid-binding globulin (CBG), fibrinogen alpha chain (FIBA), gelsolin (GELS), glutathione peroxidase 3 (GPX3), hemopexin (HEMO), heparin cofactor 2 (HEP2), histidine-rich glycoprotein (HRG), insulin-like growth factor-binding protein complex acid labile subunit (ALS), inter-alpha-trypsin inhibitor heavy chain H1 (ITIH1), inter-alpha-trypsin inhibitor heavy chain H2 (ITIH2), inter-alpha-trypsin inhibitor heavy chain H4 (ITIH4), kallistatin (KAIN), kininogen-1 (KNG1), leucine-rich alpha-2-glycoprotein (A2GL), lipopolysacchari de-binding protein (LBP), lumican (LUM), N-acetylmuramoyl-L-alanine amidase (PGRP2), phosphatidylinositol-glycan-specific phospholipase D (PHLD), pigment epithelium-derived factor (PEDF), plasma kallikrein preproprotein (KLKB1), plasma protease C1 inhibitor (IC1), plasminogen (PLMN), complement C4-A (CO4A), pregnancy zone protein (PZP), pregnancy specific beta-1-glycoprotein 1/8/4 (PSG1/PSG8/PSG4), protein AMBP preproprotein (AMBP), protein Z-dependent protease inhibitor (ZPI), prothrombin preproprotein (THRB), retinol-binding protein 4 (RET4), thyroxine-binding globulin (THBG), transforming growth factor-beta-induced protein ig-h3 (BGH3), vitamin D-binding protein (VTDB), vitronectin (VTNC), and von Willebrand factor preprotein (VWG), or fragments or derivatives thereof.

2. The panel of claim 1, wherein said two or more biomarkers comprise:
i. SHBG and INHBC;
ii. SHBG and AFAM; or
iii. SHBG and CD14.

3. A method of determining probability for preeclampsia in a pregnant female, the method comprising:
(a) detecting a measurable feature of at least two biomarkers in a biological sample obtained from said pregnant female; and
(b) analyzing said measurable feature to determine the probability for preeclampsia in said pregnant female,
wherein the at least two or more biomarkers comprise a first biomarker for sex hormone-binding globulin (SHBG) and a second biomarker for a protein selected from Inhibin beta C chain (INHBC), monocyte differentiation antigen CD14 (CD14), afamin (AFAM), alpha-1-antichymotrypsin (AACT), alpha-1Bglycoprotein (A1BG), alpha-2-antiplasmin (A2AP), alpha-2-HSglycoprotein preproprotein (FETUA), angiotensinogen preproprotein (ANGT), antithrombin-III (ANT3), apolipoprotein A-II preproprotein (APOA2), apolipoprotein A-IV (APOA4), apolipoprotein B-100 (APOB), apolipoprotein C-II (APOC2), apolipoprotein C-III (APOC3), apolipoprotein E (APOE), beta-2-glycoprotein 1 (APOH), bone marrow proteoglycan (PRG2), ceruloplasmin (CERU), cholinesterase (CHLE), coagulation factor XII (FA12), coagulation factor XIII B chain (F13B), complement C1r subcomponent (C1R), complement C1s subcomponent (C1S), complement C2 (CO2), complement C3 (CO3), complement C4-B-like preproprotein (CO4B), complement C5 preproprotein (CO5), complement component C6 (CO6), complement component C7 (CO7), complement component C8 alpha chain (CO8A), complement component C8 beta chain preproprotein (CO8B), complement component C8 gamma chain (CO8G), complement component C9 (CO9), complement factor B preproprotein (CFAB), complement factor H (CFAH), complement factor I preproprotein (CFAI), corticosteroid-binding globulin (CBG), fibrinogen alpha chain (FIBA), gelsolin (GELS), glutathione peroxidase 3 (GPX3), hemopexin (HEMO), heparin cofactor 2 (HEP2), histidine-rich glycoprotein (HRG), insulin-like growth factor-binding protein complex acid labile subunit (ALS), inter-alpha-trypsin inhibitor heavy chain H1 (ITIH1), inter-alpha-trypsin inhibitor heavy chain H2 (ITIH2), inter-alpha-trypsin inhibitor heavy chain H4 (ITIH4), kallistatin (KAIN), kininogen-1 (KNG1), leucine-rich alpha-2-glycoprotein (A2GL), lipopolysacchari de-binding protein (LBP), lumican (LUM), N-acetylmuramoyl-L-alanine amidase (PGRP2), phosphatidylinositol-glycan-specific phospholipase D (PHLD), pigment epithelium-derived factor (PEDF), plasma kallikrein preproprotein (KLKB1), plasma protease C1 inhibitor (IC1), plasminogen (PLMN), complement C4-A (CO4A), pregnancy zone protein (PZP), pregnancy specific beta-1-glycoprotein 1/8/4 (PSG1/PSG8/PSG4), protein AMBP preproprotein (AMBP), protein Z-dependent protease inhibitor (ZPI), prothrombin preproprotein (THRB), retinol-binding protein 4 (RET4), thyroxine-binding globulin (THBG), transforming growth factor-beta-induced protein ig-h3 (BGH3), vitamin D-binding protein (VTDB), vitronectin (VTNC), and von Willebrand factor preprotein (VWG).

4. The method of claim 3, wherein said two or more biomarkers comprise:
i. SHBG and INHBC;
ii. SHBG and AFAM; or
iii. SHBG and CD14.

5. The method of claim 3 or 4, wherein:
said method comprises an initial step of providing a biological sample from the pregnant female, wherein optionally the biological sample is selected from the group consisting of whole blood, plasma, and serum;
said detecting a measurable feature comprises quantifying an amount of each of the biomarkers, combinations or portions and/or derivatives thereof; or
said analysing comprises use of a predictive model or comparing said measurable feature with a reference feature.

6. The method of claim 5, wherein said analysing comprises using one or more analyses selected from the group consisting of a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, and a combination thereof.

7. The method of any one of claims 3-6, further comprising communicating said probability to a health care provider, wherein said communication informs a subsequent treatment decision for said pregnant female.

8. The method of claim 5, wherein:
(a) said quantifying comprises mass spectrometry (MS), wherein optionally said MS comprises liquid chromatography-mass spectrometry (LC-MS), multiple reaction monitoring (MRM) or selected reaction monitoring (SRM), or co-immunoprecipitation-mass spectrometry (co-IP MS); or
(b) said quantifying comprises an assay that utilizes a capture agent, wherein optionally said capture agent is selected from the group consisting of an antibody, antibody fragment, nucleic acid-based protein binding reagent, small molecule or variant thereof, wherein optionally said assay is selected from the group consisting of enzyme immunoassay (EIA), enzyme-linked immunosorbent assay (ELISA), and radioimmunoassay (RIA).

9. The method of any one of claims 3-8, further comprising detecting a measurable feature for one or more risk indicia selected from the group consisting of history of preeclampsia, first pregnancy, age, obesity, diabetes, gestational diabetes, hypertension, kidney disease, multiple pregnancy, interval between pregnancies, new paternity, migraine headaches, rheumatoid arthritis, and lupus.

10. A method of detecting two or more biomarkers, the method comprising:
(a) obtaining a biological sample from a pregnant female; and
(b) detecting whether said two or more biomarkers are present in said biological sample,
wherein said two or more biomarkers comprises sex hormone-binding globulin (SHBG) and one or more biomarkers selected from the group consisting of Inhibin beta C chain (INHBC), monocyte differentiation antigen CD14 (CD14), afamin (AFAM), alpha-1-antichymotrypsin (AACT), alpha-1Bglycoprotein (A1BG), alpha-2-antiplasmin (A2AP), alpha-2-HSglycoprotein preproprotein (FETUA), angiotensinogen preproprotein (ANGT), antithrombin-III (ANT3), apolipoprotein A-II preproprotein (APOA2), apolipoprotein A-IV (APOA4), apolipoprotein B-100 (APOB), apolipoprotein C-II (APOC2), apolipoprotein C-III (APOC3), apolipoprotein E (APOE), beta-2-glycoprotein 1 (APOH), bone marrow proteoglycan (PRG2), ceruloplasmin (CERU), cholinesterase (CHLE), coagulation factor XII (FA12), coagulation factor XIII B chain (F13B), complement C1r subcomponent (C1R), complement C1s subcomponent (C1S), complement C2 (CO2), complement C3 (CO3), complement C4-B-like preproprotein (CO4B), complement C5 preproprotein (CO5), complement component C6 (CO6), complement component C7 (CO7), complement component C8 alpha chain (CO8A), complement component C8 beta chain preproprotein (CO8B), complement component C8 gamma chain (CO8G), complement component C9 (CO9), complement factor B preproprotein (CFAB), complement factor H (CFAH), complement factor I preproprotein (CFAI), corticosteroid-binding globulin (CBG), fibrinogen alpha chain (FIBA), gelsolin (GELS), glutathione peroxidase 3 (GPX3), hemopexin (HEMO), heparin cofactor 2 (HEP2), histidine-rich glycoprotein (HRG), insulin-like growth factor-binding protein complex acid labile subunit (ALS), inter-alpha-trypsin inhibitor heavy chain H1 (ITIH1), inter-alpha-trypsin inhibitor heavy chain H2 (ITIH2), inter-alpha-trypsin inhibitor heavy chain H4 (ITIH4), kallistatin (KAIN), kininogen-1 (KNG1), leucine-rich alpha-2-glycoprotein (A2GL), lipopolysacchari de-binding protein (LBP), lumican (LUM), N-acetylmuramoyl-L-alanine amidase (PGRP2), phosphatidylinositol-glycan-specific phospholipase D (PHLD), pigment epithelium-derived factor (PEDF), plasma kallikrein preproprotein (KLKB1), plasma protease C1 inhibitor (IC1), plasminogen (PLMN), complement C4-A (CO4A), pregnancy zone protein (PZP), pregnancy specific beta-1-glycoprotein 1/8/4 (PSG1/PSG8/PSG4), protein AMBP preproprotein (AMBP), protein Z-dependent protease inhibitor (ZPI), prothrombin preproprotein (THRB), retinol-binding protein 4 (RET4), thyroxine-binding globulin (THBG), transforming growth factor-beta-induced protein ig-h3 (BGH3), vitamin D-binding protein (VTDB), vitronectin (VTNC), and von Willebrand factor preprotein (VWG), or fragments or derivatives thereof.

11. The method of claim 10, wherein:
(a) said detecting comprises mass spectrometry (MS), wherein optionally said MS comprises co-immunoprecipitation mass spectrometry (co-IP MS), liquid chromatography-mass spectrometry (LC-MS), multiple reaction monitoring (MRM) or selected reaction monitoring (SRM); or
(b) said detecting comprises an assay that utilizes a capture agent, wherein optionally said capture agent is selected from the group consisting of an antibody, antibody fragment, nucleic acid-based protein binding reagent, or small molecule, and wherein optionally said assay is selected from the group consisting of enzyme immunoassay (EIA), enzyme-linked immunosorbent assay (ELISA), and radioimmunoassay (RIA).

12. The method of any one of claims 10-11, wherein said two or more biomarkers comprise:
i. SHBG and INHBC;
ii. SHBG and AFAM; or
iii. SHBG and CD14.

13. The method of any one of claims 10-12, wherein the biological sample is selected from the group consisting of whole blood, plasma, and serum.

14. Aspirin for use in a method for preventing preeclampsia in a pregnant female, wherein the method comprises:
(a) determining the probability for preeclampsia in a pregnant female using the panel of claim 1 or 2 or the method of any one of claims 3 to 13; and
(b) administering the aspirin to a pregnant female at risk of developing preeclampsia.
